# EUROPEAN PATENT APPLICATION

(11) **EP 4 385 978 A1**
(43) Date of publication of application: **19.06.2024**
(21) Application number: 22855542.1
(22) Date of filing: 12.08.2022
(51) Int. Cl.: C07D 215/48, C07D 471/04, C07D 401/04, C07D 401/06, C07D 401/12, C07D 403/06, C07D 241/44, C07D 487/04, A61K 31/4704, A61K 31/4709, A61K 31/47, A61K 31/4745, A61K 31/498, A61K 31/55, A61K 31/5377, A61K 45/06, A61P 35/00, A61P 35/02

(54) **POLYCYCLIC COMPOUND AND USE THEREOF**

(30) Priority: 12.08.2021 CN 202110926274; 07.12.2021 CN 202111485631; 02.06.2022 CN 202210624529
(71) Applicant: TYK Medicines Inc., Huzhou, Zhejiang 313100 (CN)
(72) Inventor: LIANG, Apeng, Huzhou, Zhejiang 313100 (CN); LI, Jun, Huzhou, Zhejiang 313100 (CN); WU, Yusheng, Huzhou, Zhejiang 313100 (CN); CHEN, Shaoqing, Huzhou, Zhejiang 313100 (CN); LIU, Guangbin, Huzhou, Zhejiang 313100 (CN); YIN, Zhou, Huzhou, Zhejiang 313100 (CN); DONG, Shengli, Huzhou, Zhejiang 313100 (CN); LI, Meihua, Huzhou, Zhejiang 313100 (CN)
(74) Representative: dompatent von Kreisler Selting Werner - Partnerschaft von Patent- und Rechtsanwälten mbB
(86) International application number: PCT/CN2022/112259
(87) International publication number: WO 2023/016562

(57) **Abstract**

The present invention relates to a polycyclic compound and the use thereof. Specifically, the compound of the present invention has a structure as represented by formula (I), wherein the definitions of each group and each substituent are as described in the description. Also disclosed in the present invention are a method for preparing the compound and the use of the compound in regulating and treating related diseases caused by abnormal activity of YAP/TEAD.

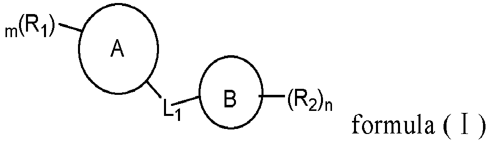

## Description

### TECHNICAL FIELD

The present invention relates to the technical field of medicine, specifically, relates to polycyclic compounds used to regulate the Hippo pathway, and preparation method therefor and use thereof. The polycyclic compounds are mainly used for the treatment or prevention of proliferative diseases (such as cancer), especially for regulating and treating related diseases caused by abnormal activity of YAP/TEAD.

### BACKGROUND

The Hippo pathway is essentially composed of a core kinase cascade containing the Ste-20 family of protein kinase MST1-2, the scaffolding protein Salvador, and large tumor suppressor kinase LATS1-2 to inhibit the transcriptional co-activators YAP (Yes1-associatedprotein) and TAZ (transcriptional co-activators with PDZ-binding motif). YAP and TAZ are the major effectors of the Hippo signaling pathway. They act as transcription factors along with TEAD (transcriptional enhanced associate domain) in the nucleus, which increases expressions of such target gene as CTGF (connective tissue growth factor) and CYR61. The Hippo pathway is an important regulatory factor for cell growth, proliferation, and migration. TEAD transcriptional factors are the core of the Hippo pathway and are crucial for regulating organ growth and wound repair. The dysregulation of TEAD as well as the regulatory cofactor thereof, Yes-associated protein (YAP), have been involved in many pathological processes of human cancers and hyper proliferation, and the disorder of this pathway is often detected in human cancers. Like the TEAD protein, the activation of YAP and TAZ have been identified in many human tumors, and are critical in tumor initiation, progression and metastasis. For example, YAP expression is increased in patients with breast cancer, ovarian cancer, colon cancer, liver cancer and pancreatic cancer, and is associated with reduced survival. Consistent with this, the activation or overexpression of YAP or TAZ enhances the expression of TEAD dependent gene (such as CCN1, CTGF, ITGB2, and Birc5/survivin) and promotes cell proliferation and migration in many cell types. On the contrary, blocking the signaling of YAP/TAZ-TEAD complex formation or intervening to block the expression of many pro mitotic TEAD target genes can significantly reduce cell proliferation and carcinogenic transformation. In addition, the Hippo pathway also cross-talks with other signaling pathways such as Wnt, Notch, Hedgehog, and MAPK, thereby affecting various biological functions. The dysfunction of Hippo pathway may be involved not only in cancer, but also in many human diseases. Therefore, YAP-TEAD complex is a promising therapeutic target.

### SUMMARY OF THE INVENTION

The purpose of the present invention is to provide a compound of formula I and a preparation method therefor and a use thereof.

In the first aspect of the present invention, provided is a compound of formula I, or a pharmaceutically acceptable salt, a solvate or a prodrug thereof, 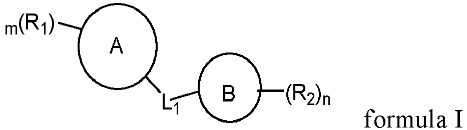 wherein,
A is selected from the group consisting of 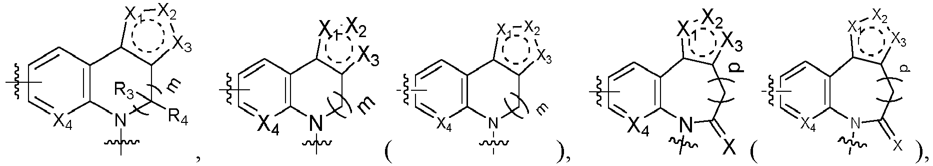 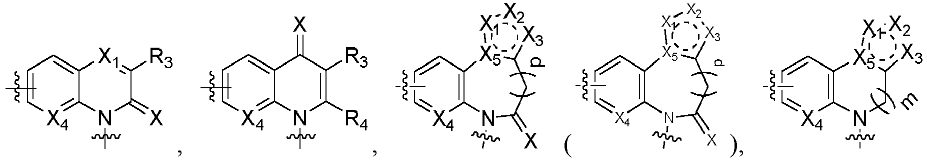 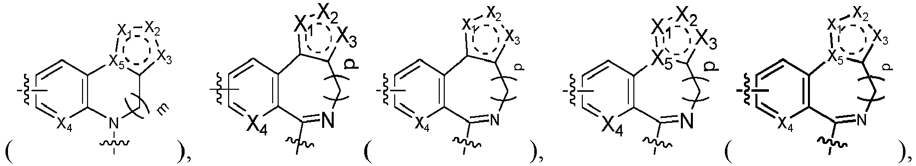 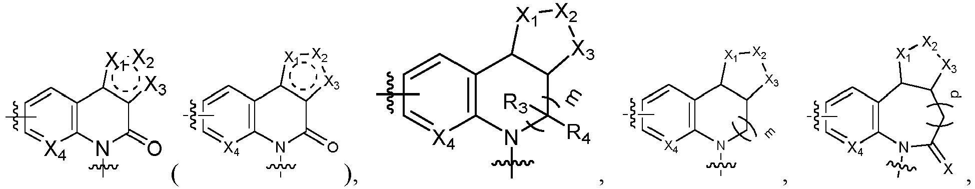 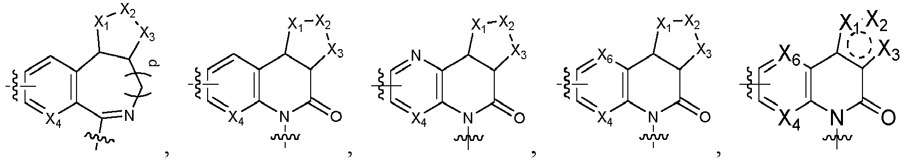 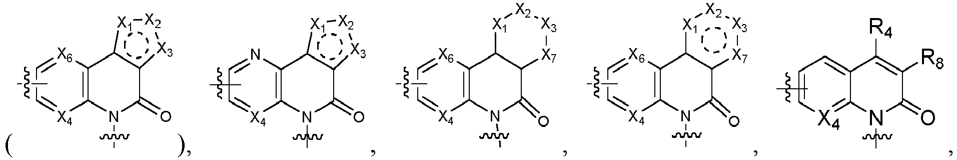 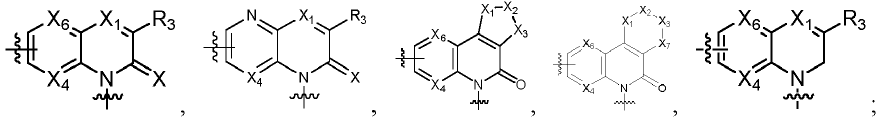
L₁ is selected from absent or CR₃R₄;
B is selected from the group consisting of C6-C10 aryl, 5-10 membered heteroaryl containing 1 to 3 heteroatoms selected from N, O and S, C5-C10 cycloalkyl;
X is selected from O, NH, CR₃R₄, and S;
X₁, X₂, X₃, X₄, X₅, X₆ and X₇ are each independently selected from the group consisting of CR₃, (CR₃)₂, N, O, S, SR₃, SR₃R₄, NR₄, CR₃R₄, (CR₃R₄)₂;
R₁ is each independently selected from H, D, halogen, CN, NH₂, ureido, carboxyl, carbamate group, C₁₋₆ alkyl, C₁₋₆ alkoxyl, C₃₋₆ cycloalkyl, C₃₋₆ cycloalkoxy, C6-C10 aryl, 5-10 membered heteroaryl containing 1 to 3 heteroatoms selected from N, O and S, 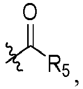 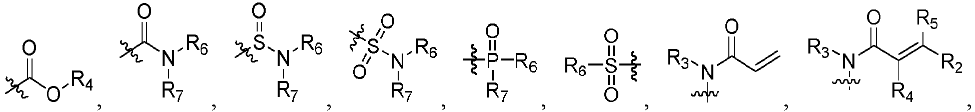 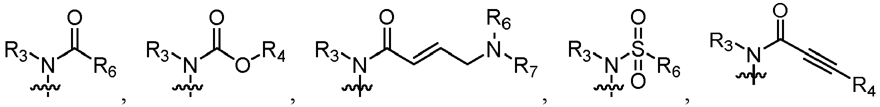 and 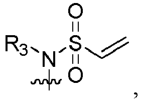 wherein NH₂, ester group, ureido, carbamate group, acylamino, C₁₋₆ alkyl, C₁₋₆ alkoxyl, C₃₋₆ cycloalkyl, C₃₋₆ cycloalkoxy, C6-C10 aryl, and 5-10 membered heteroaryl containing 1 to 3 heteroatoms selected from N, O and S are optionally substituted with 1, 2 or 3 R;
each of R₂, R₃, R₄ and R₅ are each independently selected from H, D, halogen, CN, NH₂, -CO-(C₁₋₆ alkyl), =O, -C(=O)-O-(C1-C6 alkyl), -C(=O)-O-OBi, -S(=O)₂-NR₆R₇, 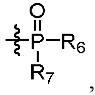 ester group, ureido, carbamate group, acylamino, C₁₋₆ alkyl, C₁₋₆ haloalkyl, C₁₋₆ alkoxyl, C₃₋₆ cycloalkyl, C₃₋₆ cycloalkoxy, C6-C10 aryl, 5-10 membered heteroaryl containing 1 to 3 heteroatoms selected from N, O and S, SF₅, wherein NH₂, ester group, ureido, carbamate group, acylamino, C₁₋₆ alkyl, C₁₋₆ alkoxy, C₃₋₆ cycloalkyl, C₃₋₆ cycloalkoxy, C6-C10 aryl, and 5-10 membered heteroaryl containing 1 to 3 heteroatoms selected from N, O and S are optionally substituted with 1, 2 or 3 R; or, X₁ and X₇ are each independently CR₃R₄, and X₁ and X₇ share one R₃, and R₃ is C1-C6 alkylene;
R₆ and R₇ are each independently selected from hydrogen, D, C₁₋₆ alkyl, C₃₋₆ cycloalkyl, C6-C10 aryl, NH₂, NH(C₁₋₆ alkyl), N(C₁₋₆ alkyl)₂, 5-10 membered heteroaryl containing 1 to 3 heteroatoms selected from N, O and S, 5-10 membered heterocyclyl containing 1 to 3 heteroatoms selected from N, O and S, -S(O)₂-(C₁₋₆ alkyl), -S(O)₂-(C₂₋₆ alkenyl), wherein C₁₋₆ alkyl, C₃₋₆ cycloalkyl, C6-C10 aryl, 5-10 membered heteroaryl containing 1 to 3 heteroatoms selected from N, O and S are optionally substituted with 1, 2 or 3 R, or R₆ and R₇ form a 3-7 membered carbocycle, or R₆ and R₇ form a 3-7 membered heterocycle containing N, O or S;
R₈ is selected from 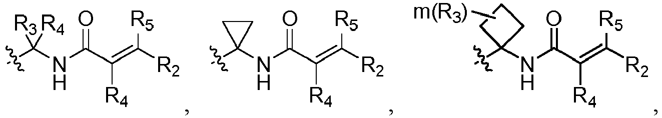 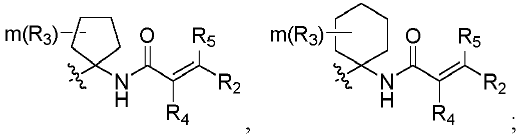
each R is independently selected from halogen, CN, OH, -(C₁₋₆ alkylene)-N(C₁₋₆ alkyl)₂, NH₂, NH(C₁₋₆ alkyl), ester group, ureido, carbamate group, acylamino, C₁₋₆ alkyl, C₁₋₆ alkoxyl, C₃₋₆ cycloalkyl, C₃₋₆ cycloalkoxy, C₂₋₆ alkenyl, C₂₋₆ alkynyl, C6-C10 aryl, 5-10 membered heterocyclyl containing 1 to 3 heteroatoms selected from N, O and S, and 5-10 membered heteroaryl containing 1 to 3 heteroatoms selected from N, O and S substituted or unsubstituted with R'; R' is independently selected from the group consisting of: C₁₋₆ alkyl, haloC1-6 alkyl, C₁₋₆ alkoxyl, NH₂, NH(C₁₋₆ alkyl), N(C₁₋₆ alkyl)₂, CN, halogen, =O;
each m and n are independently selected from 1, 2, 3 and 4;
p is selected from 0, 1 and 2.

In another preferred embodiment,
A is selected from 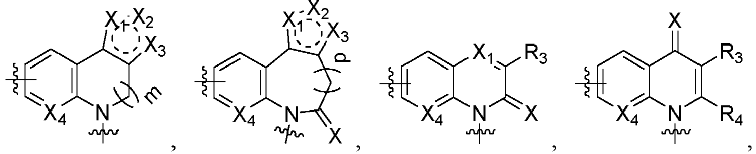 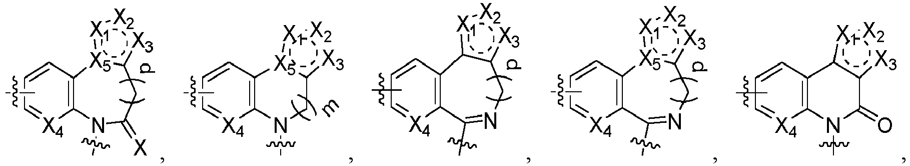 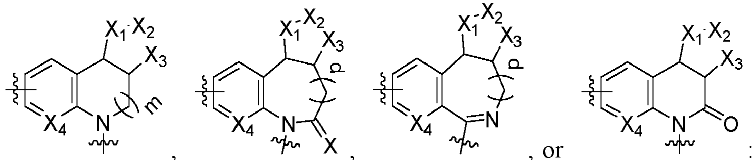
L₁ is selected from absent and CR₃R₄;
B is selected from C6-C10 aryl, 5-10 membered heteroaryl containing 1 to 3 heteroatoms selected from N, O or S, C5-C10 cycloalkyl;
X is selected from O, NH, and S;
X₁, X₂, X₃, X₄ and X₅ are each independently selected from CR₃, (CR₃)₂, N, O, S, NR₄, CR₃R₄, (CR₃R₄)₂;
R₁ is selected from H, halogen, CN, NH₂, ureido, carboxyl, carbamate group, C₁₋₆ alkyl, C₁₋₆ alkoxyl, C₃₋₆ cycloalkyl, C₃₋₆ cycloalkoxy, C6-C10 aryl, 5-10 membered heteroaryl containing 1 to 3 heteroatoms selected from N, O and S, 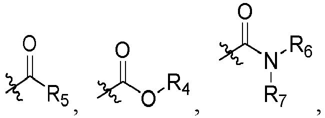 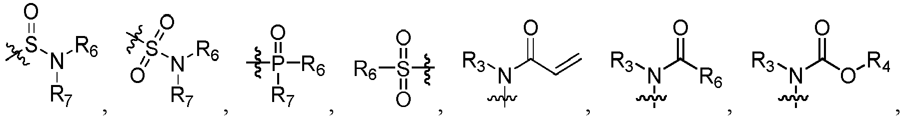 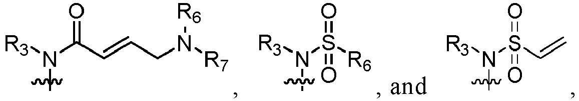 wherein NH₂, ester group, ureido, carbamate group, acylamino, C₁₋₆ alkyl, C₁₋₆ alkoxyl, C₃₋₆ cycloalkyl, C₃₋₆ cycloalkoxy, C6-C10 aryl, and 5-10 membered heteroaryl containing 1 to 3 heteroatoms selected from N, O and S are optionally substituted with 1, 2 or 3 R;
each R₂, R₃, R₄ and R₅ are independently selected from H, halogen, CN, NH₂, -CO-(C₁₋₆ alkyl), ester group, ureido, carbamate group, acylamino, C₁₋₆ alkyl, C₁₋₆ alkoxyl, C₃₋₆ cycloalkyl, C₃₋₆ cycloalkoxy, C6-C10 aryl, 5-10 membered heteroaryl containing 1 to 3 heteroatoms selected from N, O and S, SF₅, wherein NH₂, ester group, ureido , carbamate group, acylamino, C₁₋₆ alkyl, C₁₋₆ alkoxyl, C₃₋₆ cycloalkyl, C₃₋₆ cycloalkoxy, C6-C10 aryl, 5-10 membered heteroaryl containing 1 to 3 heteroatoms selected from N, O and S are optionally substituted with 1, 2 or 3 R;
R₆ and R₇ are each independently selected from hydrogen, C₁₋₆ alkyl, C₃₋₆ cycloalkyl, C6-C10 aryl, NH₂, NH(C₁₋₆ alkyl), N(C₁₋₆ alkyl)₂, 5-10 membered heteroaryl containing 1 to 3 heteroatoms selected from N, O and S, 5-10 membered heterocyclyl containing 1 to 3 heteroatoms selected from N, O and S, -S(O)₂-(C₁₋₆ alkyl), -S(O)₂-(C₂₋₆ alkenyl), wherein C₁₋₆ alkyl, C₃₋₆ cycloalkyl, C6-C10 aryl, 5-10 membered heteroaryl containing 1 to 3 heteroatoms selected from N, O and S are optionally substituted with 1, 2 or 3 R, or R₆ and R₇ form a 3-7 membered carbocycle, or R₆ and R₇ form a 3-7 membered heterocycle containing N, O or S;
each R is independently selected from halogen, CN, OH, -(C₁₋₆ alkylene)-N(C₁₋₆ alkyl)₂, NH₂, NH(C₁₋₆ alkyl), ester group, ureido, carbamate group, acylamino, C₁₋₆ alkyl, C₁₋₆ alkoxyl, C₃₋₆ cycloalkyl, C₃₋₆ cycloalkoxy, C₂₋₆ alkenyl, C₂₋₆ alkynyl, C6-C10 aryl, 5-10 membered heterocyclyl containing 1 to 3 heteroatoms selected from N, O and S, or 5-10 membered heteroaryl containing 1 to 3 heteroatoms selected from N, O and S substituted or unsubstituted with R'; R' is independently selected from the group consisting of: C₁₋₆ alkyl, haloC₁₋₆ alkyl, C₁₋₆ alkoxyl, NH₂, NH(C₁₋₆ alkyl), N(C₁₋₆ alkyl)₂, CN, halogen;
each m and n are independently selected from 1, 2, 3 and 4;
p is selected from 0, land 2.

In another preferred embodiment,
A is selected from 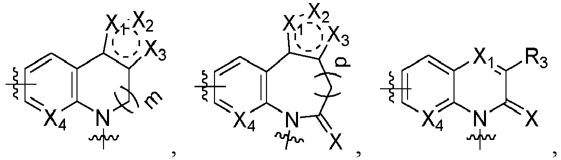 and 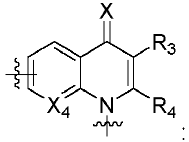
L₁ is selected from absent and CR₃R₄;
B is selected from C6-C10 aryl, or 5-10 membered heteroaryl containing 1 to 3 heteroatoms selected from N, O and S:
X is selected from O, NH, and S:
X₁, X₂, X₃ and X₄ are each independently selected from the group consisting of CR₃, N, O, S and NR₄;
R₁ is selected from the group consisting of H, halogen, CN, NH₂, ureido, carboxyl, carbamate group, C₁₋₆ alkyl, C₁₋₆ alkoxyl, C₃₋₆ cycloalkyl, C₃₋₆ cycloalkoxy, C6-C10 aryl, 5-10 membered heteroaryl containing 1 to 3 heteroatoms selected from N, O and S, 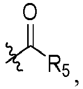 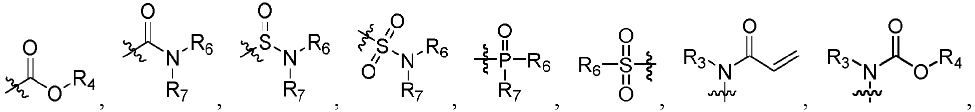 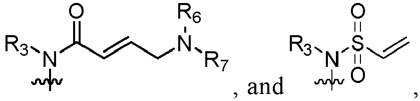 wherein NH₂, ester group, ureido, carbamate group, acylamino, C₁₋₆ alkyl, C₁₋₆ alkoxyl, C₃₋₆ cycloalkyl, C₃₋₆ cycloalkoxy, C6-C10 aryl, and 5-10 membered heteroaryl containing 1 to 3 heteroatoms selected from N, O and S are optionally substituted with 1, 2 or 3 R;
each R₂, R₃, R₄ and R₅ are independently selected from H, halogen, CN, NH₂, -CO-(C₁₋₆ alkyl), ester group, ureido, carbamate group, acylamino, C₁₋₆ alkyl, C₁₋₆ alkoxyl, C₃₋₆ cycloalkyl, C₃₋₆ cycloalkoxy, C6-C10 aryl, 5-10 membered heteroaryl containing 1 to 3 heteroatoms selected from N, O and S, wherein NH₂, ester group, ureido, carbamate group, acylamino, C₁₋₆ alkyl, C₁₋₆ alkoxyl, C₃₋₆ cycloalkyl, C₃₋₆ cycloalkoxy, C6-C10 aryl, 5-10 membered heteroaryl containing 1 to 3 heteroatoms selected from N, O and S are optionally substituted with 1, 2 or 3 R;
R₆ and R₇ are each independently selected from hydrogen, C₁₋₆ alkyl, C₃₋₆ cycloalkyl, C6-C10 aryl, 5-10 membered heteroaryl containing 1 to 3 heteroatoms selected from N, O and S, -S(O)₂-(C₁₋₆ alkyl), -S(O)₂-(C₂₋₆ alkenyl), wherein C₁₋₆ alkyl, C₃₋₆ cycloalkyl, C6-C10 aryl, 5-10 membered heteroaryl containing 1 to 3 heteroatoms selected from N, O and S are optionally substituted with 1, 2 or 3 R, or R₆ and R₇ form a 3-7 membered carbocycle, or R₆ and R₇ form a 3-7 membered heterocycle containing N, O and S;
each R is independently selected from halogen, CN, OH, NH₂, ester group, ureido, carbamate group, acylamino, C₁₋₆ alkyl, C₁₋₆ alkoxyl, C₃₋₆ cycloalkyl, C₃₋₆ cycloalkoxy, C₂₋₆ alkenyl, C₂₋₆ alkynyl, C6-C10 aryl, and 5-10 membered heteroaryl containing 1 to 3 heteroatoms selected from N, O and S;
each m and n are independently selected from 1, 2, 3 and 4;
p is selected from 0, 1 and 2.

In another preferred embodiment,
A is selected from 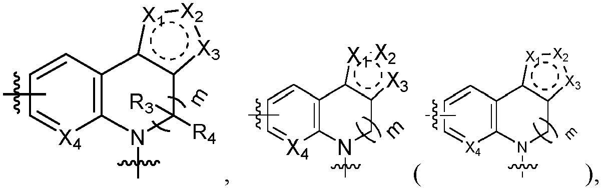 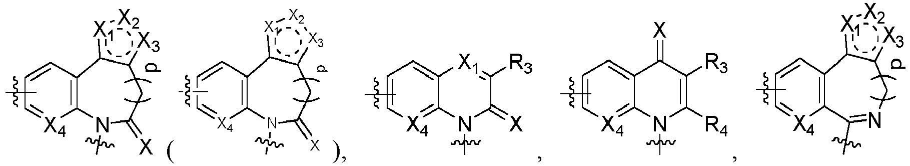 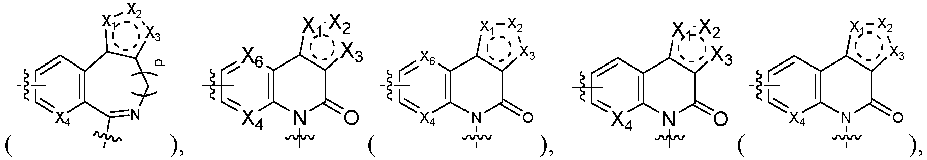 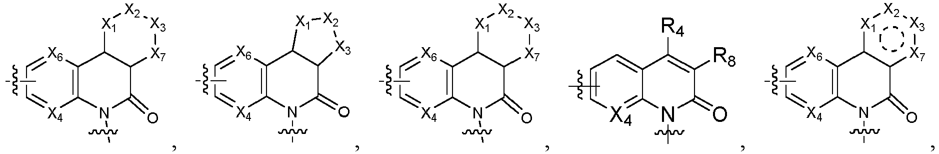 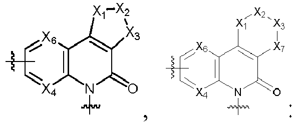
L₁ is selected from absent and CR₃R₄;
B is C6-C10 aryl;
X is O;
X₁, X₂, X₃, X₄, X₆ and X₇ are each independently selected from CR₃, N, CR₃R₄, and NR₄;
R₁ is selected from 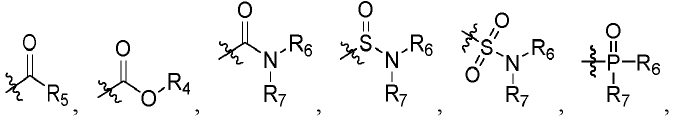 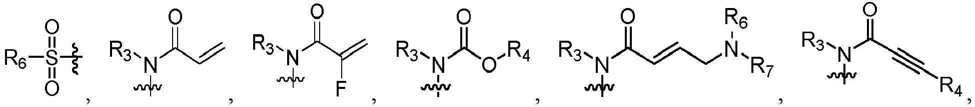 and 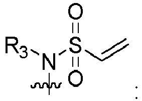
each R₂, R₃, R₄ and R₅ are independently selected form H, halogen, CN, NH₂, -CO-(C₁₋₆ alkyl), C₁₋₆ alkyl, C₁₋₆ alkoxyl, C₃₋₆ cycloalkyl, C₃₋₆ cycloalkoxy, C6-C10 aryl, 5-10 membered heteroaryl containing 1 to 3 heteroatoms selected from N, O and S, wherein NH₂, C₁₋₆ alkyl, C₁₋₆ alkoxyl, C₃₋₆ cycloalkyl, C₃₋₆ cycloalkoxy, C6-C10 aryl, 5-10 membered heteroaryl containing 1 to 3 heteroatoms selected from N, O and S are optionally substituted with 1, 2 or 3 R;
R₆ and R₇ are each independently selected from hydrogen, C₁₋₆ alkyl, C₃₋₆ cycloalkyl, C6-C10 aryl, 5-10 membered heteroaryl containing 1 to 3 heteroatoms selected from N, O and S, -S(O)₂-(C₁₋₆ alkyl), -S(O)₂-(C₂₋₆ alkenyl), wherein C₁₋₆ alkyl, C₃₋₆ cycloalkyl, C6-C10 aryl, 5-10 membered heteroaryl containing 1 to 3 heteroatoms selected from N, O and S are optionally substituted with 1, 2 or 3 R, or R₆ and R₇ form a 3-7 membered carbocycle, or R₆ and R₇ form a 3-7 membered heterocycle containing N, O or S;
R₈ is selected from 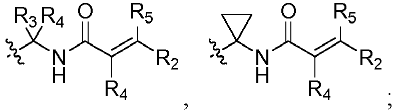
each R is independently selected from halogen, CN, OH, NH₂, C₁₋₆ alkyl, C₁₋₆ alkoxyl, C₃₋₆ cycloalkyl, C₃₋₆ cycloalkoxy, C₂₋₆ alkenyl, C₂₋₆ alkynyl, C6-C10 aryl, or 5-10 membered heteroaryl containing 1 to 3 heteroatoms selected from N, O and S;
each m and n are independently selected from 1, 2, 3 and 4;
p is selected from 0, 1 and 2.

In another preferred embodiment,
A is selected from 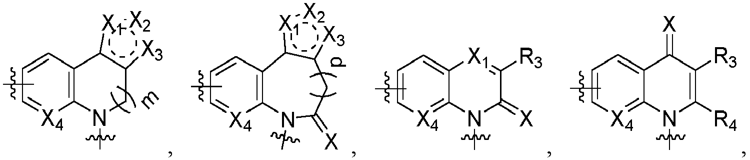 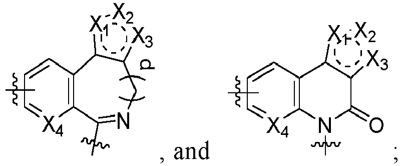
L₁ is selected from absent and CR₃R₄;
B is C6-C10 aryl;
X is O;
X₁, X₂, X₃ and X₄ are each independently selected from CR₃, N, and NR₄;
R₁ is selected from 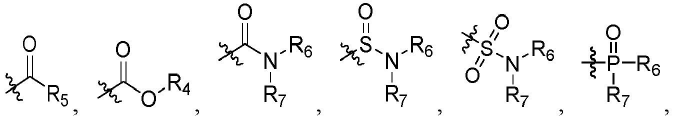 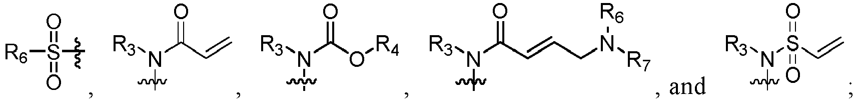
each R₂, R₃, R₄ and R₅ are independently selected form H, halogen, CN, NH₂, -CO-(C₁₋₆ alkyl), C₁₋₆ alkyl, C₁₋₆ alkoxyl, C₃₋₆ cycloalkyl, C₃₋₆ cycloalkoxy, C6-C10 aryl, 5-10 membered heteroaryl containing 1 to 3 heteroatoms selected from N, O and S, wherein NH₂, C₁₋₆ alkyl, C₁₋₆ alkoxyl, C₃₋₆ cycloalkyl, C₃₋₆ cycloalkoxy, C6-C10 aryl, 5-10 membered heteroaryl containing 1 to 3 heteroatoms selected from N, O and S are optionally substituted with 1, 2 or 3 R;
R₆ and R₇ are each independently selected from hydrogen, C₁₋₆ alkyl, C₃₋₆ cycloalkyl, C6-C10 aryl, 5-10 membered heteroaryl containing 1 to 3 heteroatoms selected from N, O and S, -S(O)₂-(C₁₋₆ alkyl), -S(O)₂-(C₂₋₆ alkenyl), wherein C₁₋₆ alkyl, C₃₋₆ cycloalkyl, C6-C10 aryl, 5-10 membered heteroaryl containing 1 to 3 heteroatoms selected from N, O and S are optionally substituted with 1, 2 or 3 R, or R₆ and R₇ form a 3-7 membered carbocycle, or R₆ and R₇ form a 3-7 membered heterocycle containing N, O or S;
each R is independently selected from halogen, CN, OH, NH₂, C₁₋₆ alkyl, C₁₋₆ alkoxyl, C₃₋₆ cycloalkyl, C₃₋₆ cycloalkoxy, C₂₋₆ alkenyl, C₂₋₆ alkynyl, C6-C10 aryl, or 5-10 membered heteroaryl containing 1 to 3 heteroatoms selected from N, O and S;
each m and n are independently selected from 1, 2, 3 and 4;
p is selected from 0, 1 and 2.

In another preferred embodiment,
A is selected from 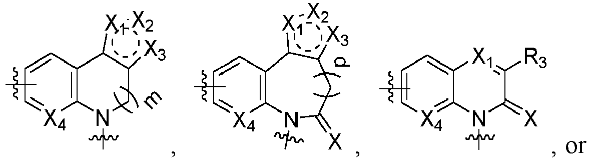 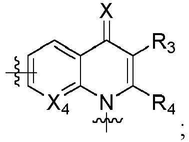
L₁ is selected from absent and CR₃R₄;
B is C6-C10 aryl;
X is O;
X₁, X₂, X₃ and X₄ are each independently selected from CR₃, N, and NR₄;
R₁ is selected from 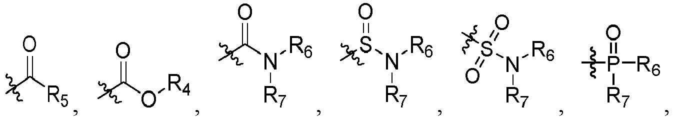 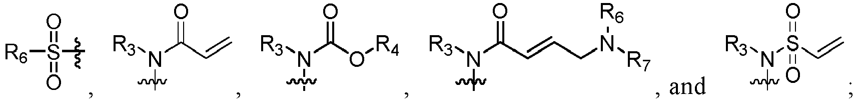
each R₂, R₃, R₄ and R₅ are independently selected form H, halogen, CN, NH₂, -CO-(C₁₋₆ alkyl), C₁₋₆ alkyl, C₁₋₆ alkoxyl, C₃₋₆ cycloalkyl, C₃₋₆ cycloalkoxy, C6-C10 aryl, 5-10 membered heteroaryl containing 1 to 3 heteroatoms selected from N, O and S, wherein NH₂, C₁₋₆ alkyl, C₁₋₆ alkoxyl, C₃₋₆ cycloalkyl, C₃₋₆ cycloalkoxy, C6-C10 aryl, 5-10 membered heteroaryl containing 1 to 3 heteroatoms selected from N, O and S are optionally substituted with 1, 2 or 3 R;
R₆ and R₇ are each independently selected from hydrogen, C₁₋₆ alkyl, C₃₋₆ cycloalkyl, C6-C10 aryl, 5-10 membered heteroaryl containing 1 to 3 heteroatoms selected from N, O and S, -S(O)₂-(C₁₋₆ alkyl), -S(O)₂-(C₂₋₆ alkenyl), wherein C₁₋₆alkyl, C₃₋₆ cycloalkyl, C6-C10 aryl, 5-10 membered heteroaryl containing 1 to 3 heteroatoms selected from N, O and S are optionally substituted with 1, 2 or 3 R, or R₆ and R₇ form a 3-7 membered carbocycle, or R₆ and R₇ form a 3-7 membered heterocycle containing N, O or S;
each R is independently selected from halogen, CN, OH, NH₂, C₁₋₆ alkyl, C₁₋₆ alkoxyl, C₃₋₆ cycloalkyl, C₃₋₆ cycloalkoxy, C₂₋₆ alkenyl, C₂₋₆ alkynyl, C6-C10 aryl, or 5-10 membered heteroaryl containing 1 to 3 heteroatoms selected from N, O and S;
each m and n are independently selected from 1, 2, 3 and 4;
p is selected from 0, 1 and 2.

In another preferred embodiment, the compound is selected from the group consisting of: 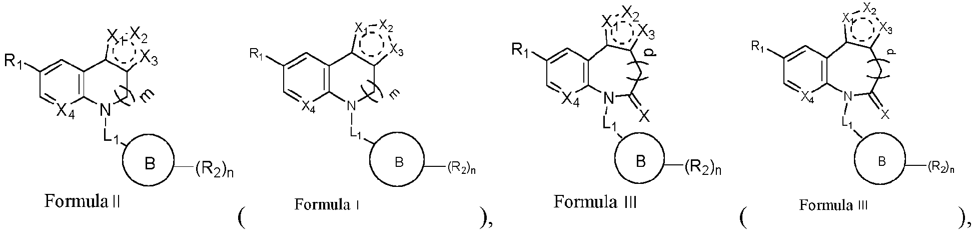 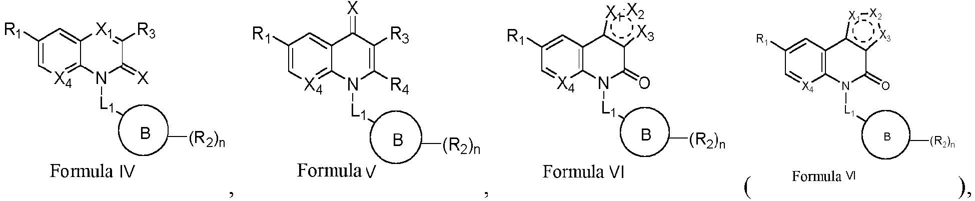 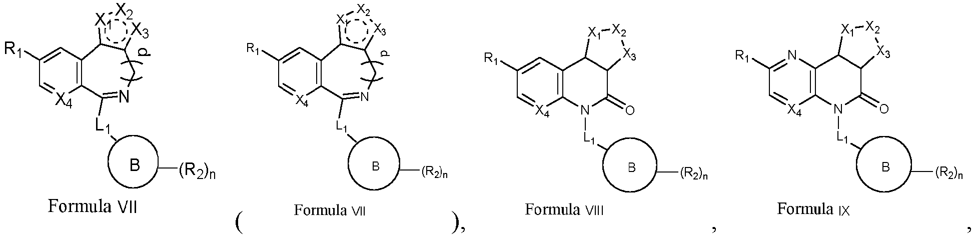 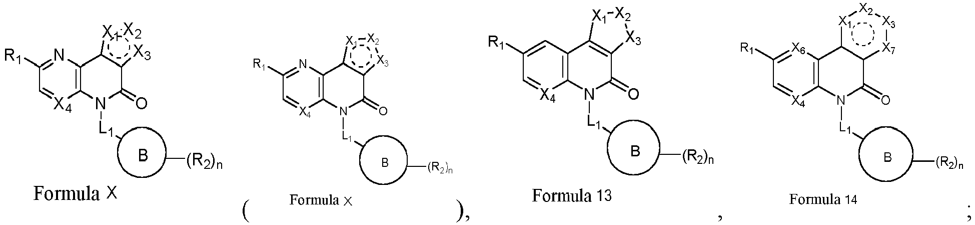 wherein, each group is as defined above.

In another preferred embodiment, the compound is selected from the group consisting of: 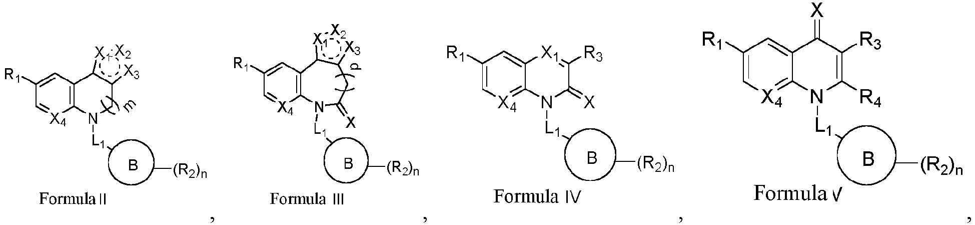 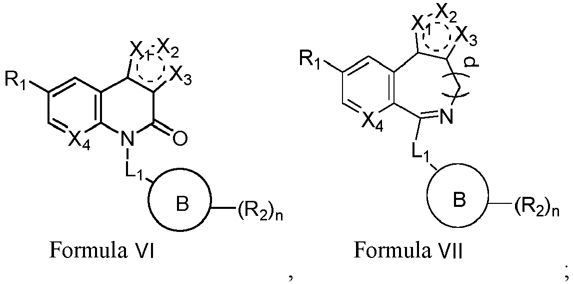 wherein, each group is as defined above.

In another preferred embodiment, the compound is selected from the group consisting of: 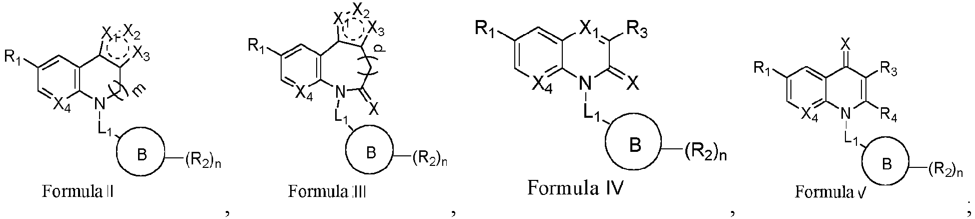 wherein, each group is as defined above.

In another preferred embodiment,
the compound is selected from the group consisting of: 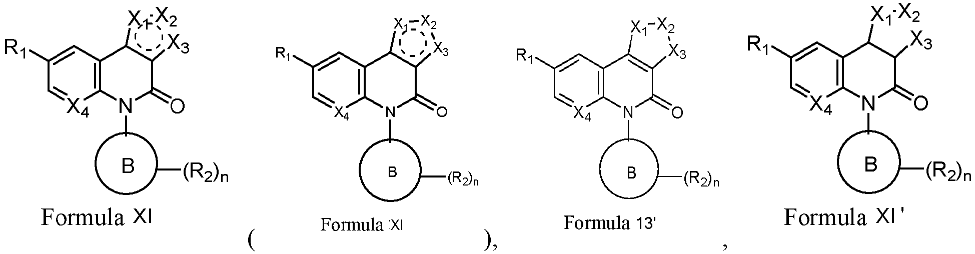 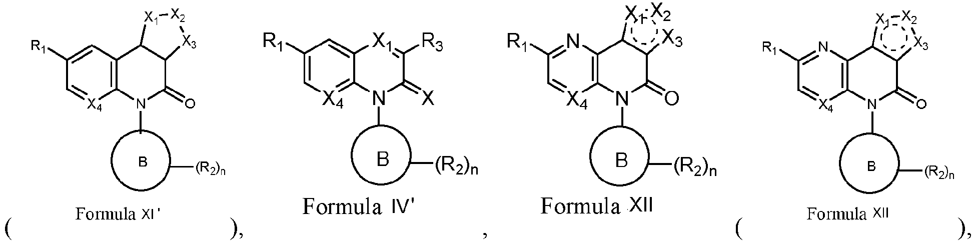 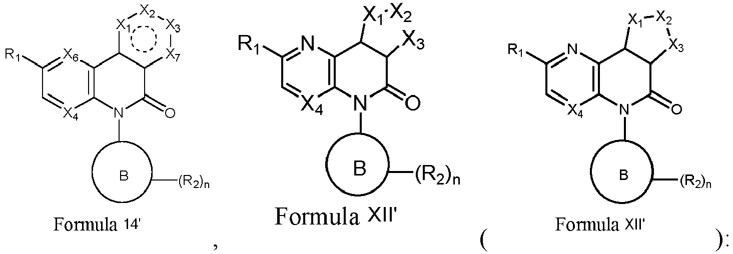
R₁ is selected from CN, ureido, carbamate group, 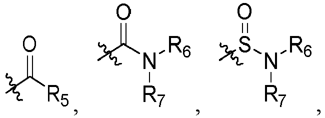 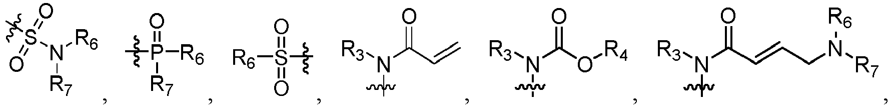 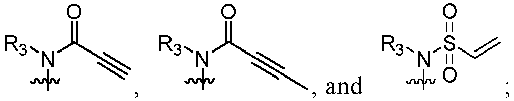
wherein, each group is as defined above.

In another preferred embodiment, R₁ is selected from CN, ureido, carbamate group,

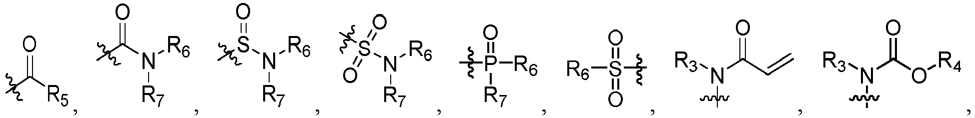

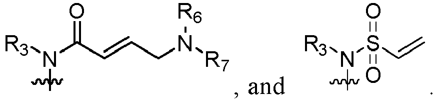

In another preferred embodiment, R₂ is selected from trifluoromethyl, fluorine, chlorine, bromine, iodine, methyl, cyclopentyl, and cyclohexyl and pentafluorothio.

In another preferred embodiment, R₂ is selected from trifluoromethyl, fluorine, chlorine, bromine, iodine, methyl, cyclopentyl, and cyclohexyl.

In another preferred embodiment, 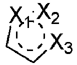 is an aromatic group or an unsaturated group.

In another preferred embodiment, 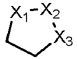 is a non-aromatic group or a saturated group. Similar format groups have similar meanings.

In another preferred embodiment, 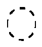 means a group containing the structure and being an aromatic group or an unsaturated group.

In another preferred embodiment, the compound is selected from the group consisting of:

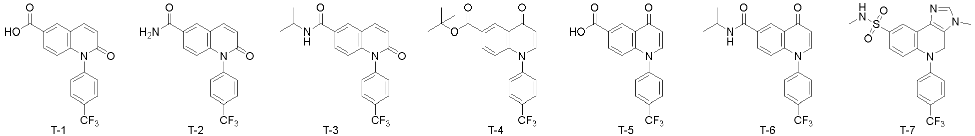

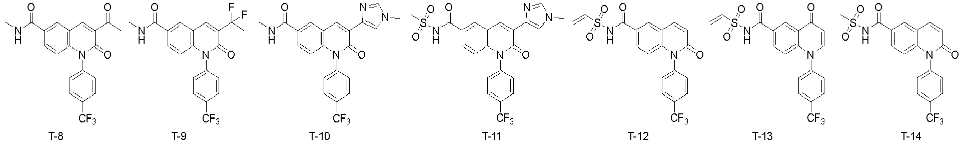

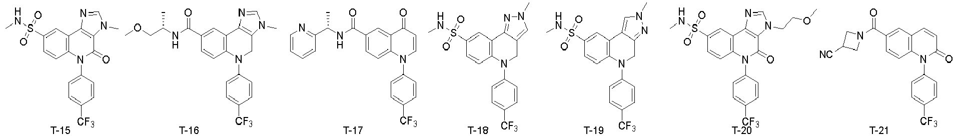

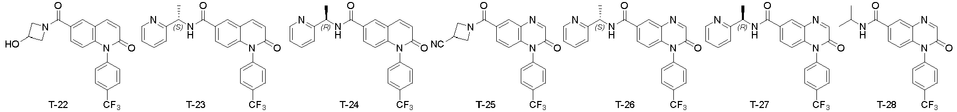

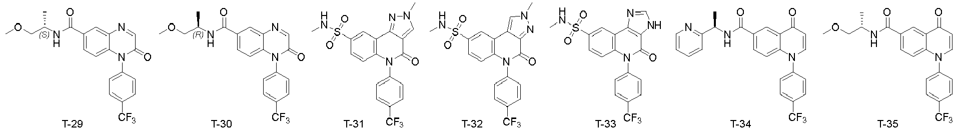

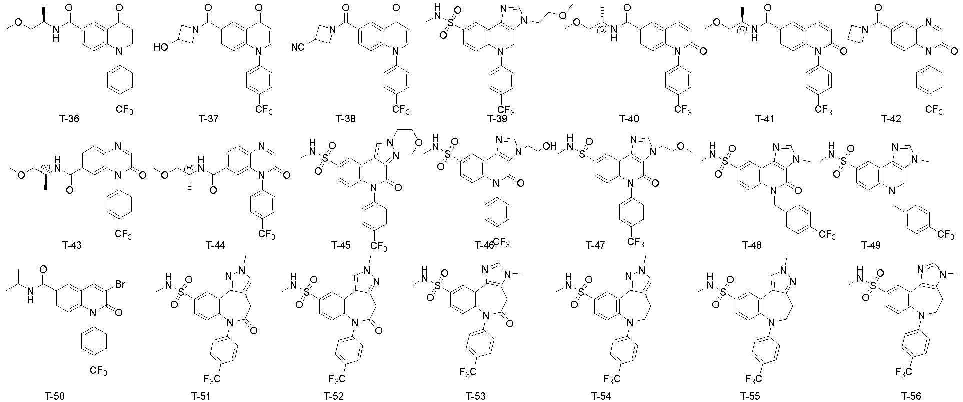

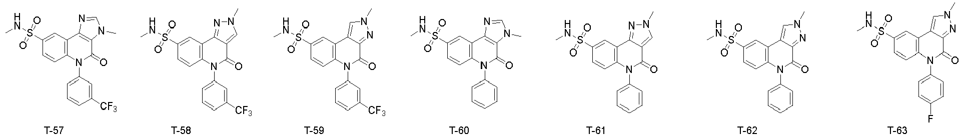

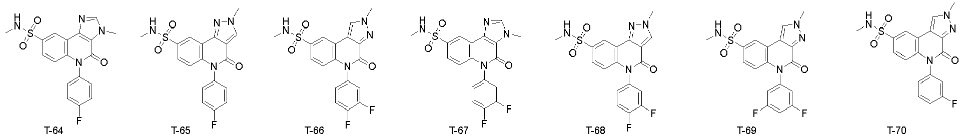

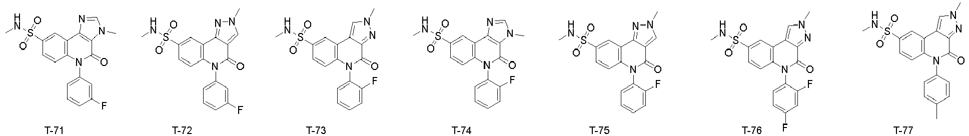

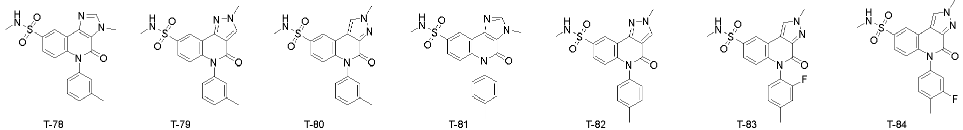

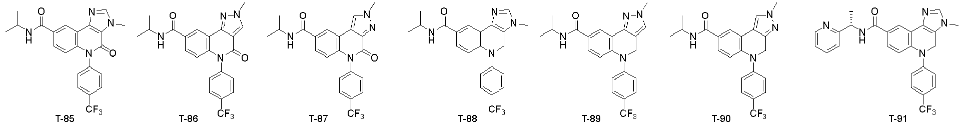

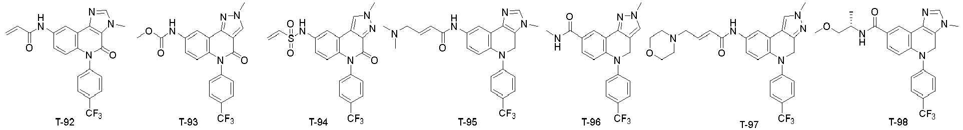

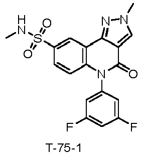

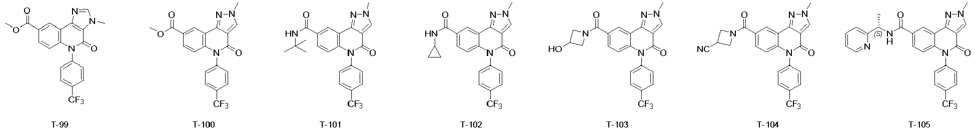

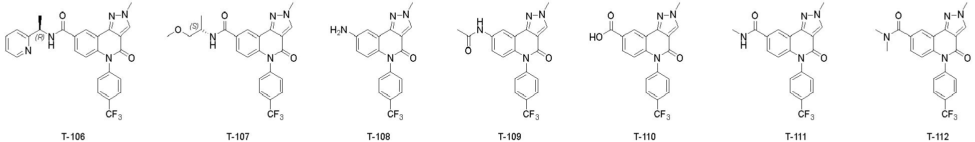

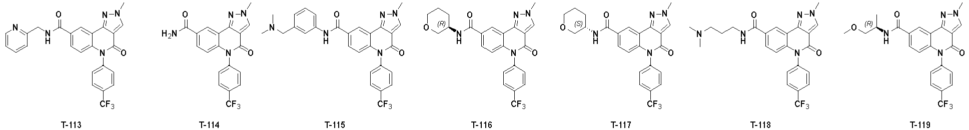

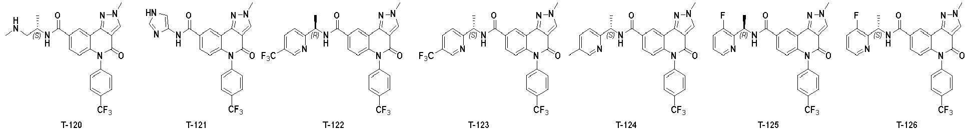

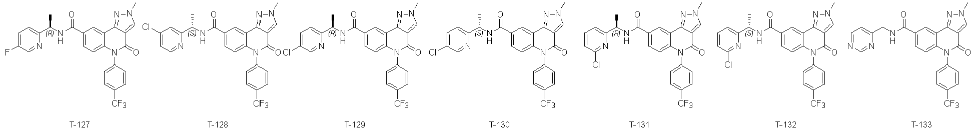

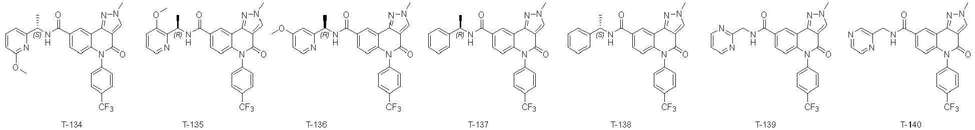

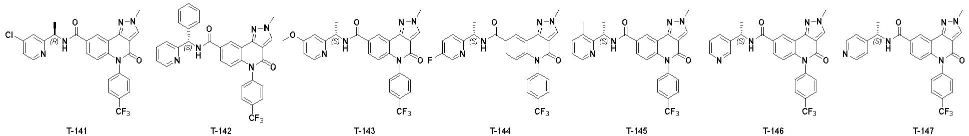

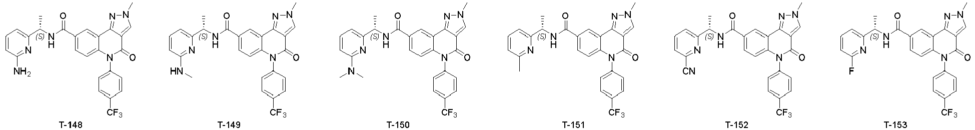

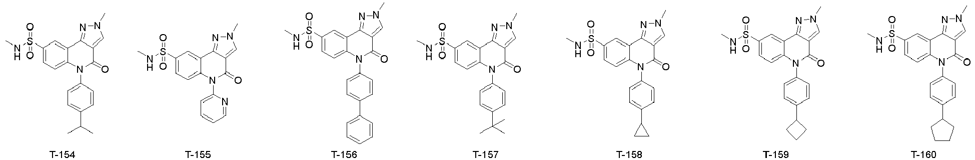

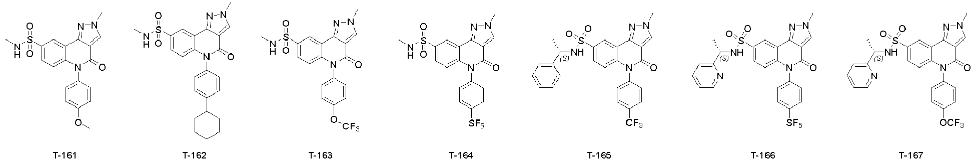

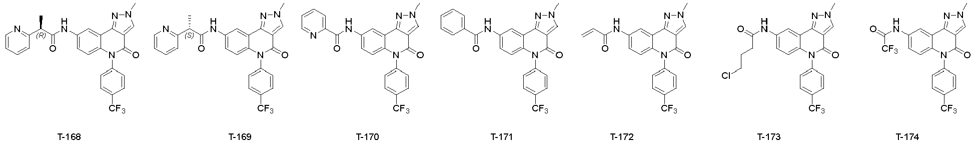

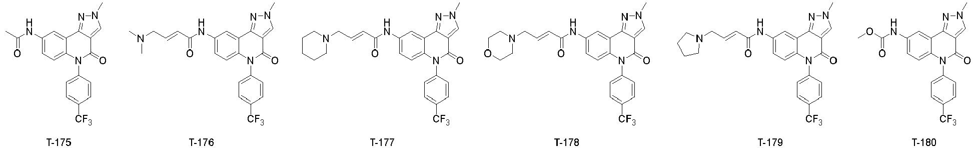

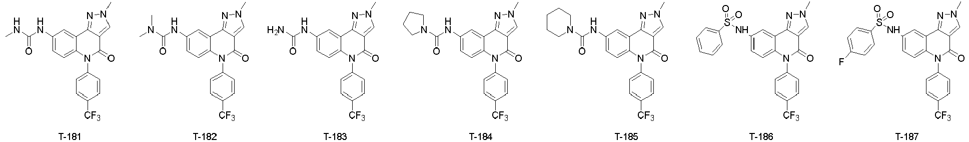

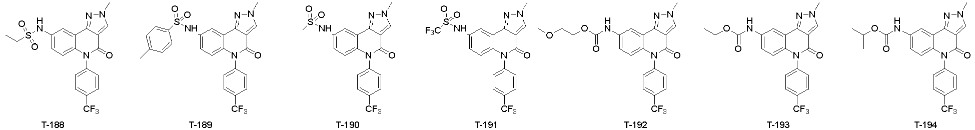

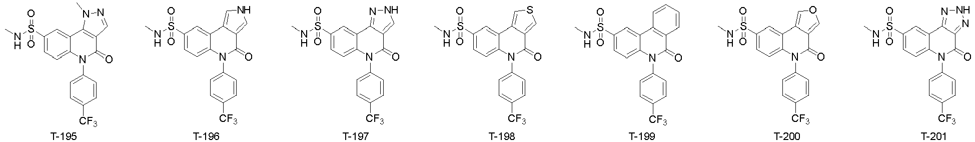

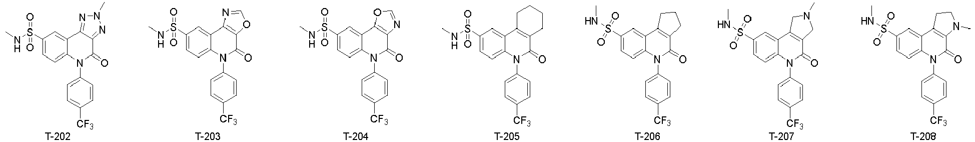

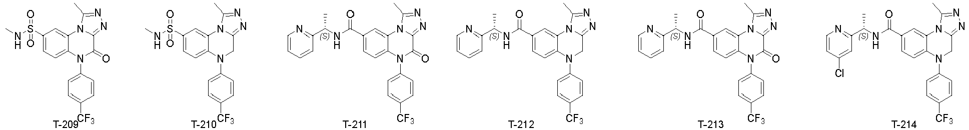

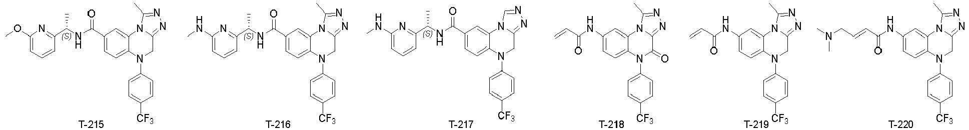

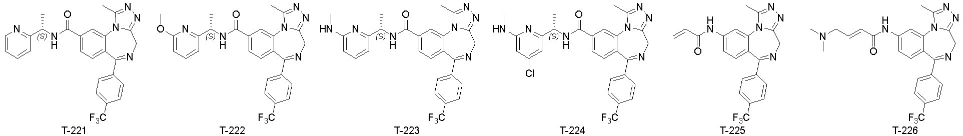

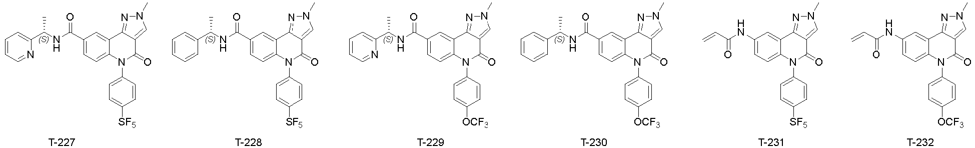

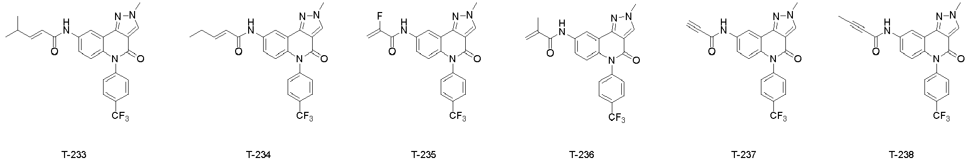

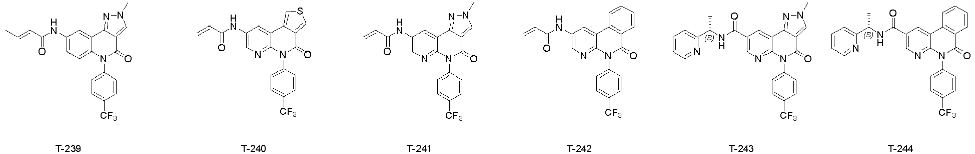

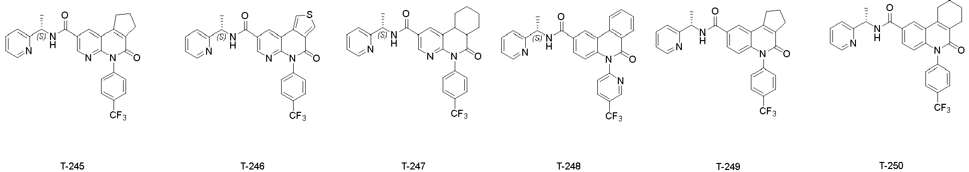

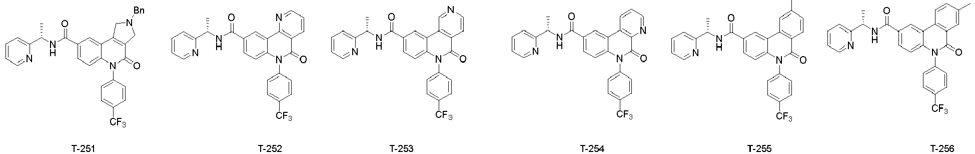

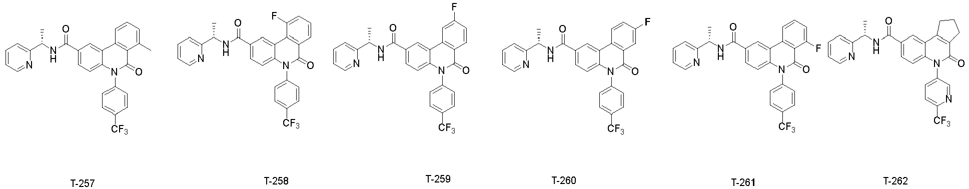

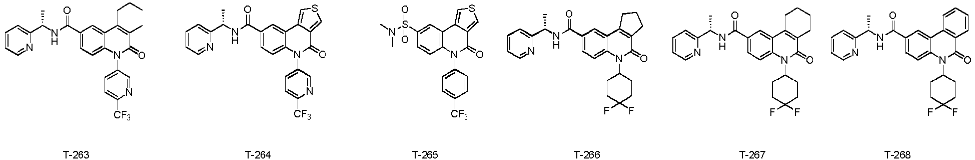

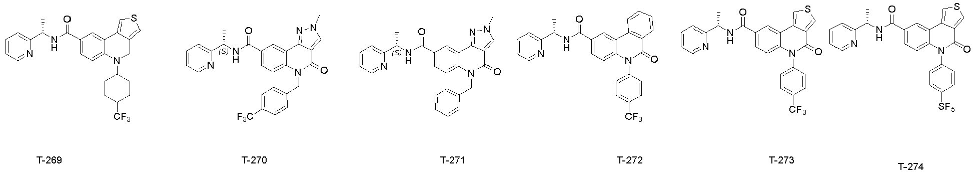

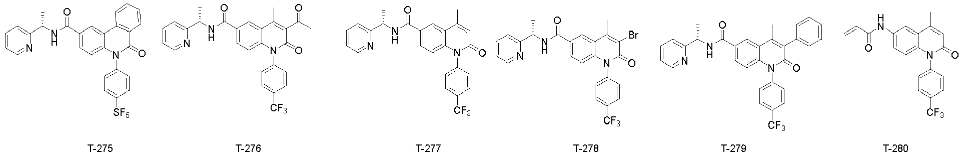

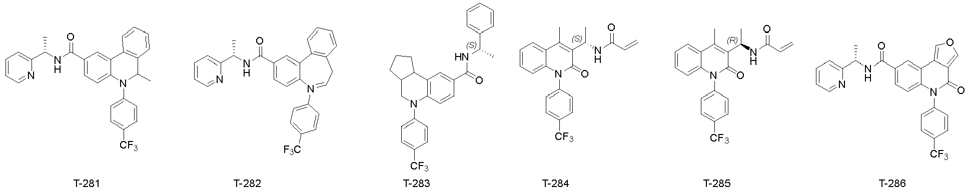

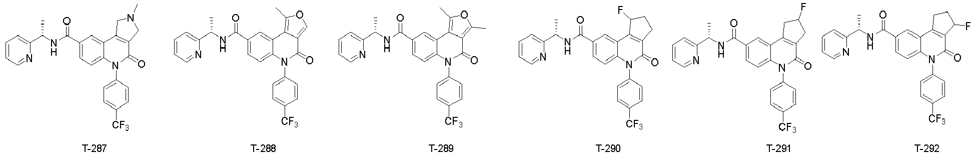

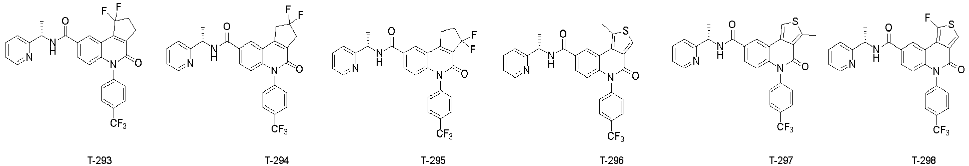

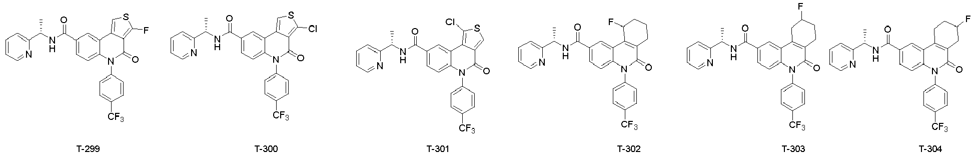

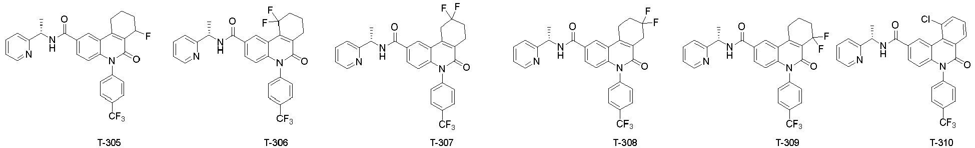

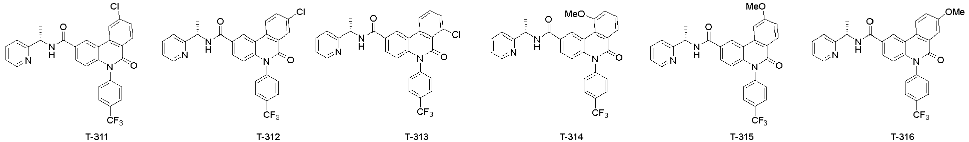

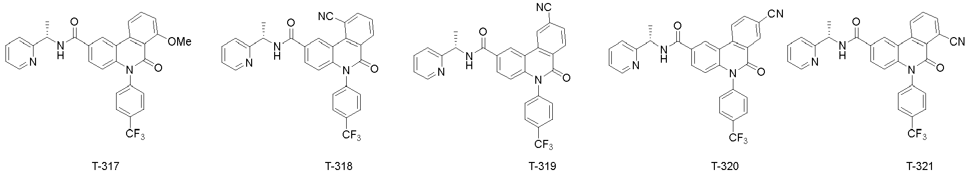

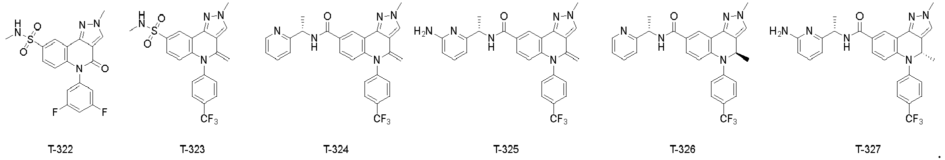

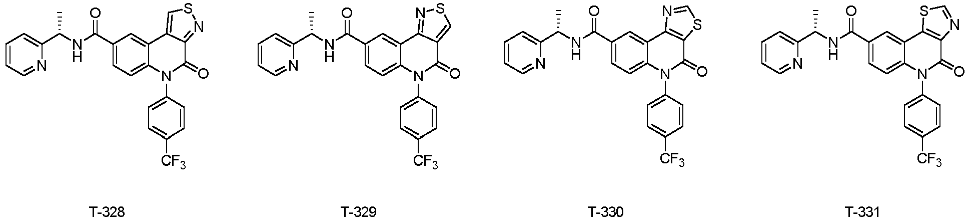

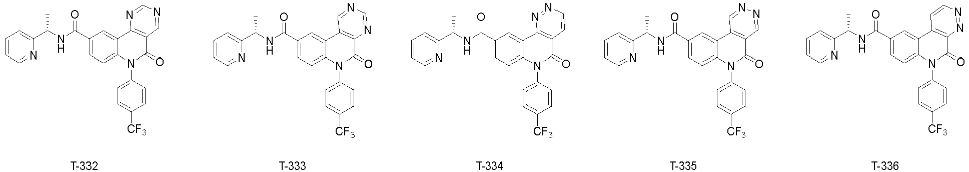

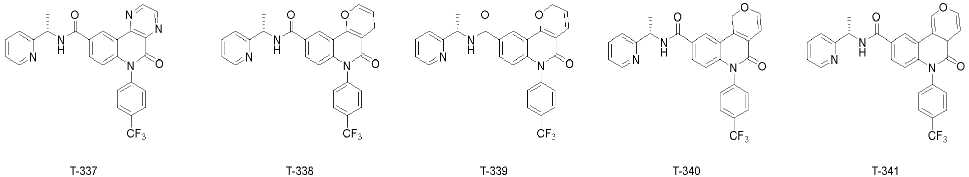

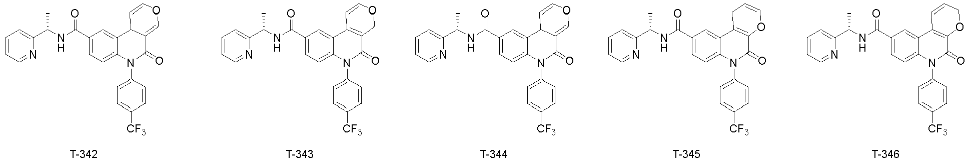

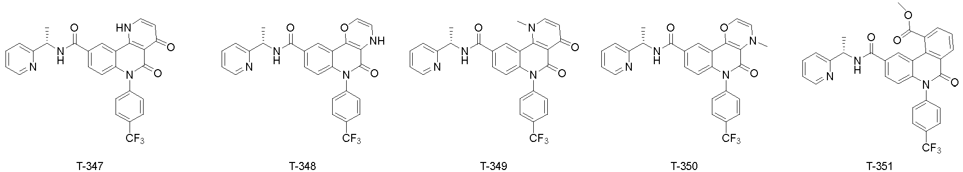

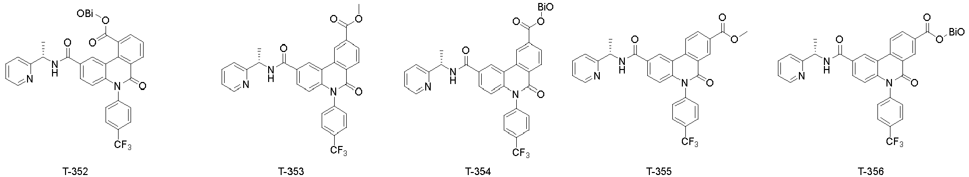

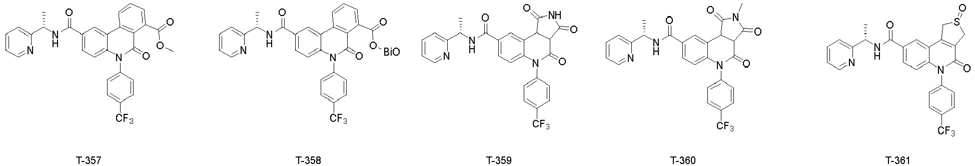

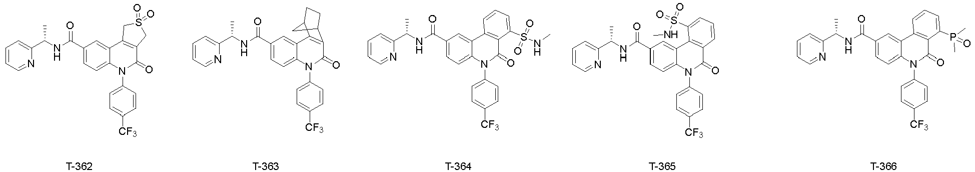

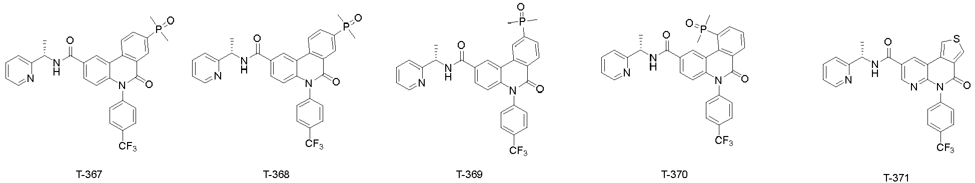

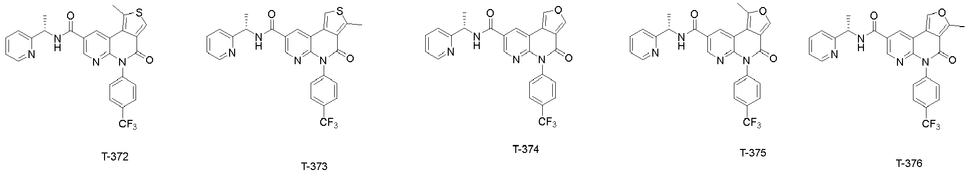

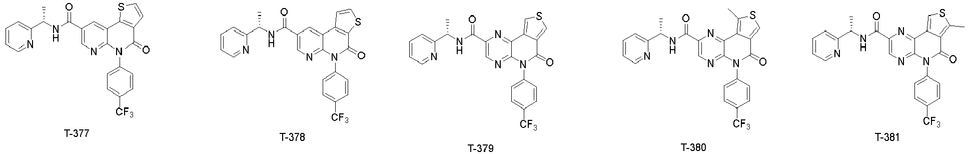

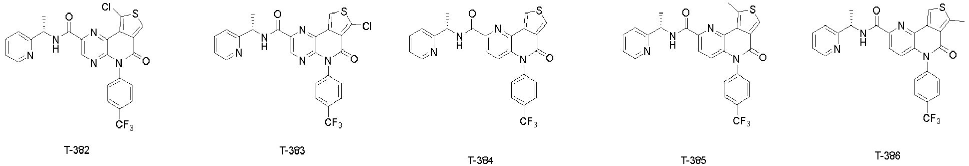

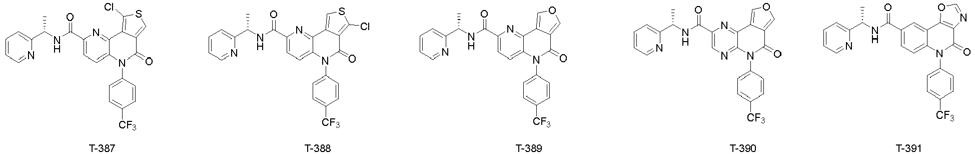

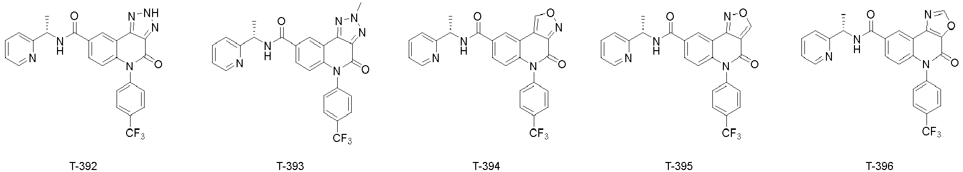

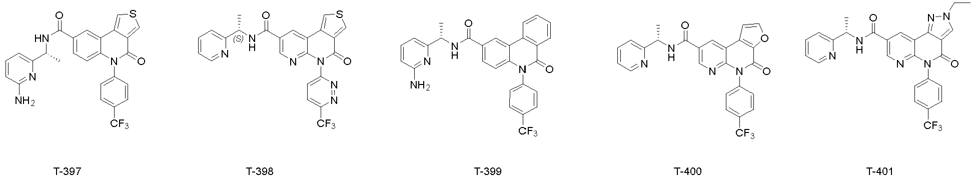

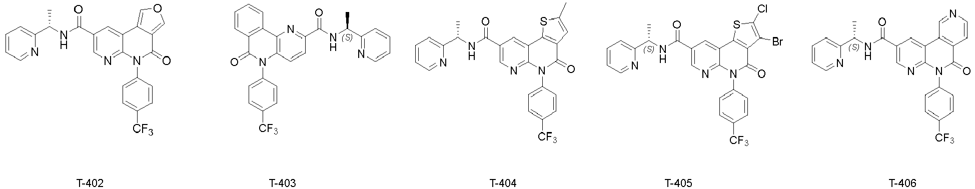

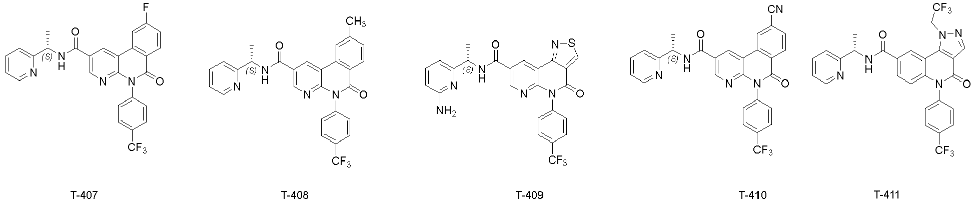

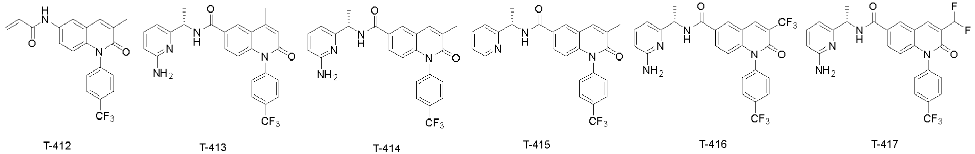

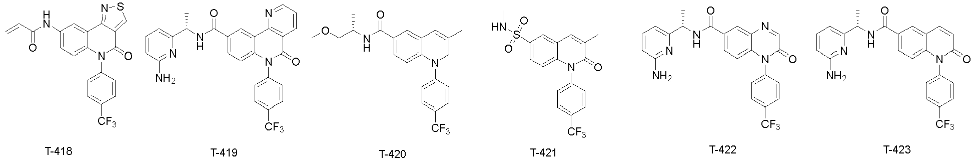

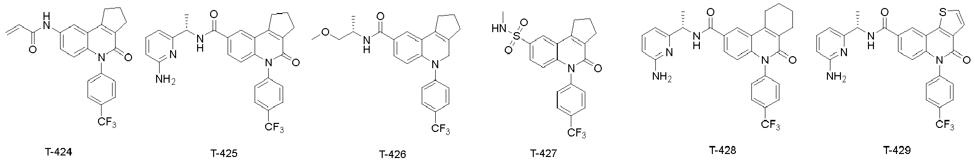

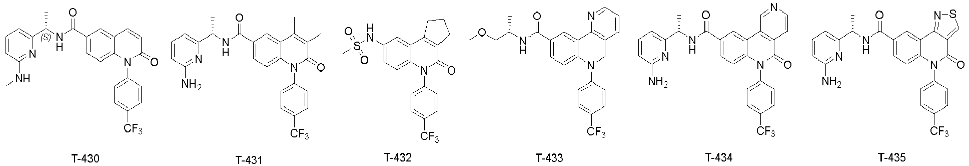

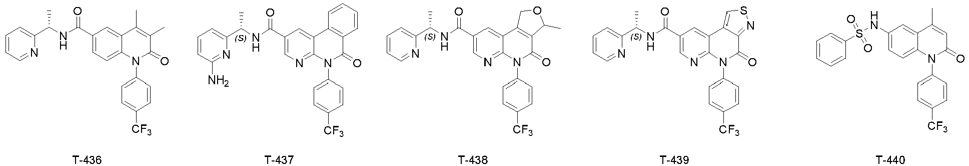

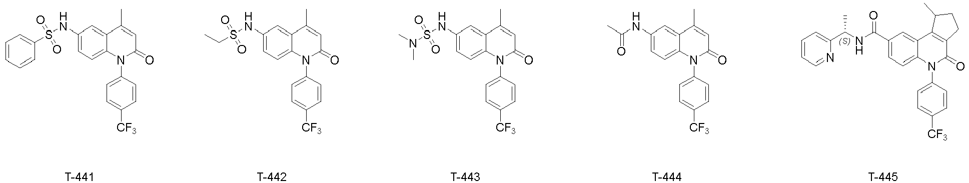

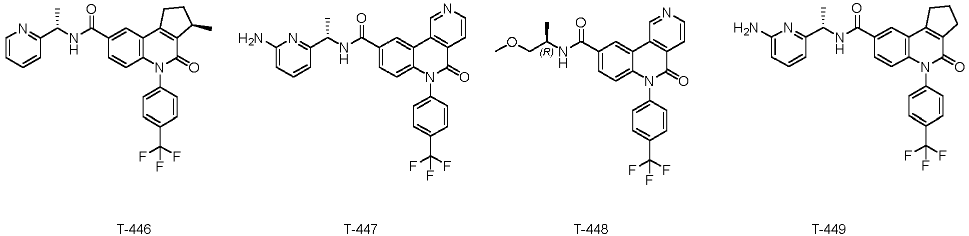

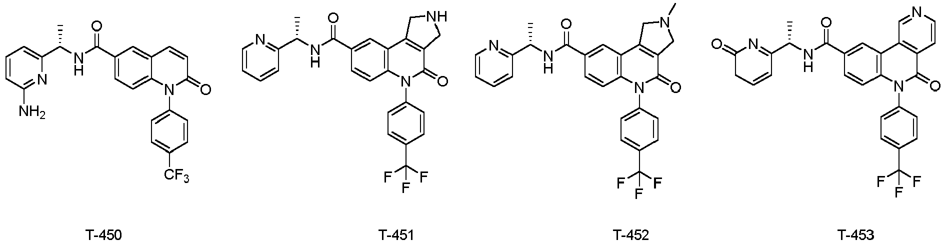

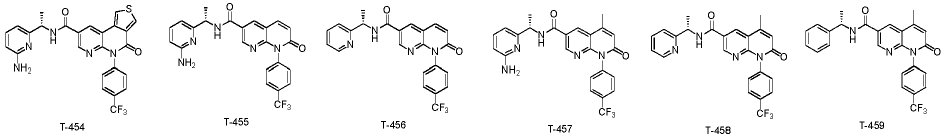

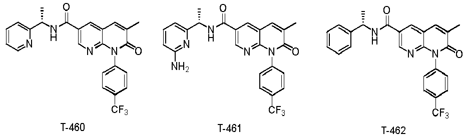

In the second aspect of the present invention, provided is a pharmaceutical composition, comprising a pharmaceutically acceptable carrier and one or more of safe and effective amount of the compound of the first aspect of the present invention or the pharmaceutically acceptable salt, the solvate or the prodrug thereof.

In the third aspect of the present invention, provided is a use of the pharmaceutical composition of the second aspect of the present invention in the preparation of a drug for the prevention and/or treatment of related diseases caused by Hippo pathway dysregulation.

In the fourth aspect of the present invention, provided is a use of the pharmaceutical composition of the second aspect of the present invention in the preparation of a drug for the prevention and/or treatment of related diseases caused by the dysregulation of YAP or TAZ or YAP/TAZ or YAP/TEAD or YAP/TAZ/TEAD.

In another preferred embodiment, the disease is selected from the group consisting of: lung cancer, breast cancer, prostate cancer, colorectal cancer, liver cancer, pancreatic cancer, ovarian cancer, leukemia, neuroblastoma, gastric cancer, kidney cancer, esophageal cancer, uterine cancer, and pleural mesothelioma.

In the fifth aspect of the present invention, provided is a combination of the compound of the first aspect of the present invention or the pharmaceutically acceptable salt, the solvate or the prodrug thereof with a second drug in the preparation of a drug for the prevention and/or treatment of cancers;
the second drug is selected from the group consisting of: ERK inhibitors, MEK inhibitors, KRAS inhibitors, BRAF inhibitors, EGFR inhibitors, Wnt inhibitors, PD-1 inhibitors, and combinations thereof.

It should be understood that within the scope of the present invention, the above technical features of the present invention and the technical features specifically described in the following (e.g., embodiments) can be combined with each other, thereby forming a new or preferred technical solution. Due to space limitations, it will not be repeated herein.

### EMBODIMENTS FOR CARRYING OUT THE INVENTION

The present inventors have unexpectedly prepared a class of compounds with excellent YAP/TEAD inhibitory activity after long-term and in-depth research. The inventor has completed the present invention on this basis.

### TERMS

In the present invention, unless specifically indicated, the terms used have the general meanings well known to those skilled in the art.

In the present invention, the term "halogen" refers to F, Cl, Br or I.

In the present invention, "C1-C6 alkyl" refers to a linear or branched alkyl including 1-6 carbon atoms, such as methyl, ethyl, propyl, isopropyl, butyl, isobutyl, tert-butyl, neopentyl, tert-pentyl, or similar groups. The term "C₁₋₆ alkyl" has a similar meaning.

In the present invention, the term "C2-C6 alkenyl" refers to a straight or branched chain alkenyl having 2 -6 carbon atoms containing a double bond, including but not limited to vinyl, propenyl, butenyl, isobutenyl, pentenyl and hexenyl, etc. The term "C₂₋₆ alkenyl" has a similar meaning.

In the present invention, the term "C2-C6 alkynyl" refers to a straight or branched chain alkynyl having 2-6 carbon atoms containing a triple bond, including but not limited to ethynyl, propynyl, butynyl, isobutynyl, pentynyl and hexynyl, etc. The term "C₂₋₆ alkynyl"has a similar meaning.

In the present invention, the term "C3-C8 cycloalkyl" refers to a cyclic alkyl having 3-8 carbon atoms in the ring, including but not limited to cyclopropyl, cyclobutyl, cyclopentyl, cyclohexyl, cycloheptyl, cyclooctyl, etc. The terms "C₃₋₈ cycloalkyl", "C₃₋₆ cycloalkyl", "C₅₋₁₀ cycloalky" have similar meanings.

In the present invention, the term "C1-C6 alkoxyl" refers to a straight or branched chain alkoxyl having 1-6 carbon atoms, including but not limited to methoxy, ethoxy, propoxy, isopropoxy and butoxy, etc., preferably C1-C4 alkoxyl. The term "C₁₋₆ alkoxyl" has a similar meaning.

In the present invention, the term "heterocyclyl" means a 4 to 8 membered heterocyclic group containing 1, 2, or 3 heteroatoms selected from N, O, and S, including, but not limited to, the following groups:

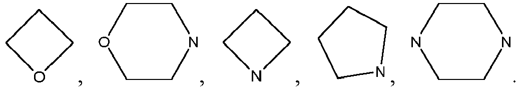

In the present invention, the term "aromatic ring" or "aryl" has the same meaning, preferably is "C6-C10 aryl". The term "C6-C10 aryl" refers to an aromatic ring having 6-10 carbon atoms in the ring that does not contain heteroatoms, such as phenyl, naphthyl and the like.

In the present invention, the term "aromatic heterocycle" or "heteroaryl" has the same meaning and refers to a heteroaromatic group containing one to more heteroatoms. For example, "C3-C10 heteroaryl" means an aromatic heterocycle containing 1 to 4 heteroatoms selected from the group consisting of oxygen, sulfur and nitrogen and 3-10 carbon atoms. Non-limiting examples include: furyl, thienyl, pyridyl, pyrazolyl, pyrrolyl, N-alkylpyrrolyl, pyrimidinyl, pyrazinyl, imidazolyl, tetrazolyl and the like. The heteroaryl ring may be fused to an aryl, heterocyclic or cycloalkyl ring, wherein the ring connected to the parent structure is the heteroaryl ring. Heteroaryl can be optionally substituted or unsubstituted.

In the present invention, the term "halogenated" means being substituted by halogen.

In the present invention, the term "deuterated" means being substituted by deuterium.

In the present invention, the term "substituted" means that one or more hydrogen atoms on a specific group is replaced with a specific substituent. The specific substituents are the substituents described correspondingly in the foregoing, or the substituents appearing in the respective embodiments. Unless otherwise specified, a substituted group may have a substituent selected from a specific group at any substitutable position of the group, and the substituent may be the same or different at each position. Those skilled in the art will understand that the combinations of substituents contemplated by the present invention are those that are stable or chemically achievable. The substituents are, for example (but not limited to): halogen, hydroxyl, carboxyl (-COOH), C1-C6 alkyl, C2-C6 alkenyl, C2-C6 alkynyl, C3-C8 cycloalkyl, 3-12 membered heterocyclyl, aryl, heteroaryl, C1-C8 aldehyde group, C2-C10 acyl, C2-C10 ester group, amino, C1-C6 alkoxyl, C1-C10 sulfonyl and the like.

In the present invention, the term "1-6" refers to 1, 2, 3, 4, 5 or 6. Other similar terms have similar meanings independently.

The term "ester group" has a structure of - C(O)-O-R' or R'-C(O)-O- , wherein R' independently represents hydrogen, C1-C6 alkyl, C3-C6 cycloalkyl, C6-C10 aryl, heteroaryl, or heterocyclyl, as defined above.

The term "ureido" has a structure of 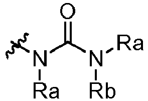 wherein Ra, Rb are each independently selected from H, C1-C6 alkyl, halo-C1-C6 alkyl, C6-C10 aryl.

The term "carbamate group" has a structure of 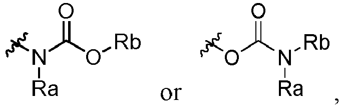 wherein Ra, Rb are each independently selected from H, C1-C6 alkyl, halo-C1-C6 alkyl, C6-C10 aryl.

Term "acylamino" refers to a group with a structure of -CONRR', wherein R and R' independently represent hydrogen, alkyl or substituted alkyl, cycloalkyl or substituted cycloalkyl, aryl or substituted aryl, heterocyclyl or substituted heterocyclyl with the definitions as described above. R and R' may be the same or different in the dialkylamino fragments.

The term "aralkyl" refers to an alkyl group substituted with an aryl or heteroaryl group, wherein the aryl, heteroaryl, and alkyl groups are as defined herein. Typically, the aryl group can have 6 to 14 carbon atoms, the heteroaryl group can have 5 to 14 ring atoms, and the alkyl group can have 1 to 6 carbon atoms. Exemplary aralkyl include but are not limited to benzyl, phenylethyl, phenylpropyl, and phenylbutyl.

It should be understood that when a certain functional group exists at multiple different positions in a compound, its definition at each position is independent from each other and can be the same or different. That is to say, the terms "selected from the group consisting of" and "independently selected from the group consisting of" have the same meaning.

### Compound

The present invention provides a compound of formula I, or a pharmaceutically acceptable salt, a solvate or a prodrug thereof, 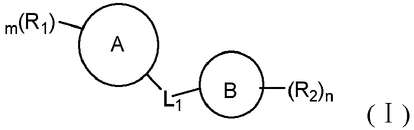 wherein, each group is as defined above.

In another preferred embodiment, A, L₁, B, R₁, R₂, m and n are each independently the corresponding group in each specific compound in the present invention.

As used herein, the term "pharmaceutically acceptable salt" refers to a salt formed by a compound of the present invention with an acid or base that is suitable for use as a medicine. Pharmaceutically acceptable salts include inorganic salts and organic salts. A group of preferred salts are salts formed of the compound of the present invention and acids. Acids suitable for forming salts include, but are not limited to, inorganic acids such as hydrochloric acid, hydrobromic acid, hydrofluoric acid, sulfuric acid, nitric acid, phosphoric acid and the like; organic acids such as methanoic acid, acetic acid, trifluoroacetic acid, propionic acid, oxalic acid, malonic acid, succinic acid, fumaric acid, maleic acid, lactic acid, malic acid, tartaric acid, citric acid, bitter acid, benzoic acid, methanesulfonic acid, ethanesulfonic acid, p-toluenesulfonic acid, benzenesulfonic acid, naphthalene sulfonic acid, and the like; and amino acids such as proline, phenylalanine, aspartic acid, glutamic acid and the like.

Another preferred class of salts are salts of the compounds of the invention formed with bases, such as alkali metal salts (such as sodium or potassium salts), alkaline earth metal salts (such as magnesium or calcium salts), ammonium salts (such as lower grades alkanol ammonium salts and other pharmaceutically acceptable amine salts), such as methylamine salt, ethylamine salt, propylamine salt, dimethylamine salt, trimethylamine salt, diethylamine salt, triethylamine salt, tert-butylamine salt, ethylenediamine salt, hydroxyethylamine salt, dihydroxyethylamine salt, trishydroxyethylamine salt, and an amine salt formed from morpholine, piperazine, and lysine, respectively.

The term "solvate" refers to a complex in which the compound of the present invention coordinates with solvent molecules at a specific ratio. "Hydrate" refers to a complex formed by the coordination of the compound of the invention with water.

Moreover, the compounds of the present invention further comprise prodrugs of the compound of formula (I). The term "prodrug" includes compounds which are themselves biologically active or inactive, when administered in an appropriate manner, undergoes metabolism or chemical reaction in the human body to convert to a compound of formula I, or a salt or a solution comprising a compound of formula I. The prodrugs include (but are not limited to) carboxylates, carbonates, phosphates, nitrates, sulfates, sulfones, sulfoxides, amino compounds, formates, azo compounds, phosphoramides, glucosides, ethers, acetals, etc..

### Pharmaceutical compositions and administration method

The present invention further provides a pharmaceutical composition, comprising a pharmaceutically acceptable carrier and one or more of safe and effective amount of the compound or the pharmaceutically acceptable salt, the solvate or the prodrug thereof.

Because the compound of the present invention has excellent anti-tumor activity, the compound of the present invention and its various crystal forms, pharmaceutically acceptable inorganic or organic salts, hydrates or solvates, and pharmaceutical compositions containing the compound of the present invention as the main active ingredient can be used to treat, prevent, and alleviate tumor-related diseases.

The pharmaceutical composition of the invention comprises the compound of the present invention or the pharmaceutically acceptable salts thereof in a safe and effective dosage range, and pharmaceutically acceptable excipients or carriers. Wherein, "safe and effective amount" means that the amount of the compound is sufficient to significantly improve the condition without causing serious side effects. Generally, the pharmaceutical composition contains 1-2000 mg of the compound of the present invention/dose, more preferably, 10-1000 mg of the compound of the present invention/dose. Preferably, the "one dose" is one capsule or one pill.

"Pharmaceutically acceptable carrier" means one or more compatible solid or liquid fillers, or gelatinous materials which are suitable for human use and should be of sufficient purity and sufficiently low toxicity. "Compatibility" means that each component in the composition can be admixed with each other and with the compounds of the present invention without significantly reducing the efficacy of the compounds. Some examples of pharmaceutically acceptable carriers include cellulose and the derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween^{®}), wetting agent (such as sodium dodecyl sulfate), coloring agents, flavoring agents, stabilizers, antioxidants, preservatives, pyrogen-free water, etc..

The pharmaceutical composition is an injection, a capsule, a tablet, a pill, powders, or granules.

There is no special limitation on administration mode for the compounds or pharmaceutical compositions of the present invention, and representative administration mode includes (but is not limited to): oral, intratumoral, rectal, parenteral (intravenous, intramuscular or subcutaneous), and topical administration.

Solid dosage forms for oral administration include capsules, tablets, pills, powders and granules. In these solid dosage forms, the active compounds are mixed with at least one conventional inert excipient (or carrier), such as sodium citrate or dicalcium phosphate, or mixed with any of the following components: (a) fillers or compatibilizer, for example, starch, lactose, sucrose, glucose, mannitol and silicic acid; (b) binders, for example, hydroxymethyl cellulose, alginates, gelatin, polyvinylpyrrolidone, sucrose and arabic gum; (c) humectant, such as, glycerol; (d) disintegrants such as agar, calcium carbonate, potato starch or tapioca starch, alginic acid, certain composite silicates, and sodium carbonate; (e) dissolution-retarding agents, such as paraffin; (f) absorption accelerators, for example, quaternary ammonium compounds; (g) wetting agents, such as cetyl alcohol and glyceryl monostearate; (h) adsorbents, for example, kaolin; and (i) lubricants such as talc, stearin calcium, magnesium stearate, solid polyethylene glycol, sodium lauryl sulfate, or a mixture thereof. In capsules, tablets, and pills, the dosage form may also include a buffer.

Solid dosage forms such as tablets, sugar pills, capsules, pills, and granules can be prepared using coating and shell materials, such as casings and other well-known materials in the art. They can contain opaque agents. The active compounds or compounds in the compositions can be released in a delayed mode in a given portion of the digestive tract. Examples of embedding components that can be used are polymeric substances and waxes. If necessary, the active compound can also form microcapsules with one or more of the aforementioned excipients.

Liquid dosage forms for oral administration include pharmaceutically acceptable emulsions, solutions, suspensions, syrups or tinctures. In addition to the active compound, the liquid dosage form may contain inert diluents conventionally used in the art, such as water or other solvents, solubilizers and emulsifiers, such as ethanol, isopropanol, ethyl carbonate, ethyl acetate, propylene glycol, 1,3-butanediol, dimethylformamide and oils, especially cottonseed oil, peanut oil, corn germ oil, olive oil, castor oil and sesame oil or mixtures of these substances.

In addition to these inert diluents, the composition can also include additives such as wetting agents, emulsifiers and suspending agents, sweeteners, corrigents, and spices.

In addition to active compounds, suspensions can include suspending agents such as ethoxylated isooctadecanol, polyoxyethylene sorbitol and dehydrated sorbitol esters, microcrystalline cellulose, methanol aluminum and agar, or mixtures of these substances.

The compositions for parenteral injection may comprise physiologically acceptable sterile aqueous or anhydrous solutions, dispersions, suspensions or emulsions, and sterile powders which can be re-dissolved into sterile injectable solutions or dispersions. Suitable aqueous and non-aqueous carriers, diluents, solvents or excipients include water, ethanol, polyols, and their suitable mixtures.

The dosage forms of the compounds of the present invention used for local administration include ointments, powders, patches, sprays, and inhalants. The active ingredients are mixed under sterile conditions with physiologically acceptable carriers and any preservatives, buffers, or propellants if necessary.

The compounds of the present invention can be administered alone or in combination with other pharmaceutically acceptable compounds (such as anti-tumor drugs).

The treatment method of the present invention can be administered alone or in combination with other treatment means or therapeutic drugs.

When the pharmaceutical composition is used, a safe and effective amount of the compound of the present invention is administered to a mammal (such as a human) in need of treatment, wherein the dosage at the time of administration is the pharmaceutically effective dosage, for people having a body weight of 60kg, the daily dose is usually 1∼2000mg, preferably 50∼1000mg. Of course, the specific dosage should also consider factors such as the route of administration and the patient's health status, which are within the skill range of a skilled physician.

Compared with the prior art, the present invention has the following main advantages:
(1) the compound possesses excellent YAP, TAZ, and/or TEAD inhibitory activity;
(2) the compound possesses excellent pharmacokinetic properties.

The present invention is further described hereafter in combination with specific embodiments. It should be understood that these examples are only used to illustrate the present invention and not to limit the scope of the invention. Experimental methods in which the specific conditions are not specified in the following examples are usually in accordance with conventional conditions such as the conditions described in Sambrook et al., Molecular Cloning: Laboratory Manual (New York: Cold Spring Harbor Laboratory Press, 1989), or in accordance with the conditions recommended by the manufacturer. Unless indicated otherwise, percentage and parts are calculated by weight.

Unless otherwise defined, all professional and scientific terminology used in the text have the same meanings as known to the skilled in the art. In addition, any methods and materials similar or equal to the recorded content can apply to the methods of the invention. The preferred embodiments and materials described herein are only for demonstration purposes.

### Example T-1

The compound synthesized by the invention:

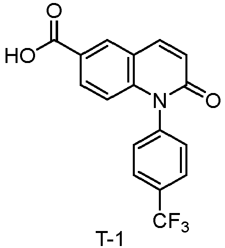

The synthetic route was as follows:

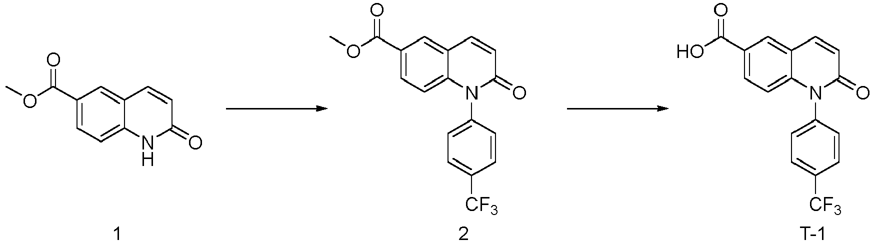

The experimental process was as follows:

### (1) Synthesis of Compound 2

50 mg (1.0eq) of Compound 1, 150mg (3.0eq) of 4-trifluoromethylphenylboronic acid, 60mg (2.4eq) of anhydrous copper acetate, 64mg (2.0eq) of DIPEA, 10ml of solvent 1,6-dioxane and 1g of 4A molecular sieve were mixed well. After replacement with nitrogen for three times, the mixture was reacted for 18 hours at room temperature under the protection of oxygen. TLC and LC-MS showed that the most are products, with a small amount of raw material remaining. The reaction solution was quenched with water and then extracted with EA. The organic phase was spin-dried and separated and purified on preparative plate to obtain 1.91mg of compound 2. HPLC purity: 98.01%.

### (2) Synthesis of Compound T-1

30mg (1.0eq) of compound 2 and 12mg (3.0eq) of anhydrous lithium hydroxide were added to the solvent (tetrahydrofuran/methanol/water = 6:3:1) (10ml) and mixed well. After replacement with nitrogen for three times, the mixture was reacted overnight at room temperature. TLC showed that the raw material disappeared; LC-MS detection showed there was mainly product. The reaction solution was quenched with 2N hydrochloric acid and the pH was adjusted to 4; then the reaction solution was extracted with EA. The organic phase was spin-dried and separated and purified on preparative plate to obtain 1.87 mg of compound T-1. HPLC purity: 97.20%.

### Example T-3

The compound synthesized by the invention:

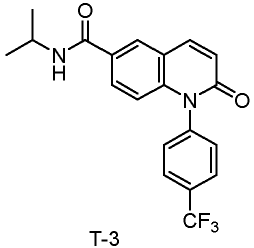

The synthetic route was as follows:

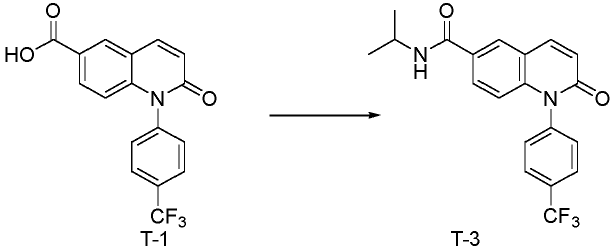

The experimental process was as follows:

### Synthesis of Compound T-3

T-1 100mg (1.0eq), isopropylamine 53mg (3.0eq), HATU 343mg (3.0eq), DIPEA 388mg (10.0eq), and the solvent DMF 5 ml were mixed well. After replacement with nitrogen for three times, the mixture was reacted for 18 hours at room temperature under the protection of nitrogen. The reaction solution was quenched by adding water and extracted with EA. The organic phases were combined, and then washed with saturated saline. Then the organic phase was spin-dried and separated and purified on preparative plate to obtain 15 mg of compound T-3. HPLC purity: 95.80%.

The following compounds were synthesized with reference to the synthesis methods of examples T-1 and T-3:

| Example | Compound structure | Compound characterization |
|---|---|---|
| Example T-21 | 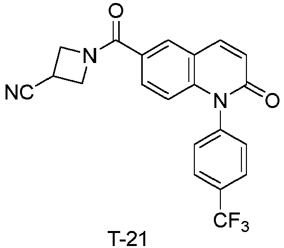 | LC-MS[M+1]: 391.0 |
| Example T-22 | 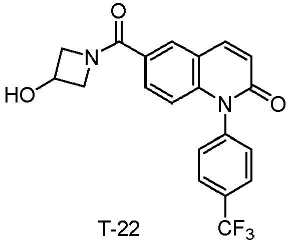 | LC-MS[M+1]: 409.0 |
| Example T-23 | 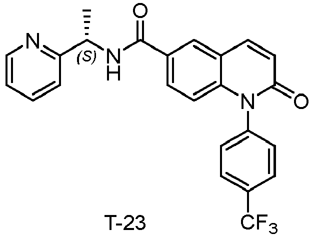 | LC-MS[M+1]: 392.0 |
| Example T-24 | 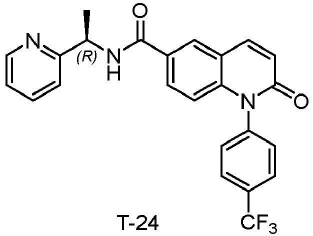 | LC-MS[M+1]: 433.1 |
| Example T-40 | 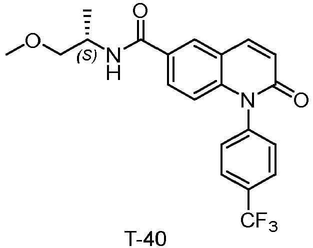 | LC-MS[M+1]: 432.1 |
| Example T-41 | 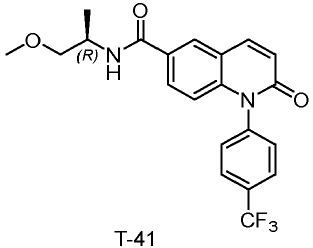 | LC-MS[M+1]: 432.1 |
| Example T-50 | 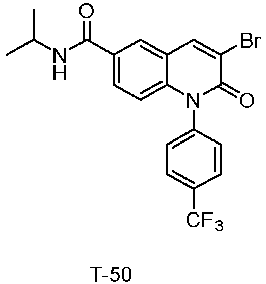 | LC-MS[M+1]: 449.0 |
| T-423 | 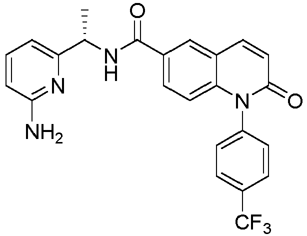 | LC-MS[M+1]: 453.1 |
| T-430 | 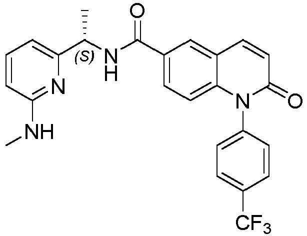 | LC-MS[M+1]: 467.1 |
| T-440 | 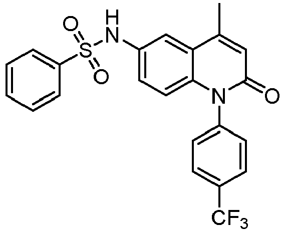 | 1H NMR (400 MHz, DMSO-d6) δ 10.39 (s, 1H), 7.95 (d, J = 8.2 Hz, 2H), 7.79 - 7.74 (m, 2H), 7.64 - 7.59 (m, 1H), 7.58 - 7.51 (m, 4H), 7.47 (d, J = 2.4 Hz, 1H), 7.15 (dd, J = 9.1, 2.5 Hz, 1H), 6.61 (d, J = 1.3 Hz, 1H), 6.44 (d, J = 9.0 Hz, 1H), 2.37 (d, J = 1.2 Hz, 3H) |
| T-441 | 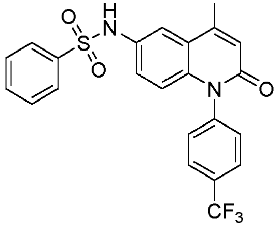 | 1H NMR (400 MHz, DMSO-d6) δ 9.78 (s, 1H), 7.99 (d, J = 8.2 Hz, 2H), 7.63 (d, J = 2.5 Hz, 1H), 7.57 (d, J = 8.1 Hz, 2H), 7.30 (dd, J = 9.0, 2.4 Hz, 1H), 6.65 (d, J = 1.4 Hz, 1H), 6.54 (d, J = 9.0 Hz, 1H), 2.97 (s, 3H), 2.49 - 2.46 (m, 3H). |
| T-442 | 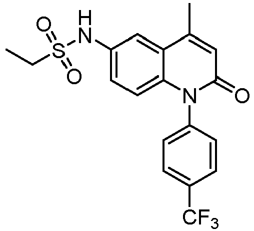 | 1H NMR (400 MHz, DMSO-d6) δ 9.85 (s, 1H), 7.99 (d, J = 8.1 Hz, 2H), 7.64 (d, J = 2.5 Hz, 1H), 7.58 (d, J = 8.1 Hz, 2H), 7.31 (dd, J = 9.0, 2.5 Hz, 1H), 6.65 (d, J = 1.4 Hz, 1H), 6.53 (d, J = 9.0 Hz, 1H), 3.07 (q, J = 7.3 Hz, 2H), 2.48 - 2.44 (m, 3H), 1.18 (t, J = 7.3 Hz, 3H). |
| T-443 | 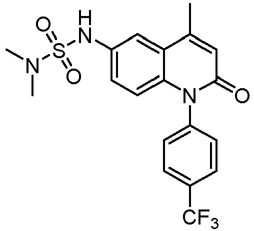 | 1H NMR (400 MHz, DMSO-d6) δ 9.98 (s, 1H), 7.98 (d, J = 8.2 Hz, 2H), 7.60 (d, J = 2.5 Hz, 1H), 7.57 (d, J = 8.1 Hz, 2H), 7.29 (dd, J = 9.0, 2.4 Hz, 1H), 6.64 (d, J = 1.3 Hz, 1H), 6.51 (d, J = 9.0 Hz, 1H), 2.69 (s, 6H), 2.46 (d, J = 1.2 Hz, 3H). |
| T-444 | 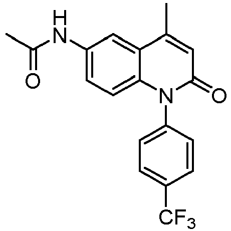 | ¹H NMR (400 MHz, DMSO-d6) δ 10.12 (s, 1H), 8.17 (t, J = 2.2 Hz, 1H), 7.99 (d, J = 8.2 Hz, 2H), 7.57 (d, J = 8.1 Hz, 2H), 7.53 (dd, J = 9.0, 2.3 Hz, 1H), 6.63 (d, J = 1.4 Hz, 1H), 6.50 (d, J = 9.1 Hz, 1H), 2.47 - 2.44 (m, 3H), 2.04 (s, 3H). |
| T-450 | 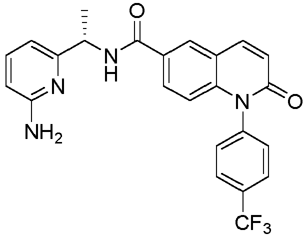 | LC-MS [M+1]:452.1460 |

### Example T-4

The compound synthesized by the invention:

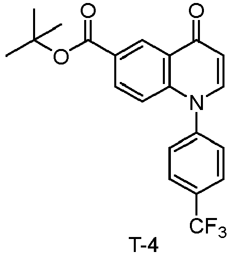

The synthetic route was as follows:

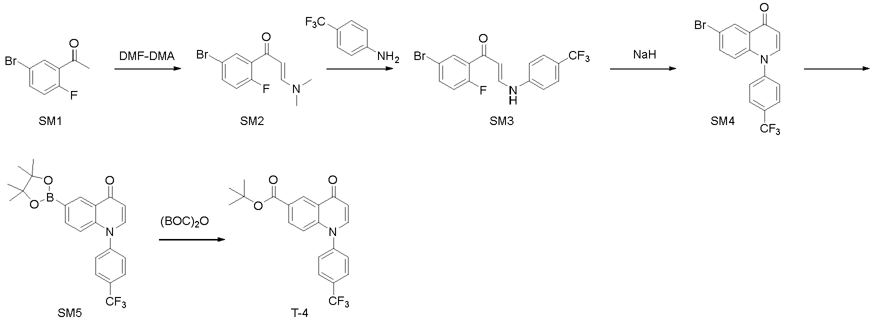

The experimental process was as follows:

### (1) Synthesis of Compound SM2

The raw material compound SM1 1.0g (1.0eq), DMF-DMA 5ml (8.2eq), p-toluenesulfonic acid monohydrate 0.13g (0.1eq), and the solvent toluene 5ml were mixed well. After replacement with nitrogen for three times, the mixture was warmed to reflux in an oil bath under the protection of nitrogen and reacted for 18 hours. TLC showed no raw material remained. The reaction solution was directly evaporated to dryness by rotary evaporation to obtain the crude compound SM2 1.3 g.

### (2) Synthesis of Compound SM3

SM2 1.2g (1.0eq), p-trifluoromethylaniline 0.85g (1.2eq), and the solvent toluene 12ml (10 times of volume) were mixed well. After replacement with nitrogen for three times, the mixture was warmed to reflux in an oil bath under the protection of nitrogen and reacted for 18 hours. TLC showed that the raw material was partially remained, and LCMS was correct. The reaction solution was directly mixed with silica gel and passed through the column to obtain SM3 70 mg.

### (3) Synthesis of Compound SM4

SM3 70mg (1.0eq), and the solvent DMF 2ml were mixed well. After replacement with nitrogen for three times, the mixture was cooled to 0 °C under ice bath under the protection of nitrogen and added in patches with NaH 10 mg. After addition, the mixture was warmed up naturally to room temperature and then stirred for 15 min. The mixture was warmed to 100 °C in an oil bath and reacted for 18 h. TLC showed no raw material remained, and LCMS was correct. The reaction solution was cooled, quenched with water and then extracted with EA. The EA phase was separated and purified on preparative plate to obtain 15mg of SM4.

### (4) Synthesis of Compound SM5

SM4 500mg (1.0eq), bis(pinacolato)diboron 518 mg (1.5eq), potassium acetate 400mg (3.0eq), Pd (dppf)Cl₂ 100mg (0.1eq), and the solvent dioxane 5ml (10 times of volume) were mixed well. After replacement with nitrogen for three times, the mixture was warmed to 90°C and reacted for 18 hours. TLC detection showed that the raw material was reacted completely. The mixture was purified by column chromatography with PE:EA=1:1 to yield 200 mg of SM5.

### (5) Synthesis of Compound T-4

200 mg of SM5 (1.0eq), 5 mg of palladium acetate (0.05eq), 19 mg of triphenylphosphine (0.15eq), di-tert-butyl dicarbonate 210 mg (2.0eq), and 1 ml of dioxane were added to the reaction system. After replacement with nitrogen for three times, the mixture was reacted at 100°C for 18 hours. TLC detection showed that the reaction was complete. The reaction solution was purified by preparative liquid chromatography to obtain 30 mg of T-4 with 99.9% purity.

¹H NMR (400 MHz, chloroformchloroform-d) δ 9.04 (d, J = 2.1 Hz, 1H), 7.92 (d, J = 8.3 Hz, 2H), 7.61 - 7.54 (m, 3H), 6.99 (d, J = 8.9 Hz, 1H), 6.43 (d, J = 7.9 Hz, 1H), 1.62 (s, 9H).

### Example T-5

The compound synthesized by the invention:

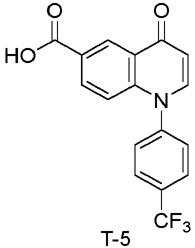

The synthetic route was as follows:

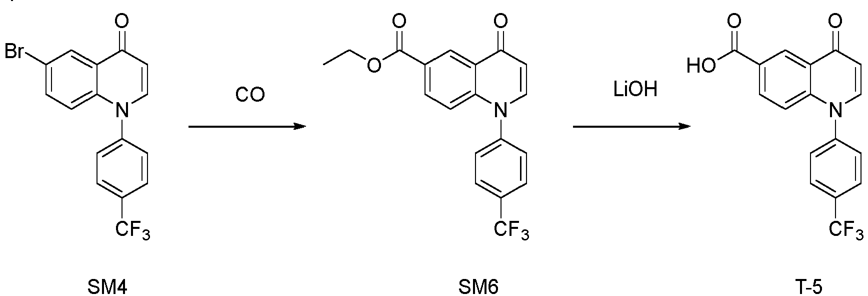

The experimental process was as follows:

### (1) Synthesis of Compound SM6

The raw material compound SM4 (5g, 1.0eq), palladium acetate (0.3g, 0.1eq), dppf (1.5g, 0.2eq), triethylamine (9ml, 5eq), the solvent ethanol (10ml), and DMF (15ml) were mixed well. After replacement with nitrogen for three times, the mixture was further replaced with CO for three times and reacted at 40°C for 18 hours under positive pressure protection with CO balloons. TLC showed no raw material remained. The reaction solution was concentrated directly and extracted with water and EA and partitioned; the EA phase was concentrated and purified by passing through the column to obtain 2.5 g of compound SM6. HPLC purity: 97.16%.

¹H NMR (400 MHz, chloroform-d) δ 9.12 (d, J = 2.1 Hz, 1H), 8.15 (dd, J = 8.9, 2.1 Hz, 1H), 7.93 (d, J= 8.2 Hz, 2H), 7.59 (dd, J = 8.1, 2.7 Hz, 3H), 7.01 (d, J = 9.0 Hz, 1H), 6.44 (d, J = 7.9 Hz, 1H), 4.42 (q, J= 7.1 Hz, 2H), 1.42 (t, J = 7.1 Hz, 3H).

### (2) Synthesis of Compound T-5

The raw material compound SM6 (2.4 g, 1.0 eq), lithium hydroxide (0.84 g, 3.0 eq), and solvents tetrahydrofuran (29 ml), methanol (14 ml) and water (5 ml) were mixed well. After replacement with nitrogen for three times, the mixture was reacted at room temperature for 18 hours. TLC showed no remaining raw material. The reaction solution was concentrated directly and added with 50 ml of water. A large amount of solids precipitated after about 10 ml of 2N hydrochloric acid being added dropwise. The solids were suction filtered. The filter cake was dissolved with ethyl acetate and then concentrated to obtain 2.3 g of compound T-5. HPLC purity: 98.10%.

¹H NMR (400 MHz, DMSO-d6) δ 8.80 (d, J = 2.1 Hz, 1H), 8.15 - 8.04 (m, 4H), 7.11 (d, J = 8.9 Hz,1H), 6.28 (d, J = 7.8 Hz, 1H)_{∘}

### Example T-6

The compound synthesized by the invention:

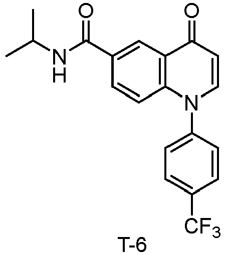

The synthetic route was as follows:

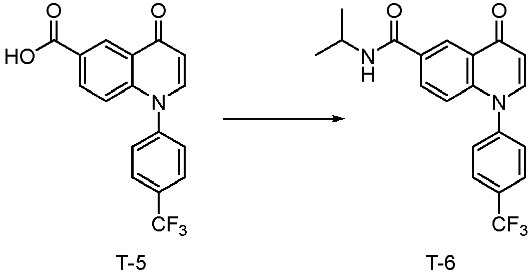

The experimental process was as follows:

### Synthesis of Compound T-6

T-5 (30mg, 1.0eq), isopropylamine (22 mg, 2.0 eq), HATU (52 mg, 1.5 eq), DIPEA (46.5 mg, 4.0 eq), and the solvent DMF (1ml) were mixed well. After replacement with nitrogen for three times, the mixture was reacted for 18 hours at room temperature under the protection of nitrogen. TLC showed no remaining raw material, and LCMS was correct. The reaction solution was quenched by adding water and extracted with EA. EA phase was separated and purified on preparative plate to obtain 12mg of Compound T-6. HPLC purity: 99.3%.

¹H NMR (400 MHz, chloroform-d) δ 8.67 (d, J = 2.2 Hz, 1H), 8.18 (dd, J = 8.9, 2.2 Hz, 1H), 7.92 (d, J = 8.2 Hz, 2H), 7.60 (dd, J = 13.7, 8.0 Hz, 3H), 7.07 (d, J = 8.9 Hz, 1H), 6.46 (d, J = 7.8 Hz, 1H), 6.35 (d, J = 7.9 Hz, 1H), 4.38 - 4.25 (m, 1H), 1.30 (d, J = 6.6 Hz, 7H).

The following compounds were synthesized with reference to the synthesis method in example T-6:

| Example | Compound structure | Compound characterization |
|---|---|---|
| Example T-17 | 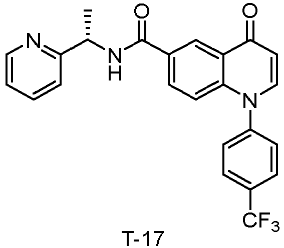 | LC-MS[M+1]: 438.1 ¹H NMR (400 MHz, chloroform-d) δ 8.87 (d, J = 2.1 Hz, 1H), 8.63 - 8.57 (m, 1H), 8.17 (dd, J = 8.9,2.2 Hz, 1H), 7.92 (d, J = 8.2 Hz, 3H), 7.69 (td, J = 7.7, 1.8 Hz, 1H), 7.59 (t, J = 7.9 Hz, 3H), 7.31 (dt, J =7.8, 1.2 Hz, 1H), 7.22 (ddd, J = 7.6, 4.9, 1.2 Hz, 2H), 7.06 (d, J = 8.9 Hz, 1H), 6.45 (d, J = 7.8 Hz, 1H),5.37 (p, J = 6.4, 6.0 Hz, 2H), 1.61 (s, 27H). |
| Example T-34 | 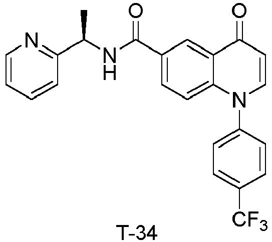 | LC-MS[M+1]: 438.1 ¹H NMR (400 MHz, chloroform-d) δ 8.87 (d, J = 2.1 Hz, 1H), 8.63 - 8.57 (m, 1H), 8.17 (dd, J = 8.9,2.2 Hz, 1H), 7.92 (d, J = 8.2 Hz, 3H), 7.69 (td, J = 7.7, 1.8 Hz, 1H), 7.59 (t, J = 7.9 Hz, 3H), 7.31 (dt, J =7.8, 1.2 Hz, 1H), 7.22 (ddd, J = 7.6, 4.9, 1.2 Hz, 2H), 7.06 (d, J = 8.9 Hz, 1H), 6.45 (d, J = 7.8 Hz, 1H),5.37 (p, J = 6.4, 6.0 Hz, 2H), 1.61 (s, 27H). |
| Example T-35 | 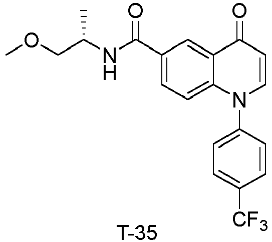 | LC-MS[M+1]: 405.1 ¹H NMR (400 MHz, chloroform-d) δ 8.65 (d, J = 2.2 Hz, 1H), 8.10 (dd, J = 8.9, 2.2 Hz, 1H), 7.85 (d, J= 8.3 Hz, 2H), 7.53 (dd, J = 11.7, 8.0 Hz, 3H), 6.99 (d, J = 8.9 Hz, 1H), 6.62 (d, J = 8.1 Hz, 1H), 6.38 (d,J = 7.9 Hz, 1H), 4.33 (dtt, J = 13.9, 6.8, 3.4 Hz, 1H), 3.41 (qd, J = 9.5, 4.3 Hz, 2H), 3.35 (s, 3H), 1.25 (d,J = 6.8 Hz, 4H). |
| Example T-36 | 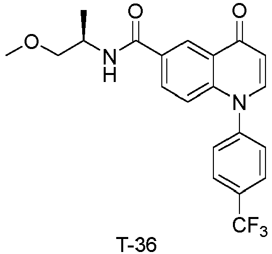 | LC-MS[M+1]: 405.1 ¹H NMR (400 MHz, DMSO-d6) δ 8.76 (d, J = 2.2 Hz, 1H), 8.60 (d, J = 8.1 Hz, 1H), 8.06 (ddd, J = 10.9,8.4, 2.3 Hz, 4H), 7.90 - 7.84 (m, 2H), 7.09 (d, J = 8.9 Hz, 1H), 6.27 (d, J = 7.7 Hz, 1H), 4.23 (dq, J =13.5, 6.7 Hz, 1H), 3.44 (dd, J = 9.5, 6.6 Hz, 1H), 3.34 - 3.29 (m, 2H), 3.27 (s, 3H), 1.15 (d, J = 6.8 Hz,3H). |
| Example T-37 | 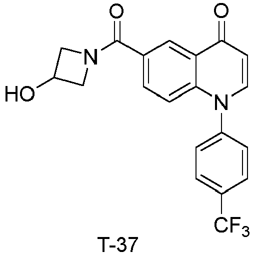 | LC-MS[M+1]: 389.1 ¹H NMR (400 MHz, ) δ 8.94 (d, J = 2.0 Hz, 1H), 8.32 - 8.22 (m, 3H), 7.37 (d, J = 8.9 Hz, 1H), 6.77 (dd,J = 7.8, 1.1 Hz, 1H), 5.04 (d, J = 39.6 Hz, 3H), 4.79 (d, J = 28.8 Hz, 3H), 4.45 (s, 2H), 3.74 (d, J = 6.4Hz, 1H). |
| Example T-38 | 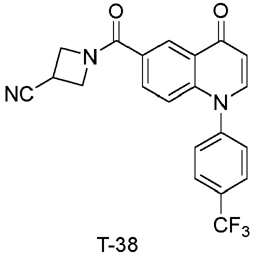 | LC-MS[M+1]: 398.1 ¹H NMR (400 MHz, DMSO-d6) δ 8.45 (d, J = 2.1 Hz, 1H), 8.08 (t, J = 8.0 Hz, 3H), 7.91 - 7.81 (m, 3H),7.07 (d, J = 8.9 Hz, 1H), 6.27 (d, J = 7.8 Hz, 1H), 4.55 (s, 2H), 4.29 (d, J = 58.3 Hz, 2H), 3.87 (p, J = 7.7Hz, 1H). |

### Example T-7 & T-15

The compound synthesized by the invention:

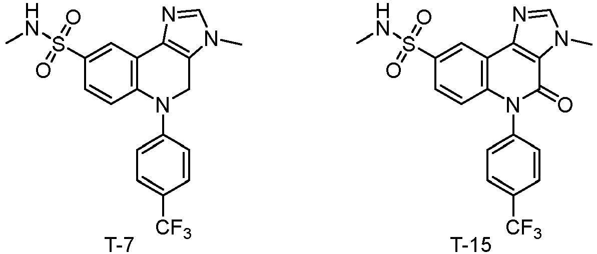

The synthetic route was as follows:

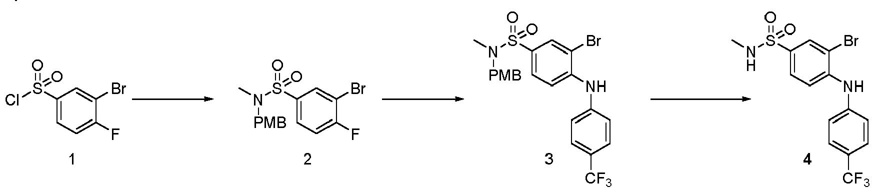

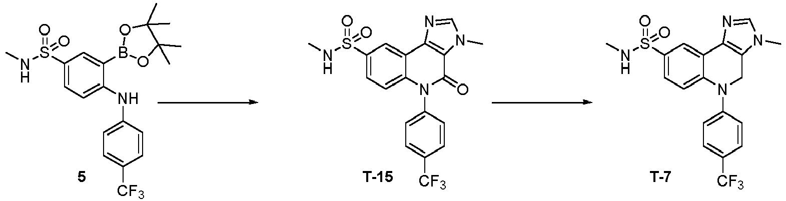

The experimental process was as follows:

### (1) Synthesis of Compound 2:

5.0 g (1.0eq) of Compound 1 was taken and added to a 250 mL flask, 60 mL of DCM and 2.85 g (1.03eq) of 4-methoxy-N-methylbenzylamine were added followed by the slow dropwise addition of 11.5 g (5eq) of DIPEA and the mixture was reacted for 2h at room temperature. After the reaction was completed, the reaction solution was added with 60mL water and extracted with DCM. The organic phases were combined and evaporated to dryness and then subjected to column chromatography to give 6.8 g of Compound 2. HPLC purity: 95.3%.

### (2) Synthesis of Compound 3:

3.33 g (1.0 eq) of p-trifluoromethylaniline was taken and added to a flask, 80mL of DMF was added and 2.48 g (3.0 eq) of NaH was added under ice bath conditions to react for 10min. The mixture was added with 8.0 g (1.0 eq) of Compound 2 subsequently, and warmed up to room temperature slowly and reacted. After the reaction was completed, the reaction was quenched by adding water and extracted with EA. After being evaporated to dryness, the residue was subjected to column chromatography to obtain 5.3g of Compound 2.

### (3) Synthesis of Compound 4:

1.0 g of Compound 3 was taken and added to a flask and 4.0 mL of TFA and 12 mL of DCM were added and the mixture was reacted at rt. After the reaction was completed, the reaction solution was added with water and extracted with DCM. After being evaporated to dryness, the residue was subjected to column chromatography to obtain 0.65g of Compound 2.

### (4) Synthesis of Compound 5:

430 mg (1.0eq) of Compound 4 was taken and added to a flask and 6 mL of dioxane, 402 mg (1.5eq) of bis(pinacolato)diboron, 206 mg (2.0eq) of KOAc, and 39 mg (0.05eq) of Pd(dppf)Cl₂ were added, and the mixture was reacted at 100 °C under the protection of N₂. After the reaction was completed, the reaction solution was added with water and extracted with EA. After being evaporated to dryness, the residue was subjected to column chromatography to obtain crude Compound 3 which was directly used in the next step.

### (5) Synthesis of Compound T-15:

200 mg (1.2eq) of Compound 5 was taken and added to a flask and 4 mL of dioxane, 0.4 mL of water, 85 mg (1.0eq) of 4-bromo-1-methyl-5-ethoxycarbonylimidazole, 21 mg (0.05eq) of Pd(PPh₃)₄, and 100 mg (2.0eq) of K₂CO₃ were added. The mixture was reacted at 100 °C under N₂ protection. After the reaction was completed, the reaction solution was added with water and extracted with EA. After being evaprated to dryness, the residue was subjected to column chromatography to obtain 120mg of Compound T-15. HPLC:97.7 %.

¹H NMR (400 MHz, DMSO-d6) δ 8.57 (d, J = 2.4 Hz, 1H), 8.43 (s, 1H), 8.06 (d, J = 8.4 Hz, 2H), 7.79 - 7.65 (m, 3H), 7.60 (m, 1H), 6.78 (d, J = 8.8 Hz, 1H), 4.06 (s, 3H), 2.41 (d, J = 5.2 Hz, 3H)_{∘}

### (6) Synthesis of compound T-7:

100 mg (1.0eq) of T-15 was taken and added to a flask, 3 mL of ultra-dry THF was added, and under an ice bath, 46 mg (5.0eq) of LiAlH₄ was added and the mixture slowly warmed up to room temperature and reacted. After the reaction was completed, the reaction solution was added with water and extracted with EA to obtain 36mg of Compound T-7. HPLC: 95.1%.

¹H NMR (400 MHz, DMSO-d6) δ 7.86 (d, J = 2.4 Hz, 1H), 7.82 (d, J = 8.4 Hz, 2H), 7.74 (s, 1H), 7.57 (d, J = 8.4 Hz, 2H), 7.29 (m, 1H), 6.82 (d, J = 8.8 Hz, 1H), 5.17 (s, 2H), 3.63 (s, 3H), 2.39 (s, 3H)_{∘}

The following compounds were synthesized with reference to the synthesis method in examples T-7 & T-15:

| Example | Compound structure | Compound characterization |
|---|---|---|
| Example T-16 | 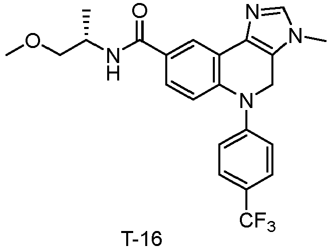 | LC-MS[M+1]: 445.2 |
| Example T-20 | 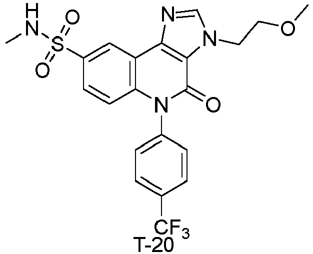 | ¹H NMR (400 MHz, DMSO-*d*₆) δ 8.59 (d, *J* = 2.4 Hz, 1H), 8.43 (s, 1H), 8.06 (d, *J* = 8.4Hz, 2H), 7.85 - 7.65 (m, 3H), 7.60 (s, 1H), 6.80 (d, *J* = 8.8 Hz, 1H), 4.62 (t, *J* = 5.2 Hz, 2H), 3.71 (t, *J* = 5.2 Hz, 2H), 3.25 (s, 3H), 2.41 (s, 3H). |
| Example T-33 | 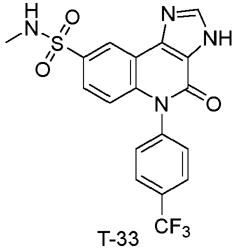 | ¹H NMR (400 MHz, DMSO-*d*₆) δ 14.02 (s, 1H), 8.60 (s, 1H), 8.46 (s, 1H), 8.06 (d, *J* = 8.0 Hz, 2H), 7.78 - 7.66 (m, 3H), 7.61 (d, *J* = 4.4 Hz, 1H), 6.79 (d, *J* = 8.8 Hz, 1H), 2.41 (t, *J* = 4.4 Hz, 3H). |
| Example T-39 | 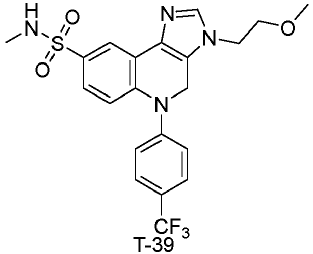 | ¹H NMR (400 MHz, DMSO-*d*₆) δ 7.87 (d, *J* = 2.4 Hz, 1H), 7.83 (s, 1H), 7.81 (s, 1H), 7.76 (s, 1H), 7.58 (s, 1H), 7.56 (s, 1H), 7.30 (m, 1H), 6.83 (d, *J* = 8.8 Hz, 1H), 5.15 (s, 2H), 4.15 (t, *J* = 5.2 Hz, 2H), 3.23 (s, 3H), 2.39 (s, 3H). |
| Example T-46 | 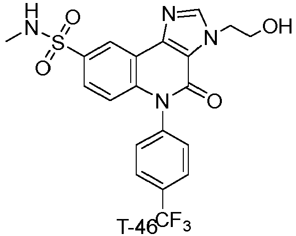 | ¹H NMR (400 MHz, DMSO-*d*₆) δ 8.59 (d, *J* = 2.4 Hz, 1H), 8.40 (s, 1H), 8.05 (d, *J* = 8.0 Hz, 2H), 7.72 (d, *J* = 8.0 Hz, 3H), 7.61 (m, 1H), 6.80 (d, *J* = 8.8 Hz, 1H), 5.00 (t, *J* = 5.2 Hz, 1H), 4.50 (t, *J* = 5.2 Hz, 2H), 3.75 (m, 2H), 2.41 (d, *J* = 4.8 Hz, 3H). |
| Example T-47 | 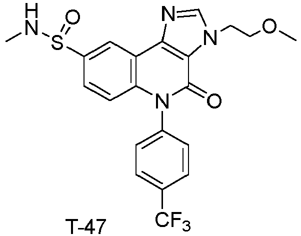 | ¹H NMR (400 MHz, DMSO-*d*₆) δ 8.15 (m, 2.3 Hz, 1H), 7.96 (s, 1H), 7.93 - 7.82 (m, 2H), 7.70 (t, *J* = 7.6 Hz, 2H), 7.43 (m, 1H), 6.87 (m, 1H), 6.74 (s, 1H), 4.37 - 4.07 (m, 2H), 3.63 (m, 2H), 3.23 (d, *J* = 1.2 Hz, 3H), 2.78 (s, 2H), 2.41 (d, *J* = 5.2 Hz, 3H). |
| Example T-48 | 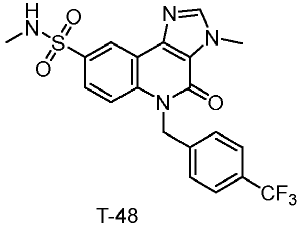 | ¹H NMR (400 MHz, DMSO-*d*₆) δ 8.55 (d, *J* = 2.4 Hz, 1H), 8.41 (s, 1H), 7.77 (m, 1H), 7.70 (s, 1H), 7.68 (s, 1H), 7.63 (d, *J* = 9.2 Hz, 1H), 7.56 (m, 1H), 7.45 (s, 1H), 7.43 (s, 1H), 5.74 (s, 2H), 4.12 (s, 3H), 2.41 (d, *J* = 5.2 Hz, 3H). |
| Example T-49 | 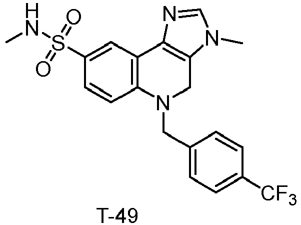 | LC-MS[M+1]: 437.0 |

### Example T-18 & T-31

The compound synthesized by the invention:

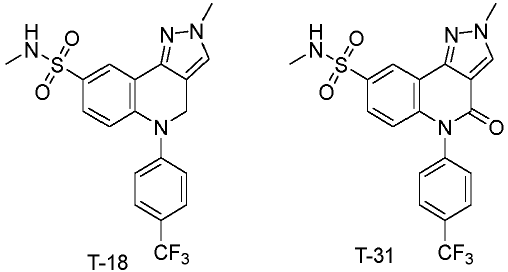

The synthetic route was as follows:

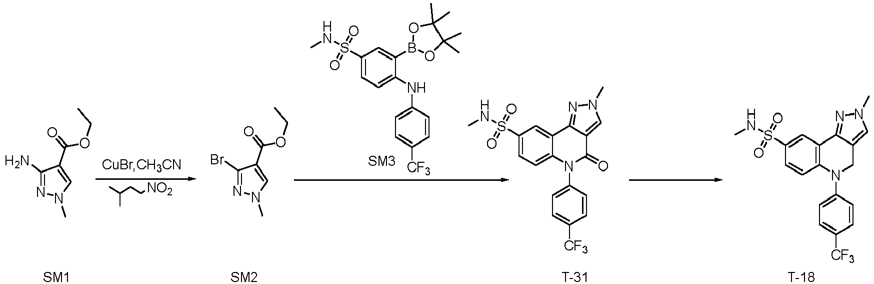

The experimental process was as follows:

### (1) Synthesis of Compound SM2:

SM1 (6.57 g, 39 mmol, 1.0 eq), CuBr (11.12 g, 78 mmol, 2.0 eq) were added to acetonitrile (245 mL) under the protection of nitrogen. Then the mixture was cooled to zero degree, and added with isopentyl nitrite (13.39 g, 105 mmol, 2.7 eq). The mixture was warmed up to 50 degree and reacted overnight. TLC (PE:EA = 5:1) showed that the raw material has completely reacted. The reaction solution was concentrated and then added with 300 mL of water and 300 mL of ethyl acetate. The mixture was filtered over diatomite, the organic layer was separated and the aqueous layer was extracted twice with 300 mL of ethyl acetate. The organic layers were combined, washed twice with 300 mL of water, washed twice with 300 mL of brine, dried over anhydrous sodium sulfate, filtered and concentrated to obtain the crude product and then subjected to silica gel column to obtain 5.5 g of product.

¹HNMR (400 MHz, MeOD) δ 8.13 (s, 1H), 4.27 (q,2H), 3.88 (s, 3H), 1.33 (t,3H)_{∘}

### (2) Synthesis of compound T-31:

SM2 (169 mg,0.73 mmol, 1.0 eq), SM3 (400 mg, 0.87 mmol, 1.2 eq), Pd(PPh₃)₄ (84 mg, 0.073 mol, 0.1 eq), and potassium carbonate (201.4 mg, 1.46 mmol, 2.0 eq) were added to 1,4-dioxane (5 mL) and water ( 0.5 mL) and the mixture was protected by nitrogen. The mixture was then reacted at 105 degree overnight and the LCMS showed the product peak. The reaction solution was cooled to room temperature and the solvent was removed under reduced pressure. The residue was added with 30 mL of water and 50 mL of ethyl acetate. The organic layer was separated, and the aqueous layer was extracted twice with 50 mL of ethyl acetate. The combined organic layer was washed with 20 mL of water for once and 20 mL of saturated saline for twice, and then dried over anhydrous sodium sulfate, concentrated and subjected to column chromatography to obtain 100 mg of product.

¹HNMR (400 MHz, CDCl3)δ 8.70 (d, 1H), 8.18 (s, 1H), 7.87 (m, 2H), 7.67 (dd, 1H), 7.42 - 7.36 (m, 2H), 6.63 (d, 1H), 4.43(m,1H), 4.13 (s, 3H), 2.63 (d, 3H).

### (3) Synthesis of compound T-18:

100 mg (1.0eq) of T-31 was taken and added to a flask, 5 mL of ultra-dry THF was added, and under an ice bath, 46 mg (5.0eq) of LiAlH₄ was added and the mixture was slowly brought to room temperature and then reacted at reflux. After the reaction was completed, the reaction solution was added with water and extracted with EA to obtain 16mg of Compound T-18. HPLC: 95.1%.

### Example T-19 & T-32

The compound synthesized by the invention:

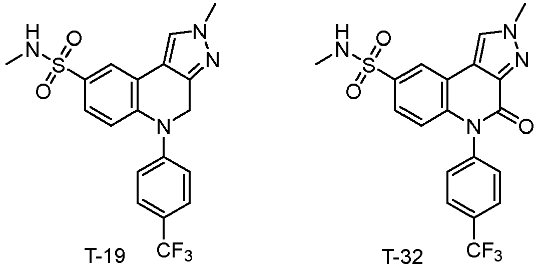

The synthetic route was as follows:

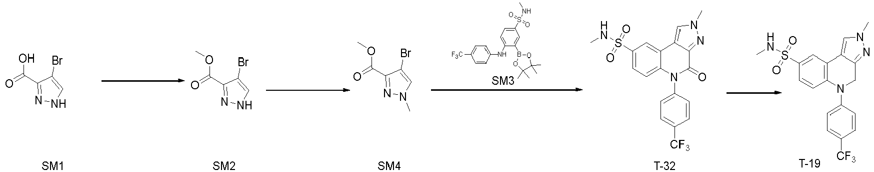

The experimental process was as follows:

### (1) Synthesis of Compound SM2:

SM1 (5.7 g, 29 mmol, 1.0 eq) was dissolved in methanol (100 mL). The mixture was cooled to 0 ° C, added with concentrated sulfuric acid (3.5 g, 1.9 mL, 1.2 eq) dropwise, and then warmed up to 70 ° C and refluxed overnight. LCMS showed that the raw material was fully reacted. The mixture was cooled to room temperature and concentrated to remove methanol. The residue was added with saturated sodium bicarbonate, then extracted with ethyl acetate, washed with saturated saline, dried over anhydrous sodium sulfate, filtrated and concentrated to give the product (5.6 g) as a white solid.

¹HNMR (400 MHz, MeOD) δ 7.79 (s, 1H), 3.91 (s, 3H)_{∘}

### (2) Synthesis of Compound SM4:

NaH (0.39 g, 9.8 mmol, 2.0 eq) was added to THF (20 mL), the mixture was cooled to zero degree, added with SM2 (1 g, 4.9 mmol, 1.0 eq) and stirred for 20 min. Then iodomethane (0.9 g, 0.4 ml, 6.4 mmol, 1.3 eq) was added dropwise. The mixture was slowly returned to room temperature and stirred overnight. The reaction solution was poured into ice water (100 ml), then extracted with 50 mL of ethyl acetate for three times and washed with saturated saline, then dried over anhydrous sodium sulfate, filtrated and concentrated to give the product (1.0 g, white solid).

¹HNMR (400 MHz, MeOD) δ 8.13 (s, 1H), 4.8 (s, 3H), 3.88 (s, 3H)_{∘}

### (3) Synthesis of compound T-32:

SM4 (169 mg,0.73 mmol, 1.0 eq), SM3 (400 mg, 0.87 mmol, 1.2 eq), Pd(PPh₃)₄ (84 mg, 0.073 mol, 0.1 eq), and potassium carbonate (201.4 mg, 1.46 mmol, 2.0 eq) were added to 1,4-dioxane (5 mL) and water ( 0.5 mL), and the mixture was protected by nitrogen. The mixture was then reacted at 105 degree overnight, and the LC-MS showed the product peak. The reaction solution was cooled to room temperature and the solvent was removed under reduced pressure. The residue was added with 30 mL of water and 50 mL of ethyl acetate. The organic layer was separated, and the aqueous layer was extracted twice with 50 mL of ethyl acetate. The combined organic layer was washed with 20 mL of water for once and 20 mL of saturated saline for twice. The combined organic layer was dried over anhydrous sodium sulfate, concentrated and subjected to column chromatography to obtain 80 mg of product.

¹HNMR (400 MHz, CDCl₃)δ 8.70 (d, 1H), 8.19 (s, 1H), 7.84 (d, 2H), 7.67 (d, 1H), 7.39 (d, 2H), 6.63 (d, 1H), 4.36 (q, 1H), 4.13 (s, 3H), 2.63 (d,3H)_{∘}

### (4) Synthesis of compound T-19:

50mg (1.0eq) of T-32 was taken and added to a flask, 3 mL of ultra-dry THF was added, and under an ice bath, 25mg (5.0eq) of LiAlH₄ was added and the mixture was slowly brought to room temperature and then reacted at reflux. After the reaction was completed, the reaction solution was added with water and extracted with EA and then subjected to preparative chromatography to obtain 12mg of Compound T-19. HPLC:97.2%.

The following compounds were synthesized with reference to the synthesis method in examples T-18, T-19, T-31 & T-32:

| Example | Compound structure | Compound characterization |
|---|---|---|
| Example T-45 | 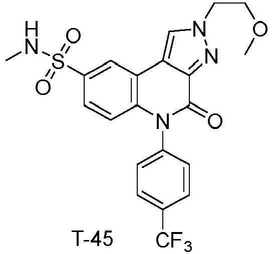 | ¹HNMR (400 MHz, CDCl₃) *δ* 8.38 (d, 1H), 8.25 (s, 1H), 7.87 (d, 2H), 7.62 (d, 1H), 7.41 (d, 2H), 6.69 (d, 1H), 4.90 (t, 2H), 4.34 (q, 1H), 3.82 (t, *J* = 5.4 Hz, 2H), 3.27 (s, 3H), 2.63 (d, *J* = 5.4 Hz, 3H) |
| Example T-51 | 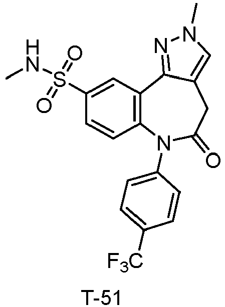 | ¹H NMR (400 MHz, chloroform-*d*) δ 7.97 (d, *J* = 2.3 Hz, 1H), 7.80 (s, 1H), 7.62 (d, *J* = 8.2 Hz, 2H), 7.57 (dd, *J* = 8.7, 2.3 Hz, 1H), 7.22 (d, *J* = 8.1 Hz, 2H), 7.03 (d, *J* = 8.6 Hz, 1H), 4.38 (d, *J* = 5.7 Hz, 1H), 4.01 (s, 3H), 3.76 (s, 2H), 2.72 (d, *J* = 5.4 Hz, 3H). |
| Example T-52 | 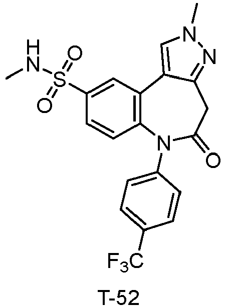 | LC-MS[M+1]: 451.1 |
| Example T-54 | 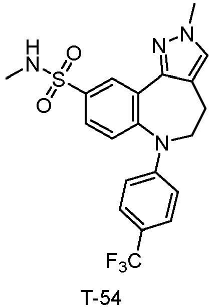 | LC-MS[M+1]: 437.0 |
| Example T-55 | 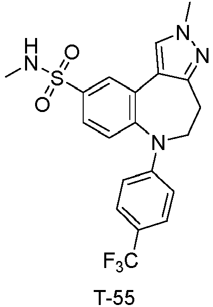 | LC-MS[M+1]: 437.0 |
| Example T-57 | 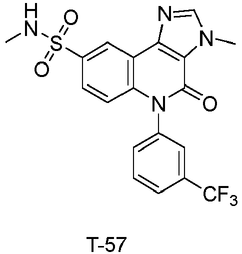 | LC-MS[M+1]: 437.0 |
| Example T-60 | 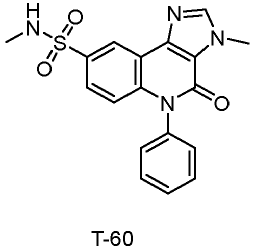 | LC-MS[M+1]: 369.1 |
| Example T-66 | 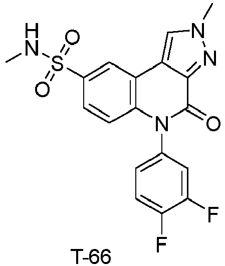 | LC-MS[M+1]: 405.0 |
| T-195 | 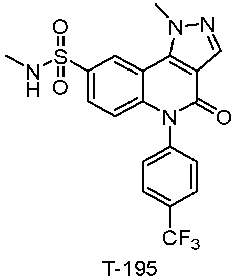 | ¹H NMR (400 MHz, chloroform-d) δ 8.66 (d, J = 2.0 Hz, 1H), 8.26 (s, 1H), 7.91 (d, J = 8.2 Hz, 2H), 7.77 (dd, J = 9.0, 2.1 Hz, 1H), 7.43 (d, J = 8.2 Hz, 2H), 6.83 (d, J = 9.0 Hz, 1H), 4.50 (s, 3H), 2.71 (d, J = 5.4 Hz, 3H) |
| T-199 | 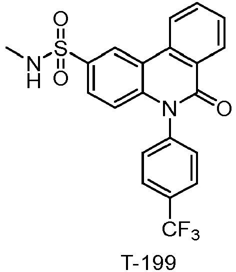 | ¹H NMR (400 MHz, DMSO-*d*₆) δ 8.84 (d, *J* = 2.1 Hz, 1H), 8.62 (d, *J* = 8.2 Hz, 1H), 8.36 (dd, *J* = 8.0, 1.4 Hz, 1H), 8.06 (d, *J* = 8.2 Hz, 2H), 8.01 (ddd, *J* = 8.4, 7.2, 1.5 Hz, 1H), 7.82 - 7.72 (m, 4H), 7.57 (q, *J* = 5.0 Hz, 1H), 6.75 (d, *J* = 8.8 Hz, 1H), 2.44 (d, *J* = 5.0 Hz, 3H). |
| T-265 | 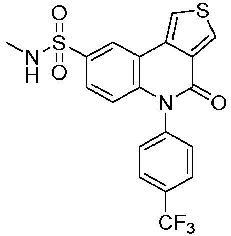 | ¹H NMR (400 MHz, chloroform-*d*) δ 8.55 - 8.52 (m, 1H), 8.50 (d, *J* = 2.0 Hz, 1H), 8.10 - 8.04 (m, 1H), 7.91 (d, *J* = 8.0 Hz, 2H), 7.66 (dt, *J* = 8.8, 2.0 Hz, 1H), 7.46 (d, *J* = 8.1 Hz, 2H), 6.65 (dd, *J* = 8.9, 1.7 Hz, 1H), 4.42 (d, *J* = 5.6 Hz, 1H), 2.70 (dd, *J* = 5.4, 1.6 Hz, 3H). |
| T-427 | 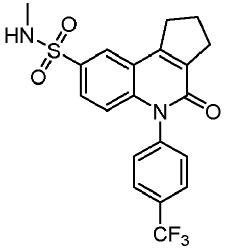 | LC-MS[M+1]: 423.0 |
| T-451 | 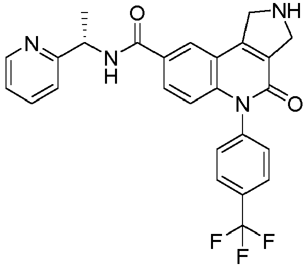 | LC-MS[M+1]: 478.2 |
| T-452 | 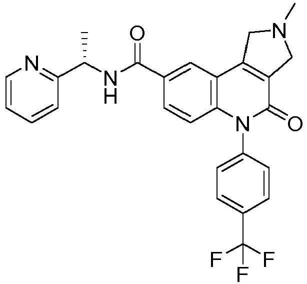 | LC-MS[M+1]: 492.2 |
| T-453 | 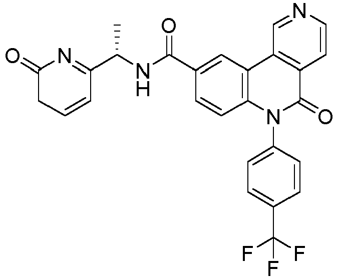 | LC-MS[M+1]: 504.1 |

### Example T-61

The compound synthesized by the invention:

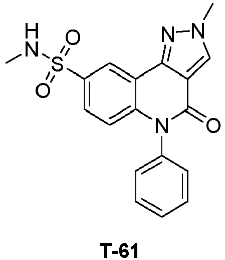

The synthetic route was as follows:

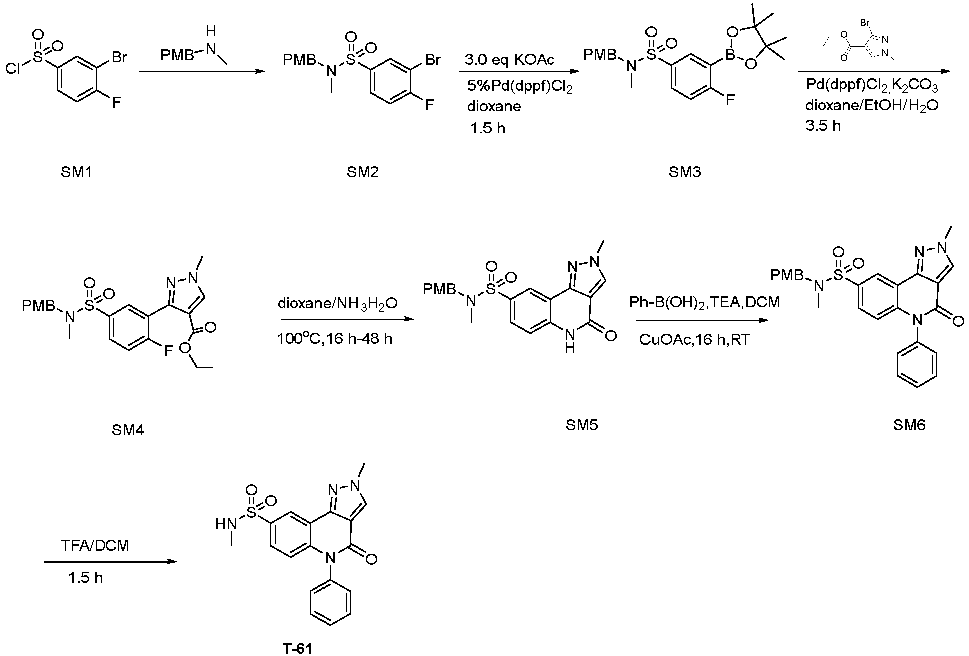

The experimental process was as follows:

### (1) Synthesis of SM2:

To a solution of SM1 (50 g, 183 mmol, 1.0 eq), and N-(4-methoxybenzyl)-N-methylamine (28 g, 185 mmol, 1.01 eq) in DCM (500 ml) was added DIEA (28.33 g, 219.6 mmol, 1.2 eq) dropwise. The mixture was protected with nitrogen, and then the temperature was maintained at 30 °C. LC (PE:EA = 2:1) showed that the raw material has completely reacted. The reaction solution was washed with NaCl (300 ml), mixed with 70 g silica gel and passed through the column chromatography to give 74.2 g of product.

### (2) Synthesis of SM3:

SM2 (5 g, 122 mmol, 1.0 eq), B₂Pin₂ (34.1 g, 134.2 mmol, 1.1 eq), Pd(dppf)Cl₂ (4.46 g, 6.1 mmol, 0.05 eq), and KOAc (35.9 g, 366 mmol, 3 eq) were added to 1,4-dioxane (764 ml). The mixture was protected by nitrogen, and then heated up to 105 °C and reacted for 1.5 h. LCMS showed that the product was obtained and was pure. The reaction solution was diluted by adding 1 L of EA, filtered through 100 g diatomite, and spin-dried. The residue was added with 250 ml of toluene, 400 ml of ethanol, and spin-dried. The residue was added with 250 ml of toluene, 400 ml of ethanol, and spin-dried. The residue was washed by adding PE to give 48 g of SM3.

### (3) Synthesis of SM4:

SM3 (4.93 g, 11.33 mmol,1.2 eq), ethyl 3-bromo-1-methyl-1H-pyrazole-4-carboxylate (2.2 g, 9.44 mmol, 1.0 eq), K₂CO₃ (2.61 g, 18.88 mmol, 2.0 eq), Pd(dppf)Cl₂ (0.345 g, 0.47 mmol, 0.05 eq) and a mixed solvent (ethanol, 1,4-dioxane, water at a ratio of 5:2:1 with a total of 35.2 mL) were added to a reaction flask, and the flask was vacuumed and exchanged with nitrogen for 3 times. The mixture was then warmed up to 95 degree and reacted for 3 hours. TLC (PE:EA = 2:1) showed that the raw material has completely reacted. The reaction was cooled to room temperature, added with 200 mL of EtOAc and 30 mL of water, and then added with diatomite for filtration. The EA layer was separated and then washed with saturated saline (30 mL*1), dried over anhydrous sodium sulfate, and concentrated to obtain the crude product, and then the crude was subjected to column chromatography to obtain 2 g product as oil.

### (4) Synthesis of SM5:

SM4 (2 g), dioxane (20 mL), and ammonia (15 mL) were added to an autoclave. The autoclave was vacuumed and exchanged with nitrogen for three times. Then the mixture was warmed up to 100 degree, and reacted for 24 h. TLC showed that the raw material has completely reacted. The reaction was cooled to room temperature, and filtrated. The filter cake was washed with water, methanol, and petroleum ether and dried in vacuum to obtain 0.8 g of product.

### (5) Synthesis of SM6:

SM5 (100 mg, 0.2424 mmol, 1 eq), phenylboronic acid (59.1 mg, 0.4848 mmol, 2 eq), Cu(OAc)₂ (4.4 mg, 0.02424 mmol, 0.1 eq), and TEA (49 mg, 0.4848 mmol, 2 eq) were added to DCM (3 ml) followed by the addition of 3 g of molecular sieves. The mixture was protected with oxygen, and reacted overnight. TLC (PE:EA = 1:2) showed that the raw material has substantially reacted. The reaction solution was diluted by adding DCM and water, filtered over diatomite, extracted with DCM, washed with saturated NaCl, dried over anhydrous sodium sulfate, mixed with silica gel, and passed through column chromatography to obtain 25 mg of SM6.

### (6) Synthesis of T-61:

SM6 (25 mg, 0.051 mmol, 1 eq), 0.83 ml TFA were added to 4.2 ml DCM, the mixture was protected by nitrogen and reacted overnight. TLC (PE:EA = 1:3) showed that the raw material has substantially reacted. The reaction solution was added with saturated NaHCO₃ solution until the solution turned alkaline, extracted with DCM, washed with saturated NaCl, dried over anhydrous sodium sulfate, mixed with silica gel, and passed through column chromatography to obtain 20 mg of T-61.

¹H NMR (400 MHz, DMSO-*d*₆) δ 8.82 - 8.77 (m, 1H), 8.56 (d, *J* = 2.2 Hz, 1H), 7.76 (m, *J* = 8.9, 2.3 Hz, 1H), 7.74 - 7.66 (m, 2H), 7.70 - 7.56 (m, 2H), 7.48 - 7.40 (m, 2H), 6.73 (d, *J* = 8.9 Hz, 1H), 4.21 (s, 3H), 2.47 (d, *J* = 5.0 Hz, 3H).

The following compounds were synthesized with reference to the synthesis method in example T-61:

| Example | Compound structure | Compound characterization |
|---|---|---|
| T-58 | 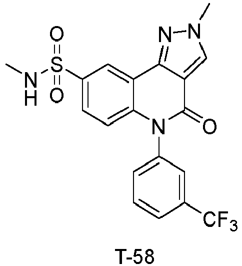 | LC-MS[M+1]: 437 |
| T-65 | 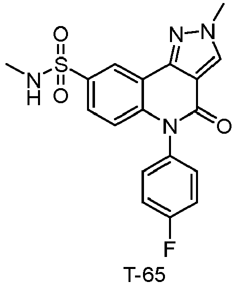 | ¹H NMR (400 MHz, chloroform-*d*) δ 8.68 (d, *J* = 2.2 Hz, 1H), 8.17 (s, 1H), 7.66 (m, *J* = 8.9, 2.2 Hz, 1H), 7.29 - 7.19 (m, 3H), 6.68 (d, *J* = 8.9 Hz, 1H), 4.34 (q, *J* = 5.4 Hz, 1H), 4.13 (s, 3H), 2.62 (d, *J* = 5.3 Hz, 3H). |
| T-68 | 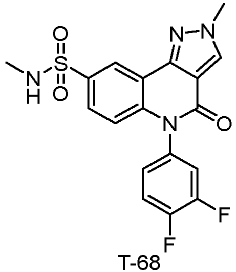 | ¹H NMR (400 MHz, DMSO-*d*₆) δ 8.76 (s, 1H), 8.50 (d, *J* = 2.2 Hz, 1H), 7.78 - 7.69 (m, 2H), 7.69 (m, *J* = 4.6, 2.2 Hz, 1H), 7.58 (q, *J* = 5.0 Hz, 1H), 7.33 m, *J* = 7.2, 3.7 Hz, 1H), 6.80 (d, *J* = 8.9 Hz, 1H), 4.15 (s, 3H), 2.41 (d, *J* = 5.0 Hz, 3H). |
| T-72 | 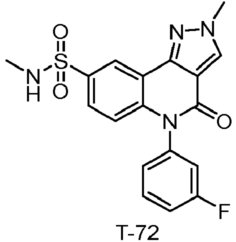 | ¹H NMR (400 MHz, DMSO-*d*₆) δ 8.76 (s, 1H), 8.50 (d, *J* = 2.3 Hz, 1H), 7.76 - 7.64 (m, 2H), 7.57 (q, *J* = 5.0 Hz, 1H), 7.50 - 7.38 (m, 2H), 7.31 - 7.24 (m, 1H), 6.73 (d, *J* = 8.9 Hz, 1H), 4.16 (s, 3H), 2.41 (d, *J* = 5.0 Hz, 3H). |
| T-75-1 | 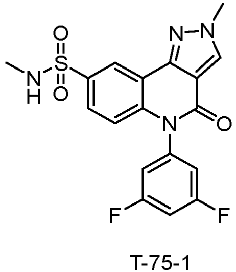 | ¹H NMR (400 MHz, DMSO-*d*₆) δ 8.77 (s, 1H), 8.50 (d, *J* = 2.2 Hz, 1H), 7.73 (m, *J* = 8.9, 2.3 Hz, 1H), 7.63 - 7.48 (m, 2H), 7.35 (m, *J* = 7.5, 2.3 Hz, 2H), 6.83 (d, *J* = 8.9 Hz, 1H), 4.15 (s, 3H), 2.41 (d, *J* = 4.9 Hz, 3H). |
| T-79 | 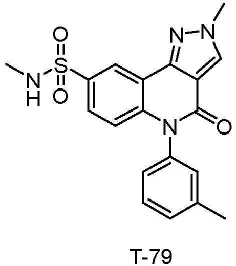 | ¹H NMR (400 MHz, DMSO-*d*₆) δ 8.81 (s, 1H), 8.57 (d, *J* = 2.3 Hz, 1H), 7.78 (m, *J* = 8.9, 2.2 Hz, 1H), 7.62 (q, *J* = 5.0 Hz, 1H), 7.51 (d, *J* = 8.0 Hz, 2H), 7.36 - 7.29 (m, 2H), 6.78 (d, *J* = 8.9 Hz, 1H), 4.23 (s, 3H), 2.54 - 2.46 (m, 6H) |
| T-82 | 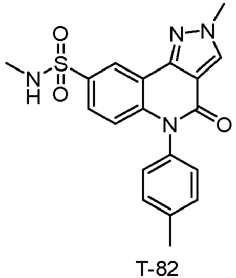 | ¹H NMR (400 MHz, DMSO-*d*₆) δ 8.73 (s, 1H), 8.49 (d, *J* = 2.2 Hz, 1H), 7.70 (m, *J* = 8.9, 2.3 Hz, 1H), 7.54 (q, *J* = 4.9 Hz, 1H), 7.44 (d, *J* = 8.0 Hz, 2H), 7.28 - 7.21 (m, 2H), 6.70 (d, *J* = 8.9 Hz, 1H), 4.15 (s, 3H), 2.46 - 2.38 (m, 6H). |
| T-154 | 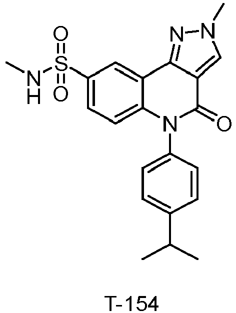 | ¹H NMR (400 MHz, DMSO-*d*₆) δ 8.73 (s, 1H), 8.50 (d, *J* = 2.2 Hz, 1H), 7.71 (m, *J* = 8.9, 2.3 Hz, 1H), 7.54 (q, *J* = 5.0 Hz, 1H), 7.54 - 7.46 (m, 2H), 7.32 - 7.24 (m, 2H), 6.68 (d, *J* = 8.9 Hz, 1H), 4.15 (s, 3H), 3.04 (m, *J* = 7.0 Hz, 1H), 2.41 (d, *J* = 5.0 Hz, 3H), 1.30 (d, *J* = 6.9 Hz, 6H). |
| T-155 | 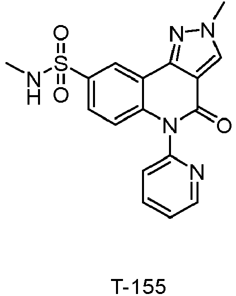 | ¹H NMR (400 MHz, DMSO-*d*₆) δ 8.80 - 8.72 (m, 2H), 8.52 (d, *J* = 2.2 Hz, 1H), 8.15 (td, *J* = 7.7, 1.9 Hz, 1H), 7.74 - 7.52 (m, 4H), 6.56 (d, *J* = 8.9 Hz, 1H), 4.16 (s, 3H), 2.41 (d, *J* = 5.0 Hz, 3H). |
| T-156 | 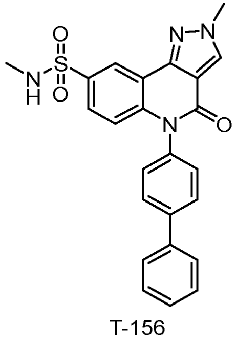 | ¹H NMR (400 MHz, DMSO-*d*₆) δ 8.77 (s, 1H), 8.52 (d, *J* = 2.2 Hz, 1H), 7.97 - 7.89 (m, 2H), 7.84 - 7.77 (m, 2H), 7.74 (m, *J* = 8.9, 2.3 Hz, 1H), 7.60 - 7.48 (m, 3H), 7.52 - 7.44 (m, 2H), 7.44 (t, *J* = 7.4 Hz, 1H), 6.80 (d, *J* = 8.9 Hz, 1H), 4.16 (s, 3H), 2.42 (d, *J* = 5.0 Hz, 3H). |
| T-157 | 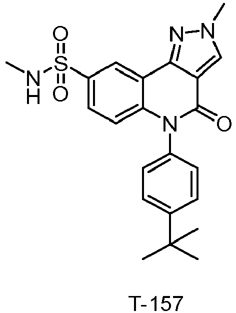 | ¹H NMR (400 MHz, DMSO-*d*₆) δ 8.75 (d, *J* = 0.6 Hz, 1H), 8.50 (d, *J* = 2.2 Hz, 1H), 7.72 (m, *J* = 8.9, 2.3 Hz, 1H), 7.69 - 7.61 (m, 2H), 7.54 (q, *J* = 5.1 Hz, 1H), 7.33 - 7.23 (m, 2H), 6.68 (d, *J* = 8.9 Hz, 1H), 4.15 (s, 3H), 2.41 (d, *J* = 5.0 Hz, 3H), 1.39 (s, 9H). |
| T-158 | 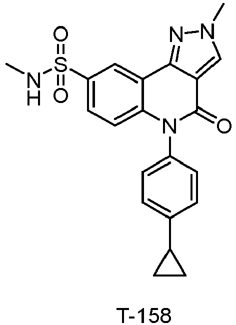 | ¹H NMR (400 MHz, DMSO-*d*₆) δ 8.73 (s, 1H), 8.49 (d, *J* = 2.3 Hz, 1H), 7.70 (m, *J* = 8.9, 2.2 Hz, 1H), 7.54 (q, *J* = 5.0 Hz, 1H), 7.35 - 7.28 (m, 2H), 7.27 - 7.19 (m, 2H), 6.70 (d, *J* = 8.9 Hz, 1H), 4.15 (s, 3H), 2.41 (d, *J* = 5.0 Hz, 3H), 1.32 - 1.13 (m, 1H), 1.10 - 1.01 (m, 2H), 0.88 - 0.75 (m, 2H). |
| T-159 | 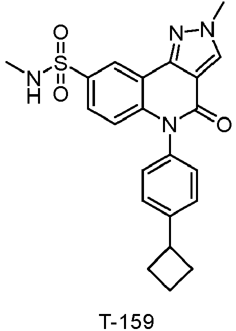 | ¹H NMR (400 MHz, DMSO-*d*₆) δ 8.74 (s, 1H), 8.49 (d, *J* = 2.2 Hz, 1H), 7.71 (m, *J* = 8.9, 2.2 Hz, 1H), 7.54 (q, *J* = 5.0 Hz, 1H), 7.51 - 7.45 (m, 2H), 7.33 - 7.25 (m, 2H), 6.70 (d, *J* = 8.9 Hz, 1H), 4.15 (s, 3H), 3.65 (p, *J* = 8.8 Hz, 1H), 2.44 - 2.35 (m, 3H), 2.35 (m, *J* = 8.2, 2.4 Hz, 2H), 2.20 (m, *J* = 9.0, 8.6, 2.5 Hz, 2H), 2.11 - 1.97 (m, 2H). |
| T-160 | 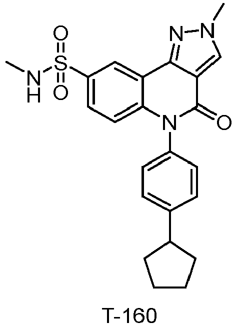 | ¹H NMR (400 MHz, DMSO-*d*₆) δ 8.74 (s, 1H), 8.49 (d, *J* = 2.2 Hz, 1H), 7.72 (dd, *J* = 8.9, 2.3 Hz, 1H), 7.58 - 7.47 (m, 3H), 7.30 - 7.23 (m, 2H), 6.70 (d, *J* = 8.8 Hz, 1H), 4.15 (s, 3H), 3.11 (t, *J* = 8.6 Hz, 1H), 2.41 (d, *J* = 5.0 Hz, 3H), 2.11 (d, *J* = 11.3 Hz, 2H), 1.82 (m, *J* = 6.4, 3.0 Hz, 2H), 1.77 - 1.58 (m, 4H). |
| T-161 | 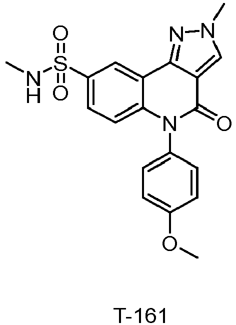 | ¹H NMR (400 MHz, DMSO-*d*₆) δ 8.74 (s, 1H), 8.49 (d, *J* = 2.3 Hz, 1H), 7.71 (dd, *J* = 8.9, 2.2 Hz, 1H), 7.55 (q, *J* = 4.9 Hz, 1H), 7.33 - 7.25 (m, 2H), 7.21 - 7.12 (m, 2H), 6.74 (d, *J* = 8.9 Hz, 1H), 4.15 (s, 3H), 3.86 (s, 3H), 2.41 (d, *J* = 5.0 Hz, 3H). |
| T-162 | 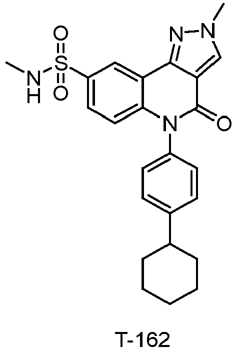 | LC-MS[M+1]: 451 |
| T-163 | 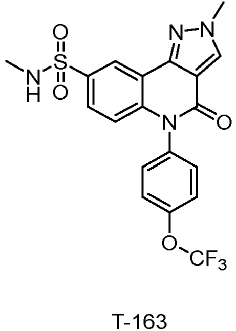 | ¹H NMR (400 MHz, DMSO-*d*₆) δ 8.77 (s, 1H), 8.51 (d, *J* = 2.2 Hz, 1H), 7.73 (m, *J* = 8.9, 2.3 Hz, 1H), 7.65 (d, *J* = 8.4 Hz, 2H), 7.62 - 7.53 (m, 3H), 6.71 (d, *J* = 8.9 Hz, 1H), 4.16 (s, 3H), 2.41 (d, *J* = 4.9 Hz, 3H). |
| T-164 | 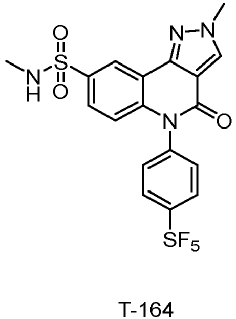 | LC-MS[M+1]: 495 |
| T-165 | 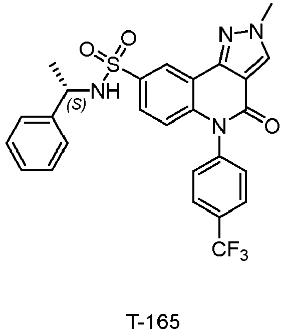 | LC-MS[M+1]: 537 |
| T-166 | 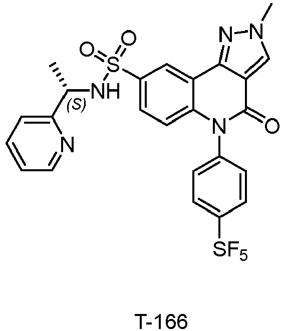 | LC-MS[M+1]: 586 |
| T-167 | 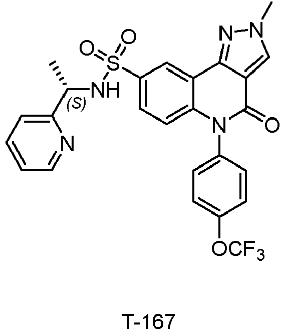 | LC-MS[M+1]: 544 |

### Examples T-86, T-100 & T-110

The compound synthesized by the invention:

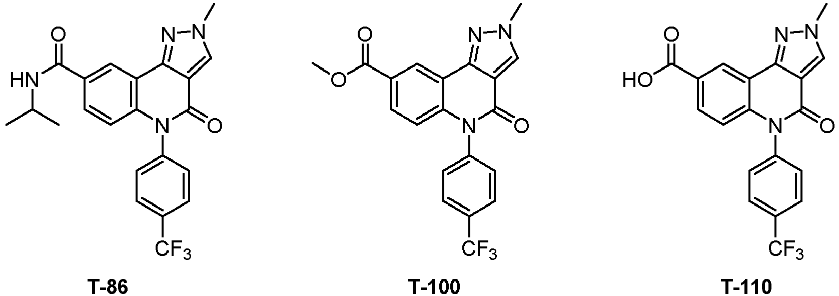

The synthetic route was as follows:

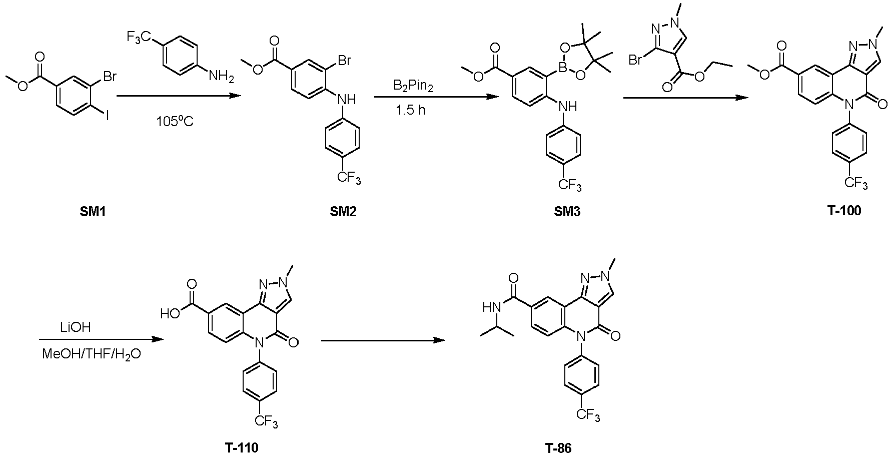

The experimental processes were as follows:

### (1) Synthesis of SM2

SM1 50g (1.0eq), cesium carbonate 72g (1.5eq), Xantphos 22g (0.25eq), palladium acetate 1.64g (0.05eq) and 1L of 1,4-dioxane were mixed well, and 4-(trifluoromethyl) aniline 31g was added slowly. The mixture was vacuumed and exchanged with nitrogen and refluxed at 105 degree, and reacted for 16 hours. TLC showed that the raw material has completely reacted. The reaction was diluted with 1L EA, then leached over diatomite; the filterate was mixed with silica gel, then subjected to column chromatography to obtain 38 g SM2.

¹H NMR (400 MHz, chloroform-*d*) δ 8.25 (d, *J* = 1.9 Hz, 1H), 7.87 (dd, *J* = 8.6, 1.9 Hz, 1H), 7.64 - 7.58 (m, 2H), 7.29 (dd, *J* = 8.5, 5.7 Hz, 4H), 6.58 (s, 1H), 3.90 (s, 3H).

### (2) Synthesis of SM3

SM2 24 g (1.0eq), B₂Pin₂ 21.5 g (1.3eq), KOAc 12.6 g (2.0eq), Pd(dppf)d₂ 2.4 g (0.05eq) and 1,4-dioxane 300 ml were mixed well. The mixture was protected with nitrogen, reacted under reflux at 105 °C for 1.5 h. The reaction was cooled to room temperature, added with 500 mL EA, and then filtered through diatomite. Then the residue was mixed evenly under ultrasound using toluene: anhydrous ethanol = 5:8, and stirred for 5 minutes, and then evaporated by rotary evaporation. This process was repeated until the rotary evaporation state turned solid, and then the solid was mixed with an appropriate amount of PE until there were solid precipitates. The mixture was leached, and the solid was collected and dried to obtain 20 g of SM3.

¹H NMR (400 MHz, DMSO-*d*₆) δ 8.47 (s, 1H), 8.22 (d, *J* = 2.2 Hz, 1H), 7.94 (dd, *J* = 8.7, 2.3 Hz, 1H), 7.65 (d, *J* = 8.4 Hz, 2H), 7.35 (t, *J* = 8.5 Hz, 2H), 3.85 - 3.71 (m, 3H), 3.36 (s, 12H).

### (3) Synthesis of T-100

SM3 18 g (1.2eq), ethyl 3-bromo-1-methyl-1H-pyrazole-4-carboxylate 21.5 g (1.3eq), K₂CO₃ 15.0 g (2.0eq), Pd(dppf)d₂ 1.3 g (0.05eq) and 250 ml of 1,4-dioxane/water/anhydrous methanol=5:2:1 were mixed well. The mixture was protected with nitrogen, reacted under reflux at 105 °C for 1.5 h. Then TLC showed the formation of the product. The reaction was cooled to room temperature, then filtered over diatomite, dried by rotary dryer, quenched with water, and extracted with EA, and subjected to column chromatography to obtain 9.5 g of T-100.

¹H NMR (400 MHz, DMSO-*d*₆) δ 8.73 (s, 1H), 8.68 (d, *J* = 2.1 Hz, 1H), 8.03 (d, *J* = 8.3 Hz, 2H), 7.89 (dd, *J* = 8.9, 2.1 Hz, 1H), 7.71 - 7.64 (m, 2H), 6.61 (d, *J* = 8.9 Hz, 1H), 4.14 (s, 3H), 3.87 (s, 3H).

### (4) Synthesis of T-110

T-100 4.5 g (1.0eq), LiOH monohydrate 1.42 g (3.0eq) and 50 ml of THF/water/anhydrous methanol = 2:1:4 were mixed evenly. The mixture was protected with nitrogen, and reacted at 50 °C for 2-5 h. Then TLC showed the formation of the product. The reaction was cooled to room temperature, dried by rotary dryer, quenched with water, and extracted with EA to remove organic impurities. The aqueous phase was adjusted to pH=2, and extracted with EA to obtain 3.6 g of T-110.

¹H NMR (400 MHz, Methanol-*d*₄) δ 8.90 (d, *J* = 2.0 Hz, 1H), 8.51 (s, 1H), 8.02 - 7.90 (m, 3H), 7.59 (d, *J* = 8.2 Hz, 2H), 6.68 (d, *J* = 8.9 Hz, 1H), 4.21 (s, 3H).

### (5) Synthesis of T-86:

A solution of T-110 (387mg, 1eq), DIPEA (258mg, 2eq) and HATU (380mg, 1eq) in DCM was stirred at room temperature for 10min, then added with isopropylamine (71mg, 1.2eq), and continued to react for 12h. After the reaction was completed, TLC (pure EA) was used for monitoring and indicated that T-110 has completely reacted. The reaction was diluted by adding about ten times of DCM, washed with 0.05% citric acid to remove DIPEA, then washed with saturated NaCl solution, and then dried. After spin-drying, the crude was dissolved by adding DCM and methanol, and then purified by PTLC or column chromatography to obtain 302mg T-86. ¹H NMR (400 MHz, chloroform-*d*) δ 8.49 (s, *J* = 2.2 Hz, 1H), 8.25 (s, 1H), 7.87 (dd, *J* = 17.7, 8.4 Hz, 3H), 7.46 (d, *J* = 8.0 Hz, 2H), 6.65 (d, *J* = 8.8 Hz, 1H), 6.10 (d, *J* = 7.6 Hz, 1H), 4.36 - 4.28 (m, 1H), 4.21 (s, 3H), 1.29 (d, *J* = 6.5 Hz,6H).

The compounds shown in the following table were synthesized with reference to the synthesis method in examples T-86, T-100 & T-110:

| Example | Compound structure | Compound characterization |
|---|---|---|
| T-99 | 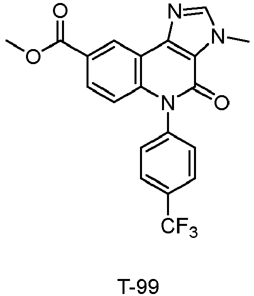 | LC-MS[M+1]: 402 |
| T-101 | 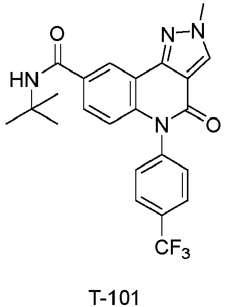 | LC-MS[M+1]: 443 |
| T-102 | 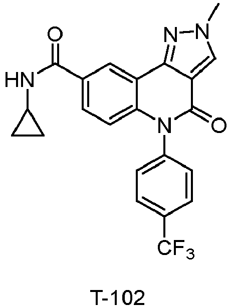 | ¹H NMR (400 MHz, chloroform-*d*) δ 8.48 (d, *J* = 2.2 Hz, 1H), 8.25 (s, 1H), 7.89 (d, *J* = 8.3 Hz, 2H), 7.84 (dd, *J* = 8.9, 2.2 Hz, 1H), 7.45 (d, *J* = 8.1 Hz, 2H), 6.64 (d, *J* = 8.8 Hz, 1H), 6.45 (s, 1H), 4.20 (s, 3H), 2.94 (tt, *J* = 7.1, 3.5 Hz, 1H), 0.90 - 0.79 (m, 2H), 0.70 - 0.62 (m, 2H). |
| T-103 | 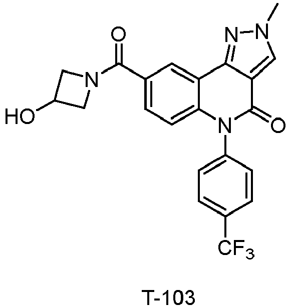 | LC-MS[M+1]: 443 |
| T-104 | 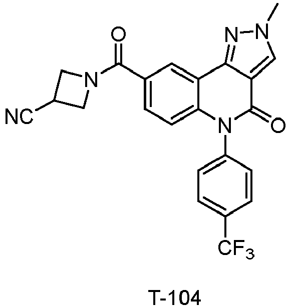 | LC-MS[M+1]: 452 |
| T-105 | 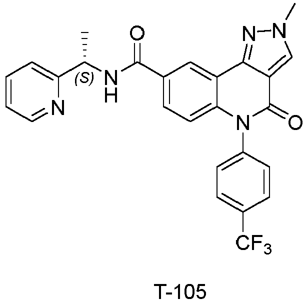 | ¹H NMR (400 MHz, DMSO-*d*₆) δ 9.13 (d, *J* = 7.8 Hz, 1H), 8.80 (s, 1H), 8.75 (s, 1H), 8.53 (d, *J* = 4.8 Hz, 1H), 8.03 (d, *J* = 8.1 Hz, 2H), 7.87 (d, *J* = 9.1 Hz, 1H), 7.77 (t, *J* = 8.0 Hz, 1H), 7.66 (d, *J* = 8.0 Hz, 2H), 7.42 (d, *J* = 8.0 Hz, 1H), 7.27 (t, *J* = 6.1 Hz, 1H), 6.57 (d, *J* = 8.9 Hz, 1H), 5.27 - 5.19 (m, 1H), 4.16 (s, 3H), 1.54 (d, *J* = 7.2 Hz, 3H). |
| T-106 | 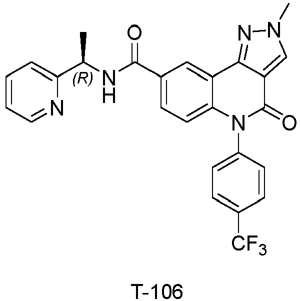 | ¹H NMR (400 MHz, chloroform-*d*) δ 8.72 (d, *J* = 2.2 Hz, 1H), 8.58 (ddd, *J* = 4.9, 1.8, 0.9 Hz, 1H), 8.24 (s, 1H), 7.90 (s, 1H), 7.90 - 7.82 (m, 2H), 7.82 (d, *J* = 7.3 Hz, 1H), 7.70 (td, *J* = 7.7, 1.8 Hz, 1H), 7.46 (dd, *J* = 7.7, 3.5 Hz, 2H), 7.32 (dt, *J* = 8.0, 1.1 Hz, 1H), 7.23 (ddd, *J* = 7.5, 4.9, 1.1 Hz, 1H), 6.64 (s, *J* = 8.8 Hz, 1H), 5.38 (m, *J* = 6.7 Hz, 1H), 4.21 (s, 3H), 1.61 (d, *J* = 6.7 Hz, 3H). |
| T-107 | 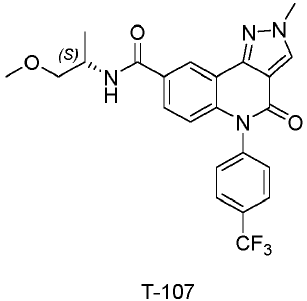 | ¹H NMR (400 MHz, DMSO-*d*₆) δ 8.75 - 8.67 (m, 2H), 8.50 (d, *J* = 8.1 Hz, 1H), 8.02 (d, *J* = 8.2 Hz, 2H), 7.82 (dd, *J* = 8.8, 2.2 Hz, 1H), 7.65 (d, *J* = 8.1 Hz, 2H), 6.60 - 6.52 (m, 1H), 4.23 (dt, *J* = 13.9, 6.9 Hz, 1H), 4.16 (s, 3H), 3.34 - 3.28 (m, 2H), 1.15 (d, *J* = 6.7 Hz, 3H). |
| T-111 | 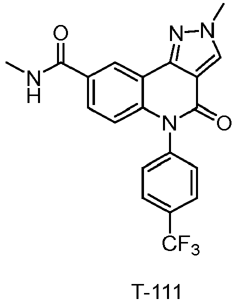 | ¹H NMR (400 MHz, chloroform-*d*) δ 8.54 (d, *J* = 2.1 Hz, 1H), 8.23 (s, 1H), 7.89 (d, *J* = 8.3 Hz, 2H), 7.82 (dd, *J* = 8.8, 2.2 Hz, 1H), 7.46 (d, *J* = 8.1 Hz, 2H), 6.64 (d, *J* = 8.8 Hz, 1H), 4.20 (s, 3H), 3.05 (d, *J* = 4.9 Hz, 3H). |
| T-112 | 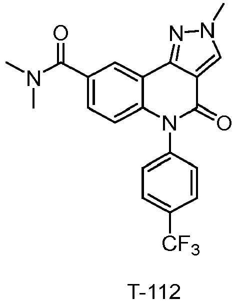 | ¹H NMR (400 MHz, chloroform-*d*) δ 8.33 (d, *J* = 2.0 Hz, 1H), 8.22 (s, 1H), 7.89 (d, *J* = 8.2 Hz, 2H), 7.46 (d, *J* = 8.1 Hz, 2H), 7.40 (dd, *J* = 8.7, 2.0 Hz, 1H), 6.61 (d, *J* = 8.7 Hz, 1H), 4.18 (s, 3H), 3.13 (s, 3H), 3.08 (s, 3H). |
| T-113 | 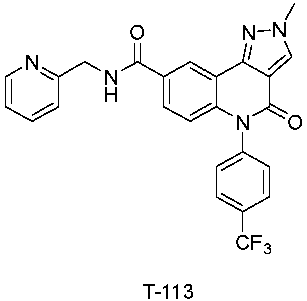 | ¹H NMR (400 MHz, Methanol-*d*₄) δ 8.81 (d, *J* = 2.2 Hz, 1H), 8.50 (s, 2H), 7.99 (d, *J* = 8.4 Hz, 2H), 7.89 - 7.78 (m, 2H), 7.60 (d, *J* = 8.2 Hz, 2H), 7.46 (d, *J* = 7.9 Hz, 1H), 7.32 (ddd, *J* = 7.6, 4.9, 1.2 Hz, 1H), 6.69 (d, *J* = 8.8 Hz, 1H), 4.72 (s, 2H), 4.20 (s, 3H). |
| T-114 | 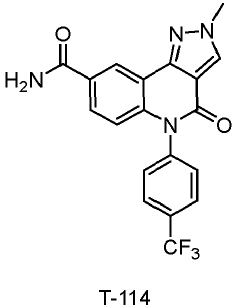 | ¹H NMR (400 MHz, DMSO-*d*₆) δ 8.74 - 8.67 (m, 1H), 8.29 (s, 1H), 8.19 (s, 1H), 8.02 (d, *J* = 8.1 Hz, 2H), 7.83 (dd, *J* = 8.8, 2.1 Hz, 1H), 7.65 (d, *J* = 8.1 Hz, 2H), 6.53 (d, *J* = 8.9 Hz, 1H), 4.15 (s, 3H) |
| T-115 | 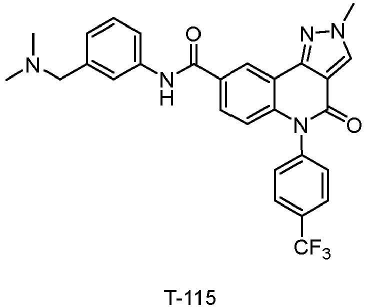 | ¹H NMR (400 MHz, Methanol-*d*₄) δ 8.84 (d, *J* = 2.2 Hz, 1H), 8.52 (d, *J* = 4.1 Hz, 1H), 8. 00 (d, *J* = 8.3 Hz, 2H), 7.91 (dd, *J* = 8.8, 2.2 Hz, 1H), 7.72 - 7.58 (m, 4H), 7.36 (t, *J* = 7.8 Hz, 1H), 7.14 (d, *J* = 7.7 Hz, 1H), 6.72 (d, *J* = 8.9 Hz, 1H), 4.21 (s, 3H), 3.54 (s, 2H), 2.30 (s, 3H). |
| T-116 | 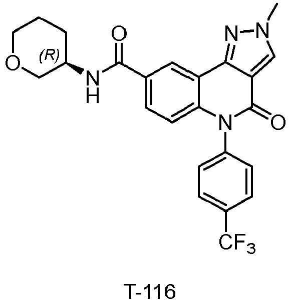 | ¹H NMR (400 MHz, Methanol-*d*₄) δ 8.70 (d, *J* = 2.1 Hz, 1H), 8.49 (s, 1H), 7.98 (d, *J* = 8.1 Hz, 2H), 7.77 (dd, *J* = 8.9, 2.2 Hz, 1H), 7.59 (d, *J* = 8.1 Hz, 2H), 6.66 (d, *J* = 8.8 Hz, 1H), 4.20 (s, 3H), 4.07 (ddd, *J* = 13.4, 8.4, 4.0 Hz, 1H), 3.95 (ddd, *J* = 10.8, 4.2, 1.6 Hz, 1H), 3.87 - 3.79 (m, 1H), 3.55 - 3.43 (m, 1H), 3.40 - 3.33 (m, 1H), 2.02 (d, *J* = 8.9 Hz, 1H), 1.83 - 1.75 (m, 1H), 1.75 - 1.67 (m, 2H). |
| T-117 | 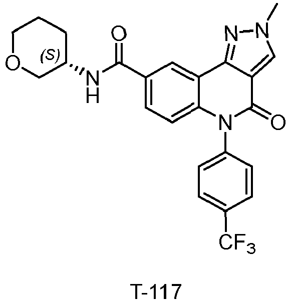 | ¹H NMR (400 MHz, Methanol-*d*₄) δ 8.69 (d, *J* = 2.1 Hz, 1H), 8.48 (s, 1H), 7.98 (d, *J* = 8.1 Hz, 2H), 7.77 (dd, *J* = 8.9, 2.2 Hz, 1H), 7.59 (d, *J* = 8.1 Hz, 2H), 6.65 (d, *J* = 8.9 Hz, 1H), 4.19 (s, 3H), 4.07 (td, *J* = 9.2, 8.8, 4.4 Hz, 1H), 3.94 (ddd, *J* = 10.9, 4.3, 1.6 Hz, 1H), 3.86 - 3.79 (m, 1H), 3.54 - 3.42 (m, 1H), 3.39 - 3.32 (m, 1H), 2.07 - 1.98 (m, 1H), 1.80 (d, *J=* 4.1 Hz, 1H), 1.75 - 1.66 (m, 2H). |
| T-118 | 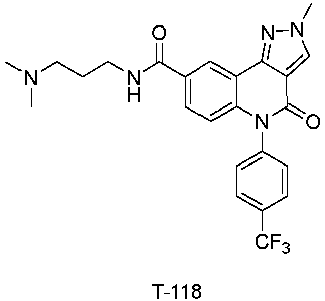 | ¹H NMR (400 MHz, Methanol-*d*₄) δ 8.76 (d, *J* = 2.1 Hz, 1H), 8.50 (d, *J* = 0.6 Hz, 1H), 7.99 (d, *J* = 8.3 Hz, 2H), 7.81 (dd, *J* = 8.9, 2.2 Hz, 1H), 7.62 - 7.56 (m, 2H), 6.67 (d, *J* = 8.9 Hz, 1H), 4.20 (s, 3H), 3.51 (t, *J* = 6.5 Hz, 2H), 3.25 - 3.16 (m, 2H), 2.92 (s, 6H), 2.05 (dt, *J* = 14.0, 6.8 Hz, 3H). |
| T-119 | 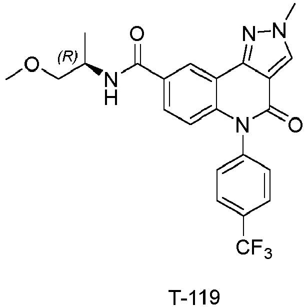 | ¹H NMR (400 MHz, Methanol-*d*₄) δ 8.72 (d, *J* = 2.1 Hz, 1H), 8.50 (s, 1H), 7.98 (d, *J* = 8.2 Hz, 2H), 7.78 (dd, *J* = 8.8, 2.2 Hz, 1H), 7.59 (d, *J* = 8.1 Hz, 2H), 6.66 (d, *J* = 8.8 Hz, 1H), 4.33 (p, *J* = 6.6 Hz, 1H), 4.20 (s, 3H), 3.55 - 3.39 (m, 2H), 3.38 (s, 3H), 1.25 (d, *J* = 6.8 Hz, 3H). |
| T-120 | 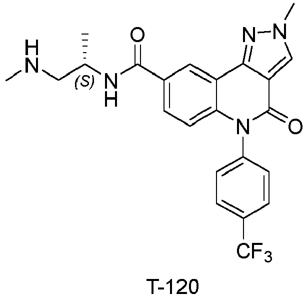 | ¹H NMR (400 MHz, DMSO-*d*₆) δ 8.75 (s, 1H), 8.68 (s, 1H), 8.54 - 8.48 (m, 1H), 8.02 (d, *J* = 8.0 Hz, 2H), 7.84 (d, *J* = 8.9 Hz, 1H), 7.65 (d, *J* = 8.0 Hz, 2H), 6.54 (d, *J* = 8.8 Hz, 1H), 4.25 (s, 1H), 4.15 (s, 3H), 2.29 (s, 6H), 1.98 (s, 1H), 1.16 (d, *J* = 6.3 Hz, 3H). |
| T-121 | 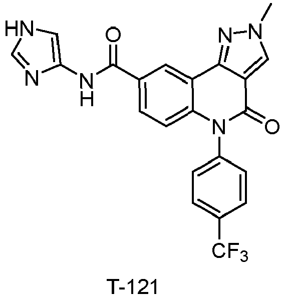 | ¹H NMR (400 MHz, Methanol-*d*₄) 8 8.78 (s, 1H), 8.50 (s, 1H), 8.35 - 8.24 (m, 1H), 7.98 (d, *J* = 8.1 Hz, 2H), 7.89 - 7.75 (m, 1H), 7.58 (d, *J* = 8.1 Hz, 2H), 7.29 (s, 1H), 6.67 (d, *J* = 8.6 Hz, 1H), 4.20 (s, 3H). |
| T-122 | 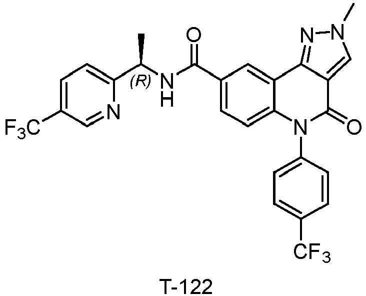 | ¹H NMR (400 MHz, DMSO-*d*₆) δ 9.20 (d, *J* = 7.4 Hz, 1H), 8.88 (s, 1H), 8.77 (d, *J* = 2.2 Hz, 1H), 8.70 (s, 1H), 8.14 (dd, *J* = 8.5, 2.4 Hz, 1H), 7.98 (d, *J* = 8.1 Hz, 2H), 7.81 (dd, *J* = 9.0, 2.2 Hz, 1H), 7.60 (t, *J* = 8.3 Hz, 2H), 6.52 (d, *J* = 8.9 Hz, 1H), 5.26 - 5.18 (m, 1H), 4.11 (s, 3H), 1.52 (d, *J* = 7.2 Hz, 3H). |
| T-123 | 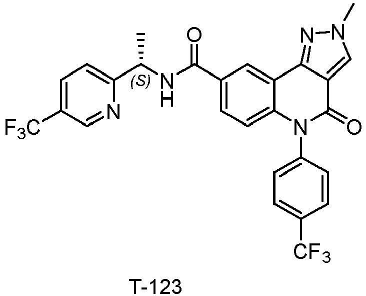 | ¹H NMR (400 MHz, DMSO-*d*₆) δ 9.25 (d, *J* = 7.3 Hz, 1H), 8.92 (d, *J* = 2.3 Hz, 1H), 8.81 (d, *J* = 2.1 Hz, 1H), 8.74 (s, 1H), 8.18 (dd, *J* = 8.4, 2.4 Hz, 1H), 8.02 (d, *J* = 8.2 Hz, 2H), 7.85 (dd, *J* = 8.8, 2.2 Hz, 1H), 7.65 (t, *J* = 8.4 Hz, 3H), 6.57 (d, *J* = 8.9 Hz, 1H), 5.27 (p, *J* = 7.2 Hz, 1H), 4.16 (s, 3H), 1.57 (d, *J* = 7.1 Hz, 3H). |
| T-124 | 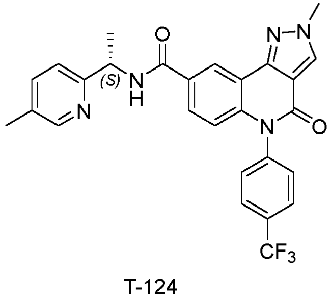 | ¹H NMR (400 MHz, DMSO-*d*₆) δ 9.07 (d, *J* = 7.7 Hz, 1H), 8.76 (d, *J* = 15.4 Hz, 2H), 8.35 (s, 1H), 8.02 (d, *J* = 8.0 Hz, 2H), 7.85 (d, *J* = 9.1 Hz, 1H), 7.66 (d, *J* = 8.0 Hz, 2H), 7.56 (d, *J* = 8.0 Hz, 1H), 7.30 (d, *J* = 8.1 Hz, 1H), 6.56 (d, *J* = 9.0 Hz, 1H), 5.23 - 5.15 (m, 1H), 4.16 (s, 3H), 1.51 (d, *J* = 7.0 Hz, 3H). |
| T-125 | 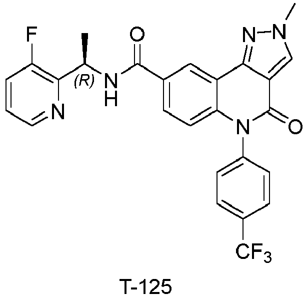 | ¹H NMR (400 MHz, DMSO-*d*₆) δ 9.13 (d, *J* = 7.4 Hz, 1H), 8.80 - 8.72 (m, 2H), 8.40 (dt, *J* = 4.6, 1.5 Hz, 1H), 8.02 (d, *J* = 8.1 Hz, 2H), 7.84 (dd, *J* = 8.9, 2.1 Hz, 1H), 7.69 (ddd, *J* = 10.1, 8.3, 1.3 Hz, 1H), 7.65 (d, *J* = 7.8 Hz, 2H), 7.39 (dt, *J* = 8.5, 4.3 Hz, 1H), 6.55 (d, *J=* 8.8 Hz, 1H), 5.49 (p, *J* = 7.0 Hz, 1H), 4.16 (s, 3H), 1.52 (d, *J=* 7.0 Hz, 3H). |
| T-126 | 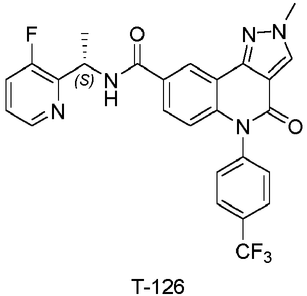 | ¹H NMR (400 MHz, DMSO-*d*₆) δ 9.20 (d, *J* = 7.4 Hz, 1H), 8.87 - 8.74 (m, 2H), 8.46 (dt, *J* = 4.7, 1.6 Hz, 1H), 8.09 (d, *J* = 8.0 Hz, 2H), 7.93 (ddd, *J* = 23.1, 8.9, 2.1 Hz, 1H), 7.80 - 7.68 (m, 3H), 7.46 (dt, *J* = 8.5, 4.4 Hz, 1H), 6.64 (dd, *J* = 20.6, 8.8 Hz, 1H), 5.56 (p, *J* = 7.1 Hz, 1H), 4.22 (s, 3H), 1.59 (d, *J* = 7.0 Hz, 3H). |
| T-127 | 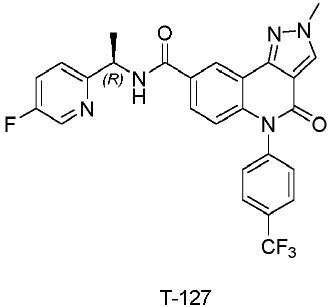 | ¹H NMR (400 MHz, DMSO-*d*₆) δ 9.14 (d, *J* = 7.6 Hz, 1H), 8.78 (s, 1H), 8.74 (s, 1H), 8.53 (d, *J* = 10.9 Hz, 1H), 8.02 (d, *J* = 8.0 Hz, 2H), 7.85 (d, *J* = 8.8 Hz, 1H), 7.68 (q, *J* = 8.7 Hz, 3H), 7.49 (q, *J* = 8.9, 6.5 Hz, 1H), 6.56 (d, *J* = 8.9 Hz, 1H), 5.28 - 5.19 (m, 1H), 4.16 (s, 3H), 1.52 (d, *J* = 7.0 Hz, 3H). |
| T-128 | 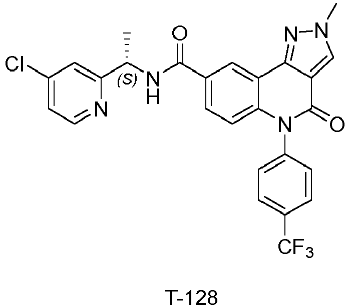 | ¹H NMR (400 MHz, DMSO-*d*₆) δ 9.14 (d, *J* = 7.7 Hz, 1H), 8.80 (d, *J* = 2.2 Hz, 1H), 8.75 (s, 1H), 8.52 (d, *J* = 5.4 Hz, 1H), 8.03 (d, *J* = 8.2 Hz, 2H), 7.86 (dd, *J* = 8.9, 2.2 Hz, 1H), 7.66 (d, *J* = 8.1 Hz, 2H), 7.51 (d, *J* = 2.0 Hz, 1H), 7.42 (dd, *J* = 5.4, 2.1 Hz, 1H), 6.57 (d, *J* = 8.9 Hz, 1H), 5.21 (p, *J* = 7.2 Hz, 1H), 4.16 (s, 3H), 1.53 (d, *J* = 7.1 Hz, 3H). |
| T-129 | 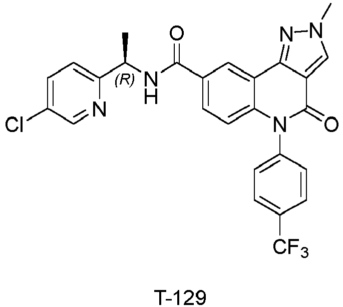 | ¹H NMR (400 MHz, DMSO-*d*₆) δ 9.16 (d, *J* = 7.7 Hz, 1H), 8.79 (s, 1H), 8.74 (s, 1H), 8.60 - 8.55 (m, 1H), 8.03 (d, *J* = 8.2 Hz, 2H), 7.89 (d, *J* = 12.4 Hz, 2H), 7.66 (d, *J* = 8.0 Hz, 2H), 7.45 (d, *J* = 8.5 Hz, 1H), 6.56 (d, *J* = 9.0 Hz, 1H), 5.30 - 5.13 (m, 1H), 4.16 (s, 3H), 1.53 (d, *J* = 7.2 Hz, 3H). |
| T-130 | 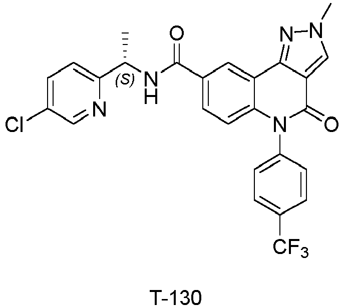 | ¹H NMR (400 MHz, DMSO-*d*₆) δ 9.16 (d, *J* = 7.5 Hz, 1H), 8.79 (s, 1H), 8.74 (s, 1H), 8.57 (s, 1H), 8.02 (d, *J* = 8.1 Hz, 2H), 7.87 (dd, *J* = 16.8, 8.6 Hz, 2H), 7.65 (d, *J* = 8.0 Hz, 2H), 7.45 (d, *J* = 8.6 Hz, 1H), 6.56 (d, *J* = 8.5 Hz, 1H), 5.20 (s, 1H), 4.16 (s, 3H), 1.52 (d, *J* = 7.0 Hz, 3H). |
| T-131 | 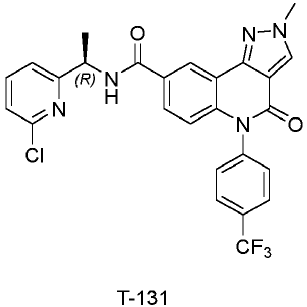 | ¹H NMR (400 MHz, DMSO-*d*₆) δ 9.20 (d, *J* = 7.5 Hz, 1H), 8.81 (s, 1H), 8.75 (s, 1H), 8.03 (d, *J=* 8.1 Hz, 2H), 7.89 - 7.79 (m, 2H), 7.66 (d, *J* = 7.9 Hz, 2H), 7.40 (dd, *J* = 12.8, 7.7 Hz, 2H), 6.57 (d, *J* = 8.9 Hz, 1H), 5.17 (q, *J* = 7.2 Hz, 1H), 4.16 (s, 3H), 1.53 (d, *J* = 7.2 Hz, 3H). |
| T-132 | 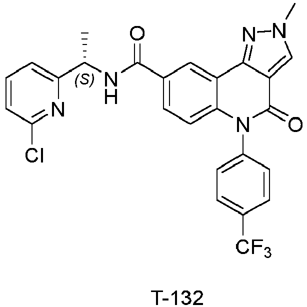 | ¹H NMR (400 MHz, DMSO-*d*₆) δ 9.20 (d, *J* = 7.5 Hz, 1H), 8.81 (d, *J* = 2.1 Hz, 1H), 8.75 (s, 1H), 8.03 (d, *J* = 8.1 Hz, 2H), 7.89 - 7.79 (m, 2H), 7.66 (d, *J* = 7.9 Hz, 2H), 7.40 (dd, *J* = 12.5, 7.8 Hz, 2H), 6.57 (d, *J* = 8.9 Hz, 1H), 5.16 (p, *J* = 7.0 Hz, 1H), 4.16 (s, 3H), 1.53 (d, *J* = 7.1 Hz, 3H). |
| T-133 | 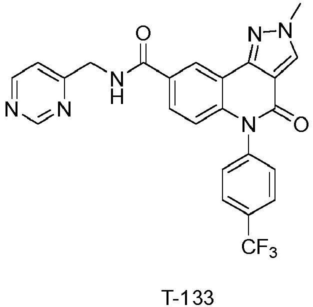 | ¹H NMR (400 MHz, DMSO-*d*₆) δ 9.42 (t, *J* = 5.7 Hz, 1H), 9.12 (s, 1H), 8.81 - 8.71 (m, 3H), 8.03 (d, *J* = 8.1 Hz, 2H), 7.89 (dd, *J* = 8.9, 2.2 Hz, 1H), 7.67 (d, *J* = 8.0 Hz, 2H), 7.43 (d, *J* = 5.3 Hz, 1H), 6.60 (d, *J* = 8.8 Hz, 1H), 4.57 (d, *J* = 4.3 Hz, 2H), 4.16 (s, 3H). |
| T-134 | 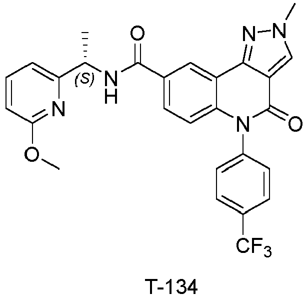 | ¹H NMR (400 MHz, DMSO-*d*₆) δ 9.06 (d, *J* = 8.0 Hz, 1H), 8.83 - 8.78 (m, 1H), 8.75 (s, 1H), 8.03 (d, *J* = 8.2 Hz, 2H), 7.90 - 7.83 (m, 1H), 7.66 (d, *J* = 7.7 Hz, 3H), 6.97 (d, *J* = 7.5 Hz, 1H), 6.67 (d, *J* = 8.2 Hz, 1H), 6.57 (d, *J* = 8.9 Hz, 1H), 5.13 (t, *J* = 7.4 Hz, 1H), 4.16 (s, 3H), 3.87 (s, 3H), 1.53 (d, *J* = 7.2 Hz, 3H). |
| T-135 | 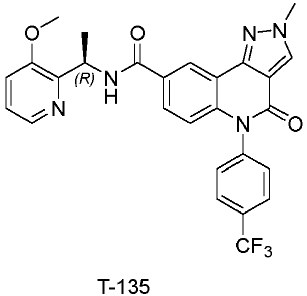 | ¹H NMR (400 MHz, DMSO-*d*₆) δ 8.88 (s, 1H), 8.77 - 8.72 (m, 1H), 8.11 (d, *J* = 4.9 Hz, 1H), 8.02 (d, *J* = 8.1 Hz, 2H), 7.84 (d, *J* = 9.0 Hz, 1H), 7.65 (d, *J* = 8.0 Hz, 2H), 7.43 (d, *J* = 8.3 Hz, 1H), 7.30 (d, *J* = 7.3 Hz, 1H), 6.55 (d, *J* = 9.1 Hz, 1H), 5.56 (s, 1H), 4.16 (s, 3H), 3.87 (s, 3H), 1.42 (d, *J* = 6.8 Hz, 3H). |
| T-136 | 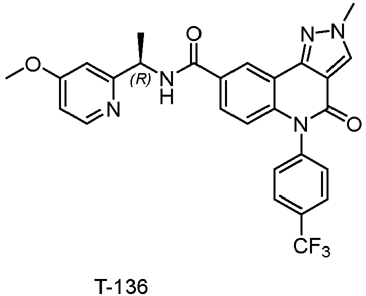 | ¹H NMR (400 MHz, DMSO-*d*₆) δ 9.06 (d, *J* = 7.8 Hz, 1H), 8.75 (d, *J* = 15.4 Hz, 2H), 8.35 (d, *J* = 5.8 Hz, 1H), 8.02 (d, *J* = 8.1 Hz, 2H), 7.85 (d, *J* = 9.0 Hz, 1H), 7.66 (d, *J* = 8.0 Hz, 2H), 6.97 (s, 1H), 6.86 (d, *J* = 5.8 Hz, 1H), 6.56 (d, *J* = 9.1 Hz, 1H), 5.17 (p, *J* = 7.2 Hz, 1H), 4.16 (s, 3H), 3.80 (s, 3H), 1.51 (d, *J* = 7.1 Hz, 3H). |
| T-137 | 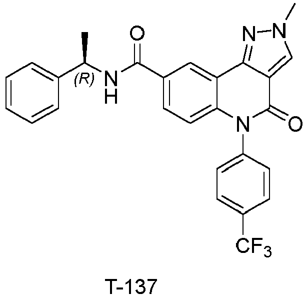 | ¹H NMR (400 MHz, DMSO-*d*₆) δ 9.08 (d, *J* = 8.0 Hz, 1H), 8.79 - 8.72 (m, 2H), 8.02 (d, *J* = 8.2 Hz, 2H), 7.84 (dd, *J=* 8.9, 2.2 Hz, 1H), 7.65 (d, *J* = 8.0 Hz, 2H), 7.44 - 7.37 (m, 2H), 7.41 - 7.18 (m, 4H), 6.55 (d, *J* = 8.9 Hz, 1H), 5.20 (p, *J* = 7.1 Hz, 1H), 4.16 (s, 3H), 1.51 (d, *J* = 7.1 Hz, 3H). |
| T-138 | 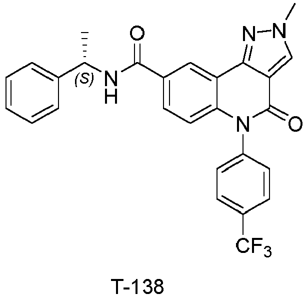 | ¹H NMR (400 MHz, DMSO-*d*₆) δ 9.09 (d, *J* = 8.1 Hz, 1H), 8.75 (d, *J* = 8.9 Hz, 2H), 8.02 (d, *J* = 8.1 Hz, 2H), 7.87 - 7.80 (m, 1H), 7.66 (d, *J* = 8.0 Hz, 2H), 7.41 (d, *J* = 7.8 Hz, 2H), 7.32 (q, *J* = 6.4, 5.4 Hz, 2H), 7.22 (t, *J* = 7.3 Hz, 1H), 6.55 (d, *J* = 8.8 Hz, 1H), 5.20 (t, *J* = 7.4 Hz, 1H), 4.16 (s, 3H), 1.51 (d, *J* = 7.1 Hz, 3H). |
| T-139 | 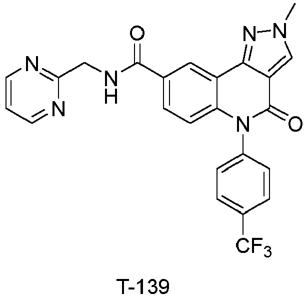 | ¹H NMR (400 MHz, DMSO-*d*₆) δ 9.29 (t, *J* = 5.9 Hz, 1H), 8.80 - 8.72 (m, 4H), 8.03 (d, *J* = 8.1 Hz, 2H), 7.88 (dd, *J* = 8.9, 2.1 Hz, 1H), 7.67 (d, *J* = 8.1 Hz, 2H), 7.40 (t, *J* = 4.8 Hz, 1H), 6.59 (d, *J* = 8.8 Hz, 1H), 4.68 (d, *J* = 5.8 Hz, 2H), 4.15 (s, 3H). |
| T-140 | 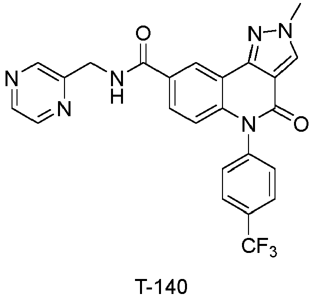 | ¹H NMR (400 MHz, DMSO-*d*₆) δ 9.41 (t, *J* = 6.0 Hz, 1H), 8.75 (d, *J* = 6.5 Hz, 2H), 8.62 (d, *J* = 16.0 Hz, 1H), 8.55 (s, 2H), 8.03 (d, *J* = 8.1 Hz, 2H), 7.87 (d, *J* = 8.9 Hz, 1H), 7.66 (d, *J* = 8.0 Hz, 2H), 6.59 (d, *J* = 8.8 Hz, 1H), 4.64 (d, *J* = 5.7 Hz, 2H), 4.15 (s, 3H). |
| T-141 | 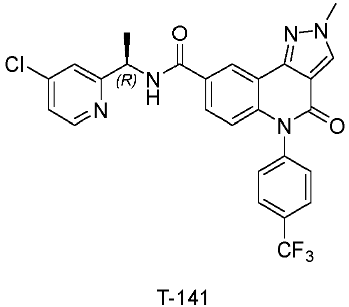 | ¹H NMR (400 MHz, DMSO-*d*₆) δ 9.14 (d, *J* = 7.5 Hz, 1H), 8.80 (s, 1H), 8.75 (s, 1H), 8.52 (d, *J* = 5.3 Hz, 1H), 8.03 (d, *J* = 7.8 Hz, 2H), 7.88 (t, *J* = 9.1 Hz, 1H), 7.66 (d, *J* = 8.0 Hz, 2H), 7.51 (s, 1H), 7.45 - 7.40 (m, 1H), 6.62 - 6.54 (m, 1H), 5.28 - 5.17 (m, 1H), 4.16 (s, 3H), 1.54 (s, 3H). |
| T-142 | 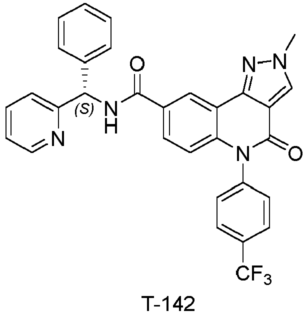 | ¹H NMR (400 MHz, DMSO-*d*₆) δ 9.53 (d, *J* = 8.3 Hz, 1H), 8.79 (d, *J* = 2.2 Hz, 1H), 8.74 (s, 1H), 8.59 - 8.53 (m, 1H), 8.03 (d, *J* = 8.1 Hz, 2H), 7.88 (dd, *J* = 8.9, 2.2 Hz, 1H), 7.81 (td, *J* = 7.7, 1.9 Hz, 1H), 7.66 (d, *J* = 8.0 Hz, 2H), 7.52 (d, *J* = 7.9 Hz, 1H), 7.41 (d, *J* = 1.6 Hz, 1H), 7.39 (s, 1H), 7.38 - 7.22 (m, 4H), 6.56 (d, *J* = 8.9 Hz, 1H), 6.44 (d, *J* = 8.2 Hz, 1H), 4.15 (s, 3H). |
| T-143 | 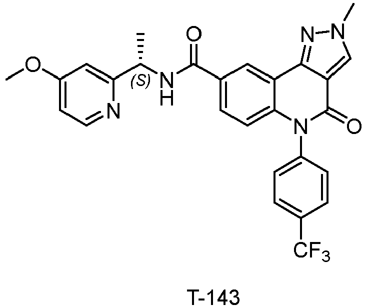 | LC-MS[M+1]: 522 |
| T-144 | 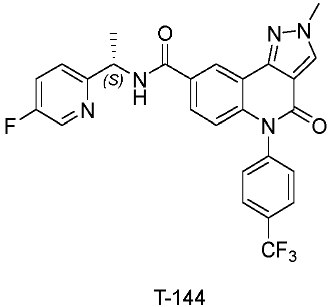 | LC-MS[M+1]: 510 |
| T-145 | 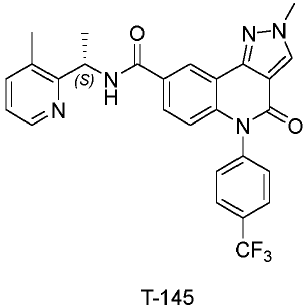 | ¹H NMR (400 MHz, DMSO-*d*₆) δ 9.03 (d, *J* = 7.5 Hz, 1H), 8.72 (s, 2H), 8.41 - 8.35 (m, 1H), 8.01 (d, *J* = 7.9 Hz, 2H), 7.82 (d, *J* = 8.9 Hz, 1H), 7.64 (d, *J* = 8.0 Hz, 3H), 7.57 (d, *J* = 7.2 Hz, 1H), 6.52 (d, *J* = 9.0 Hz, 1H), 5.45 - 5.37 (m, 1H), 4.14 (s, 3H), 2.38 (s, 3H), 1.46 (d, *J* = 6.7 Hz, 3H). |
| T-146 | 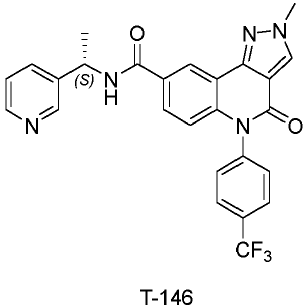 | ¹H NMR (400 MHz, DMSO-*d*₆) δ 9.17 (d, *J* = 7.7 Hz, 1H), 8.75 (d, *J* = 4.2 Hz, 2H), 8.62 (s, 1H), 8.44 (d, *J* = 4.9 Hz, 2H), 8.02 (d, *J* = 8.0 Hz, 2H), 7.82 (t, *J* = 8.9 Hz, 2H), 7.65 (d, *J* = 8.0 Hz, 1H), 7.36 (dd, *J* = 8.0, 4.7 Hz, 1H), 6.55 (d, *J* = 8.9 Hz, 1H), 5.26 - 5.18 (m, 1H), 4.16 (s, 3H), 1.54 (d, *J* = 7.0 Hz, 3H). |
| T-147 | 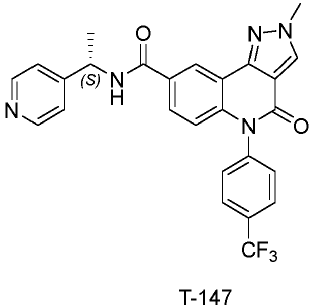 | ¹H NMR (400 MHz, DMSO-*d*₆) δ 9.25 (d, *J* = 7.7 Hz, 1H), 8.86 - 8.78 (m, 2H), 8.60 - 8.54 (m, 2H), 8.09 (d, *J* = 8.1 Hz, 2H), 7.90 (dd, *J* = 8.9, 2.2 Hz, 1H), 7.72 (d, *J* = 8.0 Hz, 2H), 7.47 - 7.41 (m, 2H), 6.62 (d, *J* = 8.8 Hz, 1H), 5.22 (p, *J* = 7.2 Hz, 1H), 4.22 (s, 3H), 1.57 (d, *J* = 7.1 Hz, 3H). |
| T-148 | 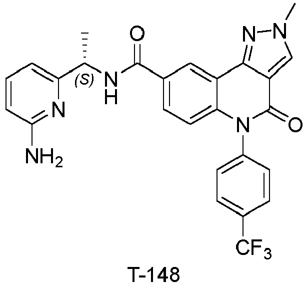 | ¹H NMR (400 MHz, DMSO-*d*₆) δ 8.97 (d, *J* = 7.8 Hz, 1H), 8.82 (d, *J* = 13.2 Hz, 2H), 8.09 (d, *J* = 8.1 Hz, 2H), 7.92 (d, *J* = 9.0 Hz, 1H), 7.72 (d, *J* = 8.1 Hz, 2H), 7.38 (t, *J* = 7.8 Hz, 1H), 6.60 (dd, *J* = 17.8, 8.1 Hz, 2H), 6.36 (d, *J* = 8.2 Hz, 1H), 5.93 (s, 2H), 5.07 - 4.99 (m, 1H), 4.22 (s, 3H), 1.52 (d, *J* = 6.9 Hz, 3H). |
| T-149 | 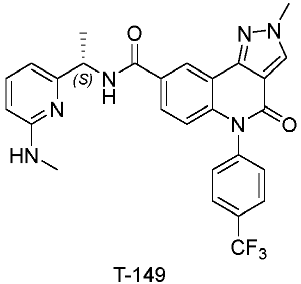 | ¹H NMR (400 MHz, DMSO-*d*₆) δ 8.93 (d, *J* = 7.8 Hz, 1H), 8.80 - 8.73 (m, 2H), 8.03 (d, *J* = 8.0 Hz, 2H), 7.89 - 7.82 (m, 1H), 7.67 (d, *J* = 8.0 Hz, 2H), 7.34 (t, *J* = 7.8 Hz, 1H), 6.57 (d, *J* = 8.8 Hz, 1H), 6.50 (d, *J* = 7.3 Hz, 1H), 6.47 - 6.40 (m, 1H), 6.28 (d, *J* = 8.2 Hz, 1H), 5.00 (t, *J* = 7.3 Hz, 1H), 4.16 (s, 3H), 2.78 (d, *J=* 4.6 Hz, 3H), 1.48 (d, *J=* 7.1 Hz, 3H). |
| T-150 | 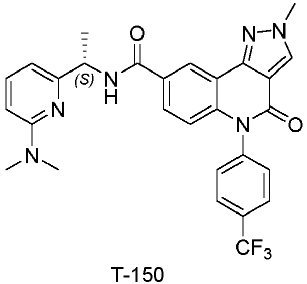 | ¹H NMR (400 MHz, DMSO-*d*₆) δ 8.92 (s, 1H), 8.75 (d, *J* = 14.2 Hz, 2H), 8.05 - 7.98 (m, 2H), 7.88 - 7.82 (m, 1H), 7.66 (s, 2H), 7.44 (s, 1H), 6.57 (s, 1H), 6.51 - 6.44 (m, 2H), 5.04 (s, 1H), 4.15 (s, 3H), 3.03 (s, 6H), 1.49 (s, 3H). |
| T-151 | 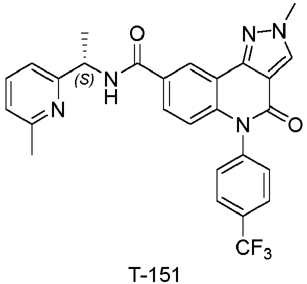 | ¹H NMR (400 MHz, DMSO-*d*₆) δ 9.09 (d, *J* = 7.8 Hz, 1H), 8.80 (s, 1H), 8.74 (s, 1H), 8.02 (d, *J* = 8.0 Hz, 2H), 7.86 (d, *J* = 9.0 Hz, 1H), 7.64 (dd, *J* = 13.8, 7.7 Hz, 3H), 7.20 (d, *J* = 7.9 Hz, 1H), 7.11 (d, *J* = 7.7 Hz, 1H), 6.56 (d, *J* = 9.0 Hz, 1H), 5.20 - 5.12 (m, 1H), 4.16 (s, 3H), 2.08 (s,3H), 1.51 (d, *J* = 7.2 Hz, 3H). |
| T-152 | 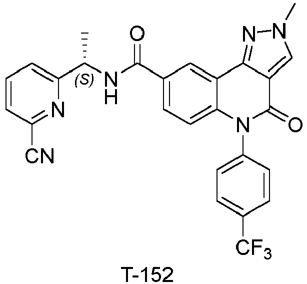 | LC-MS[M+1]: 517 |
| T-153 | 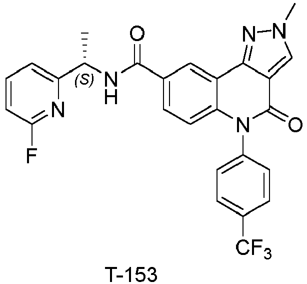 | ¹H NMR (400 MHz, DMSO-*d*₆) δ 9.19 (d, *J* = 7.6 Hz, 1H), 8.83 - 8.78 (m, 1H), 8.75 (s, 1H), 8.02 (d, *J* = 8.1 Hz, 2H), 7.95 (q, *J* = 8.0 Hz, 1H), 7.90 - 7.83 (m, 1H), 7.66 (d, *J* = 7.9 Hz, 2H), 7.35 (dd, *J* = 7.6, 2.5 Hz, 1H), 7.04 (dd, *J* = 8.2, 2.7 Hz, 1H), 6.56 (d, *J* = 8.8 Hz, 1H), 5.19 - 5.11 (m, 1H), 4.16 (s, 3H), 1.52 (d, *J=* 7.1 Hz, 3H). |
| T-227 | 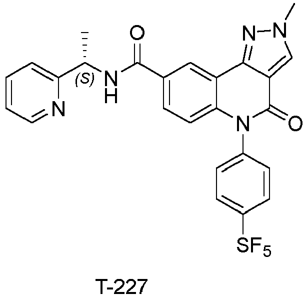 | ¹H NMR (400 MHz, DMSO-*d*₆) δ 9.22 (d, *J* = 7.7 Hz, 1H), 8.90 - 8.81 (m, 2H), 8.60 (m, *J* = 4.8, 1.8, 0.9 Hz, 1H), 8.31 - 8.24 (m, 2H), 7.95 (m, *J* = 8.9, 2.2 Hz, 1H), 7.83 (td, *J* = 7.7, 1.8 Hz, 1H), 7.76 (d, *J* = 8.3 Hz, 2H), 7.51 - 7.44 (m, 1H), 7.33 (m, *J* = 7.5, 4.8, 1.2 Hz, 1H), 6.66 (d, *J* = 8.9 Hz, 1H), 5.29 (p, *J* = 7.1 Hz, 1H), 4.23 (s, 3H), 1.60 (d, *J* = 7.1 Hz, 3H) |
| T-228 | 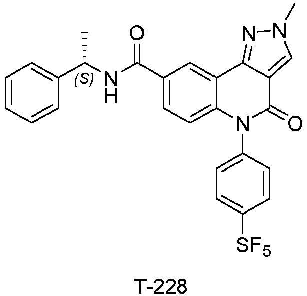 | ¹H NMR (400 MHz, DMSO-*d*₆) δ 9.12 (d, *J* = 8.0 Hz, 1H), 8.76 (dd, *J* = 3.9, 1.3 Hz, 2H), 8.24 - 8.16 (m, 2H), 7.85 (dd, *J* = 8.8, 2.2 Hz, 1H), 7.68 (d, *J* = 8.5 Hz, 2H), 7.44 - 7.37 (m, 2H), 7.37 - 7.27 (m, 2H), 7.27 - 7.18 (m, 1H), 6.58 (d, *J* = 8.9 Hz, 1H), 5.19 (p, *J* = 7.2 Hz, 1H), 4.16 (s, 3H), 1.51 (d, *J* = 7.1 Hz, 3H). |
| T-229 | 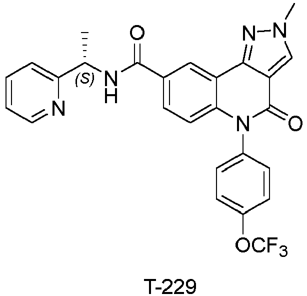 | LC-MS[M+1]: 508 |
| T-230 | 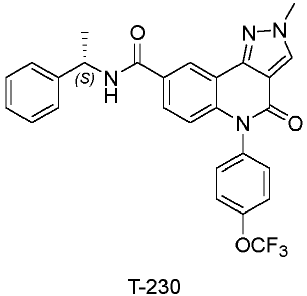 | LC-MS[M+1]: 507 |
| T-231 | 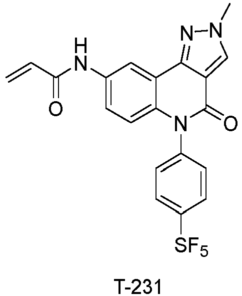 | LC-MS[M+1]: 471 |
| T-232 | 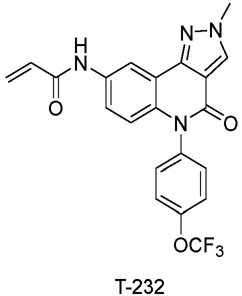 | LC-MS[M+1]: 429 |
| T-233 | 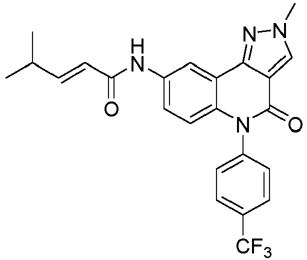 | ¹H NMR (400 MHz, chloroform-*d*) δ 8.23 - 8.15 (m, 2H), 7.87 (d, *J* = 8.2 Hz, 2H), 7.68 (d, *J* = 9.2 Hz, 1H), 7.50 - 7.43 (m, 2H), 7.38 (s, 1H), 7.03 - 6.93 (m, 1H), 6.52 (d, *J* = 9.1 Hz, 1H), 5.87 (dd, *J* = 15.3, 1.5 Hz, 1H), 4.14 (d, *J* = 4.8 Hz, 3H), 2.57 - 2.44 (m, 1H), 1.09 (d, *J* = 6.8 Hz, 6H). |
| T-234 | 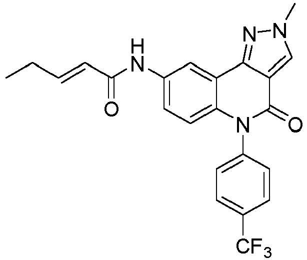 | ¹H NMR (400 MHz, chloroform-*d*) δ 8.18 - 8.09 (m, 2H), 7.80 (d, *J* = 8.1 Hz, 2H), 7.62 (d, *J* = 9.1 Hz, 1H), 7.40 (d, *J* = 8.0 Hz, 2H), 7.05 - 6.93 (m, 1H), 6.47 (d, *J* = 9.0 Hz, 1H), 5.85 (d, *J* = 15.2 Hz, 1H), 4.09 (s, 3H), 2.25 - 2.13 (m, 2H), 1.04 (t, *J* = 7.4 Hz, 3H). |
| T-235 | 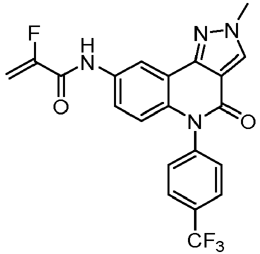 | ¹H NMR (400 MHz, DMSO-*d*₆) δ 10.50 (s, 1H), 8.69 (s, 2H), 8.00 (d, *J* = 7.8 Hz, 2H), 7.63 (d, *J* = 8.2 Hz, 3H), 6.49 (d, *J* = 9.1 Hz, 1H), 5.74 (d, *J* = 47.5 Hz, 1H), 5.45 (d, *J* = 15.5 Hz, 1H), 4.13 (s, 3H). |
| T-236 | 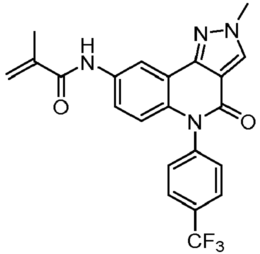 | ¹H NMR (400 MHz, DMSO-*d*₆) δ 9.96 (s, 1H), 8.67 (s, 2H), 8.00 (d, *J* = 8.0 Hz, 2H), 7.62 (d, *J* = 8.8 Hz, 3H), 6.46 (d, *J* = 9.2 Hz, 1H), 5.85 (s, 1H), 5.53 (s, 1H), 4.13 (s, 3H), 1.96 (s, 3H). |
| T-237 | 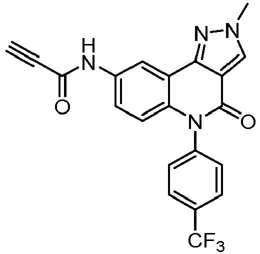 | ¹H NMR (400 MHz, DMSO-*d*₆) δ 11.05 (s, 1H), 8.74 (s, 1H), 8.67 (s, 1H), 8.06 (d, *J* = 8.1 Hz, 2H), 7.68 (d, *J* = 8.1 Hz, 2H), 7.47 (s, 1H), 6.53 (d, *J* = 9.3 Hz, 1H), 4.51 (s, 1H), 4.19 (s, 3H). |
| T-238 | 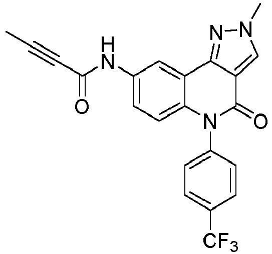 | ¹H NMR (400 MHz, DMSO-*d*₆) δ 10.86 (s, 1H), 8.75 (s, 1H), 8.68 (d, *J* = 2.5 Hz, 1H), 8.07 (d, *J* = 8.3 Hz, 2H), 7.69 (d, *J* = 8.1 Hz, 2H), 7.49 (dd, *J* = 9.1, 2.5 Hz, 1H), 6.53 (d, *J* = 9.1 Hz, 1H), 4.21 (s, 3H), 2.13 (s, 3H). |
| T-239 | 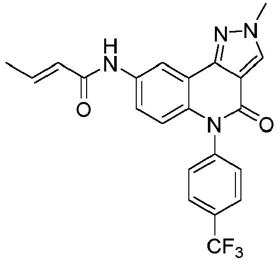 | ¹H NMR (400 MHz, DMSO-*d*₆) δ 10.13 (s, 1H), 8.69 (d, *J* = 16.2 Hz, 2H), 8.00 (d, *J* = 8.0 Hz, 2H), 7.62 (d, *J* = 8.0 Hz, 2H), 7.43 (s, 1H), 6.82 (dq, *J* = 14.3, 6.8 Hz, 1H), 6.46 (d, *J* = 9.1 Hz, 1H), 6.12 (d, *J* = 15.2 Hz, 1H), 4.13 (s, 3H), 1.87 (d, *J* = 6.9 Hz, 3H). |
| T-248 | 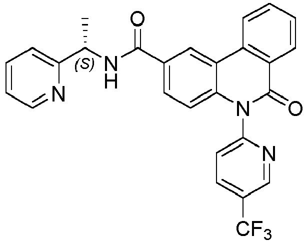 | ¹H NMR (400 MHz, DMSO-*d*₆) δ 9.22 (d, *J* = 2.5 Hz, 1H), 9.16 - 9.07 (m, 2H), 8.78 (d, *J* = 8.2 Hz, 1H), 8.64 (dd, *J* = 8.5, 2.5 Hz, 1H), 8.55 (ddd, *J* = 4.9, 1.9, 0.9 Hz, 1H), 8.38 (dd, *J* = 8.0, 1.4 Hz, 1H), 8.09 - 7.95 (m, 2H), 7.92 (dd, *J* = 8.8, 1.9 Hz, 1H), 7.84 - 7.73 (m, 2H), 7.46 (d, *J* = 7.9 Hz, 1H), 7.27 (ddd, *J* = 7.5, 4.8, 1.2 Hz, 1H), 6.61 (d, *J* = 8.8 Hz, 1H), 5.29 (h, *J* = 7.2 Hz, 1H), 1.59 (d, *J* = 7.0 Hz, 3H). |
| T-249 | 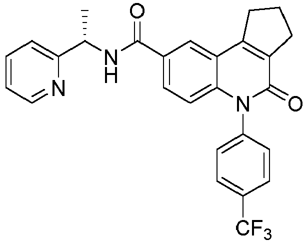 | ¹H NMR (400 MHz, chloroform-*d*) δ 8.55 (ddd, *J* = 4.8, 1.8, 0.9 Hz, 1H), 8.15 (d, *J* = 2.1 Hz, 1H), 7.95 - 7.81 (m, 3H), 7.80 - 7.66 (m, 2H), 7.46 - 7.38 (m, 2H), 7.32 (dt, *J* = 8.0, 1.1 Hz, 1H), 7.23 (ddd, *J* = 7.5, 4.9, 1.2 Hz, 1H), 6.65 (d, *J* = 8.8 Hz, 1H), 5.40 - 5.28 (m, 1H), 3.33 - 3.24 (m, 2H), 3.07 - 2.98 (m, 2H), 2.34 - 2.20 (m, 2H, 1.59 (d, *J* = 6.8 Hz, 3H). |
| T-250 | 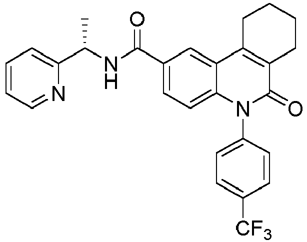 | ¹H NMR (400 MHz, chloroform-*d*) δ 8.57 - 8.53 (m, 1H), 8.36 (d, *J* = 2.0 Hz, 1H), 7.93 (s, 1H), 7.89 - 7.85 (m, 2H), 7.76 - 7.71 (m, 2H), 7.42 (d, *J* = 7.8 Hz, 2H), 7.35 (s, 1H), 7.15 (dd, *J* = 8.7, 3.3 Hz, 1H), 6.64 (d, *J* = 8.8 Hz, 1H), 5.35 (t, *J* = 6.9 Hz, 1H), 3.01 (d, *J* = 6.8 Hz, 2H), 2.68 (d, *J* = 6.8 Hz, 2H), 1.94 (d, *J* = 7.8 Hz, 2H), 1.89 - 1.83 (m, 2H). 1.60 (d, *J* = 6.6 Hz, 3H). |
| T-251 | 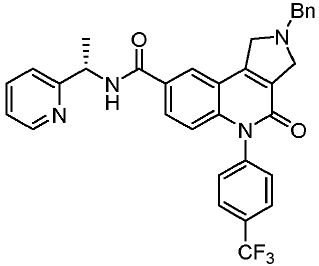 | ¹H NMR (400 MHz, chloroform-*d*) δ 8.52 (dt, *J* = 4.7, 1.4 Hz, 1H), 7.98 (d, *J* = 2.0 Hz, 1H), 7.86 (t, *J* = 8.0 Hz, 3H), 7.77 (dd, *J* = 8.9, 2.1 Hz, 1H), 7.69 (td, *J* = 7.7, 1.8 Hz, 1H), 7.46 - 7.36 (m, 6H), 7.34 - 7.27 (m, 3H), 7.23 - 7.20 (m, 1H), 6.66 (d, *J* = 8.8 Hz, 1H), 5.35 - 5.29 (m, 1H), 4.33 (t, *J* = 3.7 Hz, 2H), 4.13 (t, *J* = 3.7 Hz, 2H), 4.02 (s, 2H), 1.57 (d, *J* = 6.7 Hz, 3H). |
| T-252 | 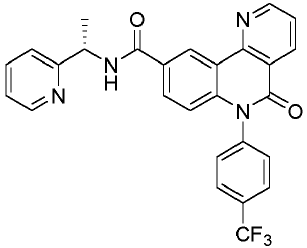 | ¹H NMR (400 MHz, chloroform-*d*) δ 9.37 (d, *J* = 2.2 Hz, 1H), 9.11 (dd, *J* = 4.6, 1.8 Hz, 1H), 8.76 (dd, *J* = 8.0, 1.9 Hz, 1H), 8.64 - 8.57 (m, 1H), 8.00 (dd, *J* = 8.8, 2.2 Hz, 1H), 7.91 (dd, *J* = 10.8, 7.6 Hz, 3H), 7.70 (td, *J* = 7.7, 1.8 Hz, 1H), 7.61 (dd, *J* = 8.0, 4.6 Hz, 1H), 7.51 (q, *J* = 5.8, 5.2 Hz, 2H), 7.34 (d, *J* = 7.8 Hz, 2H), 6.72 (d, *J* = 8.8 Hz, 1H), 5.40 (p, *J* = 6.9 Hz, 1H), 1.63 (d, *J=* 7.2 Hz, 3H). |
| T-253 | 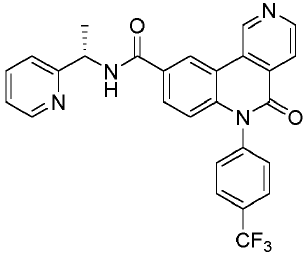 | ¹H NMR (400 MHz, chloroform-*d*) δ 9.90 (s, 1H), 9.02 (d, *J* = 2.0 Hz, 1H), 8.91 (d, *J* = 5.2 Hz, 1H), 8.57 (dt, *J* = 4.7, 1.4 Hz, 1H), 8.30 (dd, *J* = 5.2, 0.9 Hz, 1H), 8.05 (d, *J* = 7.1 Hz, 1H), 7.93 (d, *J* = 7.9 Hz, 2H), 7.85 (dd, *J* = 8.8, 2.0 Hz, 1H), 7.72 (td, *J* = 7.7, 1.8 Hz, 1H), 7.49 (d, *J* = 7.9 Hz, 2H), 7.35 - 7.32 (m, 1H), 6.73 (d, *J* = 8.8 Hz, 1H), 5.37 (p, *J* = 6.8 Hz, 1H), 1.62 (d, *J* = 6.7 Hz, 3H). |
| T-254 | 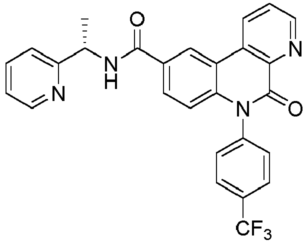 | ¹H NMR (400 MHz, chloroform-*d*) δ 9.02 (dd, *J* = 4.4, 1.5 Hz, 1H), 8.89 (d, *J* = 2.0 Hz, 1H), 8.81 (dd, *J* = 8.5, 1.6 Hz, 1H), 8.52 (ddd, *J* = 5.0, 1.8, 0.9 Hz, 1H), 8.08 (d, *J* = 7.2 Hz, 1H), 7.89 (d, *J* = 7.8 Hz, 2H), 7.86 - 7.73 (m, 2H), 7.70 (td, *J* = 7.7, 1.8 Hz, 1H), 7.52 - 7.46 (m, 2H), 7.33 (dt, *J* = 7.8, 1.1 Hz, 1H), 7.22 (ddd, *J* = 7.5, 4.9, 1.1 Hz, 1H), 6.67 (d, *J* = 8.7 Hz, 1H), 5.36 (p, *J* = 6.8 Hz, 1H), 1.59 (d, *J* = 6.8 Hz, 3H). |
| T-255 | 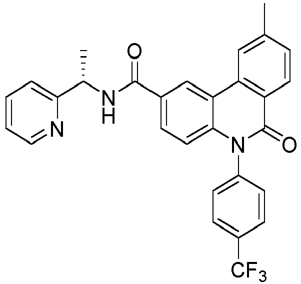 | ¹H NMR (400 MHz, chloroform-*d*) δ 8.92 (d, *J* = 2.0 Hz, 1H), 8.59 - 8.53 (m, 1H), 8.41 (d, *J* = 8.1 Hz, 1H), 8.28 (s, 1H), 7.92 (dd, *J* = 13.2, 7.4 Hz, 3H), 7.76 - 7.68 (m, 2H), 7.52 - 7.44 (m, 3H), 7.34 (d, *J* = 7.8 Hz, 1H), 7.23 (ddd, *J* = 7.5, 4.9, 1.2 Hz, 1H), 6.66 (d, *J* = 8.7 Hz, 1H), 5.38 (p, *J* = 6.8 Hz, 1H), 2.62 (s, 3H), 1.66 - 1.56 (m, 3H). |
| T-256 | 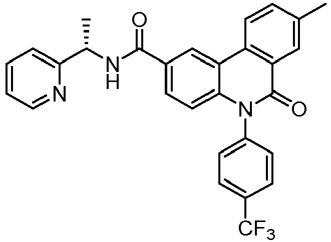 | ¹H NMR (400 MHz, chloroform-*d*) δ 8.88 (d, *J* = 2.0 Hz, 1H), 8.59 - 8.53 (m, 1H), 8.38 (d, *J* = 8.3 Hz, 1H), 8.33 (s, 1H), 7.93 (dd, *J* = 15.4, 7.4 Hz, 3H), 7.72 (ddt, *J* = 13.1, 8.1, 1.9 Hz, 3H), 7.49 (d, *J* = 7.8 Hz, 2H), 7.33 (d, *J* = 7.8 Hz, 1H), 7.23 (ddd, *J* = 7.6, 4.8, 1.1 Hz, 1H), 6.66 (d, *J* = 8.7 Hz, 1H), 5.37 (p, *J=* 6.8 Hz, 1H), 2.55 (s, 3H), 1.60 (s, 3H). |
| T-257 | 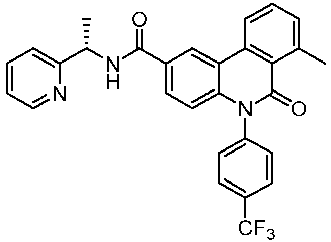 | ¹H NMR (400 MHz, ) δ 8.91 (d, *J* = 2.0 Hz, 1H), 8.60 - 8.53 (m, 1H), 8.38 (d, *J* = 8.2 Hz, 1H), 7.93 (dd, *J* = 17.0, 7.5 Hz, 3H), 7.72 (ddt, *J* = 10.3, 7.6, 1.9 Hz, 3H), 7.46 (dd, *J* = 12.5, 7.7 Hz, 3H), 7.33 (d, *J* = 7.8 Hz, 1H), 7.25 - 7.21 (m, 1H), 6.60 (d, *J* = 8.7 Hz, 1H), 5.37 (p, *J* = 6.8 Hz, 1H), 2.90 (s, 3H), 1.61 (d, *J* = 6.7 Hz, 3H). |
| T-258 | 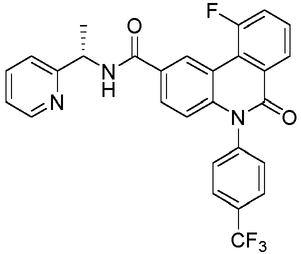 | ¹H NMR (400 MHz, chloroform-*d*) δ 9.32 (dd, *J* = 4.8, 2.0 Hz, 1H), 8.61 - 8.55 (m, 1H), 8.44 - 8.39 (m, 1H), 7.98 (d, *J* = 7.0 Hz, 1H), 7.92 (d, *J* = 8.2 Hz, 2H), 7.85 (dd, *J* = 8.8, 1.9 Hz, 1H), 7.71 (td, *J* = 7.7, 1.7 Hz, 1H), 7.65 - 7.60 (m, 2H), 7.49 (d, *J* = 8.1 Hz, 2H), 7.33 (d, *J* = 7.8 Hz, 1H), 7.24 - 7.21 (m, 1H), 5.36 (p, *J* = 6.8 Hz, 1H), 1.62 (s, 3H). |
| T-259 | 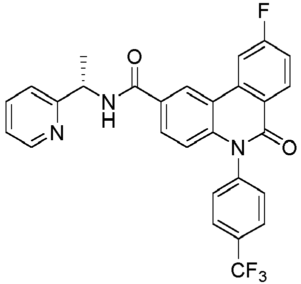 | ¹H NMR (400 MHz, chloroform-*d*) δ 8.80 (d, *J* = 2.0 Hz, 1H), 8.60 - 8.52 (m, 2H), 8.10 (dd, *J* = 10.2, 2.4 Hz, 1H), 7.98 (d, *J* = 7.1 Hz, 1H), 7.91 (d, *J* = 7.9 Hz, 2H), 7.80 (dd, *J=* 8.8, 2.0 Hz, 1H), 7.72 (td, *J* = 7.7, 1.8 Hz, 1H), 7.49 (d, *J* = 7.9 Hz, 2H), 7.41 - 7.30 (m, 2H), 7.25 - 7.22 (m, 1H), 6.69 (d, *J* = 8.7 Hz, 1H), 5.37 (p, *J* = 6.8 Hz, 1H), 1.64 - 1.58 (m, 3H). |
| T-260 | 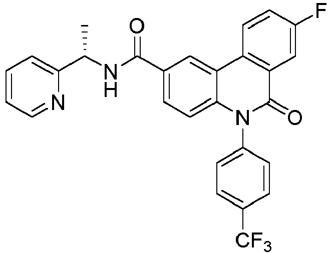 | ¹H NMR (400 MHz, chloroform-*d*) δ 8.86 (d, *J* = 2.0 Hz, 1H), 8.56 (dt, *J* = 4.8, 1.5 Hz, 1H), 8.49 (dd, *J* = 9.0, 4.8 Hz, 1H), 8.18 (dd, *J* = 8.9, 2.8 Hz, 1H), 7.97 (d, *J* = 7.0 Hz, 1H), 7.92 (d, *J* = 7.8 Hz, 2H), 7.79 - 7.67 (m, 2H), 7.60 (ddd, *J* = 8.9, 7.8, 2.9 Hz, 1H), 7.49 (d, *J* = 7.5 Hz, 2H), 7.33 (dd, *J* = 7.9, 1.1 Hz, 1H), 7.23 (ddd, *J* = 7.5, 4.9, 1.1 Hz, 1H), 6.69 (d, *J* = 8.7 Hz, 1H), 5.37 (p, *J* = 6.7 Hz, 1H), 1.61 (d, *J* = 6.7 Hz, 3H). |
| T-261 | 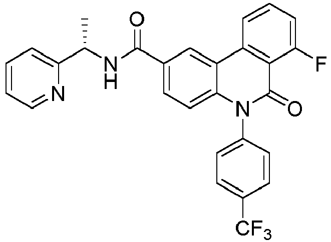 | ¹H NMR (400 MHz, chloroform-*d*) δ 8.89 (d, *J* = 1.9 Hz, 1H), 8.59 - 8.52 (m, 1H), 8.29 (d, *J* = 8.3 Hz, 1H), 7.97 (d, *J* = 7.1 Hz, 1H), 7.90 (d, *J* = 7.8 Hz, 2H), 7.83 (dt, *J* = 8.2, 4.1 Hz, 1H), 7.80 - 7.75 (m, 1H), 7.71 (td, *J* = 7.7, 1.8 Hz, 1H), 7.48 (d, *J* = 7.8 Hz, 2H), 7.37 - 7.28 (m, 2H), 7.23 (ddd, *J* = 7.6, 4.9, 1.1 Hz, 1H), 6.65 (d, *J* = 8.7 Hz, 1H), 5.36 (p, *J* = 6.8 Hz, 1H), 1.62 - 1.58 (m, 3H). |
| T-262 | 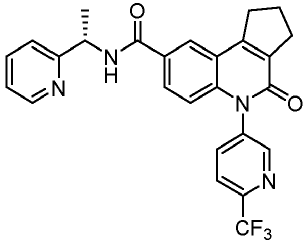 | ¹H NMR (400 MHz, chloroform-*d*) δ 8.68 (s, 1H), 8.55 (d, *J* = 5.2 Hz, 1H), 8.17 (d, *J* = 2.1 Hz, 1H), 7.98 - 7.93 (m, 1H), 7.92 - 7.85 (m, 3H), 7.79 (dd, *J* = 8.7, 2.0 Hz, 1H), 7.70 (ddt, *J* = 7.8, 3.3, 1.8 Hz, 2H), 7.59 - 7.51 (m, 1H), 7.31 (d, *J* = 7.9 Hz, 1H), 7.24 (d, *J* = 6.7 Hz, 1H), 7.17 (d, *J* = 8.5 Hz, 1H), 6.65 (d, *J* = 8.8 Hz, 1H), 5.34 (p, *J* = 6.8 Hz, 2H), 3.30 (t, *J* = 7.6 Hz, 2H), 3.03 (t, *J* = 7.3 Hz, 2H), 2.29 (p, *J* = 7.5 Hz, 2H), 1.63 - 1.55 (m, 3H). |
| T-263 | 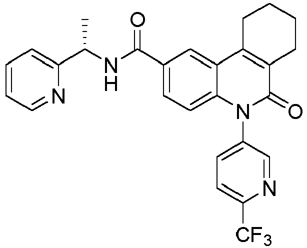 | ¹H NMR (400 MHz, chloroform-*d*) δ 8.67 (s, 1H), 8.56 (d, *J* = 4.9 Hz, 1H), 8.37 (d, *J* = 2.0 Hz, 1H), 7.95 (d, *J* = 8.2 Hz, 1H), 7.88 (d, *J* = 8.7 Hz, 2H), 7.76 (dd, *J* = 8.8, 2.0 Hz, 1H), 7.71 (td, *J* = 7.6, 1.8 Hz, 1H), 7.32 (d, *J* = 7.8 Hz, 1H), 7.24 - 7.21 (m, 1H), 6.62 (d, *J=* 8.7 Hz, 1H), 5.34 (p, *J* = 6.8 Hz, 1H), 3.02 (d, *J* = 6.8 Hz, 2H), 2.68 (d, *J* = 6.7 Hz, 2H), 1.95 (d, *J* = 5.9 Hz, 2H), 1.90 - 1.84 (m, 2H), 1.60 (d, *J* = 6.8 Hz, 3H). |
| T-264 | 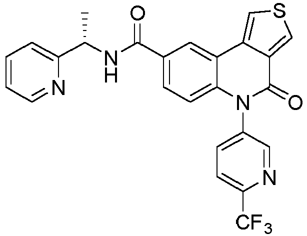 | ¹H NMR (400 MHz, DMSO-*d*₆) δ 8.92 (d, *J* = 7.8 Hz, 1H), 8.89 (d, *J=* 2.1 Hz, 1H), 8.82 (d, *J* = 2.0 Hz, 1H), 8.70 (d, *J* = 3.1 Hz, 1H), 8.60 (d, *J* = 3.1 Hz, 1H), 8.56 - 8.50 (m, 1H), 8.30 (d, *J* = 8.9 Hz, 1H), 8.24 (d, *J* = 8.3 Hz, 1H), 7.84 - 7.72 (m, 2H), 7.42 (d, *J* = 7.9 Hz, 1H), 7.26 (dd, *J* = 7.5, 4.9 Hz, 1H), 6.59 (d, *J* = 8.7 Hz, 1H), 5.25 (t, *J=* 7.3 Hz, 1H), 1.56 (d, *J* = 7.1 Hz, 3H). |
| T-266 | 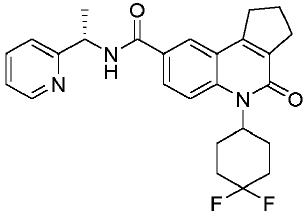 | ¹H NMR (400 MHz, chloroform-*d*) δ 8.62 - 8.56 (m, 1H), 8.10 (s, 1H), 7.93 (t, *J* = 10.1 Hz, 2H), 7.76 - 7.61 (m, 2H), 7.33 (d, *J* = 7.8 Hz, 2H), 5.36 (p, *J* = 6.7 Hz, 1H), 3.20 (t, *J* = 7.6 Hz, 2H), 2.97 (t, *J* = 7.5 Hz, 2H), 2.27 (d, *J* = 17.7 Hz, 1H), 2.29 - 2.23 (m, 2H), 2.23 - 2.17 (m, 2H), 2.17 - 1.95 (m, 4H), 1.83 (d, *J* = 12.9 Hz, 2H), 1.61 (d, *J* = 6.8 Hz, 3H). |
| T-267 | 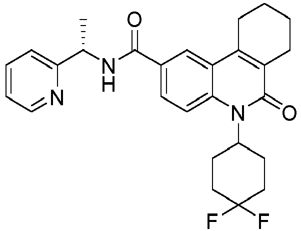 | ¹H NMR (400 MHz, chloroform-*d*) δ 8.62 - 8.56 (m, 1H), 8.31 (d, *J* = 2.1 Hz, 1H), 7.98 (s, 1H), 7.92 (dd, *J* = 8.9, 2.1 Hz, 1H), 7.74 (td, *J* = 7.7, 1.8 Hz, 1H), 7.61 (s, 1H), 7.35 (d, *J* = 7.9 Hz, 2H), 5.37 (p, *J* = 6.8 Hz, 1H), 2.92 (d, *J* = 6.6 Hz, 3H), 2.67 - 2.60 (m, 2H), 2.27 (d, *J* = 15.8 Hz, 2H), 2.12 - 1.77 (m, 10H), 1.61 (d, *J* = 6.7 Hz, 3H). |
| T-268 | 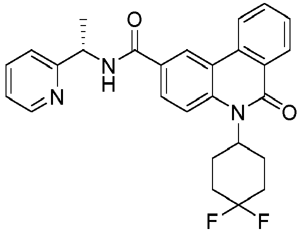 | ¹H NMR (400 MHz, chloroform-*d*) δ 8.90 (d, *J* = 2.1 Hz, 1H), 8.61 (d, *J* = 4.8 Hz, 1H), 8.50 (d, *J* = 8.0 Hz, 1H), 8.40 (d, *J* = 8.2 Hz, 1H), 8.07 (s, 1H), 7.97 (dd, *J* = 8.9, 2.1 Hz, 1H), 7.85 - 7.71 (m, 2H), 7.68 - 7.58 (m, 2H), 7.38 (d, *J* = 7.9 Hz, 2H), 5.41 (p, *J* = 6.9 Hz, 1H), 3.00 (s, 1H), 2.33 (s, 2H), 2.14 - 1.85 (m, 6H), 1.63 (s, 3H). |
| T-269 | 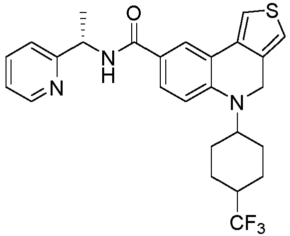 | ¹H NMR (400 MHz, DMSO-*d*₆) δ 8.94 (d, *J* = 7.7 Hz, 1H), 8.72 (d, *J* = 2.2 Hz, 1H), 8.57 - 8.50 (m, 2H), 8.44 (d, *J* = 3.1 Hz, 1H), 7.96 (m, *J* = 9.0, 2.2 Hz, 1H), 7.85 (d, *J* = 9.0 Hz, 1H), 7.77 (td, *J* = 7.7, 1.8 Hz, 1H), 7.44 (m, *J* = 8.0, 1.2 Hz, 1H), 7.27 m, *J* = 7.5, 4.8, 1.2 Hz, 1H), 5.26 (p, *J* = 7.2 Hz, 1H), 2.74 (d, *J* = 12.9 Hz, 1H), 2.69 (s, 1H), 2.01 (d, *J* = 12.2 Hz, 2H), 1.85 - 1.77 (m, 2H), 1.63 (m, *J* = 13.2, 7.1 Hz, 2H), 1.56 (d, *J* = 7.1 Hz, 2H). |
| T-270 | 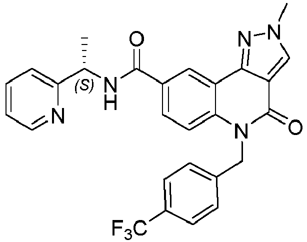 | ¹H NMR (400 MHz, DMSO-*d*₆) δ 9.14 (d, *J* = 7.8 Hz, 1H), 8.80 - 8.71 (m, 2H), 8.55 - 8.48 (m, 1H), 7.98 (dd, *J* = 8.9, 2.2 Hz, 1H), 7.74 (td, *J* = 7.7, 1.8 Hz, 1H), 7.68 (d, *J* = 8.0 Hz, 2H), 7.41 (dd, *J* = 8.4, 3.6 Hz, 4H), 7.24 (dd, *J* = 7.4, 5.0 Hz, 1H), 5.71 - 5.66 (m, 2H), 5.20 (p, *J* = 7.1 Hz, 1H), 4.16 (s, 3H), 1.47 (dd, *J* = 29.2, 7.1 Hz, 3H). |
| T-271 | 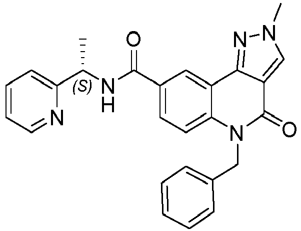 | ¹H NMR (400 MHz, DMSO-*d*₆) δ 9.14 (d, *J* = 7.7 Hz, 1H), 8.80 (d, *J* = 4.6 Hz, 2H), 8.58 (d, *J* = 4.8 Hz, 1H), 8.01 (d, *J* = 8.8 Hz, 1H), 7.81 (t, *J* = 7.8 Hz, 1H), 7.51 (d, *J* = 8.9 Hz, 1H), 7.46 (d, *J* = 7.9 Hz, 1H), 7.36 (t, *J* = 7.4 Hz, 2H), 7.28 (dd, *J* = 15.3, 7.4 Hz, 4H), 5.64 (s, 2H), 5.27 (p, *J* = 7.2 Hz, 1H), 4.22 (s, 3H), 1.58 (d, *J=* 7.1 Hz, 3H). |
| T-272 | 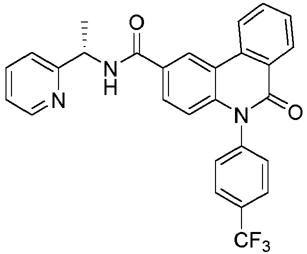 | ¹H NMR (400 MHz, DMSO-*d*₆) δ 9.19 (q, *J* = 3.9, 3.2 Hz, 2H), 8.83 (d, *J* = 8.2 Hz, 1H), 8.60 (dd, *J* = 5.0, 1.7 Hz, 1H), 8.43 (dd, *J* = 8.0, 1.4 Hz, 1H), 8.13 (s, 1H), 8.13 - 8.04 (m, 2H), 7.98 (dd, *J* = 8.8, 1.9 Hz, 1H), 7.87 - 7.75 (m, 4H), 7.50 (d, *J* = 7.9 Hz, 1H), 7.37 - 7.29 (m, 1H), 5.33 (p, *J* = 7.1 Hz, 1H), 1.64 (d, *J* = 7.1 Hz, 3H). |
| T-273 | 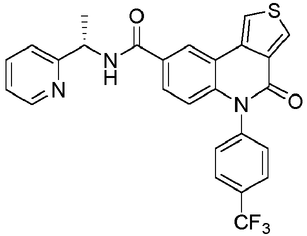 | ¹H NMR (400 MHz, DMSO-*d*₆) δ 8.94 (d, *J* = 7.6 Hz, 1H), 8.81 (s, 1H), 8.68 (s, 1H), 8.59 (s, 1H), 8.55 - 8.50 (m, 1H), 8.03 (d, *J* = 8.0 Hz, 2H), 7.83 - 7.72 (m, 2H), 7.69 (d, *J* = 7.9 Hz, 2H), 7.42 (d, *J* = 8.0 Hz, 1H), 7.26 (t, *J* = 6.4 Hz, 1H), 6.48 (d, *J* = 8.8 Hz, 1H), 5.27 - 5.19 (m, 1H), 1.55 (d, *J* = 7.0 Hz, 3H). |
| T-274 | 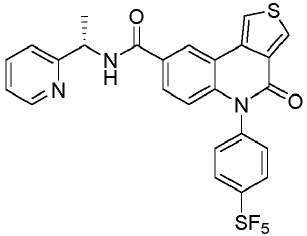 | LC-MS[M+1]: 552.61 |
| T-275 | 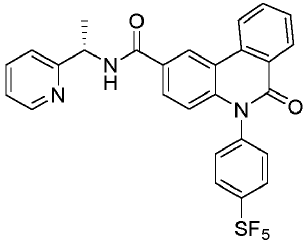 | LC-MS[M+1]: 488.32 |
| T-397 | 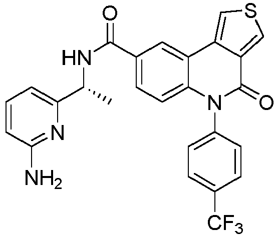 | ¹H NMR (400 MHz, chloroform-*d*) δ 8.62 (d, *J* = 2.0 Hz, 1H), 8.49 (d, *J* = 3.1 Hz, 1H), 8.10 (d, *J* = 3.2 Hz, 1H), 7.89 (d, *J* = 8.1 Hz, 2H), 7.79 (d, *J* = 7.1 Hz, 1H), 7.64 (dd, *J* = 8.8, 2.1 Hz, 1H), 7.46 (t, *J* = 8.8 Hz, 3H), 6.66 (d, *J* = 7.3 Hz, 1H), 6.55 (d, *J* = 8.8 Hz, 1H), 6.43 (d, *J* = 8.1 Hz, 1H), 5.21 (p, *J* = 6.8 Hz, 1H), 4.64 (s, 2H), 1.56 (d, *J* = 6.7 Hz, 3H). |
| T-398 | 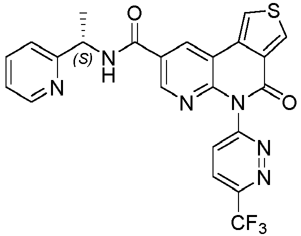 | ¹H NMR (400 MHz, chloroform-*d*) δ 8.62 (d, *J* = 2.1 Hz, 1H), 8.57 (d, *J* = 5.0 Hz, 1H), 8.52 (d, *J* = 3.1 Hz, 1H), 8.17 - 8.10 (m, 2H), 8.00 (d, *J* = 7.1 Hz, 1H), 7.92 (d, *J* = 8.7 Hz, 1H), 7.76 (t, *J* = 7.2 Hz, 1H), 7.69 (dd, *J* = 9.0, 2.1 Hz, 1H), 7.38 (d, *J* = 7.8 Hz, 1H), 6.52 (d, *J* = 8.7 Hz, 1H), 5.37 (t, *J* = 6.8 Hz, 1H), 1.62 (d, *J* = 6.8 Hz, 3H). |
| T-399 | 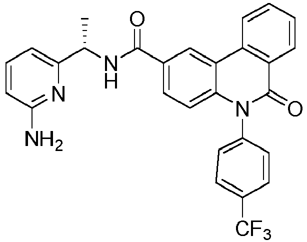 | ¹H NMR (400 MHz, DMSO-*d*₆) δ 9.10 (d, *J* = 2.0 Hz, 1H), 8.92 (d, *J* = 7.8 Hz, 1H), 8.77 (d, *J* = 8.3 Hz, 1H), 8.37 (dd, *J* = 8.1, 1.4 Hz, 1H), 8.09 - 8.00 (m, 3H), 7.92 (dd, *J* = 8.8, 1.9 Hz, 1H), 7.81 - 7.70 (m, 3H), 7.33 (dd, *J* = 8.2, 7.3 Hz, 1H), 6.62 (d, *J* = 8.8 Hz, 1H), 6.54 (d, *J* = 7.3 Hz, 1H), 6.30 (d, *J* = 8.0 Hz, 1H), 5.88 (s, 2H), 5.02 (p, *J=* 7.1 Hz, 1H), 1.50 (d, *J* = 7.1 Hz, 3H). |
| T-400 | 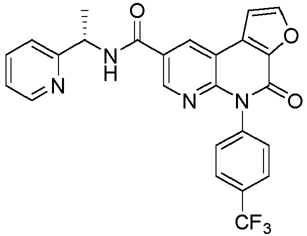 | ¹H NMR (400 MHz, DMSO-*d*₆) δ 9.14 - 9.07 (m, 2H), 8.84 (d, *J* = 2.3 Hz, 1H), 8.53 (ddd, *J* = 4.7, 1.8, 0.9 Hz, 1H), 8.43 (d, *J* = 2.0 Hz, 1H), 7.99 - 7.92 (m, 2H), 7.76 (td, *J* = 7.7, 1.9 Hz, 1H), 7.70 (d, *J* = 2.0 Hz, 1H), 7.68 - 7.61 (m, 2H), 7.46 (dt, *J* = 8.0, 1.2 Hz, 1H), 7.27 (ddd, *J* = 7.5, 4.8, 1.2 Hz, 1H), 5.24 (p, *J* = 7.1 Hz, 1H), 1.54 (d, *J* = 7.1 Hz, 3H). |
| T-401 | 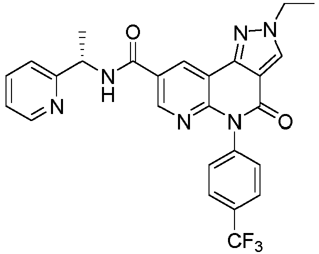 | ¹H NMR (400 MHz, chloroform-*d*) δ 8.99 (d, *J* = 2.3 Hz, 1H), 8.83 (d, *J* = 2.3 Hz, 1H), 8.50 (d, *J* = 5.1 Hz, 1H), 8.22 (s, 1H), 7.76 (d, *J* = 8.2 Hz, 3H), 7.35 (d, *J* = 8.1 Hz, 3H), 7.27 (s, 1H), 5.33 (q, *J* = 7.0 Hz, 1H), 4.39 (q, *J* = 7.4 Hz, 2H), 1.60 (s, 3H), 1.58 (s, 3H). |
| T-402 | 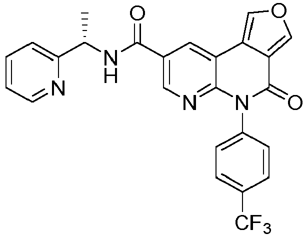 | ¹H NMR (400 MHz, chloroform-*d*) δ 8.96 (d, *J=* 7.4 Hz, 1H), 8.78 (d, *J* = 2.2 Hz, 1H), 8.74 - 8.69 (m, 2H), 8.53 (d, *J=* 2.2 Hz, 1H), 8.43 (dd, *J* = 5.0, 1.7 Hz, 1H), 7.77 (d, *J* = 8.3 Hz, 3H), 7.45 (d, *J* = 8.2 Hz, 2H), 7.41 (d, *J* = 7.9 Hz, 1H), 7.24 (dd, *J* = 7.5, 5.0 Hz, 1H), 5.09 (p, *J* = 7.1 Hz, 1H), 1.39 (d, *J=* 7.1 Hz, 3H). |
| T-403 | 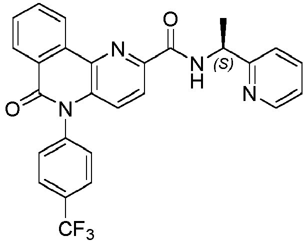 | ¹H NMR (400 MHz, DMSO-d6) δ 9.52 (d, J = 8.1 Hz, 1H), 9.26 (d, J = 8.1 Hz, 1H), 8.65 (d, J = 4.8 Hz, 1H), 8.38 (d, J = 8.0 Hz, 1H), 8.10 - 8.03 (m, 4H), 7.94 - 7.74 (m, 4H), 7.53 (d, J = 7.9 Hz, 1H), 7.35 (t, J = 6.5 Hz, 1H), 7.14 (d, J = 8.8 Hz, 1H), 5.32 (t, J = 7.6 Hz, 1H), 1.64 (dd, J = 7.0, 0.0 Hz, 3H). |
| T-404 | 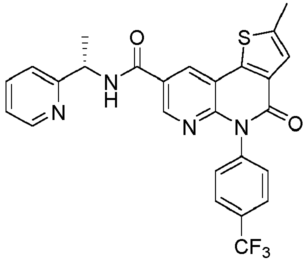 | ¹H NMR (400 MHz, DMSO-*d*₆) δ 9.17 (d, *J* = 7.7 Hz, 1H), 8.79 (q, *J* = 2.2 Hz, 2H), 8.54 (ddd, *J* = 4.8, 1.9, 0.9 Hz, 1H), 7.94 (d, *J* = 8.2 Hz, 2H), 7.77 (td, *J* = 7.7, 1.8 Hz, 1H), 7.63 (d, *J* = 8.1 Hz, 2H), 7.47 - 7.40 (m, 2H), 7.27 (ddd, *J* = 7.5, 4.8, 1.2 Hz, 1H), 5.24 (p, *J* = 7.1 Hz, 1H), 2.66 (d, *J* = 1.2 Hz, 3H), 1.55 (d, *J* = 7.1 Hz, 3H). |
| T-405 | 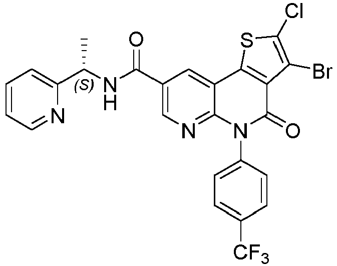 | ¹H NMR (400 MHz, DMSO-*d*₆) δ 9.14 (d, *J* = 7.7 Hz, 1H), 8.91 (dd, *J* = 4.8, 2.1 Hz, 2H), 8.56 - 8.50 (m, 1H), 7.96 (d, *J* = 8.2 Hz, 2H), 7.77 (td, *J* = 7.6, 1.8 Hz, 1H), 7.64 (d, *J* = 8.0 Hz, 2H), 7.44 (d, *J* = 7.9 Hz, 1H), 7.27 (dd, *J* = 7.5, 4.8 Hz, 1H), 5.23 (t, *J* = 7.3 Hz, 1H), 1.55 (d, *J* = 7.1 Hz, 3H). |
| T-406 | 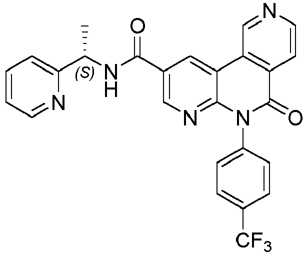 | ¹H NMR (400 MHz, chloroform-d) δ 9.85 (s, 1H), 9.28 (d, J = 2.1 Hz, 1H), 8.99 - 8.88 (m, 2H), 8.56 - 8.50 (m, 1H), 8.33 (dd, J = 5.2, 0.8 Hz, 1H), 8.24 (d, J = 6.9 Hz, 1H), 7.87 (d, J = 8.2 Hz, 2H), 7.72 (td, J = 7.7, 1.8 Hz, 1H), 7.45 (d, J = 8.1 Hz, 2H), 7.32 (d, J = 7.8 Hz, 1H), 7.24 (dd, J = 4.9, 1.1 Hz, 1H), 5.36 (p, J = 6.7 Hz, 1H), 1.61 (d, J = 6.7 Hz, 3H). |
| T-407 | 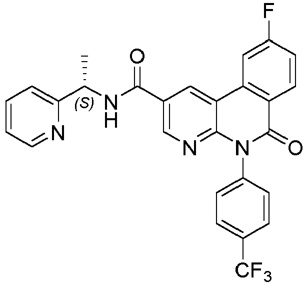 | ¹H NMR (400 MHz, DMSO-d6) δ 9.40 (d, J = 2.2 Hz, 1H), 9.08 (d, J = 7.7 Hz, 1H), 8.82 (d, J = 2.1 Hz, 1H), 8.60 (d, J = 2.5 Hz, 1H), 8.58 - 8.52 (m, 1H), 8.45 (dd, J = 8.9, 5.9 Hz, 1H), 7.95 (d, J = 8.3 Hz, 2H), 7.79 (td, J = 7.7, 1.9 Hz, 1H), 7.66 (td, J = 8.8, 2.3 Hz, 3H), 7.48 (d, J = 7.9 Hz, 1H), 7.29 (ddd, J = 7.6, 4.8, 1.2 Hz, 1H), 5.27 (p, J = 7.2 Hz, 1H), 1.58 (d, J = 7.0 Hz, 3H). |
| T-408 | 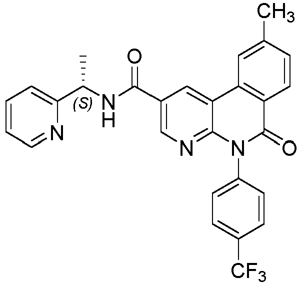 | ¹H NMR (400 MHz, DMSO-d6) δ 9.35 (d, J = 2.2 Hz, 1H), 9.15 (d, J = 7.7 Hz, 1H), 8.80 (d, J = 2.1 Hz, 1H), 8.55 (dd, J = 8.0, 2.4 Hz, 2H), 8.28 (d, J = 8.2 Hz, 1H), 7.94 (d, J = 8.3 Hz, 2H), 7.80 (td, J = 7.7, 1.9 Hz, 1H), 7.67 - 7.60 (m, 3H), 7.48 (d, J = 7.9 Hz, 1H), 7.34 - 7.26 (m, 1H), 5.27 (p, J = 7.2 Hz, 1H), 2.63 (s, 3H), 1.58 (d, J = 7.1 Hz, 3H). |
| T-409 | 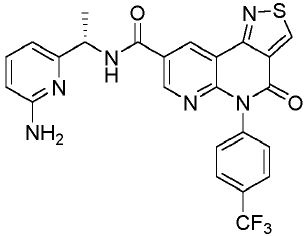 | LC-MS[M+1]: 511.0 |
| T-410 | 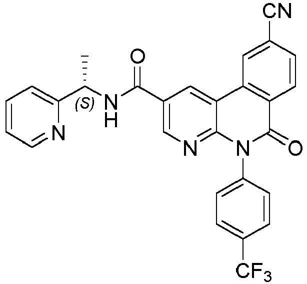 | ¹H NMR (400 MHz, chloroform-d) δ 9.16 (d, J = 2.2 Hz, 1H), 8.92 (d, J = 2.1 Hz, 1H), 8.76 (s, 1H), 8.67 (d, J = 8.2 Hz, 1H), 8.54 (d, J = 5.0 Hz, 1H), 8.26 (d, J = 6.9 Hz, 1H), 7.97 - 7.91 (m, 1H), 7.87 (d, J = 8.1 Hz, 2H), 7.80 - 7.71 (m, 1H), 7.45 (d, J = 8.1 Hz, 2H), 7.35 (d, J = 7.9 Hz, 1H), 7.28 (d, J = 6.2 Hz, 1H), 5.37 (p, J = 6.7 Hz, 1H), 1.62 (d, J = 6.7 Hz, 3H). |
| T-411 | 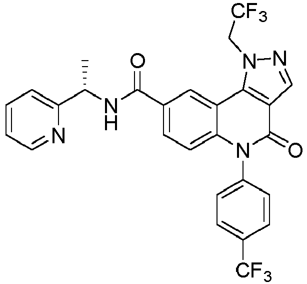 | ¹H NMR (400 MHz, chloroform-d) δ 8.73 (d, J = 2.2 Hz, 1H), 8.57 (ddd, J = 4.9, 1.8, 0.9 Hz, 1H), 8.39 (s, 1H), 7.91 (t, J = 8.2 Hz, 3H), 7.84 (dd, J = 8.9, 2.2 Hz, 1H), 7.70 (td, J = 7.7, 1.8 Hz, 1H), 7.46 (tdd, J = 9.9, 8.8, 3.2, 1.7 Hz, 2H), 7.33 (dt, J = 7.8, 1.1 Hz, 1H), 7.23 (ddd, J = 7.5, 4.9, 1.1 Hz, 1H), 6.61 (d, J = 8.8 Hz, 1H), 5.38 (h, J = 7.0 Hz, 1H), 4.98 (q, J = 8.1 Hz, 2H), 1.60 (d, J = 6.8 Hz, 3H). |
| T-419 | 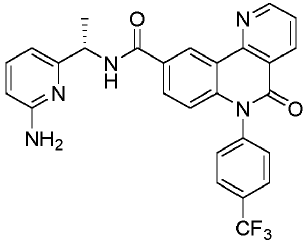 | LC-MS[M+1]: 504.1 |
| T-425 | 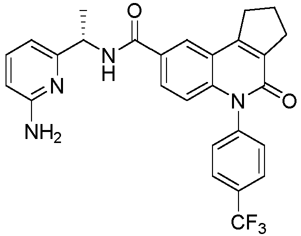 | LC-MS[M+1]: 493.1 |
| T-426 | 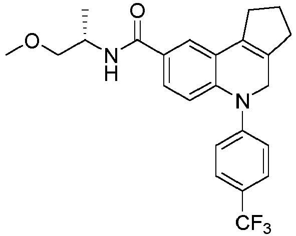 | LC-MS[M+1]: 431.2 |
| T-428 | 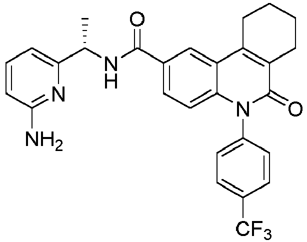 | LC-MS[M+1]: 507.1 |
| T-429 | 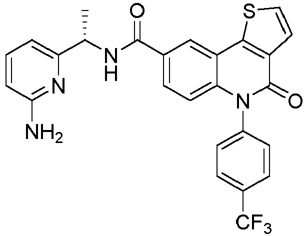 | LC-MS[M+1]: 509.1 |
| T-433 | 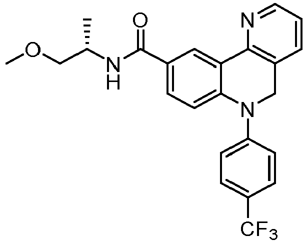 | LC-MS[M+1]: 442.2 |
| T-434 | 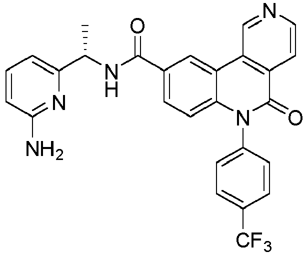 | LC-MS[M+1]: 504.1 |
| T-43 5 | 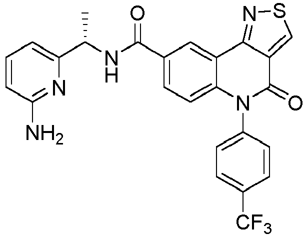 | LC-MS[M+1]: 510.1 |
| T-437 | 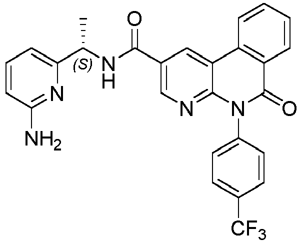 | LC-MS[M+1]: 504.1 |
| T-438 | 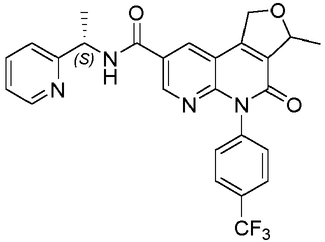 | ¹H NMR (400 MHz, DMSO-*d*₆) δ 9.05 (d, *J* = 7.8 Hz, 1H), 8.91 - 8.82 (m, 1H), 8.57 (d, *J=* 2.3 Hz, 1H), 8.55 - 8.48 (m, 1H), 7.94 (d, *J* = 8.5 Hz, 2H), 7.76 (td, *J* = 7.7, 1.8 Hz, 1H), 7.58 - 7.46 (m, 2H), 7.41 (d, *J* = 7.9 Hz, 1H), 7.27 (ddd, *J* = 7.6, 4.8, 1.2 Hz, 1H), 5.49 (dd, *J* = 14.2, 4.3 Hz, 1H), 5.42 - 5.30 (m, 2H), 5.21 (q, *J* = 7.3 Hz, 1H), 1.51 (dd, *J* = 15.5, 6.6 Hz, 3H), 1.50 (dd, 3H). |
| T-439 | 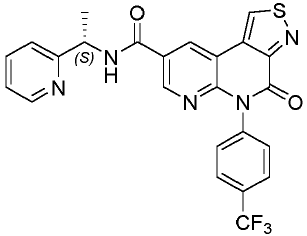 | LC-MS[M+1]: 495.4802 |
| T-445 | 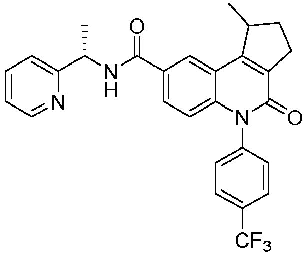 | ¹H NMR (400 MHz, chloroform-d) δ 8.58 - 8.48 (m, 1H), 8.24 (dd, J = 4.4, 2.0 Hz, 1H), 7.90 (dd, J = 16.9, 7.1 Hz, 3H), 7.78 - 7.66 (m, 2H), 7.43 (t, J = 11.0 Hz, 2H), 7.31 (d, J = 7.8 Hz, 1H), 7.22 (ddd, J = 7.6, 4.9, 1.2 Hz, 1H), 6.66 (d, J = 8.8 Hz, 1H), 5.33 (dq, J = 12.3, 6.5, 4.9 Hz, 1H), 3.77 (t, J = 7.5 Hz, 1H), 3.09 - 2.89 (m, 2H), 2.50 - 2.34 (m, 1H), 1.94 (dd, J = 12.4, 7.6 Hz, 1H), 1.58 (s, 3H), 1.41 (dd, J = 7.1, 2.7 Hz, 3H). |
| T-446 | 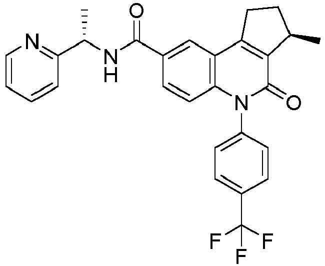 | ¹H NMR (400 MHz, chloroform-*d*) δ 8.48 (d, *J* = 4.8 Hz, 1H), 8.08 (d, *J* = 2.0 Hz, 1H), 7.81 (dd, *J* = 7.3, 4.2 Hz, 3H), 7.69 (dd, *J* = 8.8, 2.1 Hz, 1H), 7.63 (td, *J* = 7.7, 1.8 Hz, 1H), 7.42 - 7.32 (m, 2H), 7.25 (d, *J* = 7.8 Hz, 1H), 7.18 - 7.13 (m, 1H), 6.59 (d, *J* = 8.8 Hz, 1H), 5.27 (p, *J* = 6.9 Hz, 1H), 3.42 (d, *J* = 7.6 Hz, 1H), 3.23 (dt, *J* = 15.9, 7.6 Hz, 1H), 3.15 - 3.02 (m, 1H), 2.49 - 2.33 (m, 1H), 2.02 - 1.85 (m, 1H), 1.79 (ddt, *J* = 13.6, 9.3, 4.9 Hz, 1H), 1.30 (d, *J* = 7.0 Hz, 3H). |
| T-447 | 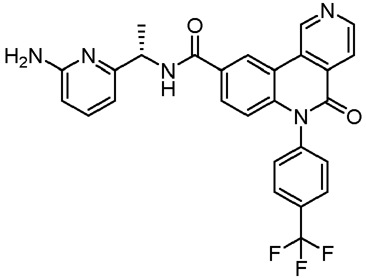 | ¹H NMR (400 MHz, DMSO-*d*₆) δ 10.15 (s, 1H), 9.29 (s, 1H), 8.91 (dd, *J* = 12.4, 6.5 Hz, 2H), 8.18 (d, *J* = 5.2 Hz, 1H), 8.08 (d, *J* = 8.1 Hz, 2H), 8.00 - 7.93 (m, 1H), 7.75 (d, *J* = 7.9 Hz, 2H), 7.33 (t, *J* = 7.8 Hz, 1H), 6.66 (d, *J* = 8.8 Hz, 1H), 6.55 (d, *J* = 7.3 Hz, 1H), 6.31 (d, *J* = 8.2 Hz, 1H), 5.88 (d, *J* = 6.4 Hz, 1H), 5.04 (q, *J* = 7.3 Hz, 1H), 1.51 (d, *J* = 7.1 Hz, 3H). |
| T-448 | 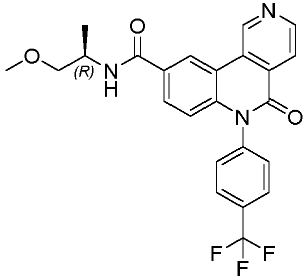 | ¹H NMR (400 MHz, DMSO-*d*₆) δ 10.11 (s, 1H), 9.19 (d, *J* = 2.0 Hz, 1H), 8.92 (d, *J* = 5.2 Hz, 1H), 8.46 (d, *J* = 8.0 Hz, 1H), 8.17 (dd, *J=* 5.3, 0.8 Hz, 1H), 8.07 (d, *J* = 8.3 Hz, 2H), 7.95 (dd, *J* = 8.8, 1.9 Hz, 1H), 7.74 (d, *J* = 8.1 Hz, 2H), 6.66 (d, *J* = 8.8 Hz, 1H), 4.27 (dt, *J* = 13.3, 6.6 Hz, 1H), 3.47 (dd, *J* = 9.5, 6.2 Hz, 2H), 3.30 (s, 3H), 1.22 (d, *J* = 6.8 Hz, 3H). |
| T-449 | 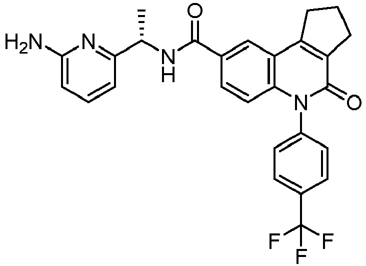 | ¹H NMR (400 MHz, DMSO-*d*₆) δ 8.78 (d, *J* = 7.9 Hz, 1H), 8.25 (d, *J* = 2.1 Hz, 1H), 8.02 (d, *J* = 8.2 Hz, 2H), 7.90 (dd, *J* = 8.9, 2.0 Hz, 1H), 7.62 (d, *J* = 8.0 Hz, 2H), 7.32 (t, *J* = 7.8 Hz, 1H), 6.59 (d, *J* = 8.8 Hz, 1H), 6.50 (d, *J* = 7.3 Hz, 1H), 6.30 (d, *J* = 8.2 Hz, 1H), 5.88 (s, 2H), 4.98 (p, *J* = 7.1 Hz, 1H), 2.87 (t, *J* = 7.5 Hz, 2H), 2.21 (q, *J* = 7.6 Hz, 2H), 1.46 (d, *J* = 7.0 Hz, 3H). |

### Example T-170

The compound synthesized by the invention:

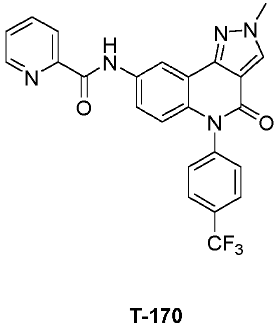

The synthetic route was as follows:

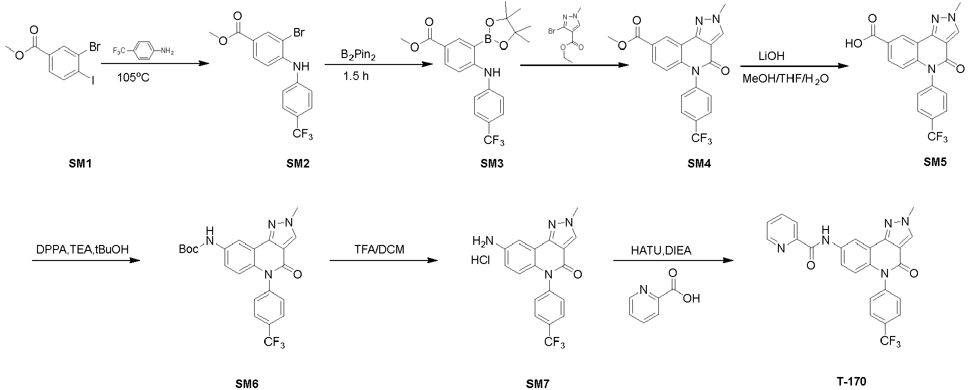

The experimental processes were as follows:

### (1) Synthesis of SM2:

SM1 50g (1.0eq), cesium carbonate 72g (1.5eq), Xantphos 22g (0.25eq), palladium acetate 1.64g (0.05eq) and 1L of 1,4-dioxane were mixed well, and 4-(trifluoromethyl)aniline 31g was added slowly. The mixture was vacuumed and exchanged with nitrogen and refluxed at 105 degree, and reacted for 16 hours. TLC showed that the raw material has completely reacted. The reaction was diluted with 1L of EA, then suction-filtered over diatomite, and the filtrate was added with silica gel and mixed and dried, then subjected to column chromatography to obtain 38 g SM2.

¹H NMR (400 MHz, chloroform-*d*) δ 8.25 (d, *J* = 1.9 Hz, 1H), 7.87 (dd, *J* = 8.6, 1.9 Hz, 1H), 7.64 - 7.58 (m, 2H), 7.29 (dd, *J* = 8.5, 5.7 Hz, 4H), 6.58 (s, 1H), 3.90 (s, 3H).

### (2) Synthesis of SM3:

SM2 24 g (1.0eq), B₂Pin₂ 21.5 g (1.3eq), KOAc 12.6 g (2.0eq), Pd(dppf)d₂ 2.4 g (0.05eq) and 1,4-dioxane 300 ml were mixed well. The mixture was protected with nitrogen, reacted under reflux at 105 °C for 1.5 h. The reaction solution was cooled to room temperature, added with 500 mL of EA, and then filtered through diatomite. Then the residue was mixed evenly under ultrasound with toluene: anhydrous ethanol = 5:8, and stirred for 5 minutes, and evaporated by rotary evaporation. This process was repeated until the rotary evaporation state turned solid, and then the solid was mixed with an appropriate amount of PE until there were solid precipitates. After suction filteration, the solid was collected and dried to obtain 20 g of SM3.

¹H NMR (400 MHz, DMSO-*d*₆) δ 8.47 (s, 1H), 8.22 (d, *J* = 2.2 Hz, 1H), 7.94 (dd, *J* = 8.7, 2.3 Hz, 1H), 7.65 (d, *J* = 8.4 Hz, 2H), 7.35 (t, *J* = 8.5 Hz, 2H), 3.85 - 3.71 (m, 3H), 3.36 (s, 12H).

### (3) Synthesis of SM4:

SM3 18 g (1.2eq), ethyl 3-bromo-1-methyl-1H-pyrazole-4-carboxylate 21.5 g (1.3eq), K₂CO₃ 15.0 g (2.0eq), Pd(dppf)d₂ 1.3 g (0.05eq) and 250 ml of 1,4-dioxane/water/anhydrous methanol at 5:2:1 were mixed well. The mixture was protected with nitrogen, reacted under reflux at 105 °C for 1.5 h. Then TLC showed the formation of the product. The reaction was cooled to room temperature, then filtered over diatomite, dried by rotary dryer, quenched with water, and extracted with EA, then subjected to column chromatography to obtain 9.5 g of SM4.

¹H NMR (400 MHz, DMSO-*d*₆) δ 8.73 (s, 1H), 8.68 (d, *J* = 2.1 Hz, 1H), 8.03 (d, *J* = 8.3 Hz, 2H), 7.89 (dd, *J* = 8.9, 2.1 Hz, 1H), 7.71 - 7.64 (m, 2H), 6.61 (d, *J* = 8.9 Hz, 1H), 4.14 (s, 3H), 3.87 (s, 3H).

### (4) Synthesis of SM5:

SM4 4.5 g (1.0eq), LiOH monohydrate 1.42 g (3.0eq) and 50 ml of THF/water/anhydrous methanol at 2:1:4 were mixed evenly. The mixture was protected with nitrogen, reacted at 50 °C for 2-5 h. Then TLC showed the formation of the product. The reaction was cooled to room temperature, dried by rotary dryer, quenched with water, and extracted with EA to remove organic impurities. The aqueous phase was adjusted to pH=2, and extracted with EA to obtain 3.6 g of SM5.

¹H NMR (400 MHz, Methanol-*d*₄) δ 8.90 (d, *J* = 2.0 Hz, 1H), 8.51 (s, 1H), 8.02 - 7.90 (m, 3H), 7.59 (d, *J* = 8.2 Hz, 2H), 6.68 (d, *J* = 8.9 Hz, 1H), 4.21 (s, 3H).

### (5) Synthesis of SM6:

SM5 1.0 g (1.0eq), TEA 0.4 g (1.5 eq) and 20 ml of t-BuOH were mixed evenly. The mixture was refluxed at 80 °C under nitrogen protection, added with 0.85 g (1.2eq) of DPPA dropwise, and reacted for 2 hours. TLC showed the formation of the product. The reaction was cooled to room temperature, dried by rotary dryer, quenched with water, extracted with EA, and subjected to column chromatography to obtain 2.3 g of SM6.

### (5) Synthesis of SM7:

2.3g of SM6, 23 ml of TEA and 30 ml of DCM were mixed evenly. The mixture was reacted overnight at ordinary temperature under nitrogen protection. The reaction solution was dried by rotary dryer, quenched with water, extracted with DCM, and subjected to column chromatography to obtain 280 mg of SM7.

¹H NMR (400 MHz, Methanol-*d*₄) δ 8.40 (s, 1H), 7.93 (d, *J* = 8.2 Hz, 2H), 7.58 (t, *J* = 7.9 Hz, 1H), 7.54 - 7.48 (m, 4H), 7.22 - 7.12 (m, 1H), 4.16 (s, 3H).

### (7) Synthesis of T-170:

2-pyridinecarboxylic acid (147.6mg, 1.2eq), HATU (465.6mg, 1.2eq), DIPEA (490mg, 4.0eq) and 2ml of anhydrous dichloromethane were mixed evenly and the mixture was reacted at room temperature for 20min, then compound SM7 (394mg, 1.0eq) was added to the reaction solution and the reaction solution was reacted at ordinary temperature for 18 h. TLC showed that the raw material has disappeared, and LCMS was correct. The reaction solution was spin-dried, quenched by adding water, and extracted with EA. The EA phase was washed once with 0.5% citric acid and dried over anhydrous sodium sulfate. The EA phase was separated and purified by preparative plate to obtain product T-170.

¹H NMR (400 MHz, Methanol-*d*₄) δ 8.96 (d, 1H), 8.74 (d, *J* = 2.5 Hz, 1H), 8.68 (s, 1H), 8.18 - 8.16 (d, H), 8.10-8.06 (m, 1H), 8.01 - 7.99 (d, 1H), 7.76 - 7.60 (m, 4H), 4.14 (s, 3H).

### Example T-172

The compound synthesized by the invention:

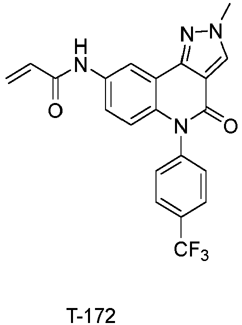

The synthetic route was as follows:

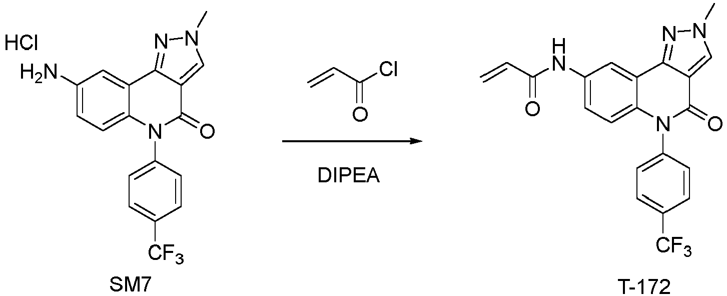

The experimental process was as follows:

### Synthesis of T-172:

SM7 (394mg, 1.2eq), DIPEA (490mg, 4.0eq) and 2ml of anhydrous dichloromethane were mixed evenly and the mixture was reacted at room temperature for 20min; then compound acryloyl chloride (108mg, 1.2eq) was added to the reaction solution and the reaction solution was reacted at room temperature for 18 h. TLC showed that the raw material had disappeared, and LCMS was correct. The reaction solution was spin-dried, quenched by adding water, and extracted with EA. The EA phase was washed once with 0.5% citric acid and dried over anhydrous sodium sulfate. The EA phase was separated and purified by preparative liquid chromatography to obtain product T-172.

¹H NMR (400 MHz, DMSO-d6) δ 10.35 (s, 1H), 8.73 (d, J = 2.5 Hz, 1H), 8.68 (s, 1H), 8.00 (d, J = 8.3 Hz, 2H), 7.62 (d, J = 8.1 Hz, 2H), 7.45 (dd, J = 9.1, 2.5 Hz, 1H), 6.51 - 6.36 (m, 2H), 6.27 (dd, J = 17.0, 2.1 Hz, 1H), 5.77 (dd, J = 10.0, 2.1 Hz, 1H), 4.13 (s, 3H).

### Example T-186

The compound synthesized by the invention:

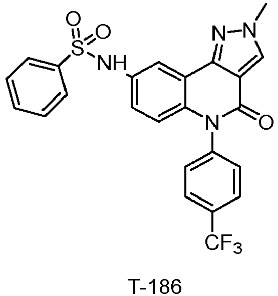

The synthetic route was as follows:

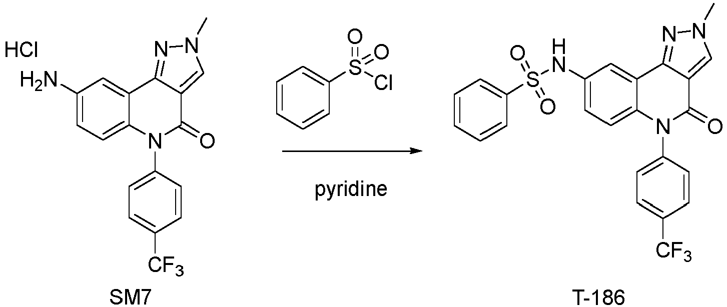

The experimental process was as follows:

### Synthesis of T-186:

SM7 (394mg, 1.0eq), pyridine (237mg, 3.0eq), benzenesulfonyl chloride (352mg, 2.0eq) and 2ml of solvent anhydrous dichloromethane were mixed evenly, and the mixture was reacted at ordinary temperature for 18 hours. TLC showed that the raw material had disappeared, and LCMS was correct. The reaction solution was spin-dried, quenched by adding water, and extracted with EA. The EA phase was washed once with 0.5% citric acid and dried over anhydrous sodium sulfate. The EA phase was separated and purified by preparative plate to obtain product T-186.

¹H NMR (400 MHz, Methanol-*d*₄) δ 8.39 (s, 1H), 8.00 - 7.87 (m, 5H), 7.87 - 7.69 (m, 3H), 7.60 - 7.36 (m, 1H), 7.08 (dd, *J* = 9.1, 2.6 Hz, 1H), 6.44 (d, *J* = 9.1 Hz, 1H), 5.50 (s, 1H), 4.15 (s, 3H).

The following compounds were synthesized with reference to the synthesis method in examples T-170, T-172 & T-186:

| Example | Compound structure | Compound characterization |
|---|---|---|
| T-168 | 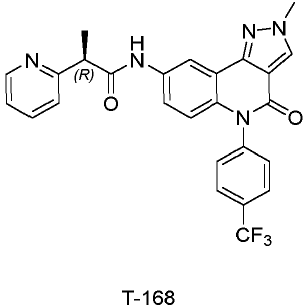 | ¹H NMR (400 MHz, Methanol-*d*₄) δ 8.14 (s, 1H), 8.06 (d, *J* = 2.5 Hz, 1H), 7.92 - 7.82 (m, 2H), 7.58 - 7.42 (m, 2H), 7.34 - 7.26 (m, 2H), 7.25 - 6.98 (m, 4H), 6.12 (d, *J* = 9.1 Hz, 1H), 3.97 (s, 3H), 3.75 - 3.49 (m, 1H), 1.37 (dd, *J* = 28.6, 7.0 Hz, 3H) |
| T-169 | 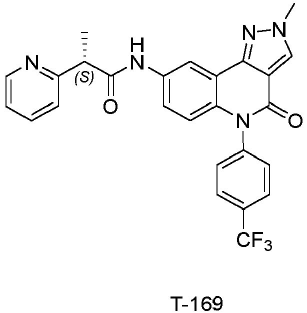 | ¹H NMR (400 MHz, Methanol-*d*₄) δ 8.19 (s, 1H), 8.11 (d, *J* = 2.5 Hz, 1H), 7.97 (m, *J* = 13.1, 8.3, 2.2 Hz, 2H), 7.69 - 7.51 (m, 2H), 7.47 - 7.35 (m, 2H), 7.32 - 7.13 (m, 4H), 6.16 (d, *J* = 9.1 Hz, 1H), 4.04 (s, 3H), 3.69 (m, *J* = 7.1 Hz, 1H), 1.50 (d, *J* = 7.0 Hz, 3H). |
| T-171 | 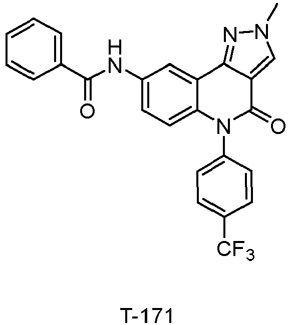 | ¹H NMR (400 MHz, DMSO-*d*₆) δ 10.44 (s, 1H), 8.80 (d, *J* = 2.5 Hz, 1H), 8.68 (s, 1H), 8.04 - 7.96 (m, 4H), 7.72 - 7.50 (m, 6H), 6.50 (d, *J* = 9.1 Hz, 1H), 4.14 (s, 3H). |
| T-173 | 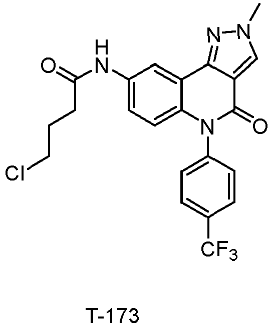 | ¹H NMR (400 MHz, DMSO-*d*₆) δ 10.16 (s, 1H), 8.70 - 8.62 (m, 2H), 7.99 (d, *J* = 8.2 Hz, 2H), 7.61 (d, *J* = 8.0 Hz, 2H), 7.37 (dd, *J* = 9.1, 2.6 Hz, 1H), 6.44 (d, *J* = 9.0 Hz, 1H), 4.12 (d, *J* = 3.4 Hz, 3H), 3.69 (dt, *J* = 17.3, 6.5 Hz, 2H), 2.49 - 2.32 (m, 2H), 2.08 - 1.86 (m, 2H), 1.22 (s, 1H). |
| T-174 | 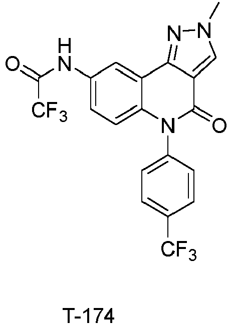 | ¹H NMR (400 MHz, DMSO-*d*₆) δ 11.43 (s, 1H), 8.68 (s, 1H), 8.61 (d, *J* = 2.5 Hz, 1H), 8.00 (d, *J* = 8.2 Hz, 2H), 7.63 (d, *J* = 8.1 Hz, 2H), 7.55 (dd, *J* = 9.1, 2.6 Hz, 1H), 6.53 (d, *J* = 9.1 Hz, 1H), 4.13 (s, 3H). |
| T-175 | 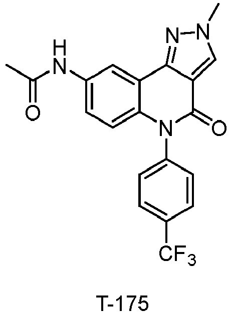 | ¹H NMR (400 MHz, chloroform-*d*) δ 8.20 (d, *J* = 2.0 Hz, 2H), 7.87 (d, *J* = 8.2 Hz, 2H), 7.59 (dd, *J* = 9.1, 2.5 Hz, 1H), 7.45 (d, *J* = 8.1 Hz, 2H), 7.23 (s, 1H), 7.00 (s, 1H), 6.54 (d, *J* = 9.1 Hz, 1H), 4.17 (s, 3H), 2.21 (s, 3H). |
| T-176 | 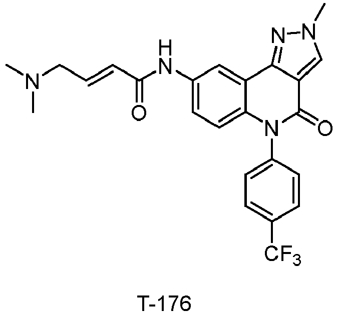 | LC-MS[M+1]: 470 |
| T-177 | 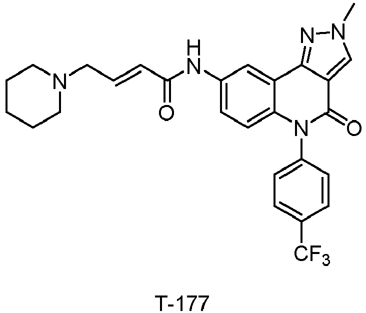 | LC-MS[M+1]: 510 |
| T-178 | 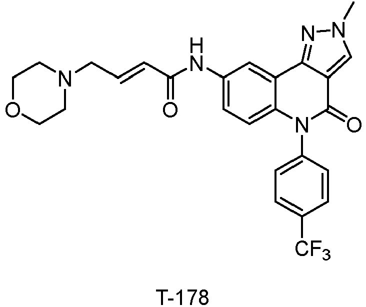 | LC-MS[M+1]: 512 |
| T-179 | 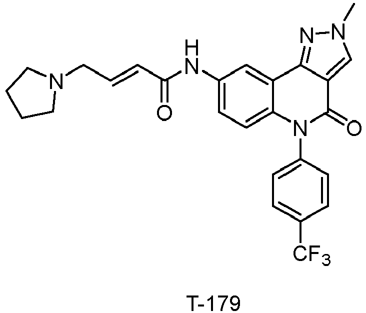 | LC-MS[M+1]: 496 |
| T-180 | 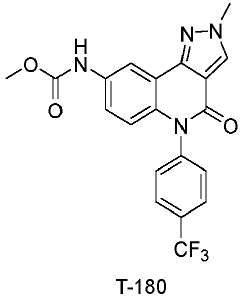 | LC-MS[M+1]: 417 |
| T-181 | 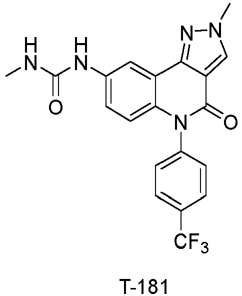 | LC-MS[M+1]: 416 |
| T-182 | 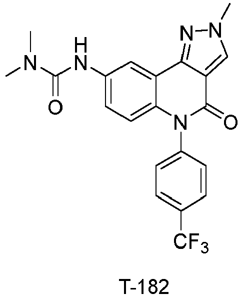 | LC-MS[M+1]: 430 |
| T-183 | 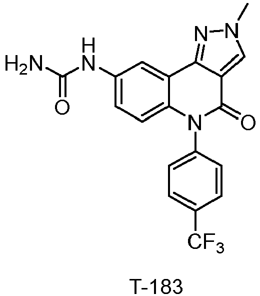 | LC-MS[M+1]: 402 |
| T-184 | 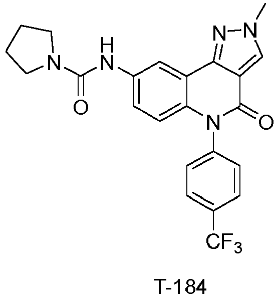 | LC-MS[M+1]: 456 |
| T-185 | 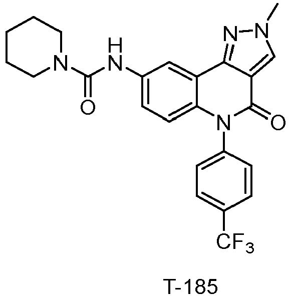 | LC-MS[M+1]: 470 |
| T-187 | 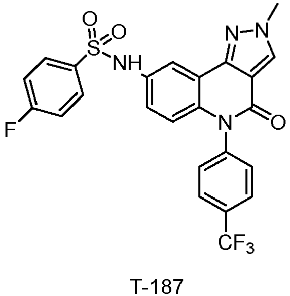 | ¹H NMR (400 MHz, DMSO-*d*₆) δ 10.48 (s, 1H), 8.66 (s, 1H), 7.97 (d, *J* = 8.3 Hz, 2H), 7.88 (d, *J* = 2.6 Hz, 1H), 7.86 - 7.76 (m, 2H), 7.58 (d, *J* = 8.1 Hz, 2H), 7.45 - 7.34 (m, 2H), 7.10 (dd, *J* = 9.1, 2.6 Hz, 1H), 6.40 (d, *J* = 9.1 Hz, 1H), 4.11 (s, 3H). |
| T-188 | 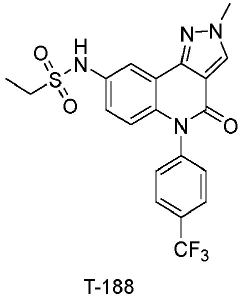 | LC-MS[M+1]: 451 |
| T-189 | 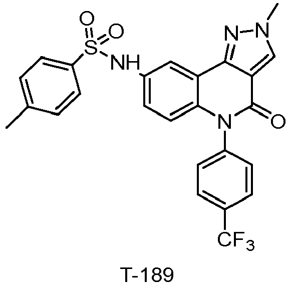 | ¹H NMR (400 MHz, DMSO-*d*₆) δ 10.37 (s, 1H), 8.64 (s, 1H), 7.96 (d, *J* = 8.4 Hz, 2H), 7.89 (d, *J* = 2.6 Hz, 1H), 7.68 - 7.61 (m, 2H), 7.60 - 7.53 (m, 2H), 7.41 - 7.29 (m, 2H), 7.10 (dd, *J* = 9.1, 2.6 Hz, 1H), 6.38 (d, *J* = 9.1 Hz, 1H), 4.11 (s, 3H), 2.30 (s, 3H). |
| T-190 | 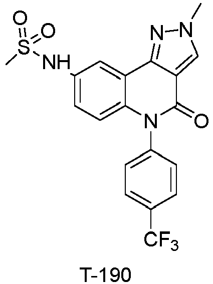 | LC-MS[M+1]: 437 |
| T-191 | 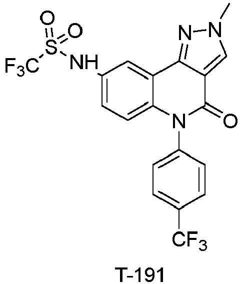 | LC-MS[M+1]: 491 |
| T-192 | 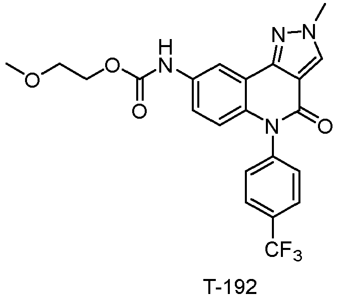 | LC-MS[M+1]: 461 |
| T-193 | 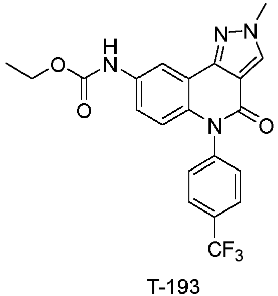 | LC-MS[M+1]: 431 |
| T-194 | 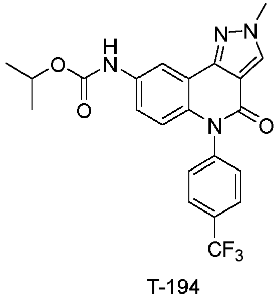 | LC-MS[M+1]: 445 |
| T-240 | 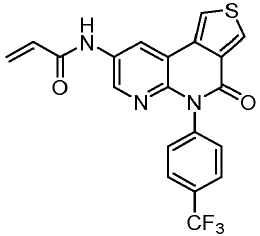 | ¹H NMR (400 MHz, DMSO-*d*₆) δ 10.51 (s, 1H), 8.92 (d, *J* = 2.4 Hz, 1H), 8.69 (d, *J* = 3.0 Hz, 1H), 8.56 (d, *J* = 3.1 Hz, 1H), 8.33 (d*, J* = 2.4 Hz, 1H), 7.92 (d, *J* = 8.3 Hz, 2H), 7.60 (d, *J* = 8.1 Hz, 2H), 6.47 (dd, *J* = 16.9, 10.1 Hz, 1H), 6.32 (dd, *J* = 17.0, 2.1 Hz, 1H), 5.83 (dd, *J* = 10.0, 2.1 Hz, 1H). |
| T-241 | 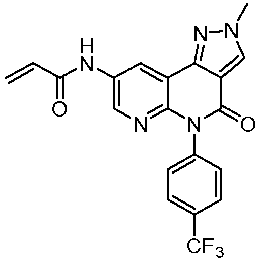 | ¹H NMR (400 MHz, DMSO-*d*₆) δ 10.48 (s, 1H), 8.97 (d, *J* = 2.6 Hz, 1H), 8.67 (s, 1H), 8.34 (d, *J* = 2.5 Hz, 1H), 7.84 (d, *J* = 8.0 Hz, 2H), 7.50 (d, *J* = 8.0 Hz, 2H), 6.38 (dd, *J* = 16.9, 10.0 Hz, 1H), 6.25 (d, *J* = 16.8 Hz, 1H), 5.76 (d, *J* = 10.2 Hz, 1H). |
| T-242 | 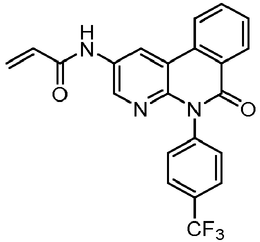 | ¹H NMR (400 MHz, DMSO-*d*₆) δ 10.57 (s, 1H), 9.24 (d, *J* = 2.4 Hz, 1H), 8.50 - 8.35 (m, 3H), 8.04 - 7.91 (m, 3H), 7.78 (t, *J* = 7.6 Hz, 1H), 7.64 (d, *J* = 8.1 Hz, 2H), 6.48 (dd, *J* = 17.0, 10.0 Hz, 1H), 6.34 (dd, *J* = 16.9, 2.1 Hz, 1H), 5.88 - 5.81 (m, 1H). |
| T-418 | 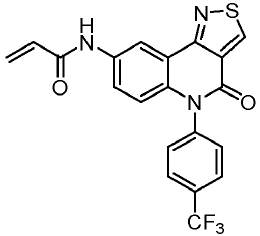 | LC-MS[M+1]: 416.0 |
| T-424 | 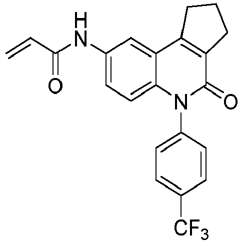 | LC-MS[M+1]: 399.1 |
| T-432 | 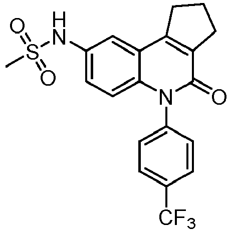 | LC-MS[M+1]: 423.1 |

### Example T-243

The compound synthesized by the invention:

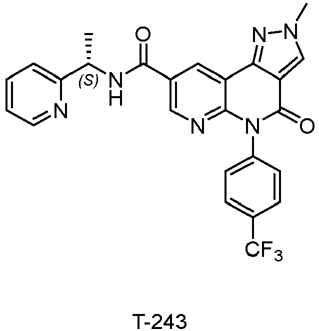

The synthetic route was as follows:

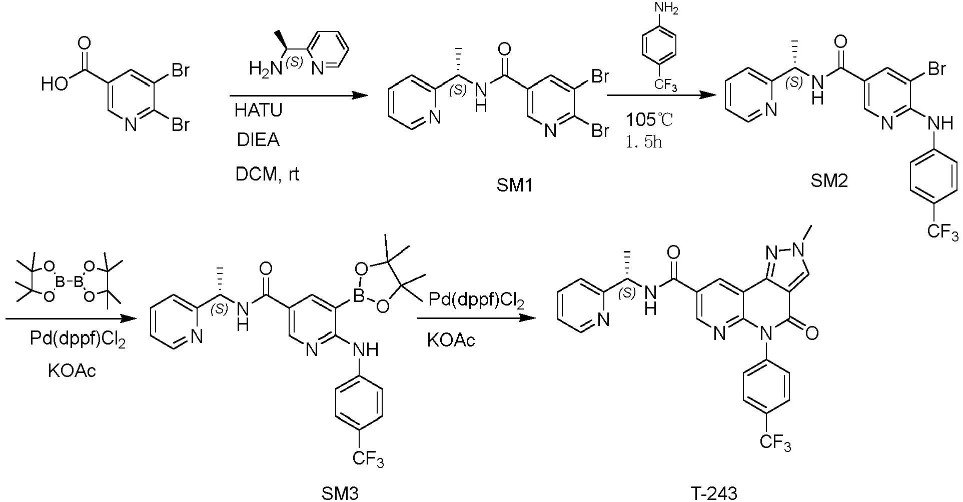

### (1) Synthesis of SM1

25g of 5,6-dibromonicotinic acid (1eq), 23.13g of DIPEA (2eq, when d is hydrochloride, 3eq) and 34.083g of HATU (1eq) were dissolved in DCM (250ml). The mixture was stirred at rt for 10min, and then added with 13.14g of compound pyridine ethylamine (1.2eq), and then continued to react for 1.5h. After the reaction was completed, TLC (pure EA) was used for monitoring, and indicated that 5,6-dibromonicotinic acid has completely reacted. The reaction was diluted by adding 5 times of DCM, washed with 0.1% hydrochloric acid to remove DIPEA, washed with saturated NaCl solution, then dried. The residue was mixed with 2~2.5 times of silica gel and passed through column chromatography with PE:EA=3:1 to produce 30.7g of SM1 as yellow viscous liquid.

### (2) Synthesis of SM2

30.7g of SM1 (1eq), 52.2g of Cs₂CO₃ (2eq), 4.6g of Xantphos (0.1eq) and 12.9g of p-fluoroaniline (1eq) were dissolved in the ultra-dry 1,4-dioxane solution (300ml), and added into a round bottom flask. Then the mixture was added with 0.9g of Pd(OAc)₂ ( 0.05eq) and replaced with nitrogen for 2-3 times, warmed to 105 °C and reacted for 1.5 h. After the reaction was completed, TLC (PE:EA = 1:1) was used for monitoring, and indicated that the raw material has completely reacted. The reaction was diluted with ten times of EA, suction-filtered over diatomite, mixed with silica gel and dried, and separated by chromatography column with PE:EA=2:1 to obtain the product, 15.2g of SM2 as yellow solid.

### (3) Synthesis of SM2

13.2g of SM2 (1eq), 14.5g of Bis(pinacolato)diboron (2eq), and 1.04g of Pd (dppf)Cl₂ (0.05eq) were dissolved in 132ml of 1,4-dioxane solution successively, and the mixture was stirred in a round bottom flask. Then the mixture was added with 5.6g of KOAc (2eq) and replaced with nitrogen for 2-3 times, warmed to 105 °C in an oil bath and reacted for 3 h. After the reaction was completed, TLC (PE:EA = 1:1.5) was used for monitoring, and indicated that the raw material has completely reacted. Fumigating with chromogen alizarin, SM3 appeared yellow. The reaction was diluted with ten times of EA, suction-filtered over diatomite, and spin-dried to remove solvent. The residue was added with a solution of toluene and ethanol at 5:8, stirred for 5 minutes, and the solvent was spin dried. The previous step was repeated and the solvent was spin-dried. Then a small amount of PE was added, the mixture was shook thoroughly, and suction-filtered to obtain 13.1g of SM3 as gray-brown solid.

### (4) Synthesis of TM

To a round-bottomed flask was added a solution of SM3 (1eq) in 1,4-dioxane solution and then K₂CO₃ (3eq), Pd(dppf)Cl₂ (0.16eq) and ethyl 4-bromo-1-methylpyrazole-3-carboxylate (1eq) successively. The mixture was replaced with nitrogen 2 to 3 times, warmed to 105°C, and reacted for 1 hour. After the reaction was completed, TLC (PE:EA = 3:1) was used for monitoring, and indicated that SM3 has completely reacted and the product had a relative high polarity. TLC (PE:EA=1:1) was used for monitoring, and indicated that SM4 completely crawled away from the far point. The reaction solution was suction-filtered, added with EA, mixed with silica gel and dried, and then subjected to chromatography column (PE:EA=1: 1) to separate the pass-position product point as beige brown solid (24mg).

The following compounds were synthesized with reference to the synthesis method in example T-243:

| Example | Compound structure | Compound characterization |
|---|---|---|
| T-244 | 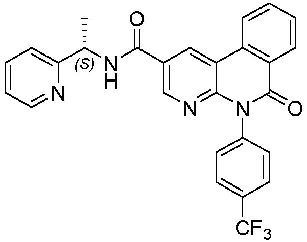 | ¹H NMR (400 MHz, DMSO-*d*₆) δ 9.45 (d, *J* = 2.2 Hz, 1H), 9.18 (d, *J* = 7.7 Hz, 1H), 8.84 (d, *J* = 2.1 Hz, 1H), 8.78 (d, *J* = 8.2 Hz, 1H), 8.61 - 8.55 (m, 1H), 8.43 (dd, *J* = 8.0, 1.4 Hz, 1H), 8.13 - 8.04 (m, 1H), 7.99 (d, *J* = 8.2 Hz, 2H), 7.89 - 7.76 (m, 2H), 7.69 (d, *J* = 8.1 Hz, 2H), 7.50 (d, *J* = 7.9 Hz, 1H), 7.35 - 7.27 (m, 1H), 5.38 - 5.26 (m, 1H), 1.61 (d, *J* = 7.1 Hz, 3H). |
| T-245 | 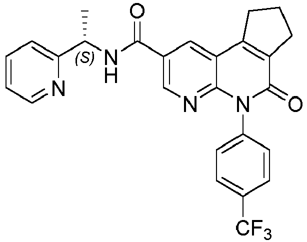 | ¹H NMR (400 MHz, DMSO-*d*₆) δ 9.45 (d, *J* = 2.2 Hz, 1H), 9.18 (d, *J* = 7.7 Hz, 1H), 8.84 (d, *J* = 2.1 Hz, 1H), 8.78 (d, *J* = 8.2 Hz, 1H), 8.61 - 8.55 (m, 1H), 8.43 (dd, *J* = 8.0, 1.4 Hz, 1H), 8.13 - 8.04 (m, 1H), 7.99 (d, *J* = 8.2 Hz, 2H), 7.89 - 7.76 (m, 2H), 7.69 (d, *J* = 8.1 Hz, 2H), 7.50 (d, *J* = 7.9 Hz, 1H), 7.35 - 7.27 (m, 1H), 5.38 - 5.26 (m, 1H), 1.61 (d, *J* = 7.1 Hz, 3H). |
| T-246 | 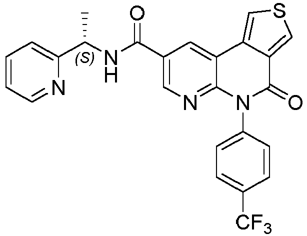 | ¹H NMR (400 MHz, chloroform-*d*) δ 8.84 (d, *J* = 2.2 Hz, 1H), 8.76 (d, *J* = 2.3 Hz, 1H), 8.53 (dd, *J* = 7.8, 3.8 Hz, 2H), 8.13 - 8.08 (m, 2H), 7.85 (d, *J* = 8.1 Hz, 2H), 7.71 (td, *J* = 7.7, 1.8 Hz, 1H), 7.44 (d, *J* = 8.1 Hz, 2H), 7.30 (d, *J* = 7.8 Hz, 1H), 7.23 (ddd, *J* = 7.6, 4.9, 1.1 Hz, 1H), 5.34 (q, *J* = 6.8 Hz, 1H), 1.59 (d, *J* = 6.7 Hz, 3H). |
| T-247 | 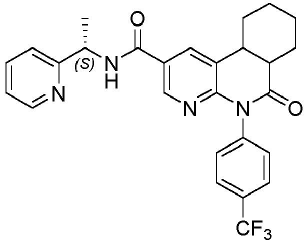 | ¹H NMR (400 MHz, chloroform-*d*) δ 8.82 (d, *J* = 2.2 Hz, 1H), 8.61 (d, *J* = 2.2 Hz, 1H), 8.52 (dd, *J* = 4.6, 1.6 Hz, 1H), 8.08 (t, *J* = 6.4 Hz, 1H), 7.83 (d, *J* = 8.1 Hz, 1H), 7.71 (td, *J* = 7.7, 1.8 Hz, 1H), 7.40 (d, *J* = 8.0 Hz, 2H), 7.30 (d, *J* = 7.9 Hz, 1H), 7.23 (d, *J* = 2.6 Hz, 1H), 5.32 (p, *J* = 6.7, 6.0 Hz, 1H), 2.96 (d, *J* = 6.8 Hz, 2H), 2.70 (d, *J* = 6.8 Hz, 2H), 1.95 (q, *J=* 5.0 Hz, 2H), 1.90 - 1.85 (m, 2H), 1.58 (d, 3H) |
| T-328 | 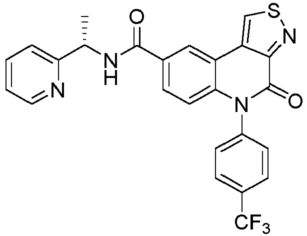 | LC-MS[M+1]: 494.49 ¹H NMR (400 MHz, chloroform-d) δ 9.47 (s, 1H), 8.54 (d, J = 2.1 Hz, 1H), 8.48 (d, J = 4.9 Hz, 1H), 7.94 (d, J = 7.1 Hz, 1H), 7.85 (d, J = 7.9 Hz, 2H), 7.67 (ddd, J = 15.1, 8.7, 4.4 Hz, 2H), 7.43 (d, J = 7.9 Hz, 2H), 7.27 (d, J = 7.8 Hz, 1H), 6.58 (d, J = 8.8 Hz, 1H), 5.29 (q, J = 6.8 Hz, 1H), 1.18 (s, 3H). |
| T-329 | 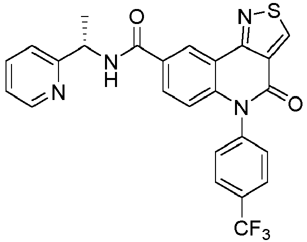 | LC-MS[M+1]: 494.49 |
| T-330 | 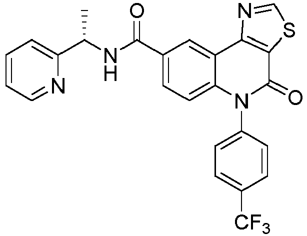 | LC-MS[M+1]: 494.49 |
| T-331 | 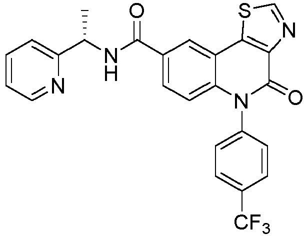 | LC-MS[M+1]: 494.49 |
| T-332 | 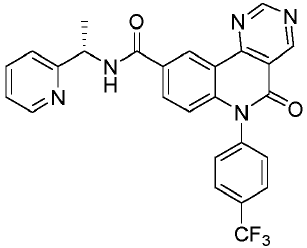 | LC-MS[M+1]: 489.14 |
| T-333 | 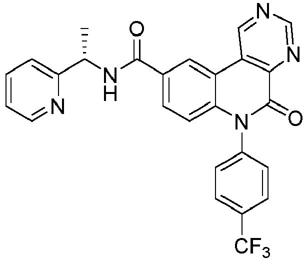 | LC-MS[M+1]: 489.14 |
| T-334 | 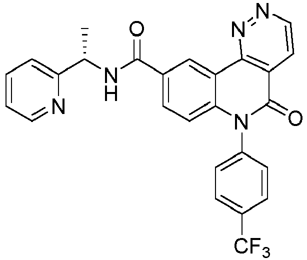 | LC-MS[M+1]: 489.14 |
| T-335 | 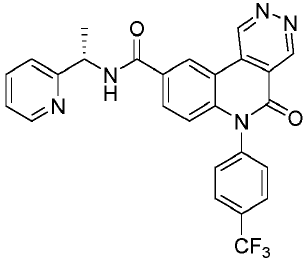 | LC-MS[M+1]: 489.14 |
| T-336 | 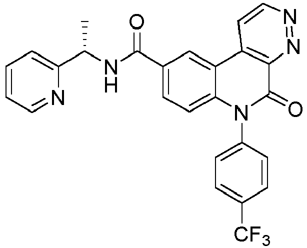 | LC-MS[M+1]: 489.14 |
| T-337 | 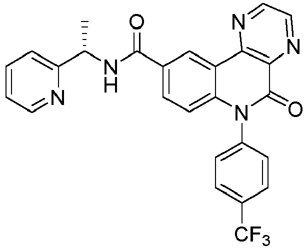 | LC-MS[M+1]: 489.14 |
| T-338 | 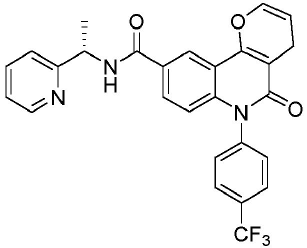 | LC-MS[M+1]: 491.14 |
| T-339 | 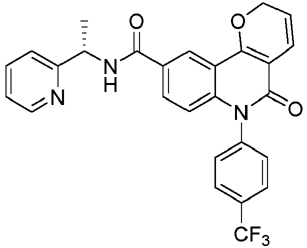 | LC-MS[M+1]: 489.14 |
| T-340 | 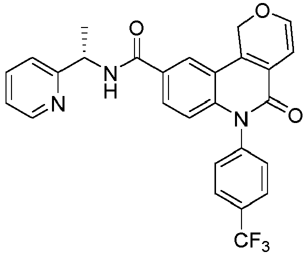 | LC-MS[M+1]: 489.14 |
| T-341 | 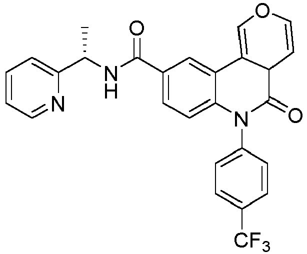 | LC-MS[M+1]: |
| T-342 | 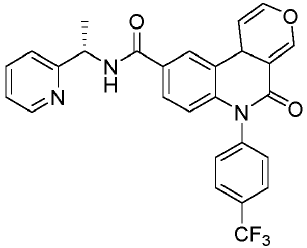 | LC-MS[M+1]: 489.14 |
| T-343 | 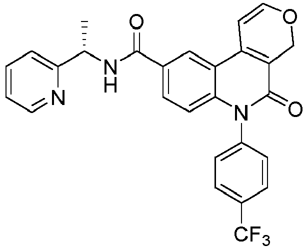 | LC-MS[M+1]: 489.14 |
| T-344 | 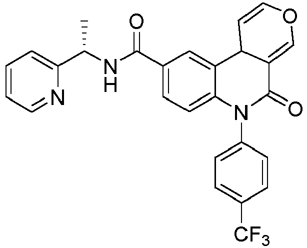 | LC-MS[M+1]: 489.14 |
| T-345 | 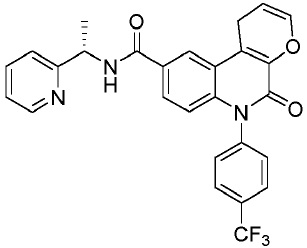 | LC-MS[M+1]: 489.14 |
| T-346 | 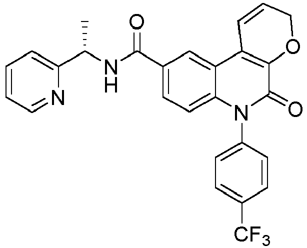 | LC-MS[M+1]: 489.14 |
| T-347 | 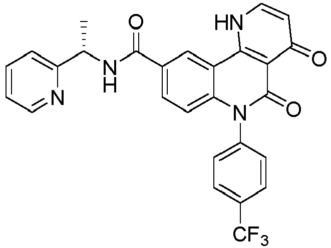 | LC-MS[M+1]: 504.14 |
| T-348 | 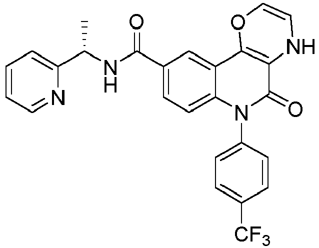 | LC-MS[M+1]: 492.14 |
| T-349 | 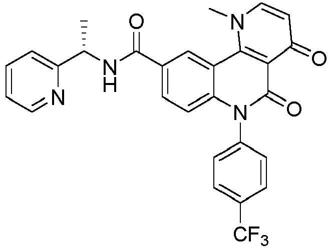 | LC-MS[M+1]: 518.15 |
| T-350 | 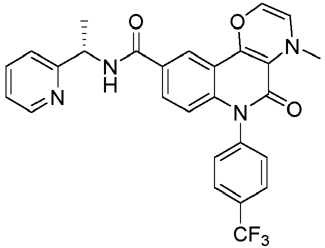 | LC-MS[M+1]: 506.15 |
| T-351 | 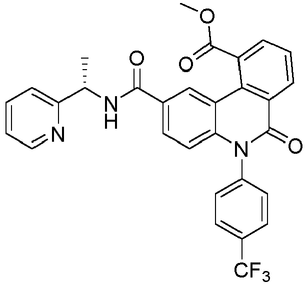 | LC-MS[M+1]: 545.15 |
| T-352 | 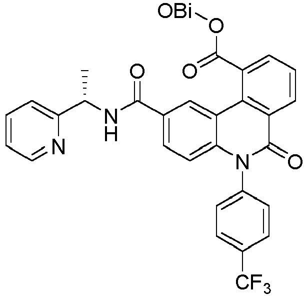 | LC-MS[M+1]: 755.10 |
| T-353 | 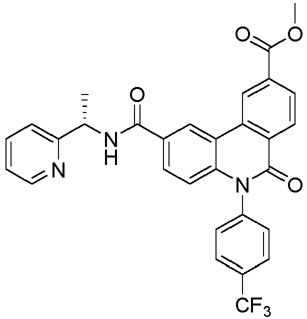 | LC-MS[M+1]: 545.15 |
| T-354 | 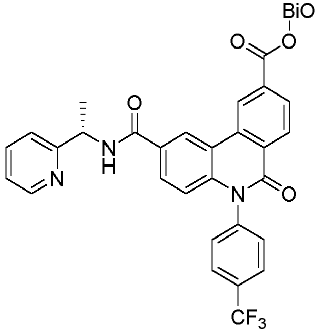 | LC-MS[M+1]: 755.10 |
| T-355 | 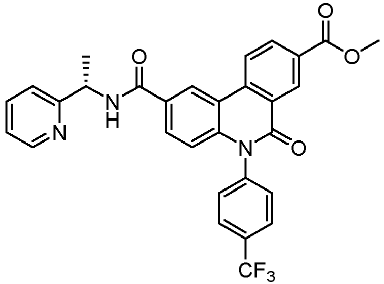 | LC-MS[M+1]: 545.15 |
| T-356 | 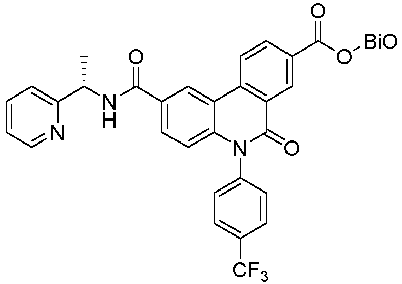 | LC-MS[M+1]: 755.10 |
| T-357 | 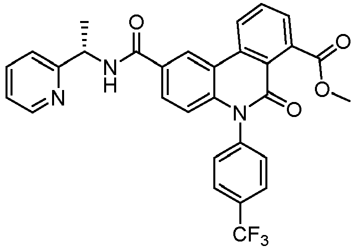 | LC-MS[M+1]: 545.15 |
| T-358 | 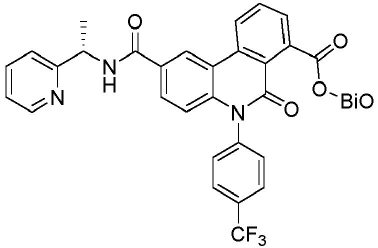 | LC-MS[M+1]: 755.10 |
| T-359 | 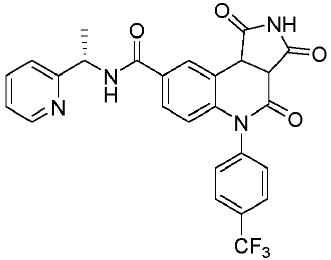 | LC-MS[M+1]: 508.13 |
| T-360 | 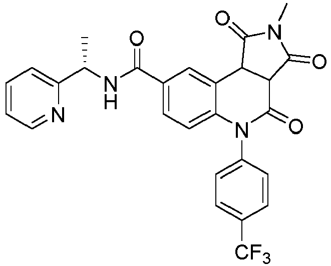 | LC-MS[M+1]: 522.15 |
| T-361 | 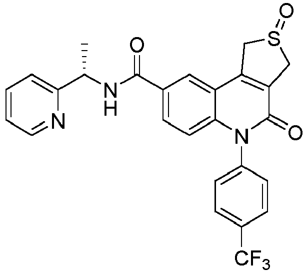 | LC-MS[M+1]: 511.12 |
| T-362 | 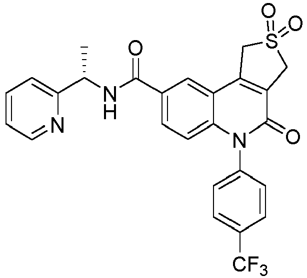 | LC-MS[M+1]: 527.11 |
| T-363 | 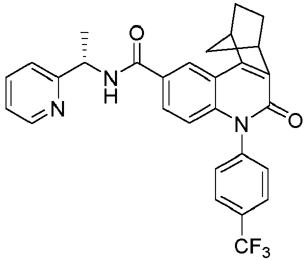 | LC-MS[M+1]: 503.18 |
| T-364 | 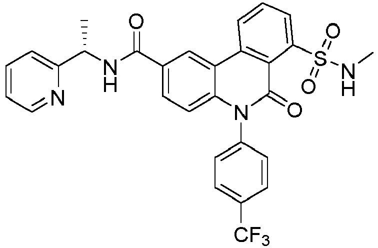 | LC-MS[M+1]: 580.58 |
| T-365 | 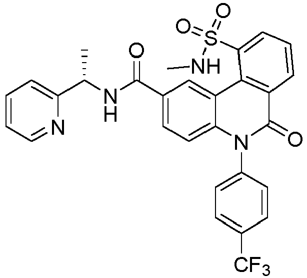 | LC-MS[M+1]: 580.58 |
| T-366 | 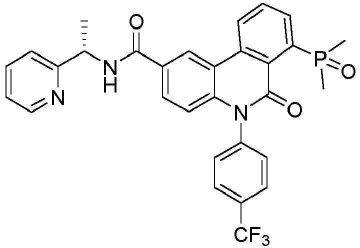 | LC-MS[M+1]: 560.15 |
| T-367 | 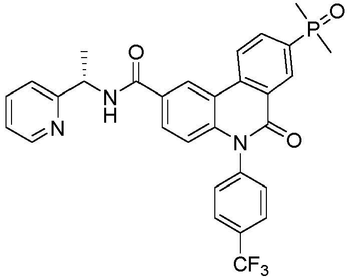 | LC-MS[M+1]: 560.15 |
| T-368 | 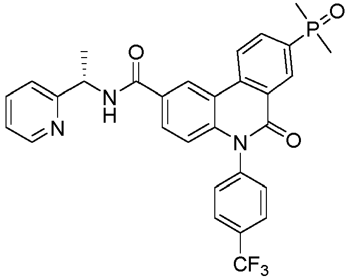 | LC-MS[M+1]: 560.15 |
| T-369 | 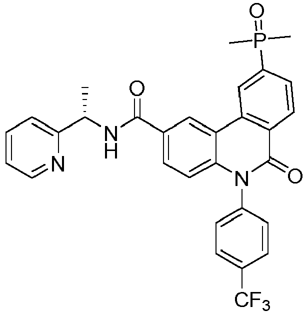 | LC-MS[M+1]: 560.15 |
| T-370 | 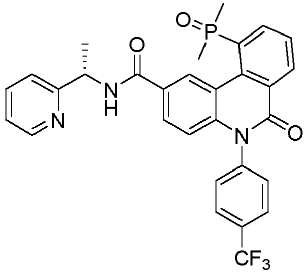 | LC-MS[M+1]: 560.15 |
| T-454 | 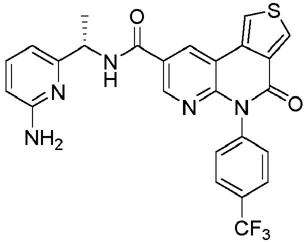 | LC-MS[M+1]: 510.5 |
| T-372 | 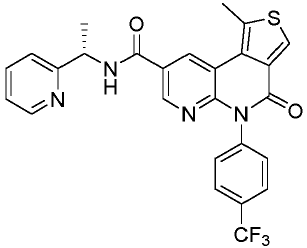 | ¹H NMR (400 MHz, chloroform-d) δ 8.98 (d, J = 2.2 Hz, 1H), 8.73 (d, J = 2.2 Hz, 1H), 8.52 (d, J = 4.8 Hz, 1H), 8.28 (s, 1H), 8.24 (s, 1H), 7.83 (d, J = 8.1 Hz, 2H), 7.76 (t, J = 7.8 Hz, 1H), 7.42 (d, J = 8.1 Hz, 2H), 7.35 (d, J = 7.8 Hz, 1H), 7.28 (s, 1H), 5.34 (p, J = 6.7 Hz, 1H), 3.05 (s, 3H), 1.62 (d, J = 6.7 Hz, 3H). |
| T-373 | 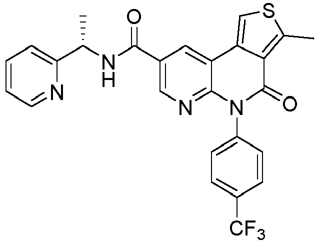 | LC-MS[M+1]: 508.11 |
| T-374 | 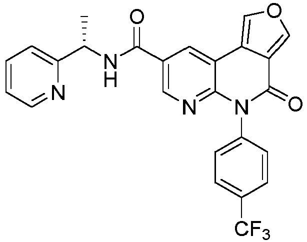 | LC-MS[M+1]: 478.12 |
| T-375 | 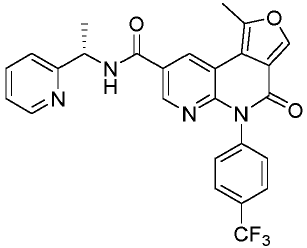 | LC-MS[M+1]: 492.14 |
| T-376 | 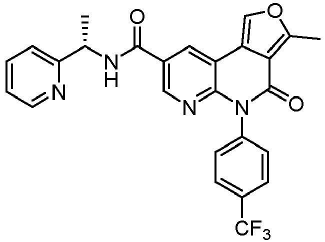 | ¹H NMR (400 MHz, DMSO-*d*₆) δ 9.05 (d, *J* = 7.8 Hz, 1H), 8.91 - 8.82 (m, 1H), 8.57 (d, *J* = 2.3 Hz, 1H), 8.55 - 8.48 (m, 1H), 7.94 (d, *J* = 8.5 Hz, 2H), 7.76 (td, *J* = 7.7, 1.8 Hz, 1H), 7.58 - 7.46 (m, 2H), 7.41 (d, *J* = 7.9 Hz, 1H), 7.27 (ddd, *J* = 7.6, 4.8, 1.2 Hz, 1H), 5.49 (dd, *J* = 14.2, 4.3 Hz, 1H), 5.42 - 5.30 (m, 2H), 5.21 (q, *J* = 7.3 Hz, 1H), 1.51 (dd, *J* = 15.5, 6.6 Hz, 3H), 1.50 (dd, 3H). |
| T-377 | 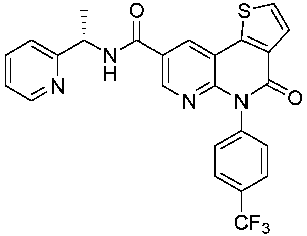 | ¹H NMR (400 MHz, DMSO-*d*₆) δ 9.20 (d, *J* = 7.7 Hz, 1H), 8.94 (d, *J* = 2.2 Hz, 1H), 8.81 (d, *J* = 2.2 Hz, 1H), 8.58 - 8.52 (m, 1H), 8.02 (d, *J* = 5.2 Hz, 1H), 7.95 (d, *J* = 8.2 Hz, 2H), 7.79 (td, *J* = 7.7, 1.9 Hz, 1H), 7.71 (d, *J* = 5.2 Hz, 1H), 7.65 (d, *J* = 8.0 Hz, 2H), 7.46 (d, *J* = 7.9 Hz, 1H), 7.29 (ddd, *J* = 7.6, 4.8, 1.2 Hz, 1H), 5.25 (p, *J* = 7.1 Hz, 1H), 1.56 (d, *J* = 7.0 Hz, 3H). |
| T-378 | 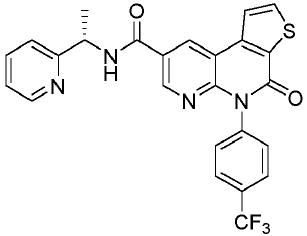 | ¹H NMR (400 MHz, DMSO-*d*₆) δ 9.19 (d, *J* = 2.2 Hz, 1H), 9.08 (d, *J* = 7.7 Hz, 1H), 8.83 (d, *J* = 2.2 Hz, 1H), 8.55 (dd, *J* = 4.9, 2.2 Hz, 1H), 8.40 (d, *J* = 5.3 Hz, 1H), 8.25 (d, *J* = 5.2 Hz, 1H), 7.96 (d, *J* = 8.2 Hz, 2H), 7.79 (td, *J* = 7.7, 1.9 Hz, 1H), 7.67 (d, *J* = 8.1 Hz, 2H), 7.47 (d, *J* = 7.9 Hz, 1H), 7.29 (dd, *J* = 7.5, 4.9 Hz, 1H), 5.25 (p, *J* = 7.0 Hz, 1H), 1.56 (d, *J* = 7.1 Hz, 3H). |
| T-379 | 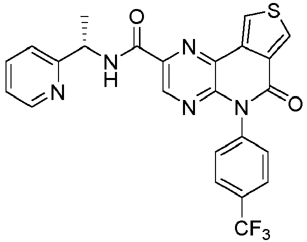 | LC-MS[M+1]: 495.09 |
| T-380 | 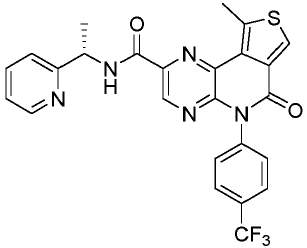 | LC-MS[M+1]: 509.11 |
| T-381 | 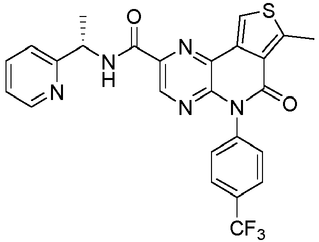 | LC-MS[M+1]: 509.11 |
| T-382 | 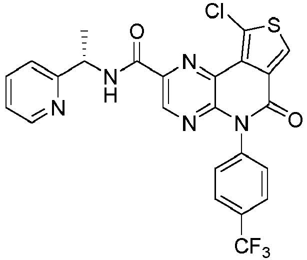 | LC-MS[M+1]: 529.05 |
| T-383 | 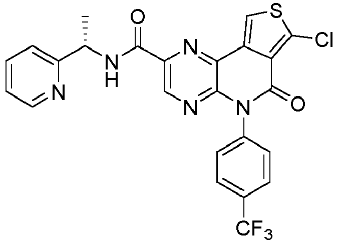 | LC-MS[M+1]: 529.05 |
| T-384 | 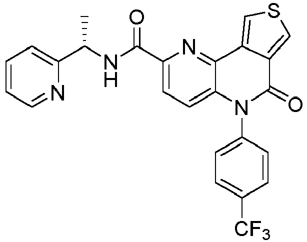 | LC-MS[M+1]: 494.10 |
| T-385 | 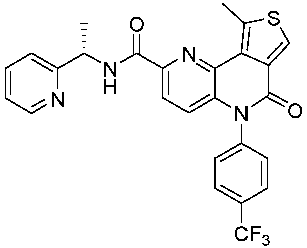 | LC-MS[M+1]: 508.11 |
| T-386 | 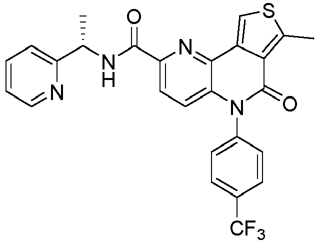 | LC-MS[M+1]: 508.11 |
| T-387 | 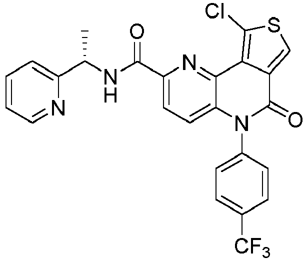 | LC-MS[M+1]: 528.06 |
| T-388 | 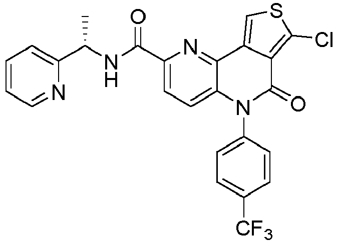 | LC-MS[M+1]: 528.06 |
| T-389 | 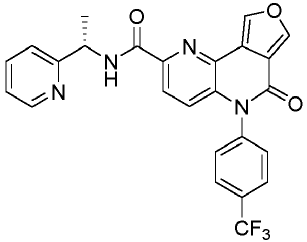 | LC-MS[M+1]: 478.12 |
| T-390 | 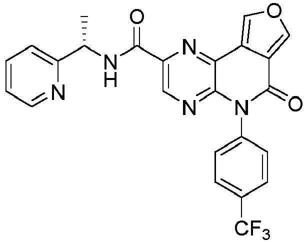 | LC-MS[M+1]: 479.12 |
| T-391 | 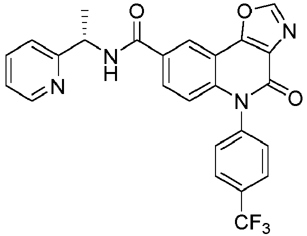 | LC-MS[M+1]: 478.12 |
| T-392 | 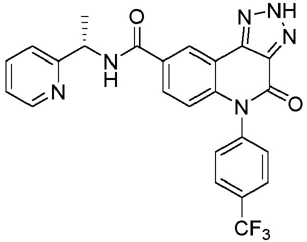 | LC-MS[M+1]: 478.13 |
| T-393 | 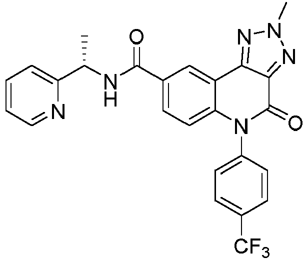 | LC-MS[M+1]: 492.12 |
| T-394 | 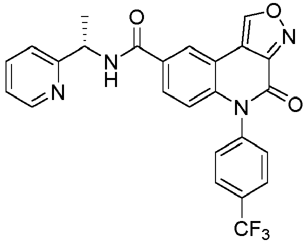 | LC-MS[M+1]: 478.12 |
| T-395 | 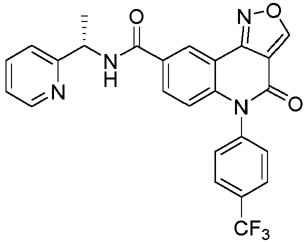 | LC-MS[M+1]: 478.12 |
| T-396 | 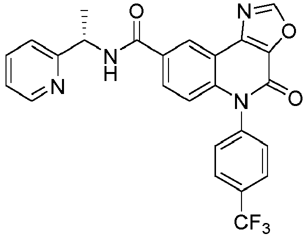 | LC-MS[M+1]: 478.12 |

### Example T-276

The compound synthesized by the invention:

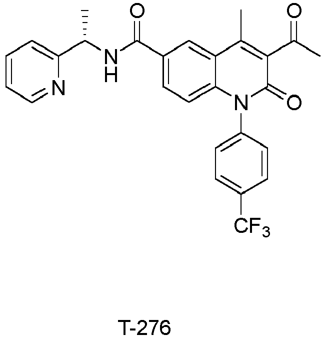

The synthetic route was as follows:

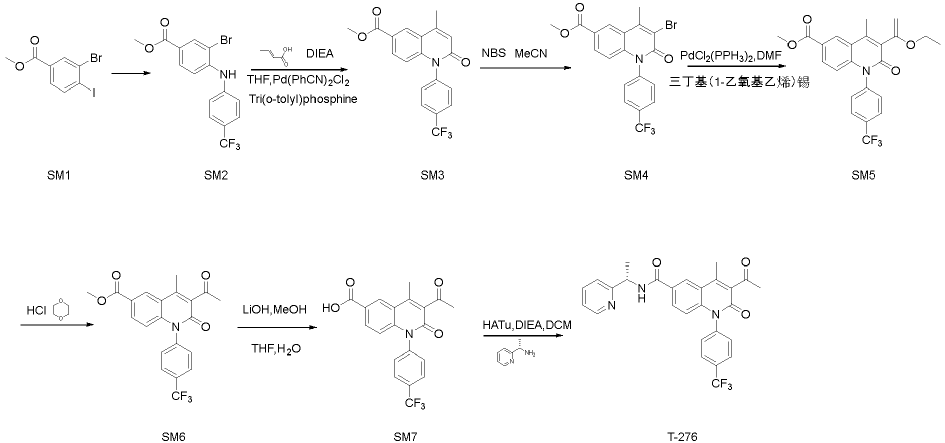

The experimental processes were as follows:

### Synthesis of SM3:

A 50ml round-bottom flask was first protected with nitrogen, followed by the addition of SM2 (2 g, 5.362 mmol, 1 eq), crotonic acid (1.154 g, 13.4 mmol, 2.5 eq), DIEA (6.92 g, 53.62 mmol, 10 eq) and THF (10 ml). Then the round-bottom flask was replaced with nitrogen, followed by the addition of Pd(PHCN)₃Cl₂ (102.8 mg, 0.268 mmol, 0.05 eq), Tri(o-tolyl)phosphine (81.6 g, 0.26 mmol, 0.05 eq), and acetic anhydride (1.68 ml). The reaction system was protected by nitrogen, heated to 70°C, refluxed, and reacted overnight. TLC (PE:EA = 10: 1) showed that all raw materials were fully reacted. The reaction was cooled, added with 20ml of 2N HCl and 10 ml of water, extracted with 50ml of EA, then washed with NaHCO₃, extracted with EA (20ml × 3), washed with saturated sodium chloride (20ml × 2), dried over anhydrous sodium sulfate, mixed with silica gel and dried, and subjected to column chromatography to obtain a total of 892mg of SM5.

### Synthesis of SM4:

SM3 (85 mg, 0.235 mmol, 1 eq), LiOH (16.9 mg, 0.705 mmol, 3 eq), MeOH (1.2ml), H2O (0.3ml), and THF (0.6ml) were reacted under nitrogen protection for 4.5h. LC-MS showed that the reaction was complete. The reaction solution was cooled, evaporated by rotary evaporation, and added with DCM and spin dried to obtain 80 mg of product.

### Synthesis of SM5:

SM4 (640 mg, 1.458 mmol, 1 eq), tributyl(1-ethoxyethylene)tin (789.8 mg, 52.187 mmol, 1.5 eq), Pd(PPh₃)Cl₂ (102.3mg, 0.1458 mmol, 0.1 eq), and DMF (13 ml) were put into a round bottom flask. The mixture was protected under nitrogen, heated to 120°C, refluxed for 0.5h. TLC (PE:EA = 3:1) showed that the raw material had been fully reacted. The reaction was cooled, added with 15 ml of water, filtered over diatomite, extracted with EA (30 ml × 3), washed with saturated sodium chloride (40 ml × 2), dried over anhydrous sodium sulfate, mixed with silica gel and dried, and subjected to column chromatography to obtain a total of 363mg of SM4.

### Synthesis of SM6:

SM5(363 mg, 0.842 mmol, 1 eq), methanol (10 ml) and HCl 1,4-dioxane (10 ml) were added to a round bottom flask, and the mixture was protected under nitrogen and reacted overnight. TLC (PE:EA = 3:1) showed that the raw material had been fully reacted. The mixture was dried by rotary dryer to obtain 312mg SM6.

### Synthesis of SM7:

SM6 (312 mg, 0.774 mmol, 1 eq), LiOH (55.6 mg, 2.322 mmol, 3 eq), MeOH (6ml), H2O (1.5ml), and THF (3ml) were reacted under nitrogen protection at 25 °C overnight. TLC (DCM:MeOH=10:1) showed that the reaction was complete. The reaction solution was cooled, evaporated by rotary evaporation, added with 5 ml of water and 2 ml of 2N HCl. A large amount of white solid precipitated. After filteration, the filtrate was extracted with EA, washed with saturated NaCl, dried over anhydrous sodium sulfate, and spin-dried to obtain a total of 280 mg of SM7.

### Synthesis of T-276:

SM7 (280 mg, 0.72 mmol, 1 eq), pyridine ethylamine (105.4 mg, 0.864 mmol, 1.2 eq), HATu (237.8 mg, 0.72 mmol, 1 eq), and DIEA (185.8mg, 1.44 mmol, 2 eq) were added to 5ml of DCM. The mixture was protected under nitrogen, and reacted overnight at ordinary temperature. TLC (EA) showed that the raw material has completely reacted and the product has been generated. The reaction solution was added with water and extracted with EA, washed with 0.5% citric acid, washed with saturated sodium chloride, dried over anhydrous sodium sulfate, and subjected to column chromatography to obtain 335 mg of product. HPLC:97%

1H NMR (400 MHz, Methanol-d4) δ 8.55 (d, J = 2.0 Hz, 1H), 8.50 (dd, J = 5.0, 1.8 Hz, 1H), 7.97 (dd, J = 8.5, 2.8 Hz, 3H), 7.80 (td, J = 7.7, 1.8 Hz, 1H), 7.58 (d, J = 8.1 Hz, 2H), 7.46 (d, J = 7.9 Hz, 1H), 7.29 (dd, J = 7.6, 5.0 Hz, 1H), 6.74 (d, J = 8.9 Hz, 1H), 5.27 (q, J = 7.1 Hz, 1H), 2.58 (s, 3H), 2.53 (s, 3H), 1.60 (d, J = 7.1 Hz, 3H).

The following compounds were synthesized with reference to the synthesis method in example T-276:

| Example | Compound structure | Compound characterization |
|---|---|---|
| T-277 | 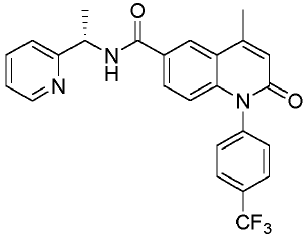 | ¹H NMR (400 MHz, Methanol-*d*₄) δ 8.49 (m, *J* = 10.9, 3.3 Hz, 2H), 8.01 - 7.92 (m, 3H), 7.81 (td, *J* = 7.7, 1.8 Hz, 1H), 7.55 (d, *J* = 8.1 Hz, 2H), 7.48 (d, *J* = 7.9 Hz, 1H), 7.31 (ddd, *J* = 7.6, 4.9, 1.2 Hz, 1H), 6.73 (d, *J* = 8.9 Hz, 2H), 5.28 (q, *J* = 7.1 Hz, 1H), 2.68 (d, *J* = 1.2 Hz, 3H), 1.61 (d, *J* = 7.1 Hz, 3H). |
| T-278 | 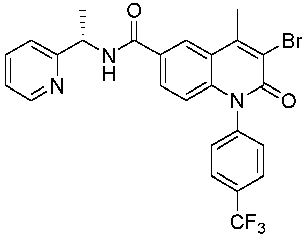 | ¹H NMR (400 MHz, Methanol-*d*₄) δ 8.48 (d, *J* = 2.0 Hz, 1H), 8.41 (m, *J* = 4.9, 1.8, 0.9 Hz, 1H), 7.92 - 7.84 (m, 3H), 7.71 (td, *J* = 7.7, 1.8 Hz, 1H), 7.48 (d, *J* = 8.2 Hz, 2H), 7.44 - 7.35 (m, 1H), 7.21 (m, *J* = 7.6, 5.0, 1.2 Hz, 1H), 6.64 (d, *J* = 8.8 Hz, 1H), 5.19 (q, *J* = 7.1 Hz, 1H), 2.81 (s, 3H), 1.51 (d, *J* = 7.1 Hz, 3H). |
| T-279 | 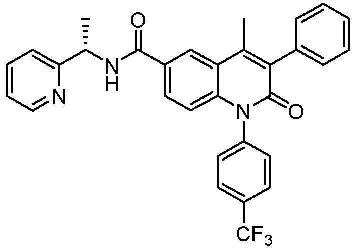 | ¹H NMR (400 MHz, DMSO-*d*₆) δ 9.30 (d, *J* = 7.8 Hz, 1H), 8.64 - 8.56 (m, 2H), 8.12 - 8.05 (m, 3H), 7.83 (td, *J* = 7.7, 1.9 Hz, 1H), 7.74 (d, *J* = 7.9 Hz, 2H), 7.61 - 7.50 (m, 1H), 7.53 (s, 1H), 7.51 (s, 1H), 7.52 - 7.42 (m, 1H), 7.46 - 7.36 (m, 2H), 7.32 (ddd, *J* = 7.5, 4.8, 1.2 Hz, 1H), 6.69 (d, *J* = 8.8 Hz, 1H), 5.31 (p, *J* = 7.2 Hz, 1H), 2.53 (s, 3H), 1.62 (d, *J* = 7.1 Hz, 3H). |
| T-280 | 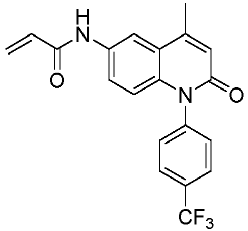 | ¹H NMR (400 MHz, DMSO-*d*₆) δ 10.42 (s, 1H), 8.37 (d, *J* = 2.4 Hz, 1H), 8.06 (d, *J* = 8.1 Hz, 2H), 7.66 (t, *J* = 10.1 Hz, 3H), 6.70 (s, 1H), 6.60 (d, *J* = 9.1 Hz, 1H), 6.49 (dd, *J* = 6.9, 10.0 Hz, 1H), 6.33 (dd, *J* = 17.0, 2.1 Hz, 1H), 5.84 (d, *J* = 2.0 Hz, 1H). |

### Example T-281

The compound synthesized by the invention:

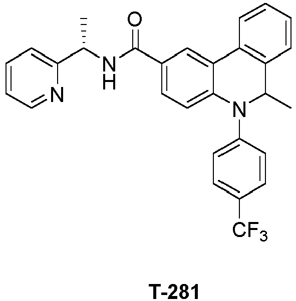

The synthetic route was as follows:

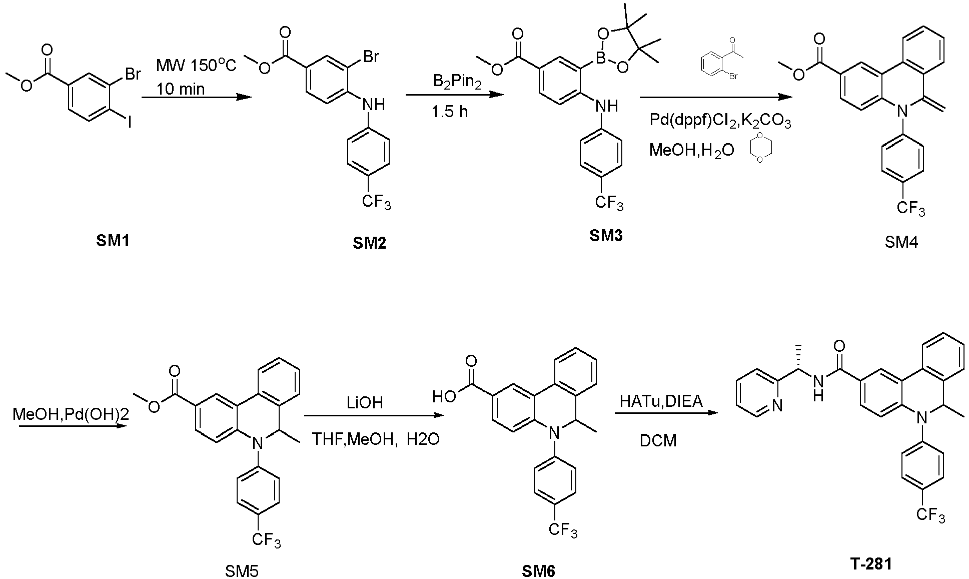

The experimental processes were as follows:

### Synthesis of SM4:

SM3 (2.54 g, 6.02 mmol, 1.2 eq), o-bromoacetophenone (1 g, 5.02 mmol, 1eq), Pd(dppf)C12 (183.7 mg, 0.251 mmol, 0.05 eq), K2CO3 (1.385 mg, 10.04 mmol, 2eq), methanol (10 ml), water (5 ml), and 1,4-dioxane (25 ml) were put into a round bottom flask. The mixture was protected under nitrogen, heated to 90°C, refluxed, and reacted overnight. TLC (PE:EA=3: 1) showed that the raw material has mostly reacted. The reaction was cooled, added with 15 ml of water, filtered over diatomite, extracted with EA (50 ml × 3), washed with saturated sodium chloride (50 ml × 2), dried over anhydrous sodium sulfate, mixed with silica gel and dried, and subjected to column chromatography to obtain a total of 161 mg of SM4.

### Synthesis of SM5:

SM4 (161 mg, 0.393 mmol, 1eq), Pd(OH)₂ (20 mg, 0.142 mmol, 0.36 eq), and ethanol (3ml) were put into a round bottom flask. The mixture was reacted at ordinary temperature under nitrogen protection. TLC (PE:EA = 5:1) showed that the raw material had been fully reacted. The reaction solution was filtered over diatomite, mixed with silica gel and dried, and subjected to column chromatography to obtain a total of 100mg of SM5.

### Synthesis of SM6:

SM5 (100 mg, 0.252 mmol, 1 eq), LiOH (18.1 mg, 0.756 mmol, 3 eq), MeOH (2ml), H₂O (0.5ml), and THF (1ml) were reacted overnight at 25 °C under nitrogen protection. TLC (DCM:MeOH=10:1) showed that the reaction was complete. The reaction solution was cooled, evaporated by rotary evaporation, and added with water and 2N HCl with a large amount of white solid precipitated, and filtered to afford 85 mg of SM6.

### Synthesis of T-281

SM6 (85 mg, 0.222 mmol, 1 eq), pyridine ethylamine (32.5 mg, 0.2664 mmol, 1.2 eq), HATu (84.4 mg, 0.222 mmol, 1 eq), and DIEA (57.3mg, 0.444 mmol, 2 eq) were added to 2 ml of DCM. The mixture was protected under nitrogen, and reacted overnight at ordinary temperature. TLC (PE:EA=1:3) showed that the raw material has been completely reacted and the product has been generated. The reaction solution was added with water and extracted with EA, washed with 0.5% citric acid, washed with saturated sodium chloride, dried over anhydrous sodium sulfate, and subjected to flat-bed chromatography to obtain 62 mg of product. HPLC:99.03%

¹H NMR (400 MHz, chloroform-*d*) δ 8.59 (t, *J* = 5.3 Hz, 1H), 8.42 (s, 1H), 7.93 (d, *J* = 7.8 Hz, 1H), 7.78 (d, *J* = 7.2 Hz, 1H), 7.68 (dt, *J* = 15.6, 6.7 Hz, 2H), 7.54 (d, *J* = 8.4 Hz, 2H), 7.44 - 7.38 (m, 1H), 7.32 (q, *J* = 8.6 Hz, 5H), 7.13 (d, *J* = 7.5 Hz, 1H), 5.38 (dt, *J* = 12.6, 6.2 Hz, 1H), 5.03 (q, *J* = 6.7 Hz, 1H), 1.62 (d, *J* = 6.7 Hz, 3H), 1.40 (d, *J* = 6.7 Hz, 3H).

### Example T-282

The compound synthesized by the invention:

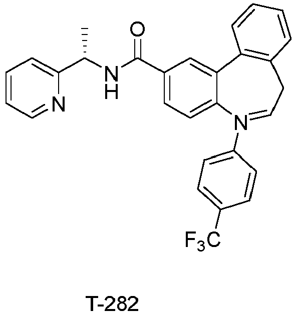

The synthetic route was as follows:

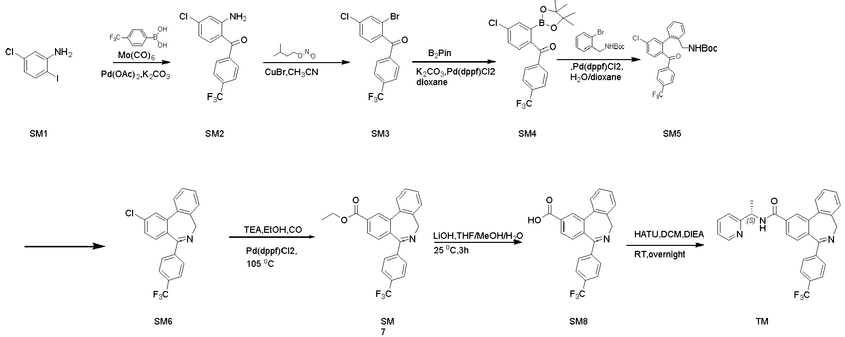

The experimental processes were as follows:

### Synthesis of SM2:

SM1 (15 g, 59.18 mmol, 1.0 eq), p-trifluoromethylphenylboronic acid (16.86 g, 88.77 mmol, 1.5 eq), Mo(CO)₆ (15.62 g, 39.18 mmol, 1.0 eq), potassium carbonate (24.54 g, 177.54 mmol, 3.0 eq), palladium acetate (398.59 mg, 1.78 mmol, 0.03 eq) and anisole (150 ml) were added sequentially into a 500 ml three-necked flask. The flask was evacuated and replaced with nitrogen, and then the mixture was warmed up to 105 degree and reacted for 16h. TLC (PE:EA = 10:1, UV) showed that the raw material has been completely consumed.

The reaction was cooled to room temperature, and 300 ml of methanol was added to dilute the reaction system. Then the reaction system was suction-filtered using a Buchner funnel with diatomaceous earth, and the filter cake was washed with methanol. The filtrate was concentrated at 45 °C and add with 300ml of EA and 300ml of water. The organic layer was separated. The aqueous layer was extracted with EA (300ml * 2). The organic layers were combined, washed with saturated sodium chloride and dried over sodium sulfate. After concentration, the residue was added with 40g of silica gel, mixed and dried and subjected to column chromatography to obtain 2.8g of SM2 (yellow oil)

### Synthesis of SM3

SM2 (2.8g, 0.009 mol, 1.0 eq), cuprous bromide (2.68 g, 0.0186 mol, 2.0 eq) and acetonitrile (50 mL) were added into a 100ml reaction flask. Isoamyl nitrite (2.87 g, 3.3 ml, 0.0243 mol, 2.7 eq) was added dropwise at 0 °C, then the mixture was heated up to 50 °C and reacted for 16 hours. TLC showed that there was a little remaining SM2.

The reaction system was cooled to room temperature, diluted with EA (200 ml), and suction-filtered using a Buchner funnel with diatomite. The filtrate was added with water (200ml) and the EA layer was separated. The aqueous layer was extracted with EA (200ml * 2). The organic layers were combined and dried by adding saturated sodium sulfate, then concentrated and subjected to column chromatography to obtain 2.2 g of product SM3.

### Synthesis of SM4

SM3 (2.0g, 5.5 mmol, 1.0 eq), pinacol borate ester (1.7 g, 6.6 mmol, 1.2 eq), anhydrous potassium acetate (1.6 g, 16.5 mmol, 3.0 eq), Pd(dppf)Cl₂ (0.201 g, 0.28 mmol, 5%) and ultra-dry dioxane (20 mL) were added to a reaction flask; then the flask was vacuumed and exchanged with nitrogen. The mixture was warmed up to 100 degree and reacted for 16 hours. After cooling to room temperature, the reaction solution was diluted by adding ethyl acetate and suction-filtered with diatomite. The filtrate was concentrated and then added with toluene: ethanol (6:1) successively and concentrated to obtain 2.2g of SM4.

### Synthesis of SM5

SM4 (1.38g, 4.8 mmol,1.0 eq), tert-butyl (2-bromobenzyl)carbamate (2.8 g, 6.8 mmol, 1.4 eq), potassium carbonate (1.33 g, 9.7 mmol, 2.0 eq), Pd(dppf)Cl₂ (0.177 g, 0.24 mmol, 5% eq) and dioxane/water (50 ml: 5 ml) were added to a reaction flask; the flask was vacuumed and exchanged with nitrogen. LCMS showed that the raw material has been completely reacted. The reaction was cooled to room temperature, added with EA (100 ml) and water (50 ml), and the organic layer was separated. The aqueous layer was extracted with EA (100 ml * 2), and the combined organic layers were washed with saturated saline, concentrated after drying, and subjected to column chromatography to obtain 800 mg of the product.

### Synthesis of SM6:

To a solution of SM5 (540 mg) in methanol (2 ml) was added hydrochloric acid/dioxane (10 ml), and stirred overnight at room temperature. TLC showed that the raw material has been completely reacted (PE:EA=5:1). The reaction solution was dried and added with saturated sodium bicarbonate solution and extracted with EA. The organic phase was separated, dried, concentrated, and subjected to column chromatography to give 300 mg of product SM6.

### Synthesis of SM7

SM6 (300 mg, 0.806 mmol, 1.0 eq), triethylamine (244 mg, 334 µL, 2.4 mmol, 3.0 eq), Pd(dppf)Cl₂ (30 mg, 0.0403 mmol, 5%) and ethanol (35 mL) were added to an autoclave; then the autoclave was evacuated for exchange with carbon monoxide. The mixture was warmed up to 100 degree and reacted for 24h. 1TLC showed that there was still a small amount of raw material remaining. The reaction solution was cooled to room temperature and suction-filtered using a Buchner funnel with diatomite. The filtrate was diluted by adding EA, then mixed with silica gel (100-200 mesh, 3 g) and dried and then separated and purified by preparative chromatography to obtain 200 mg of SM7 and 80 mg of raw material SM6.

### Synthesis of SM8

To a reaction flask was added SM7 (200 mg, 0.), THF/MeOH/H2O (4ml: 2ml: 1ml), and then lithium hydroxide (35.2 mg, 1.46 mmol, 3.0 eq). The mixture was stirred overnight at room temperature. TLC showed that the raw material has been completely reacted. After cooling to room temperature, the reaction solution was added with 5ml of water and added a few drops of concentrated hydrochloric acid to adjust the pH to acidity. There was white solid precipitated. 150mg of white solid was obtained after suction filtration.

### Synthesis of TM:

SM9 (150 mg, 0.39 mmol, 1.0 eq), HATU (147.38 mg, 0.387 mmol, 1.0 eq), DIEA (100 mg, 141 µl, 0.775 mmol, 2.0 eq), and DCM (2 ml) were added to a reaction tube and stirred at room temperature for 10 minutes. Then, (S) -1- (pyridin-2-yl) ethan-1-amine (56.82mg, 0.465 mmol, 1.2 eq) was added and the mixture was reacted overnight at room temperature. LCMS showed that the raw material has been completely reacted. The reaction solution was diluted by adding DCM (10 ml), then washed with 0.5% citric acid and saturated saline. The mixture was dried, concentrated and subjected to column chromatography to obtain 161 mg of white solid.

¹HNMR (400 MHz, MeOD) δ 8.55-8.50 (t, 1H), 8.39-8.35 (d, 1H), 7.96-7.89(m,2H),7.86-7.78(m,1H),7.70-7.68(d,2H),7.61-7.59(d,2H),7.55-7.47(m,4H),7.43-7.41 (d,1H), 7.34-7.31(m,1H),5.33-5.31(q,1H),3.98-3.96(d,1H), 1.64-1.62(q,3H)

### Example T-283

The compound synthesized by the invention:

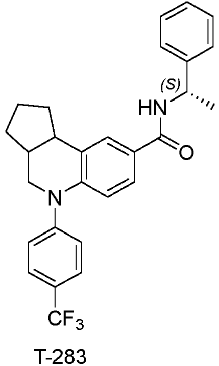

The synthetic route was as follows:

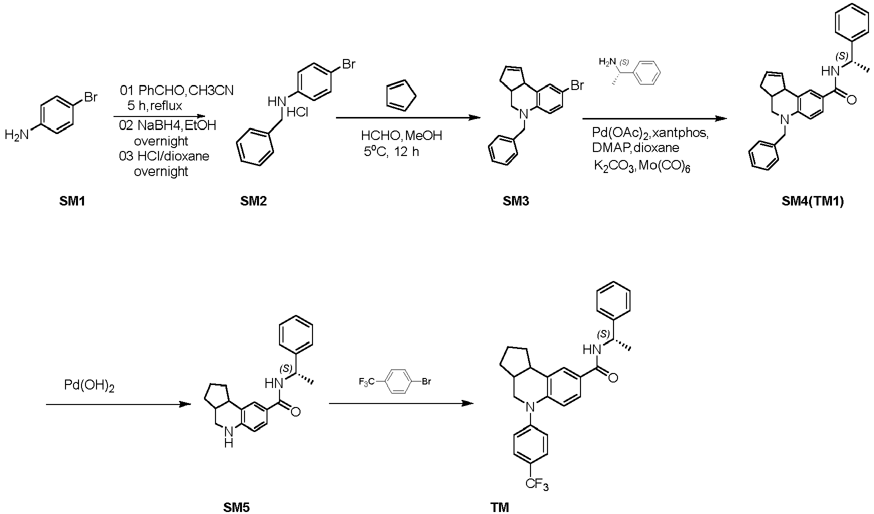

The experimental processes were as follows:

### Synthesis of SM2

SM1 (5.3 g, 0.05 mol, 1.0 eq), benzaldehyde (9.46 g, 0.055 mol, 1.1 eq), and ethanol (75 ml) were added to a reaction flask. The flask was evacuated and replaced with nitrogen, and then the mixture was warmed up to 80 degree and reacted for 3.5h. Then the reaction was cooled to room temperature, added with NaBH₄ (2.28g, 0.06 mol, 1.2 eq) in batches in an ice bath, and stirred overnight at room temperature. The reaction system was poured into a 1L erlenmeyer flask, and slowly added with 1N HCl (200 ml), and the mixture was extracted with EA (150 ml * 3), washed with saturated saline, dried and concentrated to obtain the crude product. Then the crude product was added with 20ml of methanol and 100ml of HCl/dioxane solution, and the mixture was stirred overnight at room temperature. After removing the hydrochloric acid gas with a water pump, the mixture was spin-dried to remove solvent to obtain SM2 (11.6g, white solid SM2).

### Synthesis of SM3

The purchased dicyclopentadiene underwent atmospheric distillation using an electric heating sleeve at 180 degree and fractions at 40-42 degrees were collected to obtain cyclopentadiene monomer for SM3 synthesis.

SM2 (5.5 g), methanol (55 ml), cyclopentadiene (3.3 g), and aqueous formaldehyde solution (2.86 g) were added to a 250ml three-necked flask at around 0 degree, and then the mixture was reacted at room temperature for 72 h. TLC (PE:EA = 10:1) showed that the raw material has been completely reacted with precipitation of white solids. The reaction solution was suction-filtered using a sand core funnel, the filter cake was dried at 25 degree in a vacuum drying chamber to obtain the target product (about 2.6 g)

### Synthesis of SM4:

In 2 batches, SM3 (425 mg, 1.25 mmol, 1.0 eq), anhydrous potassium phosphate (1.59 g, 7.49 mmol, 5.0 eq), palladium acetate (20.19 mg, 0.09 mmol, 8%), (S) -1- (pyridin-2-yl) ethan-1-amine (457.79 mg, 3.75 mmol, 3.0 eq), DMAP (302.72 mg, 2.5 mmol, 2.0 eq), Mo(CO)₆ (108.16 mg, 0.409 mmol, 0.32 eq), Xantphos (106.96 mg, 0.184 mmol, 14% eq), and dioxane(10 ml) were added into a microwave reaction tube, then the mixture was reacted at 120 degree for 20 minutes. TLC (PE:EA = 1:1) showed that the raw material has been completely reacted. The reaction was cooled to room temperature and the two batches of reaction solution were combined for treatment. The combined solution was added with EA (100 ml) and water (50 ml). The EA layer was separated, and the aqueous layer was extracted with EA (50 ml * 2), The combined organic layers were washed with saturated sodium chloride, dried over sodium sulfate, then concentrated, and subjected to preparative chromatography to obtain 630 mg SM4 (yellow solid).

### Synthesis of SM5

To a solution of SM4 (200 mg) in ethanol (10 ml) was added palladium hydroxide (30 mg, w/w 15%) and then 0.2 ml of 2N HCl. The mixture was vacuumed and exchanged with nitrogen for 3 times, then again vacuumed and exchanged with hydrogen for three times (hydrogen balloon). The mixture was warmed up to 80 degree and reacted at reflux for 19h. TLC showed that the raw material has been completely reacted (PE:EA=1: 1). The reaction was cooled to room temperature, added with EA (30 ml), suction-filtered over diatomite, and then the filtrate was mixed with silica gel and dried and subjected to preparative chromatography to obtain SM5 (60 mg, white solid).

### Synthesis of T-283

SM5 (60 mg, 0.186 mmol,1.0 eq), p-trifluoromethyl bromobenzene (50.4 mg, 0.224 mol, 1.2 eq), cesium carbonate (121.64 mg, 0.373 mmol, 2.0 eq), palladium acetate(2.1 mg, 0.01 mmol, 5% eq), xantphos (10.8 mg, 0.018 mmol, 10% eq) and anhydrous dioxane (1 mL) were added to a reaction flask. Then the mixture was warmed up to 105 degree and reacted for 16 hours. TLC showed that the raw material has been completely reacted. The reaction solution was cooled to room temperature, added with 1 ml of water and extracted with EA (6 ml*2), then separated using PTLC to obtain SM6 (28 mg, white solid).

1HNMR (400 MHz, CDCl3) δ 8.50-8.49 (d, 1H), 8.39-8.35 (d, 1H), 7.71-7.51 (m, 2H),7.49-7.44(m,3H),7.41-7.36(d,1H),7.257.23(d,2H),7.157.12(m,2H),6.926.86(m,1H),5.295.2 5(m,1H),4.04-3.50(m,1H),3.20-3.07(d,1H),2.43-2.26(m,2H), 1.96-1.87(m,2H), 1.74-1.60(m,2H),1.49 (m,3H) ,1.40-1.26(m,2H)

### Example T-285

The compound synthesized by the invention:

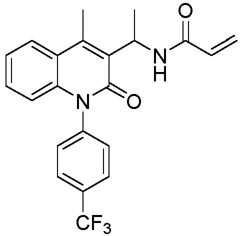

The synthetic route was as follows:

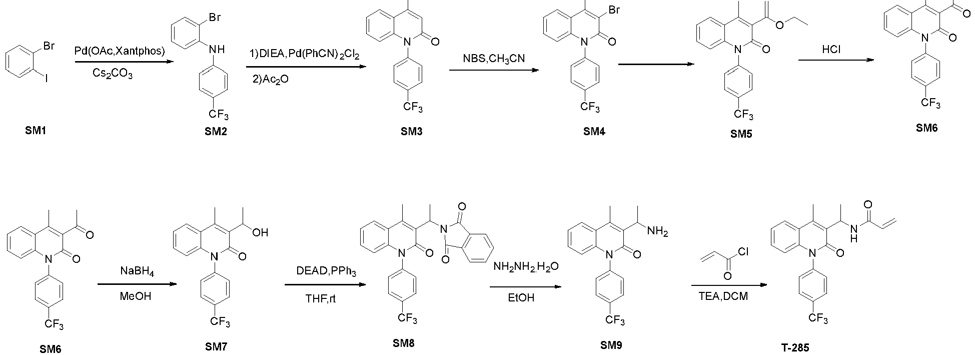

The experimental processes were as follows:
Synthesis of **SM2**: Compound SM1 (1 g, 1.0 eq), p-trifluoroaniline (728 mg, 1.28 eq), Cs₂CO₃ (1.71 g, 1.5 eq), Pd(OAc)₂ (39 mg, 0.05 eq), and Xantphos (506 mg, 0.25 eq) were dissolved in dioxane (20 mL), and the mixture was stirred under N₂ protection at 105 °C for 14 h. The raw material has been basically reacted. Post-treatment: The reaction solution was diluted by adding EA followed by the addition of a small amount of water, then extracted with EA to obtain the organic phase. The organic phase was washed with saturated saline, and dried over anhydrous sodium sulfate. The filtrate was concentrated to dryness to obtain crude **SM3** (1.02 g).

### Synthesis of SM3

Compound SM2 (200 mg, 1.0 eq), crotonic acid (137 mg, 2.5 eq), and DIEA (813 mg, 10 eq) were added to THF. After replacement with N₂, The mixture was added with Pd(PhCN)₂Cl₂ (12 mg, 0.05 eq) and 3(tolyl)phosphorus (10 mg, 0.05 eq), and reacted overnight at 70 °C. The raw material has been basically reacted, but a large number of unclosed ring products were generated. Therefore, supplementary acetic anhydride (0.2 mL) was added to promote ring-closing reaction. After 1 hour, the most of them were ring-closed. LCMS was correct. Post-treatment: the reaction solution was diluted by adding EA, added with a small amount of water, and extracted with EA to obtain the organic phase. The organic phase was washed with saturated saline, dried over anhydrous sodium sulfate. The filtrate was concentrated to dryness to obtain crude **SM3** (126 mg).

### Synthesis of SM4

Compound SM3 (6.0 g, 1.0 eq), and NBS (7.13 g, 2 eq) were added to acetonitrile (60 mL). After replacement with N₂, the mixture was reacted overnight at 90 °C. The raw material has been basically reacted. LCMS was correct. Post-treatment: the reaction solution was diluted by adding EA, added with a small amount of water, and extracted with EA to obtain the organic phase. The organic phase was washed with saturated saline, dried over anhydrous sodium sulfate, subjected to column chromatography (PE→PE:EA=50:1→20:1→10:1) to obtain **SM4** (7.42 g).

### Synthesis of SM5

Compound **SM4** (4 g, 1.0 eq), tributyl(1-ethoxyethylene) tin (5.67 g, 1.5 eq), and Pd(PPh₃)Cl₂ (738mg, 0.1 eq) were added to DMF (40 mL). After replacement with N₂, the mixture was reacted overnight at 120 °C. The raw material has been basically reacted. LCMS was correct. Post-treatment: the reaction solution was diluted by adding EA, added with a small amount of water, and extracted with EA to obtain the organic phase. The organic phase was washed with saturated saline, dried over anhydrous sodium sulfate, and subjected to preparative chromatography (PE:EA=5:1) to obtain **SM5** (970 mg). 1HNMR was correct.

### Synthesis of SM6

Compound **SM5** (970 mg, 1.0 eq) was added to MeOH (10 mL), and hydrochloric acid dioxane (10 ml) was added. After reacting overnight at room temperature, the raw material has been basically reacted, and LCMS was correct. Post-treatment: the reaction solution was directly dried by rotary dryer to obtain crude product **SM6** (728 mg).

### Synthesis of SM7

Compound **SM6** (728 mg, 1.0 eq) was added to MeOH (5 mL), and sodium borohydride (200 mg, 2.5 eq) was added under an ice bath. The mixture returned to room temperature and reacted; subsequent reaction cannot proceed. Supplementary sodium borohydride was added till 10 times the equivalent. After reacting for half an hour, the raw material has been basically reacted. LCMS was correct. Post-treatment: the reaction solution was diluted by adding EA, added with a small amount of water, and extracted with EA to obtain the organic phase. The organic phase was washed with saturated saline, dried over anhydrous sodium sulfate to obtain crude product **SM7** (734 mg). 1HNMR was correct.

### Synthesis of SM8

Compound **SM7** (690 mg, 1.0 eq), phthalimide (438 mg, 1.5 eq), and triphenylphosphine (1.563 g, 3 eq) were added to THF (15 mL). DEAD (865 mg, 2.5 eq) was added under an ice bath. The mixture returned to room temperature and reacted. After reacting over weekend, the raw material has been basically reacted with new point produced. Post-treatment: the reaction solution was directly dried by rotary dryer, subjected to preparative chromatography (PE:EA=5: 1) to obtain the product **SM8** (2.55 g).

### Synthesis of SM9

Compound **SM8** (2.5 g, 1.0 eq), and hydrazine hydrate (2.683 g, 10 eq) were added to EtOH (25 mL). The mixture was reacted overnight under reflux. Post-treatment: the reaction solution was directly dried by rotary dryer and subjected to column chromatography to obtain crude product **SM9** (327 mg).

### Synthesis of T-285

Compound SM9 (166 mg, 1.0 eq), and TEA (145 mg, 3 eq) were added to DCM (1 mL). Acryloyl chloride (50 mg, 1.15 eq) was added dropwise under an ice bath. The mixture returned to room temperature and reacted. After reacting overnight, the raw material has been basically reacted, and LCMS was correct. Post-treatment: the reaction solution was directly dried by rotary dryer, and subjected to column chromatography (PE→PE:EA=5:1→PE:EA=2:1→1:1) to obtain the product **T-285** (33 mg).

¹H NMR (400 MHz, chloroform-*d*) δ 7.89 (ddd, *J* = 7.7, 6.2, 2.1 Hz, 3H), 7.71 (d, *J* = 9.4 Hz, 1H), 7.50 - 7.39 (m, 2H), 7.35 (ddd, *J* = 8.5, 7.1, 1.5 Hz, 1H), 7.31 - 7.28 (m, 1H), 6.61 (dd, *J* = 8.3, 1.3 Hz, 1H), 6.26 (dd, *J* = 17.0, 1.6 Hz, 1H), 6.06 (dd, *J* = 16.9, 10.2 Hz, 1H), 5.83 (dq, *J* = 9.4, 7.0 Hz, 1H), 5.60 (dd, *J* = 10.2, 1.5 Hz, 1H), 2.72 (s, 3H), 1.54 (d, *J* = 7.0 Hz, 3H).

### Example T-412

The compound synthesized by the invention:

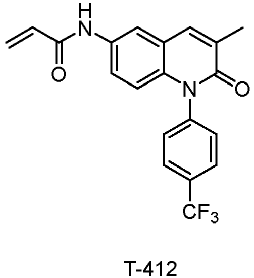

The synthetic route was as follows:

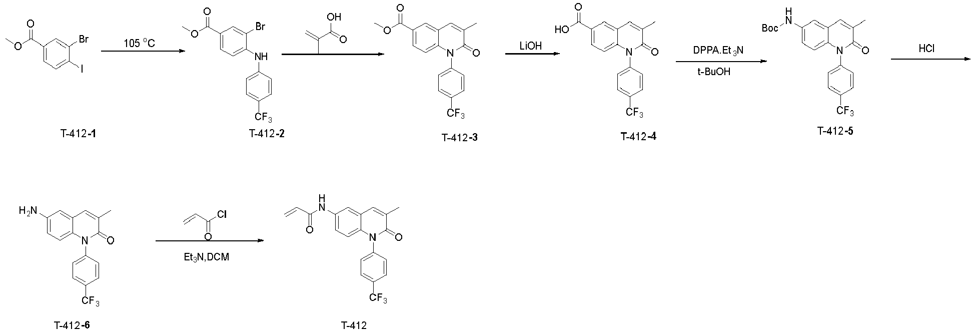

The experimental processes were as follows:

### Synthesis of T-412-2

Compound T-412-1 (20 g, 1.0 eq), p-trifluoroaniline (12.12 g, 1.28 eq), Cs₂CO₃ (28.75 g, 1.5 eq), Pd(OAc)₂ (660 mg, 0.05 eq), and Xantphos (850 mg, 0.25 eq) were dissolved in dioxane (400 mL), and the mixture was stirred under N₂ protection at 105 °C for 14 h. The raw material has been basically reacted. Post-treatment: the reaction solution was diluted by adding EA, added with a small amount of water, and extracted with EA to obtain the organic phase. The organic phase was washed with saturated saline, and dried over anhydrous sodium sulfate. The filtrate was concentrated to dryness and purified (PE→PE:EA=50:1) to obtain crude product T-412-2 (20.7 g). Note: If the purity of T-412-2 was insufficient, the strategy of hydrolysis and esterification can be adopted, and 1HNMR was correct.

### Synthesis of T-412-3

Compound T-412-2 (10.6 g, 1.0 eq), crotonic acid (6.11 g, 2.5 eq), and DIEA (36.09 g, 10 eq) were added to THF. After replacement with N₂, the mixture was added with Pd(PhCN)₂Cl₂ (545 mg, 0.05 eq) and 3(tolyl)phosphorus (432 mg, 0.05 eq) and reacted overnight at 70 °C. The raw material has been basically reacted, but a large amount of unclosed product was generated. Therefore, supplementary acetic anhydride (8.9 mL) was added to activate and promote ring-closing reaction. After 2 hours, all of them produced as ring-closed product. LCMS was correct. Post-treatment: the reaction solution was diluted by adding EA, added with a small amount of water, and extracted with EA to obtain the organic phase. The organic phase was washed with saturated saline, and dried over anhydrous sodium sulfate. The filtrate was concentrated to dryness and subjected to preparative chromatography (PE:EA=5:1) to obtain crude T-412-3 (4.6 g).

### Synthesis of T-412-4

Compound T-412-3 (4.6 g, 1.0 eq), and LiOH (917.5 mg, 2 eq) were dissolved in MeOH/THF/H2O (76 ml /38 mL/19 mL). The mixture was stirred under N2 protection at 50 room temperature. The raw material has been basically reacted, and LCMS was correct. Post-treatment: the reaction solution was diluted by adding EA, added with a small amount of water, separated to obtain the aqueous layer (at this point, the product was salt, in the aqueous phase). The aqueous phase was added with 1N HCl to adjust the pH to about 3, extracted with EA to obtain organic phase (at this point, the product was in the organic phase). The organic phase was washed with saturated saline, and dried over anhydrous sodium sulfate. The filtrate was concentrated to dryness to obtain crude T-412-4 (3.9 g). LCMS was correct.

### Synthesis of T-412-5

Compound T-412-4 (3.9 g, 1.0 eq), DPPA (4.33 g, 1.4 eq) and Et3N(2.04g, 1.8g) were added to Tert butanol (80 ml) and the mixture was reacted overnight at 90°C. The raw material has been basically reacted. LCMS was correct. Post-treatment: the reaction solution was diluted by adding EA, added with a small amount of water, and extracted with EA to obtain the organic phase. The organic phase was washed with saturated saline, dried over anhydrous sodium sulfate, and subjected to preparative chromatography (PE:EA=5:1) to obtain T-412-5 (944 mg). 1HNMR was correct.

### Synthesis of T-412-6

Compound T-412-5 (944 mg, 1.0 eq) was added to MeOH (15 mL), and 4M hydrochloric acid dioxane (15 ml) was added under an ice bath. After reacting overnight, the raw material has been basically reacted. Post-treatment: the reaction solution was directly dried by rotary dryer to obtain the crude T-412-6 (709 mg).

### Synthesis of T-412

Compound T-412-6 (709 mg, 1.0 eq), and TEA (684 mg, 3 eq) were added to DCM (15 mL). Acryloyl chloride (244 mg, 1.15 eq) was added dropwise under an ice bath. The mixture returned to room temperature and reacted. After reacting overnight, the raw material has been basically reacted, and LCMS was correct. Post-treatment: the reaction solution was directly dried by rotary dryer and subjected to column chromatography to obtain the product T-425 (536 mg). The product was subjected to further purification to obtain 363 mg, with a purity of 93.3%; continued to crystallize to obtain 160 mg with a purity of 97.6%, and the remaining mixed product was 220 mg.

¹H NMR (400 MHz, DMSO-d6) δ 10.30 (s, 1H), 8.15 (d, J = 2.4 Hz, 1H), 8.00 (d, J = 8.2 Hz, 2H), 7.93 (d, J = 1.4 Hz, 1H), 7.60 (d, J = 8.1 Hz, 2H), 7.49 (dd, J = 9.1, 2.4 Hz, 1H), 6.47 (d, J = 9.3 Hz, 1H), 6.45 - 6.37 (m, 1H), 6.26 (dd, J = 17.0, 2.1 Hz, 1H), 5.76 (dd, J = 10.0, 2.1 Hz, 1H), 2.15 (d, J = 1.2 Hz, 3H).

The following compounds were synthesized with reference to the methods in examples T-243 and T-412:

| Example | Compound structure | Compound characterization |
|---|---|---|
| T-413 | 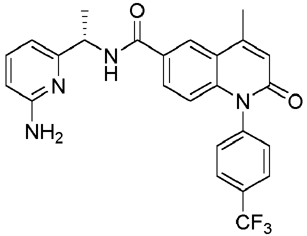 | ¹H NMR (400 MHz, DMSO-d6) δ 8.82 (d, J = 7.8 Hz, 1H), 8.37 (d, J = 2.0 Hz, 1H), 8.04 - 7.99 (m, 2H), 7.94 (dd, J = 8.8, 2.0 Hz, 1H), 7.62 (d, J = 8.0 Hz, 2H), 7.31 (t, J = 7.8 Hz, 1H), 6.69 (d, J = 1.3 Hz, 1H), 6.60 (d, J = 8.8 Hz, 1H), 6.50 (d, J = 7.4 Hz, 1H), 6.29 (d, J = 8.1 Hz, 1H), 5.86 (d, J = 6.3 Hz, 1H), 4.98 (p, J = 7.1 Hz, 1H), 2.60 (d, J = 1.2 Hz, 3H), 1.46 (d, J = 7.0 Hz, 3H). |
| T-414 | 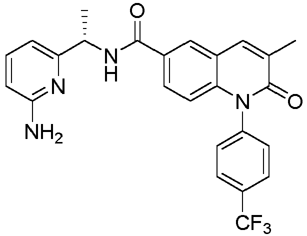 | ¹H NMR (400 MHz, DMSO-d6) δ 8.70 (d, J = 7.8 Hz, 1H), 8.28 (d, J = 2.0 Hz, 1H), 8.06 - 7.99 (m, 3H), 7.86 (dd, J = 8.8, 2.1 Hz, 1H), 7.64 (d, J = 8.1 Hz, 2H), 6.52 (dd, J = 16.6, 8.1 Hz, 2H), 6.29 (d, J = 8.2 Hz, 1H), 5.89 (s, 2H), 4.94 (p, J = 7.0 Hz, 1H), 2.18 (d, J = 1.3 Hz, 3H), 1.43 (d, J = 7.0 Hz, 3H) |
| T-415 | 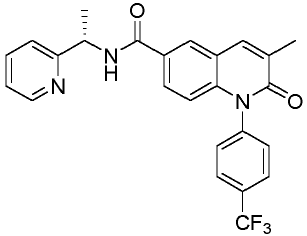 | ¹H NMR (400 MHz, chloroform-d) δ 8.56 (d, J = 5.0 Hz, 1H), 8.12 (s, 1H), 7.90 (dd, J = 14.9, 6.6 Hz, 3H), 7.74 (dq, J = 15.4, 8.3, 7.8 Hz, 3H), 7.43 (d, J = 8.0 Hz, 2H), 7.32 (d, J = 7.8 Hz, 1H), 7.23 (d, J = 6.4 Hz, 1H), 6.62 (d, J = 8.8 Hz, 1H), 5.38 - 5.30 (m, 1H), 2.30 (s, 3H), 1.59 (d, J = 6.8 Hz, 3H). |
| T-416 | 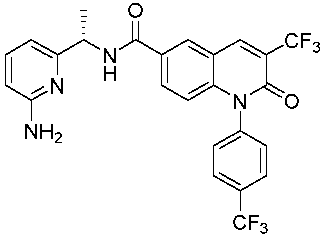 | LC-MS[M+1]: 521.1 |
| T-417 | 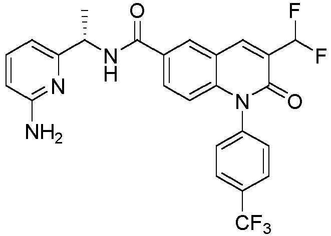 | LC-MS[M+1]: 503.1 |
| T-420 | 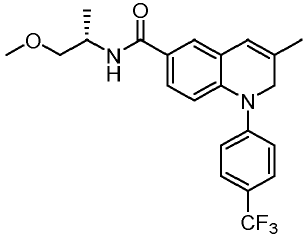 | LC-MS[M+1]: 405.2 |
| T-421 | 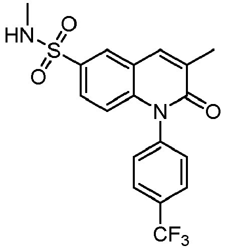 | LC-MS[M+1]: 397.1 |
| T-431 | 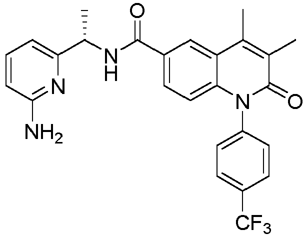 | LC-MS[M+1]: 481.2 |
| T-436 | 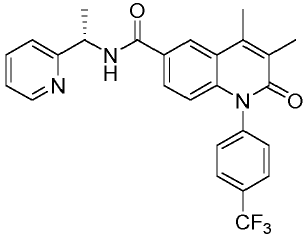 | LC-MS[M+1]: 466.1 |
| T-455 | 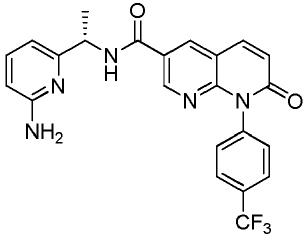 | LC-MS[M+1]: 454.2 |
| T-456 | 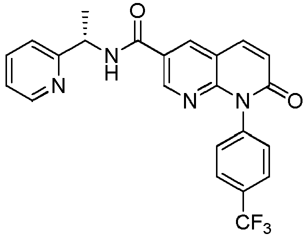 | LC-MS[M+1]: 439.1 |
| T-457 | 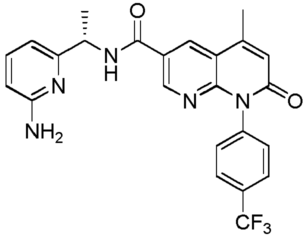 | LC-MS[M+1]: 468.1 |
| T-458 | 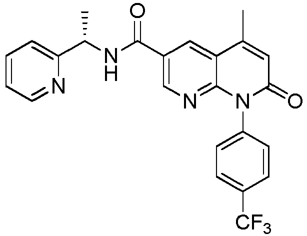 | LC-MS[M+1]: 453.1 |
| T-459 | 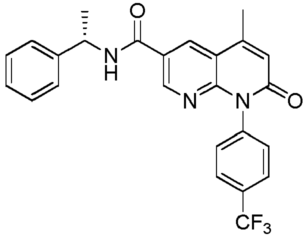 | LC-MS[M+1]: 452.1 |
| T-460 | 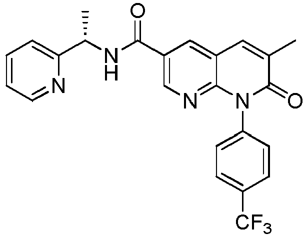 | LC-MS[M+1]: 453.1 |
| T-461 | 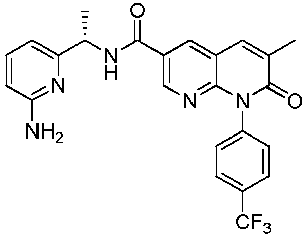 | LC-MS[M+1]: 468.1 |
| T-462 | 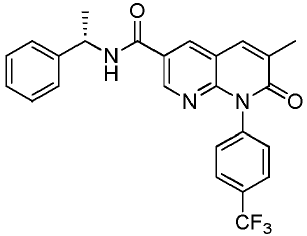 | LC-MS[M+1]: 452.1 |

Biological activity testing experimental process was as follows:

### Test Example 1 Cell Anti-proliferation Experiment

### Determination of proliferation inhibition of YAP-TEAD inhibitor compounds on human pleural mesothelioma cells NCI-H226

Experimental materials and equipments: Human pleural mesothelioma cells NCI-H226 was purchased from NANJING COBIOER BIOSCIENCES CO., LTD. RPMI-1640 medium (Bio-Channel), DMSO (dimethyl sulfoxide), CCK8 (WST-8) Cell Assay Kit (Beyotime), 0.25% EDTA-Tripsin (trypsin digest), 1xPBS (phosphate buffer, PH7.2), 96-well plate (Corning), fetal bovine serum (FBS), 10,000 U/mL penicillin-G/streptomycin, high-speed freezing centrifuge (EPPENDORF 5810R), enzyme-linked immunosorbent assay (Tecan Spark).

### Experimental preparation:

### 1. Cell plating

A) Tumor cells were cultured to 80-90% density in RPMI-1640 (containing 10% FBS and 100U/mL penicillin-G/streptomycin) under the condition of 37°C, 5% CO2 and saturated humidity.
B) The culture medium was removed from the 10cm culture dish.
C) Cells were washed with 10 ml 1xPBS.
D) 4 ml of 0.25% EDTA-Tripsin was added to digest pancreatin for 5 minutes in an incubator at 37°C and 5% CO2. Then the mixture was transferred to a 15 ml centrifuge tube, and centrifuged for 5 minutes at 200g. The supernatant was discarded to obtain cell precipitation.
E) The cell precipitation was resuspended with 4 ml of DMEM medium, counted and adjusted to 10,000 cells/ml.
F) The cell suspension was added to a 96-well plate with a volume of 100 µL per well and cultured overnight in an incubator at 37°C and 5% CO2.

### 2. Compound Treatment

### Compound dilution

A) Preparation of gradient dilution solutions of the tested compounds: the tested compounds was configured to 1 mM stock solution. Then 1.5 µl of stock solution was dissolved in 1.5ml of DMSO-free culture solution, and then 3-fold continuous gradient dilution with 0.1% DMSO culture solution was performed to get a total of 9 concentrations. The diluted concentrations of the compound were as follows:
   333.33 nM, 111.11 nM, 37.03 nM, 12.35 nM, 4.15 nM, 1.37 nM, 0.46 nM, 0.15 nM
B) After full mixing, 100 µL of culture compound solution was taken to replace the culture solution in the cell culture plate, with 4 duplicated wells at each concentration;
C) The cells were transferred to the incubator to incubate for 3 days.

### 3. CCK8 (WST-8) cells analysis and detection

A) the cell culture plate was taken out and added with 10 µl of CCK-8 (WST-8) solution per well in the biosafety cabinet;
B) The cell culture plate was put back into the incubator and continued to incubate for 3 hours;
C) 450 nm wavelength was selected to measure absorbance value on TECAN enzyme linked immunosorbent assay detector.

### 4. Data Analysis

The following formula was used to calculate the cell viability (% Cell Viability); $Cell Viability \left(%\right) = \left[A\left(Drug-added\right)-A\left(Blank\right)\right] / \left[A\left(0 Drug-added\right)-A\left(blank\right)\right] \times 100$

A (Drug-added): The absorbance of wells containing cells, CCK8 solution, and drug solution.

A (Blank): The absorbance of wells containing culture medium and CCK8 solution, but without cells.

A (0 Drug-added): The absorbance of wells containing cells, CCK8 solution, but without drug solution.

Cell viability: Cell proliferation viability or cytotoxicity viability was determined by the IC50 value obtained by curve fitting using GraphPad Prism 8 software.

### Example 2: YAP-TEAD Inhibitor Activity was detected by Nano Luciferase Method

(1) Experimental materials and equipments: 293T cells were purchased from NANJING COBIOER BIOSCIENCES CO., LTD. DMEM medium (High sugar, no phenol red, Bio-Channel), DMSO (dimethyl sulfoxide), Lipo6000^{™} transfection reagent (Beyotime), pGL3B-8xGTiiC-nLuc-CMV-fLuc plasmid, 0.25% EDTA-Tripsin (trypsin digest), 1xPBS (phosphate buffer, PH7.2), 96-well white cell culture plate (PerkinElmer), fetal bovine serum (FBS), 10,000 U/mL penicillin-G/streptomycin, high-speed freezing centrifuge (EPPENDORF 5810R), 37°C CO2 incubator, Vi-cell^{®} Cell Counter, Envision Microplate Reader (PerkinElmer).

### Reagent

| Reagent | | Catalog number | concentration |
|---|---|---|---|
| Nano-luciferase detection reagent | MES pH 6.0 | 69892-25G | 100 mM |
| | CDTA | CAS:125572-95-4 | 1 mM |
| | Tergitol | NP10-100ML | 0.5% (v/v) |
| | Mazu DF 204 | A8311-50ML | 0.05% (v/v) |
| | KCl | CAS:7447-40-7 | 150 mM |
| | DTT | Roche 10197777001 | 1 mM |
| | thiourea | T8656-50G | 35 mM |
| | Furimazine | 1374040-24-0 | 20 µM, adding before use |

### (2) 293T cell transient transfection

The revived 293T cells were inoculated into a 10cm culture dish and placed in an incubator with 5% CO2, and cultured at constant temperature of 37 °C. To ensure transfection efficiency, cells at logarithmic phase (cell density of approximately 50% -70%) should be used.

On the day before transfection, cells at the logarithmic phase were digested with Trypsin-EDTA, and culture medium was added to terminate digestion. The cell suspension was prepared by blowing and mixing using a pipette.

Cell concentration was measured using Vi-cell, and the cell suspension was diluted to a suspension of 5 * 10^{^ 5} cells per milliliter. After preparing the cell suspension, the suspension was gently mixed. 10ml of liquid was added to a 10cm culture dish. In this way, the number of cells in each 10cm culture dish was 5 * 10 ^{^ 6}. The cells were incubated at a constant temperature of 37 °C for 1 day in an incubator with 5% CO2.

Two clean and sterile centrifuge tubes were taken and added with 750µL opti-MEM Medium without antibiotics and serum, respectively. Then one tube was added with 15 µg Plasmid (pGL3B-8xGTiiC-nLuc-CMV-fLuc), and gently blew and mixed well using a pipette; the other tube was added with Lipo6000 transfection reagent, and gently blew and mixed using a pipette. After standing at room temperature for 5 minutes, the culture solution containing DNA was added gently to the culture solution containing Lipo6000 transfection reagent, and mixed well by gently inverting the centrifuge tube, then standing at room temperature for 5 minutes.

The above mixture was added evenly to a 10cm culture dish and replaced with fresh complete culture medium after 6 hours of cultivation.

### (3) 96-well plates plating

After transfection for 1 day, cells were digested with Trypsin EDTA, and culture medium was added to terminate the digestion. The cell suspension was prepared by blowing and mixing using a pipette.

Cell concentration was measured using Vi-cell, and the cell suspension was diluted to a suspension of 20000 cells per milliliter.

After preparing the cell suspension, the suspension was gently mixed, and added to 96-well plate with 100 µL per well. Thus, the density of the cells to be tested is 2000 cells per well.

### (4) addition of compounds

The inoculated cell culture plate was placed in the incubator for cultivation, and after about 24 hour, compounds with concentration gradient were added.

10mM compound stock solution was diluted with culture medium to 50 µM. Compound solution of 50 µM was added to the third column of the deep well plate, 216 µL of culture medium containing 0.5% DMSO was added to the fourth to eleventh columns, successively.

Gradient dilution: taking 100 µL of solution from the third column and adding the solution to the fourth column and mixing well; then taking 100 µL of solution from the fourth column again and adding the solution to the fifth column, repeating this operation until the eleventh column.

Taking 25 µE of compound solution from the deep well plate and adding it to a 96-well culture plate using a multi-channel pipette. Repeating four times on the 96 well plate for each compound. Finally, a concentration gradient of 1 :3.16 was formed in the 96-well plate with a maximum concentration of 10,000 nM. The

### (5) addition of Nano luciferase detection reagent and read

The 96-well plates were incubated at a constant temperature of 37 °C for 48 hours in an incubator with 5% CO₂, and then the 96-well plates were taken out for equilibrium at room temperature for 10 minutes.

Each well was added with 100 µL of detection reagent, placed on a horizontal shaker and shaken at low speed for 10 minutes to fully lyse cells. The fluorescence value of each well was detected using the PerkinElmer Envision Microplate Reader.

### (6) Result Calculation

Taking 0nM as a control, the value of each well was converted into percentage, and IC50 was calculated by nonlinear fitting using [Inhibitor] vs. response (three parameters) in GrahpPad prism software.

As shown in table 1, wherein A≤1µM; 1µM<B<5µM; C≥5µM

**Table 1**

| Example | Structure | NCI-H226 activity (IC₅₀) | Nano-luciferase detection (IC₅₀) |
|---|---|---|---|
| T-1 | 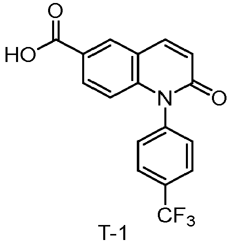 | B | B |
| T-3 | 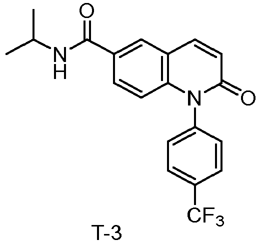 | A | A |
| T-4 | 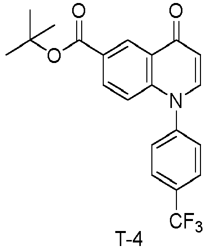 | | B |
| T-5 | 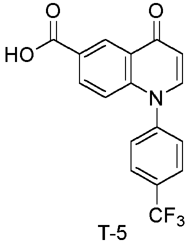 | B | A |
| T-6 | 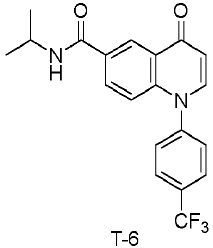 | A | A |
| T-7 | 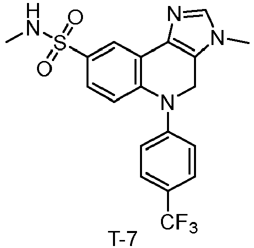 | A | A |
| T-15 | 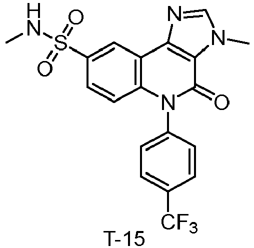 | A | A |
| T-16 | 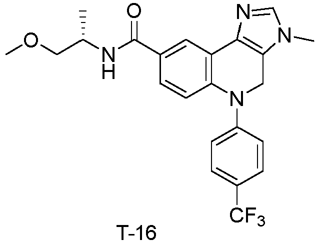 | A | A |
| T-17 | 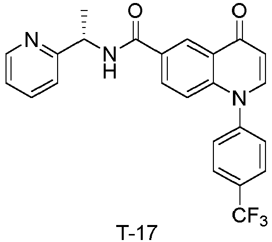 | B | A |
| T-18 | 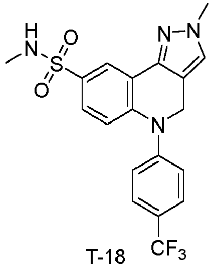 | A | A |
| T-19 | 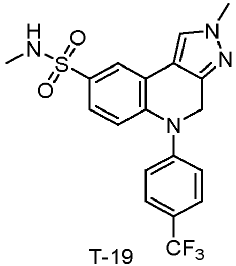 | A | A |
| T-20 | 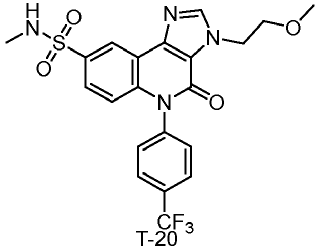 | A | A |
| T-21 | 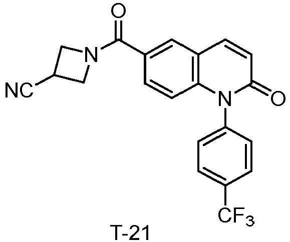 | B | B |
| T-22 | 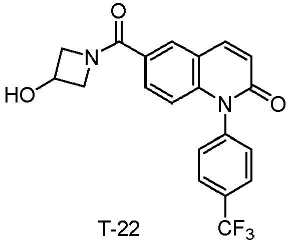 | B | B |
| T-23 | 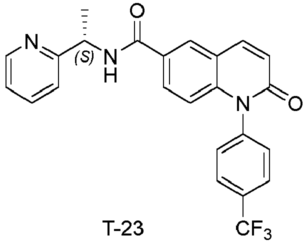 | B | A |
| T-24 | 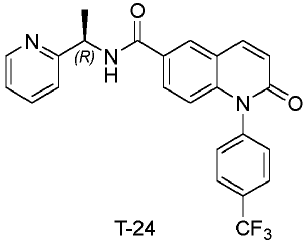 | B | B |
| T-31 | 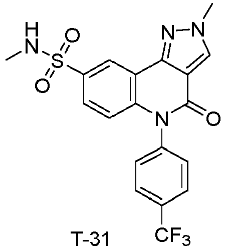 | A | A |
| T-32 | 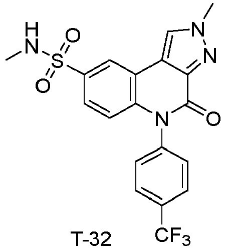 | A | A |
| T-33 | 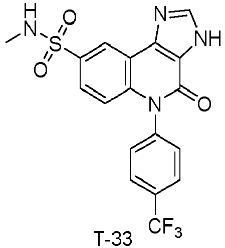 | A | A |
| T-34 | 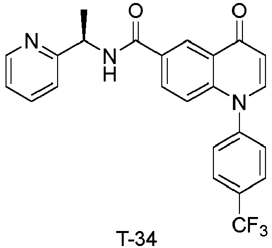 | B | A |
| T-35 | 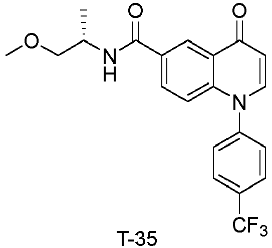 | B | A |
| T-36 | 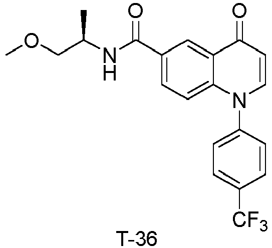 | | B |
| T-37 | 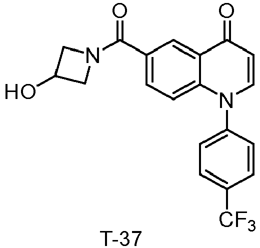 | | B |
| T-38 | 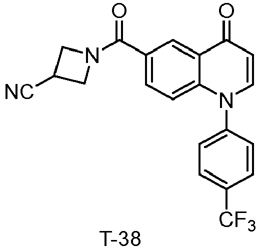 | B | B |
| T-39 | 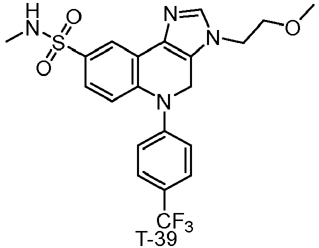 | A | A |
| T-40 | 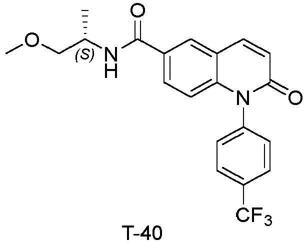 | B | B |
| T-41 | 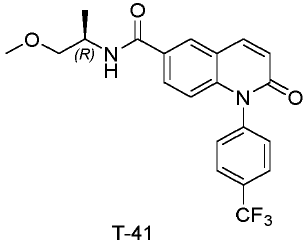 | | B |
| T-45 | 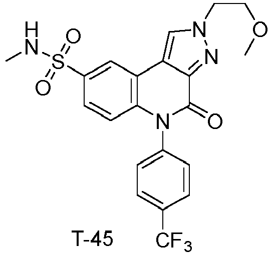 | A | A |
| T-46 | 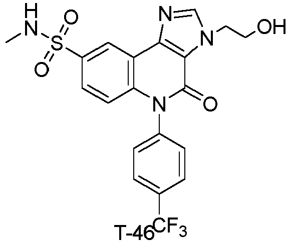 | A | A |
| T-47 | 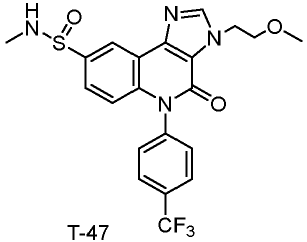 | A | A |
| T-48 | 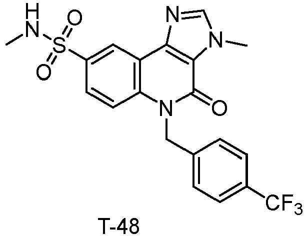 | A | A |
| T-49 | 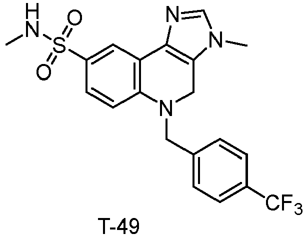 | A | A |
| T-50 | 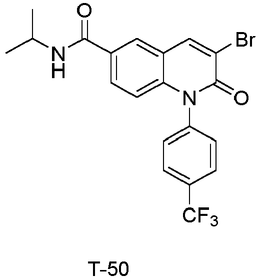 | B | B |
| T-51 | 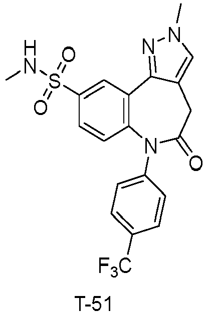 | A | A |
| T-52 | 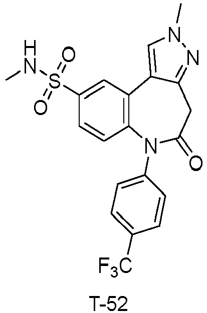 | A | A |
| T-54 | 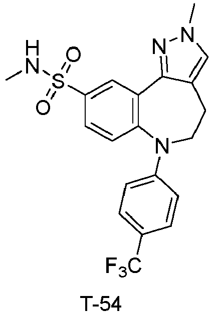 | A | A |
| T-55 | 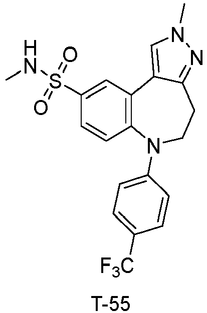 | A | A |
| T-57 | 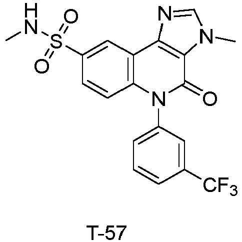 | A | A |
| T-58 | 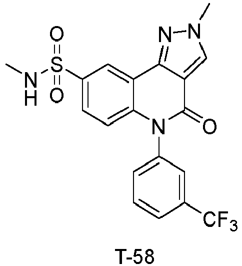 | A | A |
| T-60 | 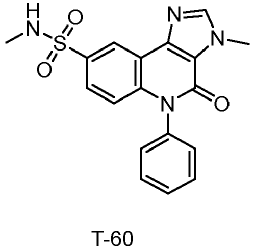 | A | A |
| T-61 | 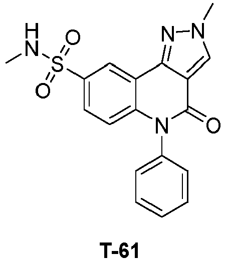 | A | A |
| T-65 | 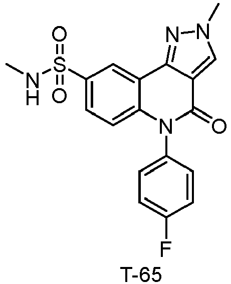 | A | A |
| T-66 | 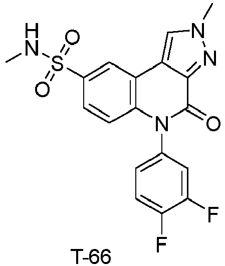 | A | A |
| T-68 | 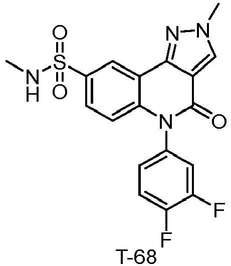 | A | A |
| T-72 | 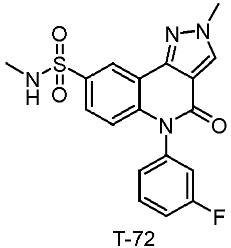 | A | A |
| T-75 | 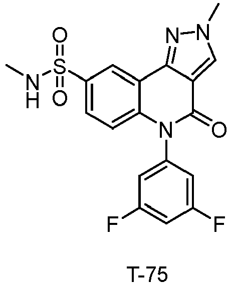 | A | A |
| T-79 | 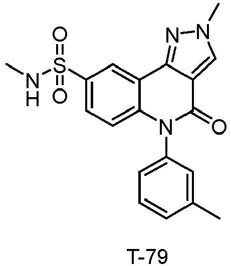 | B | A |
| T-82 | 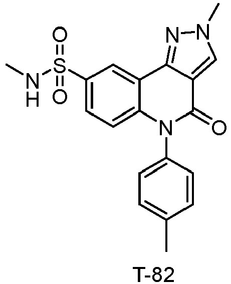 | A | A |
| T-86 | 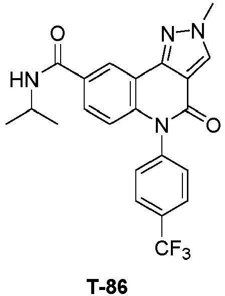 | A | A |
| T-99 | 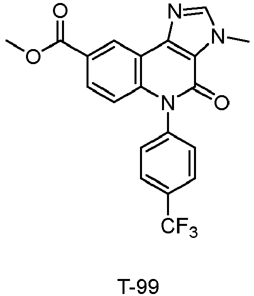 | B | B |
| T-100 | 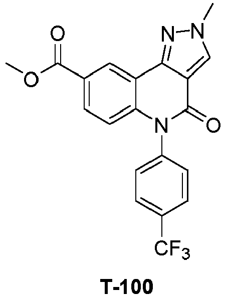 | B | B |
| T-101 | 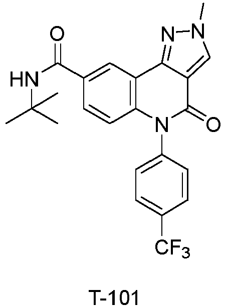 | A | A |
| T-102 | 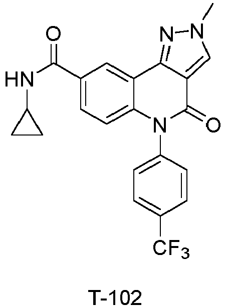 | A | A |
| T-103 | 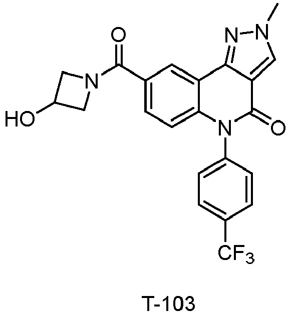 | B | |
| T-104 | 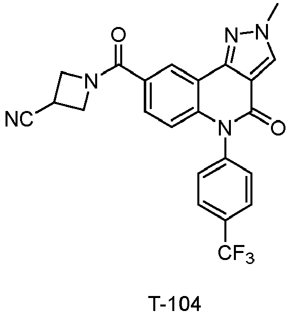 | B | |
| T-105 | 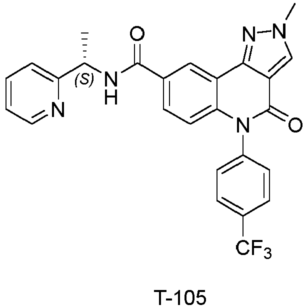 | A | A |
| T-106 | 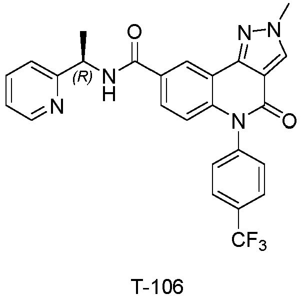 | B | |
| T-107 | 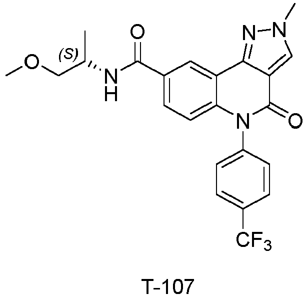 | A | A |
| T-110 | 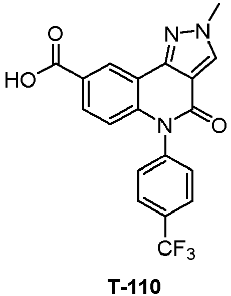 | B | B |
| T-111 | 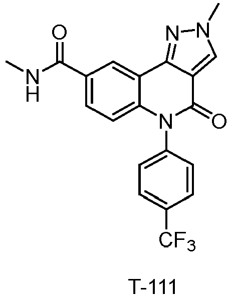 | A | A |
| T-112 | 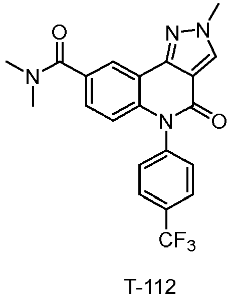 | B | |
| T-113 | 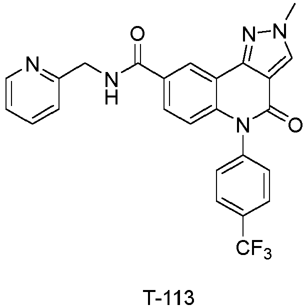 | A | A |
| T-114 | 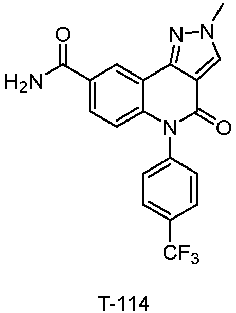 | A | B |
| T-115 | 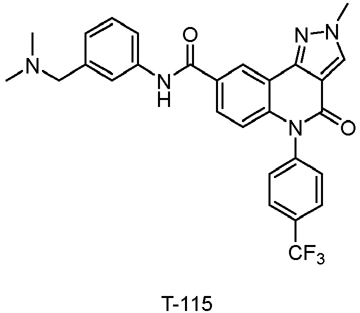 | B | B |
| T-116 | 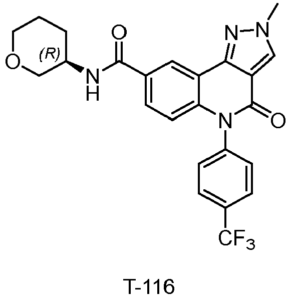 | | |
| T-117 | 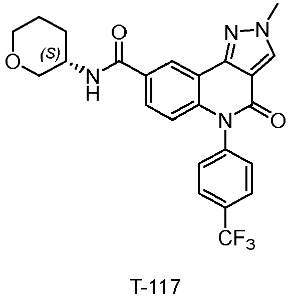 | | |
| T-118 | 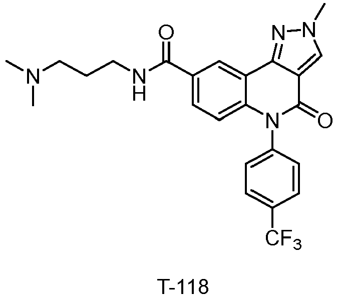 | B | B |
| T-119 | 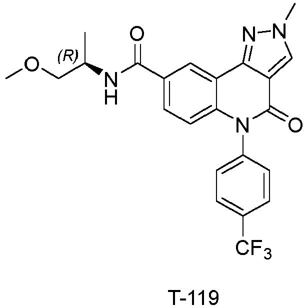 | B | |
| T-120 | 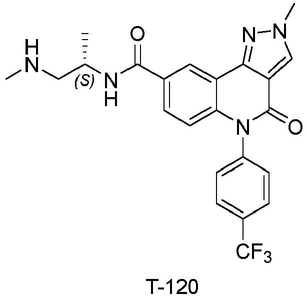 | B | B |
| T-121 | 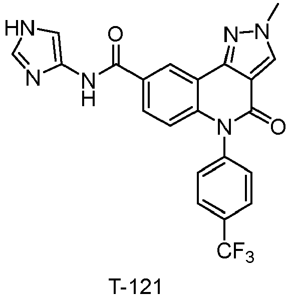 | B | B |
| T-122 | 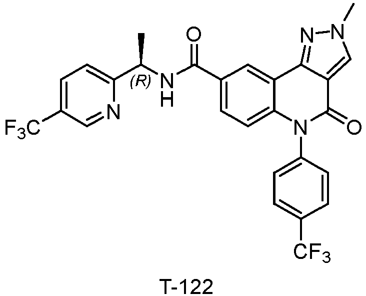 | | |
| T-123 | 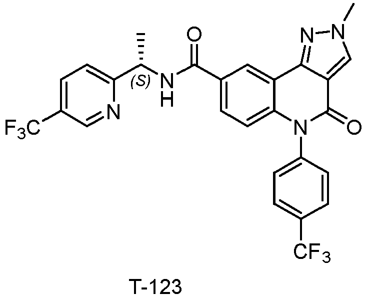 | B | B |
| T-124 | 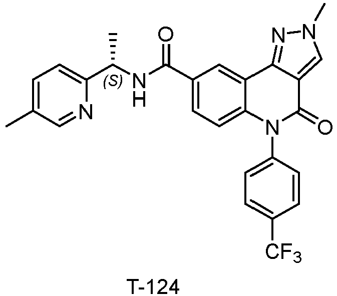 | B | B |
| T-125 | 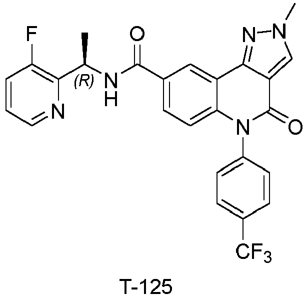 | B | B |
| T-126 | 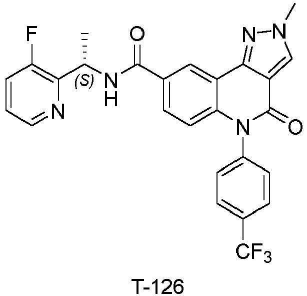 | A | A |
| T-127 | 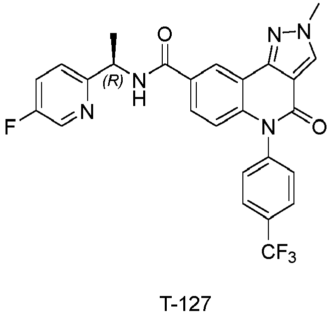 | B | B |
| T-128 | 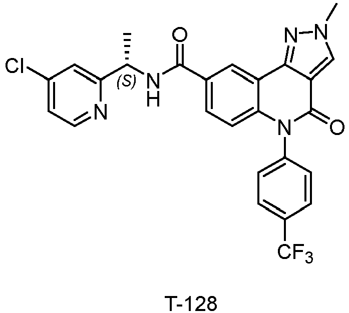 | A | A |
| T-129 | 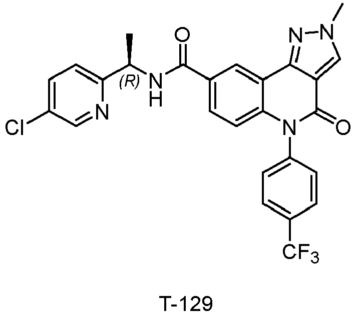 | | |
| T-130 | 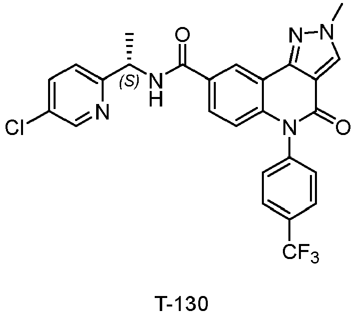 | B | B |
| T-131 | 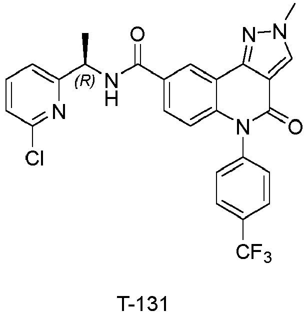 | | |
| T-132 | 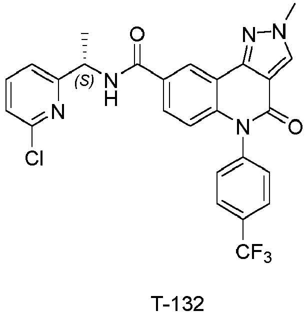 | B | B |
| T-133 | 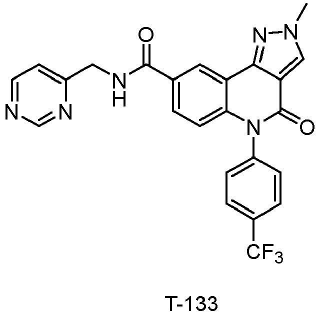 | B | B |
| T-134 | 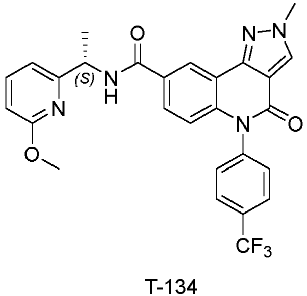 | A | A |
| T-135 | 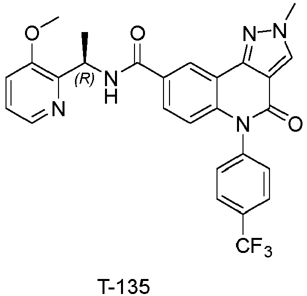 | B | B |
| T-136 | 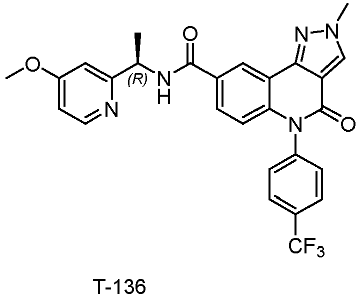 | B | B |
| T-137 | 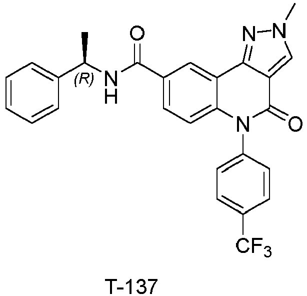 | | |
| T-138 | 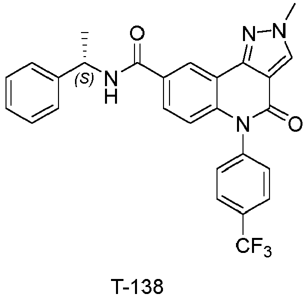 | A | A |
| T-139 | 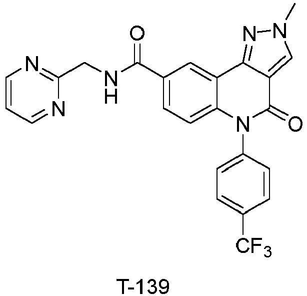 | B | B |
| T-140 | 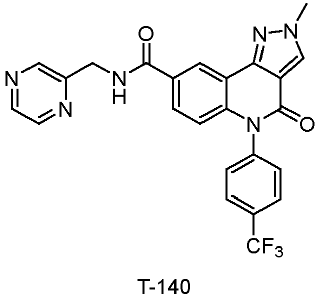 | B | B |
| T-141 | 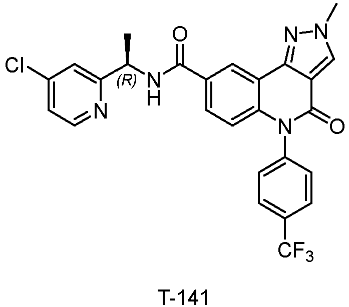 | | |
| T-142 | 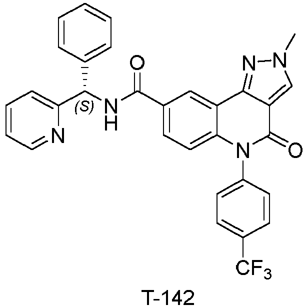 | B | B |
| T-143 | 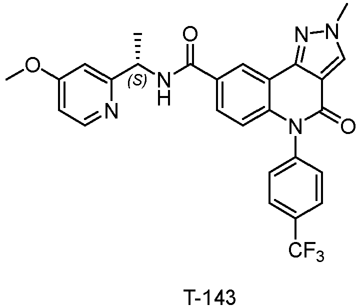 | B | B |
| T-144 | 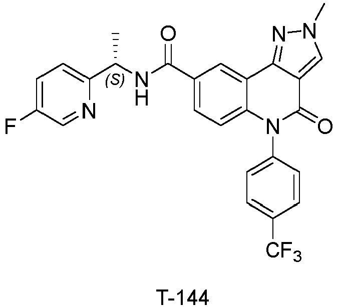 | B | B |
| T-145 | 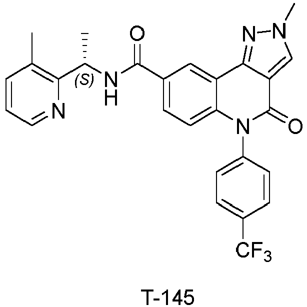 | A | A |
| T-146 | 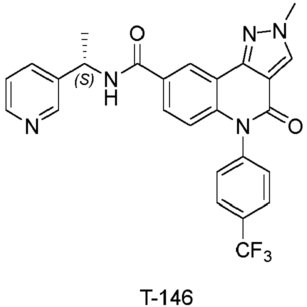 | B | B |
| T-147 | 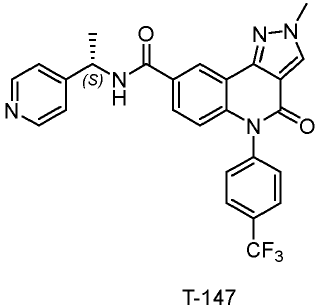 | B | B |
| T-148 | 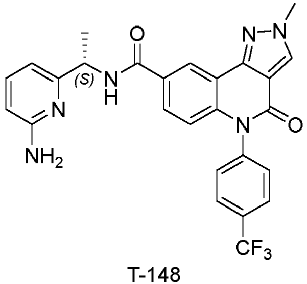 | A | A |
| T-149 | 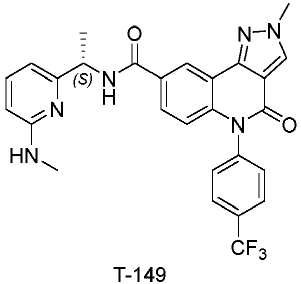 | A | A |
| T-150 | 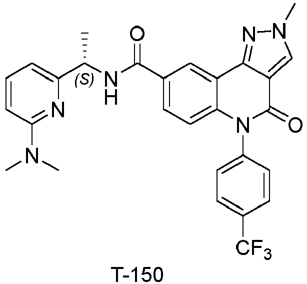 | A | A |
| T-151 | 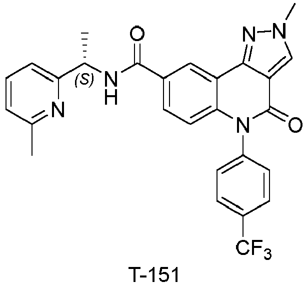 | A | A |
| T-152 | 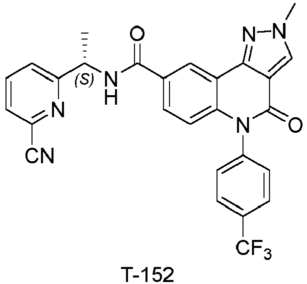 | A | A |
| T-153 | 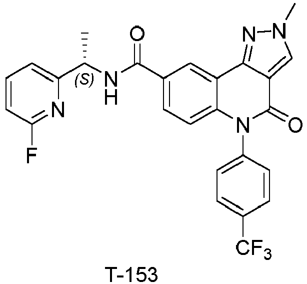 | A | A |
| T-154 | 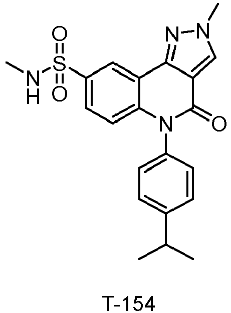 | A | A |
| T-155 | 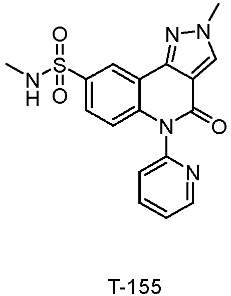 | B | B |
| T-156 | 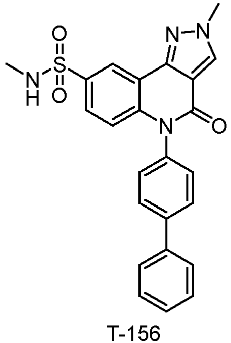 | B | B |
| T-157 | 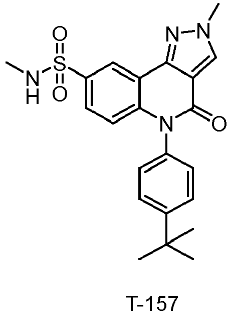 | A | A |
| T-158 | 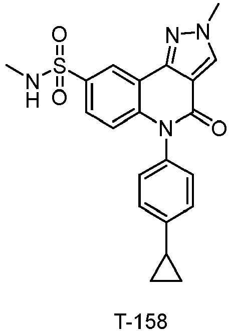 | A | A |
| T-159 | 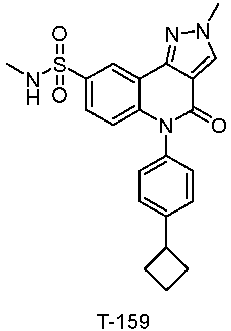 | A | A |
| T-160 | 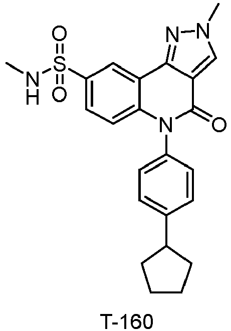 | A | A |
| T-161 | 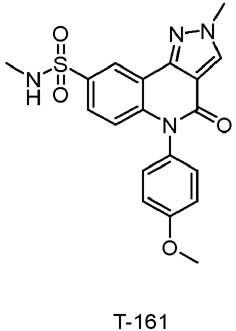 | B | B |
| T-162 | 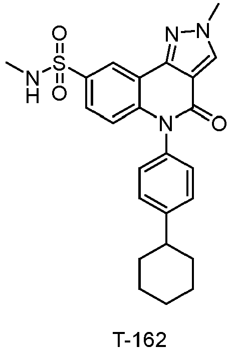 | B | B |
| T-163 | 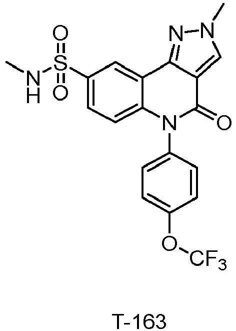 | A | A |
| T-164 | 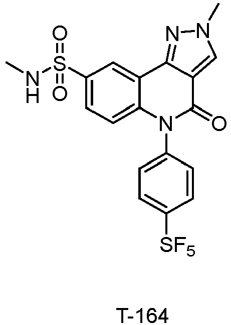 | A | A |
| T-165 | 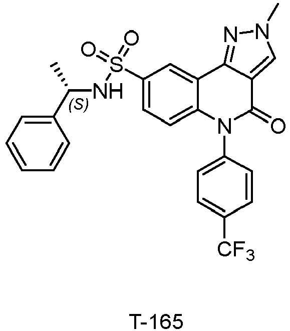 | B | B |
| T-166 | 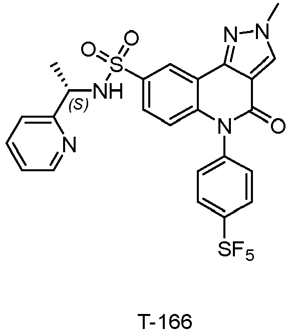 | B | B |
| T-167 | 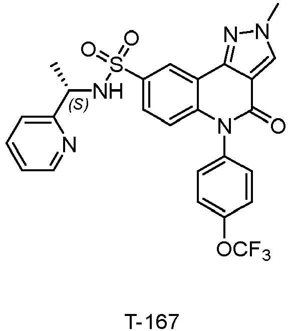 | B | B |
| T-170 | 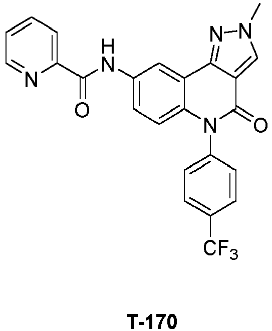 | A | A |
| T-172 | 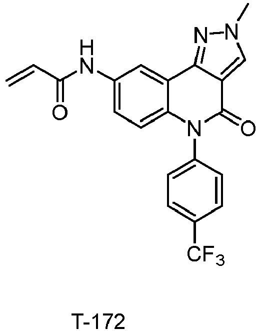 | A | A |
| T-186 | 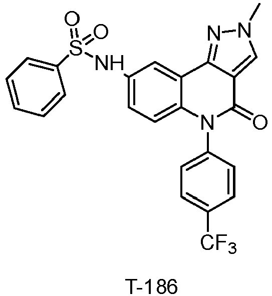 | A | A |
| T-168 | 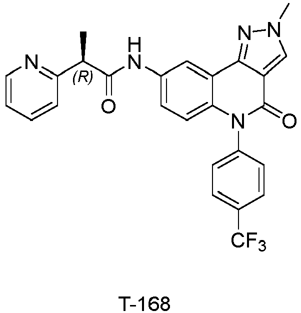 | B | B |
| T-169 | 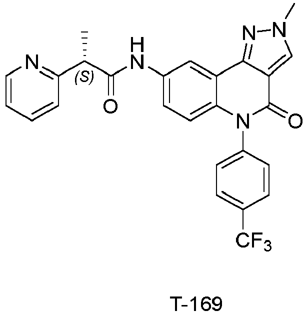 | B | B |
| T-171 | 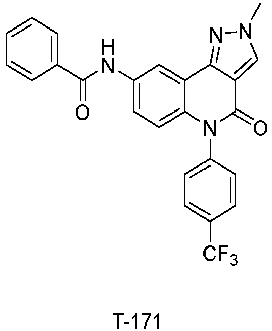 | A | A |
| T-173 | 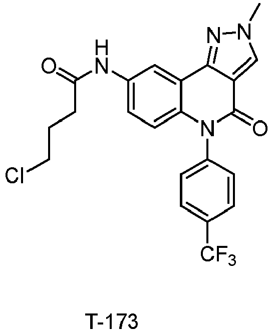 | B | B |
| T-174 | 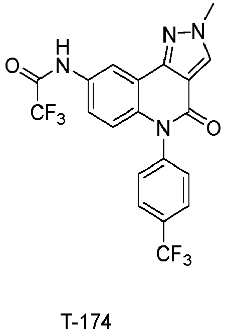 | A | A |
| T-175 | 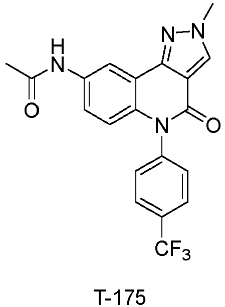 | A | A |
| T-176 | 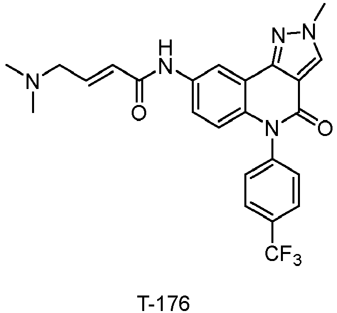 | A | A |
| T-177 | 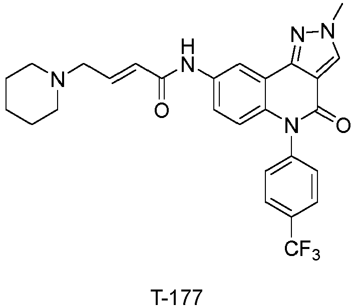 | A | A |
| T-178 | 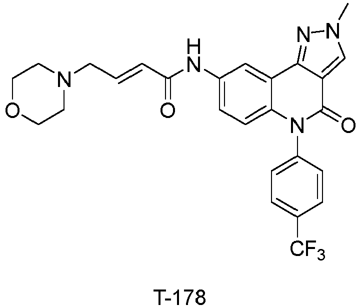 | A | A |
| T-179 | 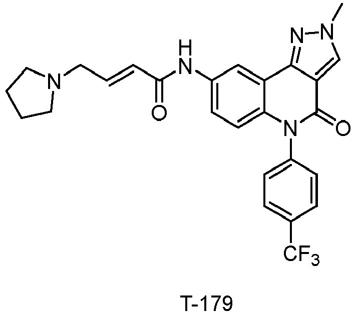 | A | A |
| T-180 | 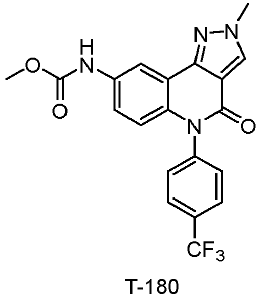 | A | A |
| T-181 | 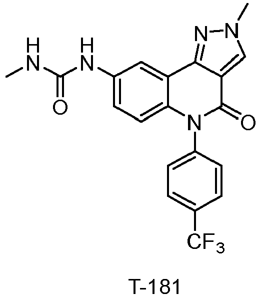 | A | A |
| T-182 | 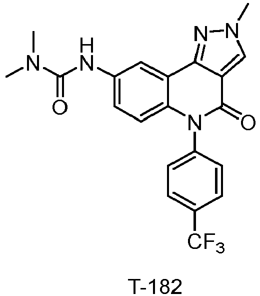 | A | A |
| T-183 | 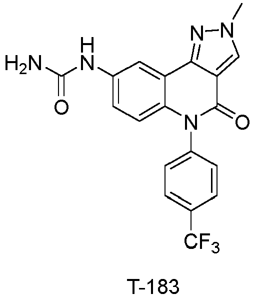 | A | A |
| T-184 | 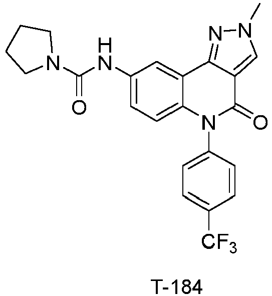 | A | A |
| T-185 | 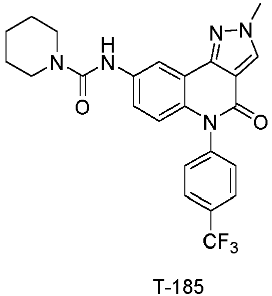 | A | A |
| T-187 | 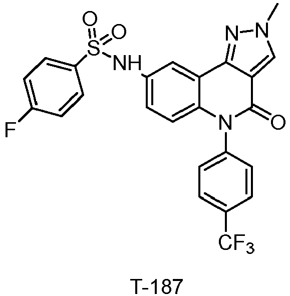 | A | A |
| T-188 | 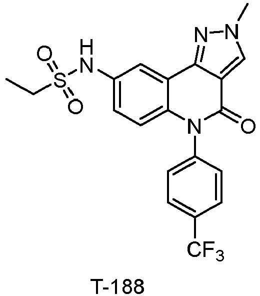 | A | A |
| T-189 | 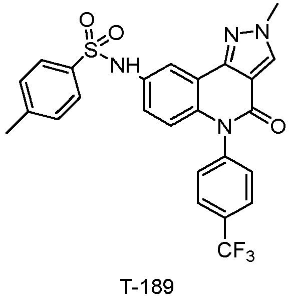 | A | A |
| T-190 | 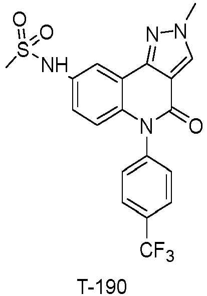 | A | A |
| T-191 | 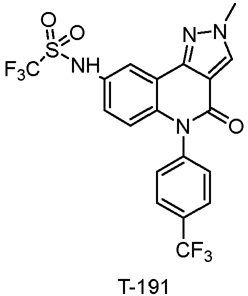 | A | A |
| T-192 | 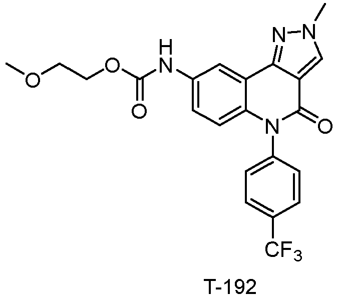 | A | A |
| T-193 | 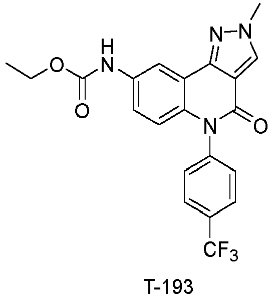 | A | A |
| T-194 | 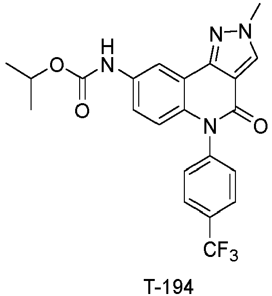 | A | A |
| T-195 | 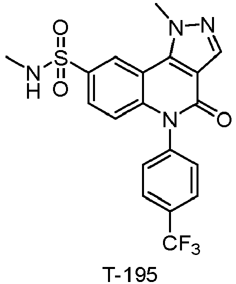 | B | B |
| T-199 | 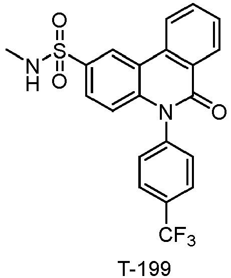 | B | B |
| T-227 | 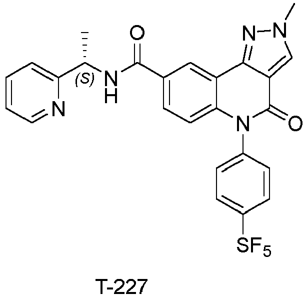 | A | A |
| T-228 | 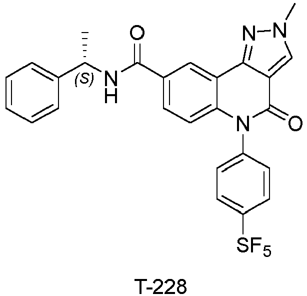 | A | A |
| T-229 | 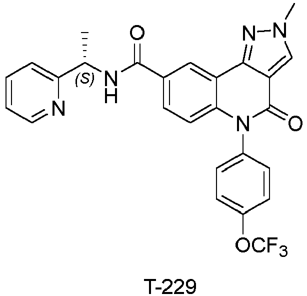 | A | A |
| T-230 | 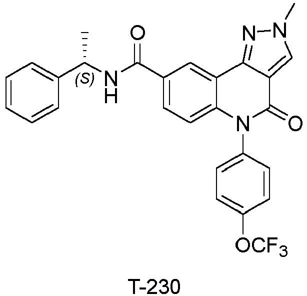 | A | A |
| T-231 | 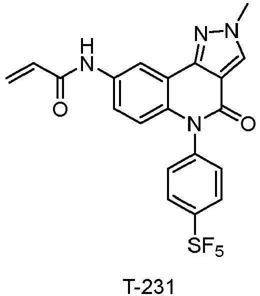 | A | A |
| T-232 | 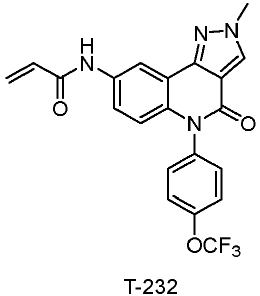 | A | A |
| T-233 | 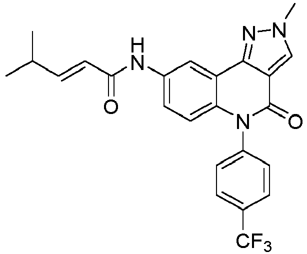 | A | A |
| T-234 | 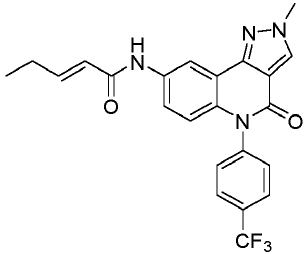 | A | A |
| T-235 | 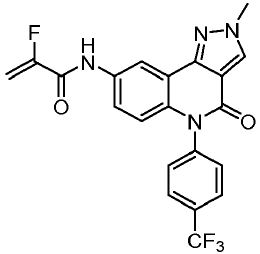 | A | A |
| T-236 | 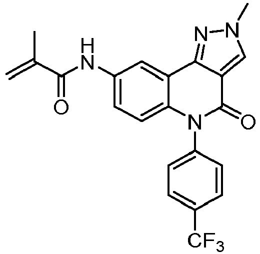 | A | A |
| T-237 | 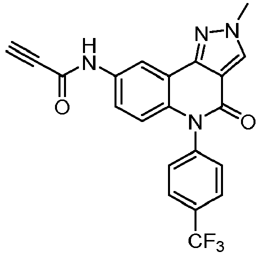 | A | A |
| T-238 | 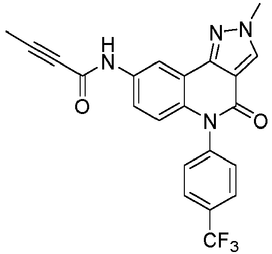 | A | A |
| T-239 | 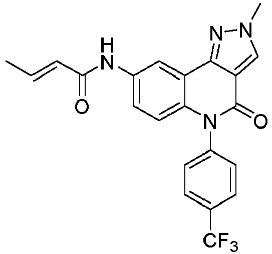 | A | A |
| T-240 | 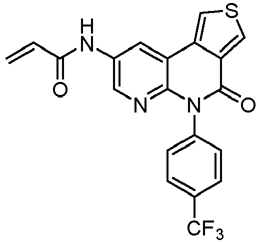 | A | A |
| T-241 | 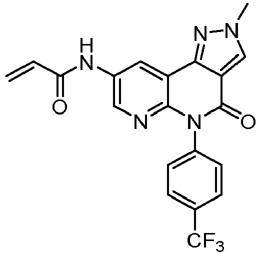 | A | A |
| T-242 | 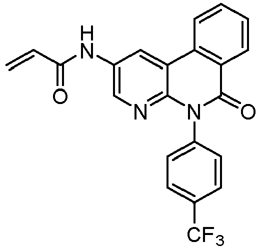 | A | A |
| T-243 | 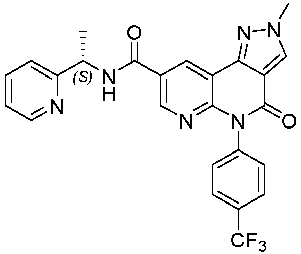 | A | A |
| T-244 | 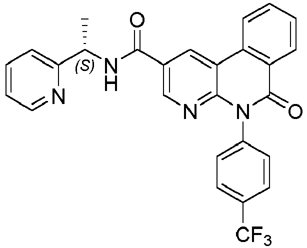 | A | A |
| T-245 | 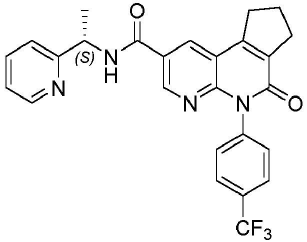 | A | A |
| T-246 | 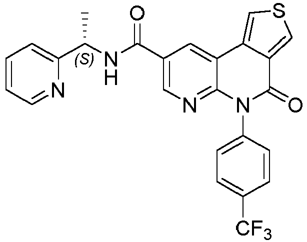 | A | A |
| T-247 | 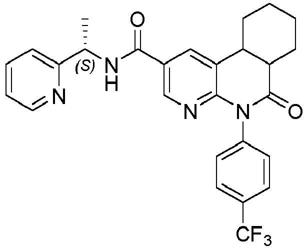 | A | A |
| T-248 | 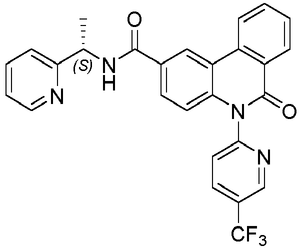 | A | A |
| T-249 | 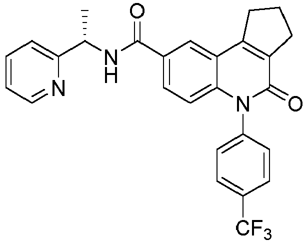 | A | A |
| T-250 | 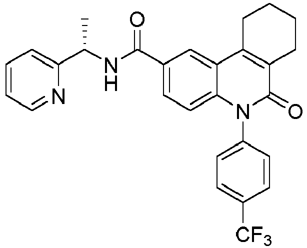 | A | A |
| T-251 | 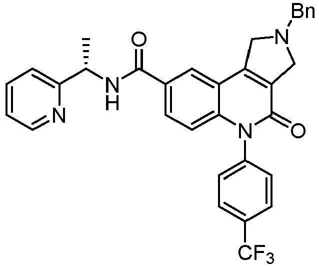 | B | B |
| T-252 | 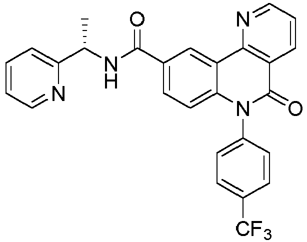 | A | A |
| T-253 | 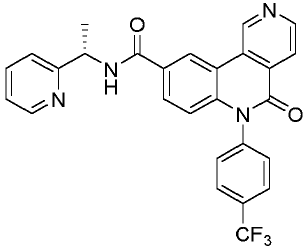 | A | A |
| T-254 | 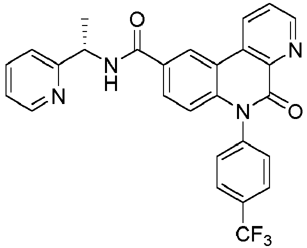 | A | A |
| T-255 | 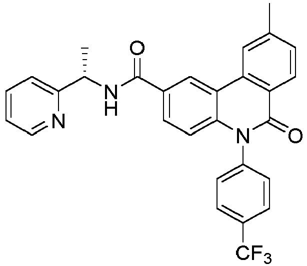 | A | A |
| T-256 | 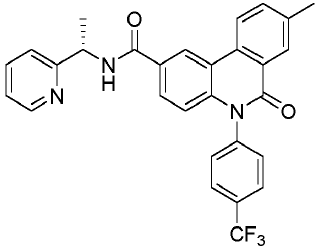 | A | A |
| T-257 | 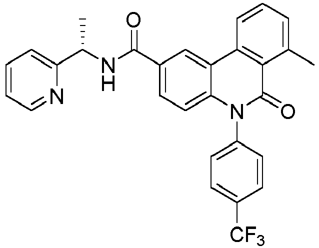 | A | A |
| T-258 | 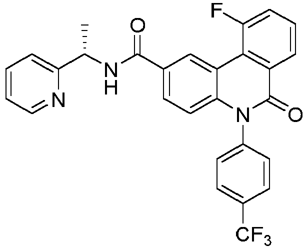 | A | A |
| T-259 | 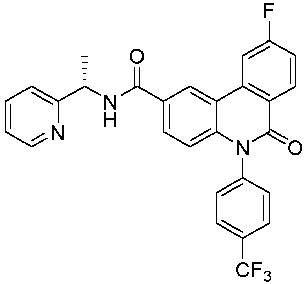 | A | A |
| T-260 | 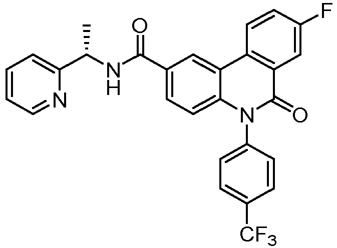 | A | B |
| T-261 | 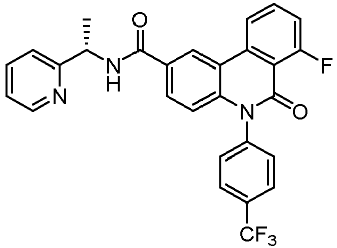 | A | B |
| T-262 | 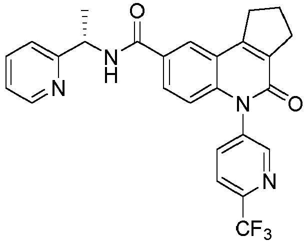 | A | A |
| T-263 | 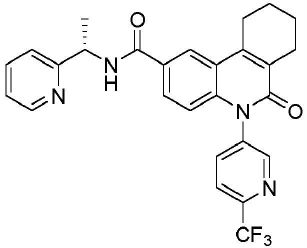 | A | A |
| T-264 | 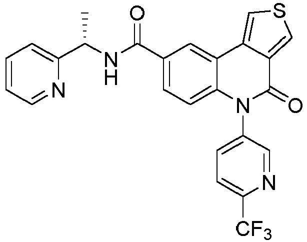 | A | A |
| T-265 | 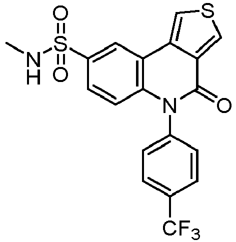 | A | A |
| T-266 | 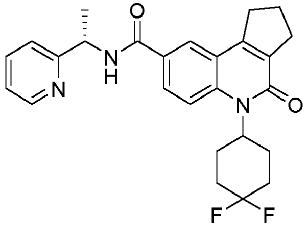 | A | A |
| T-267 | 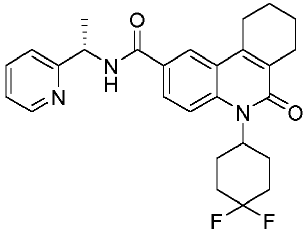 | A | A |
| T-268 | 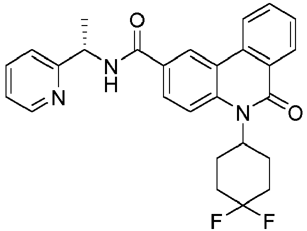 | A | A |
| T-269 | 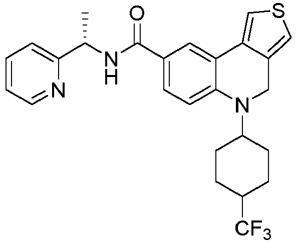 | A | A |
| T-270 | 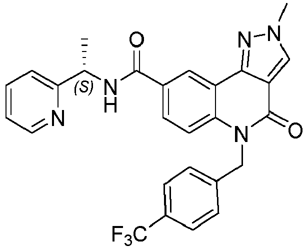 | A | A |
| T-271 | 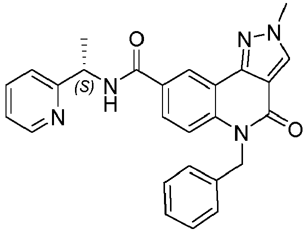 | A | A |
| T-272 | 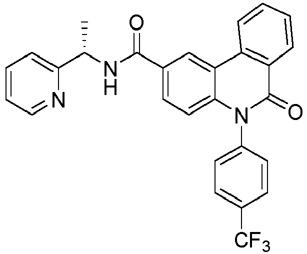 | A | A |
| T-273 | 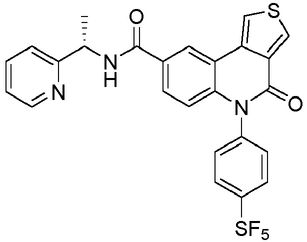 | A | A |
| T-274 | 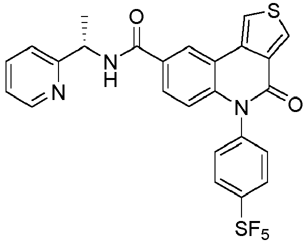 | A | A |
| T-275 | 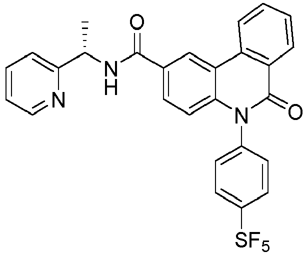 | A | A |
| T-276 | 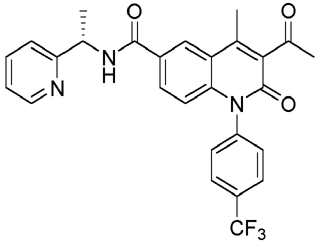 | B | B |
| T-277 | 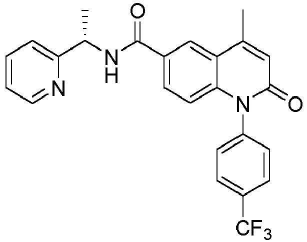 | A | A |
| T-278 | 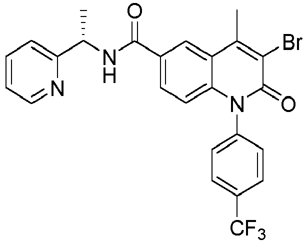 | A | A |
| T-279 | 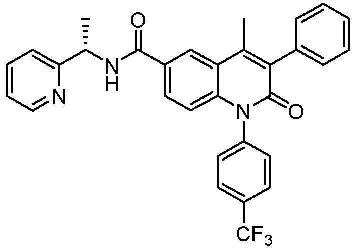 | A | B |
| T-280 | 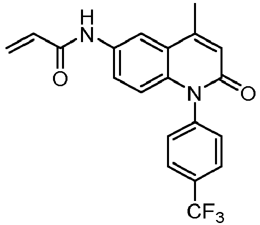 | A | A |
| T-281 | 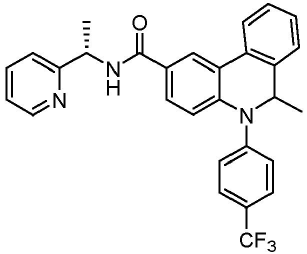 | A | A |
| T-282 | 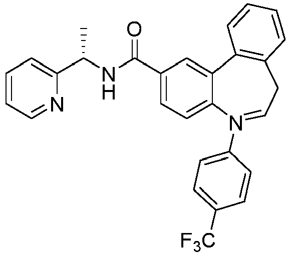 | B | B |
| T-283 | 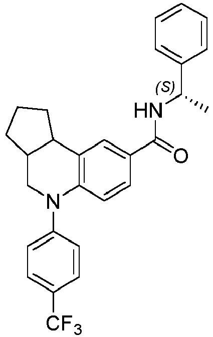 | B | B |
| T-284 | 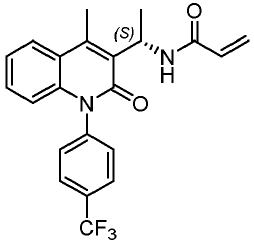 | B | B |
| T-285 | 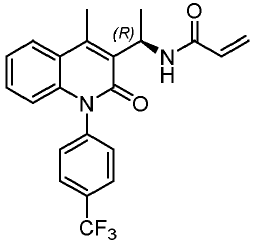 | B | B |
| T-286 | 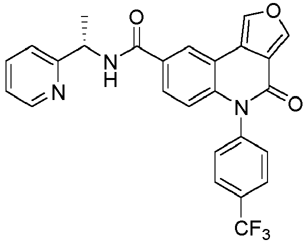 | A | A |
| T-287 | 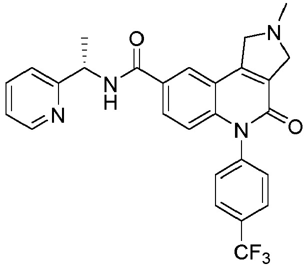 | A | A |
| T-288 | 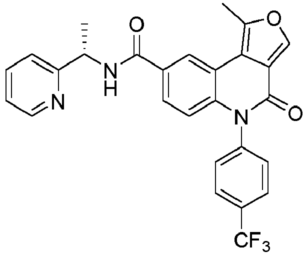 | A | A |
| T-289 | 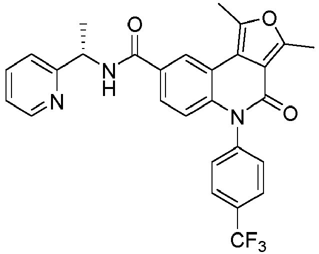 | A | A |
| T-290 | 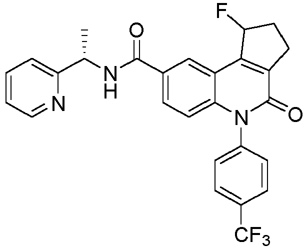 | A | A |
| T-291 | 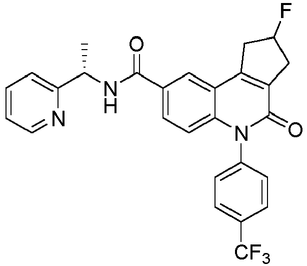 | A | A |
| T-292 | 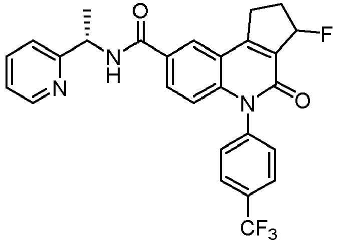 | A | A |
| T-293 | 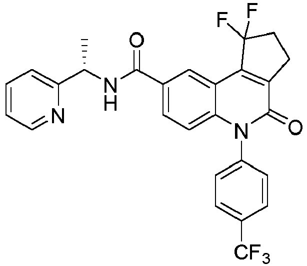 | A | A |
| T-294 | 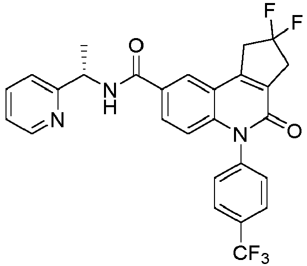 | A | A |
| T-295 | 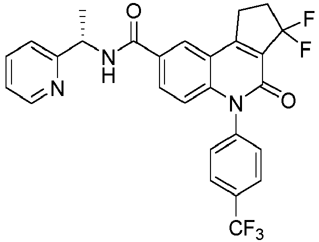 | A | A |
| T-296 | 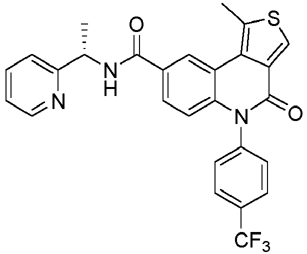 | A | A |
| T-297 | 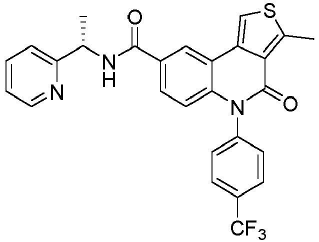 | A | A |
| T-298 | 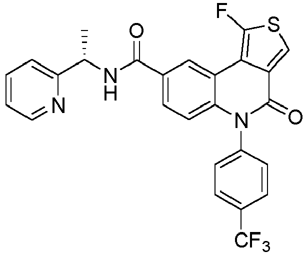 | A | A |
| T-299 | 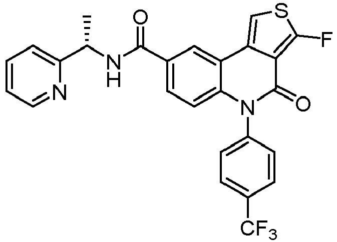 | A | A |
| T-300 | 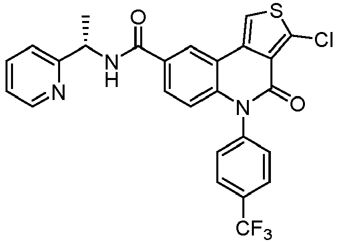 | A | A |
| T-301 | 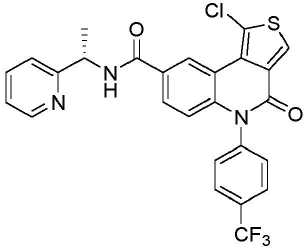 | A | A |
| T-302 | 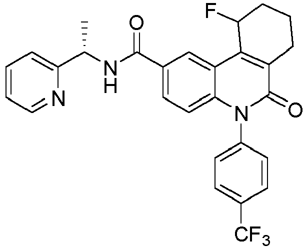 | A | A |
| T-303 | 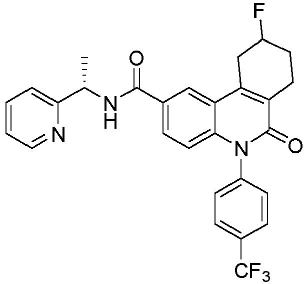 | A | A |
| T-304 | 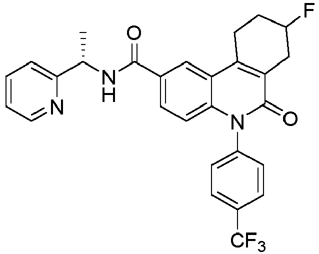 | NA | A |
| T-305 | 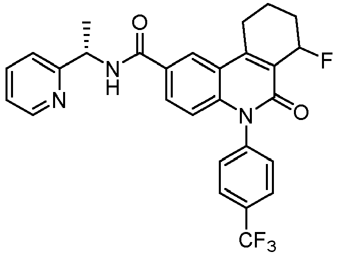 | NA | A |
| T-306 | 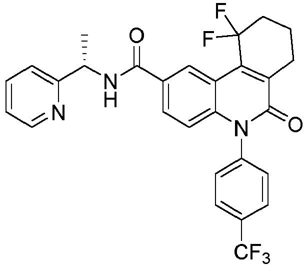 | A | A |
| T-307 | 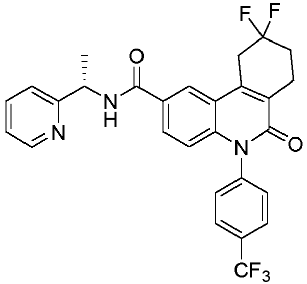 | A | A |
| T-308 | 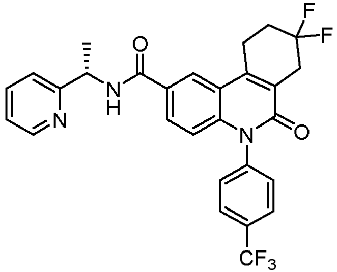 | NA | A |
| T-309 | 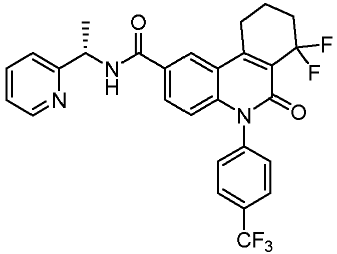 | NA | A |
| T-311 | 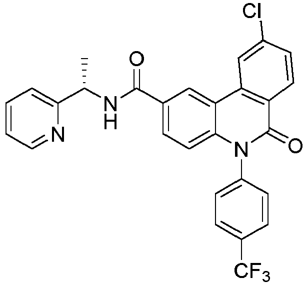 | A | A |
| T-315 | 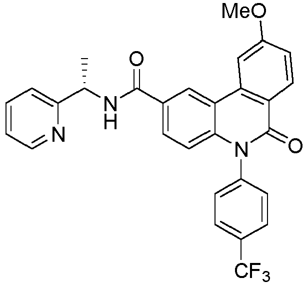 | A | A |
| T-318 | 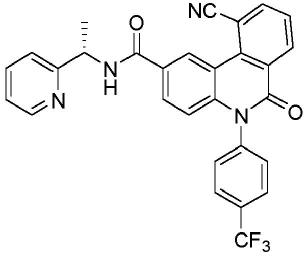 | A | A |
| T-319 | 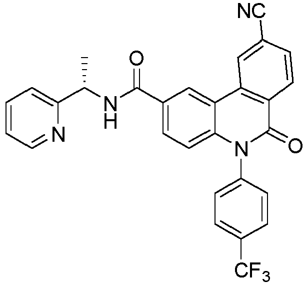 | A | A |
| T-320 | 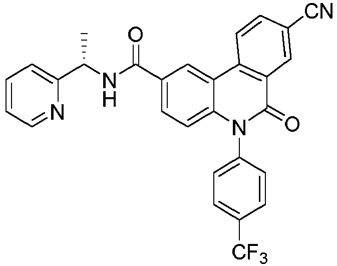 | A | A |
| T-321 | 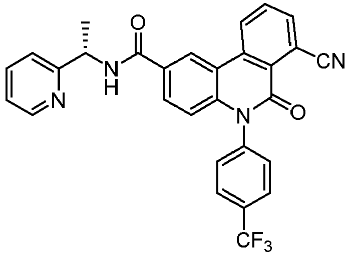 | A | A |
| T-322 | 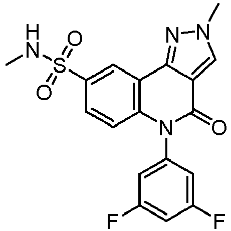 | A | A |
| T-324 | 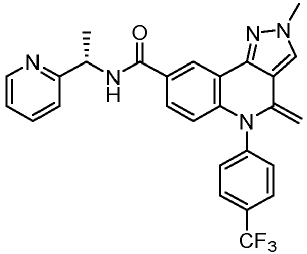 | A | A |
| T-326 | 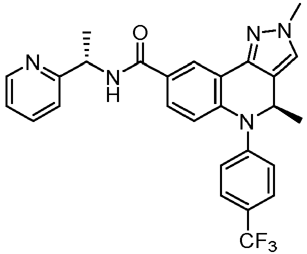 | A | A |
| T-327 | 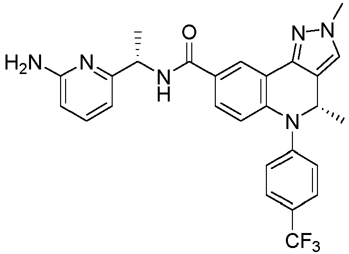 | A | A |
| T-328 | 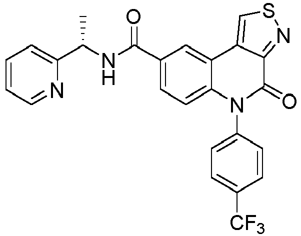 | A | A |
| T-329 | 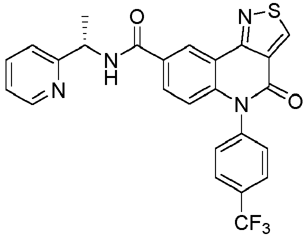 | A | A |
| T-330 | 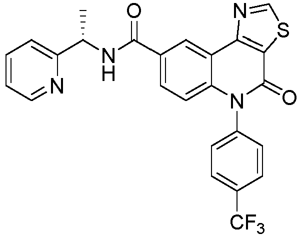 | A | A |
| T-331 | 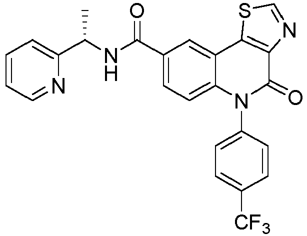 | A | A |
| T-332 | 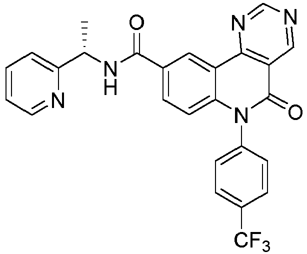 | A | A |
| T-333 | 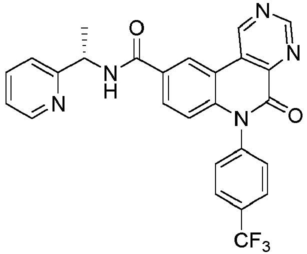 | A | A |
| T-334 | 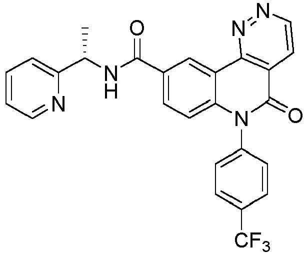 | A | A |
| T-335 | 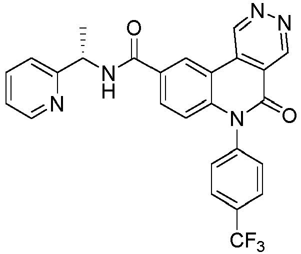 | A | A |
| T-336 | 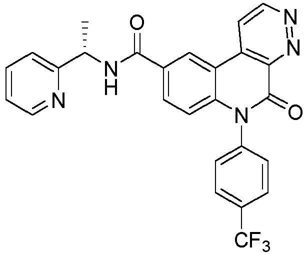 | A | A |
| T-337 | 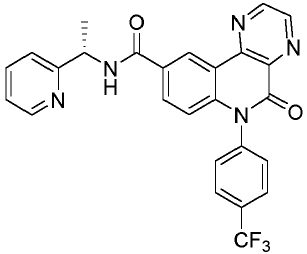 | A | A |
| T-454 | 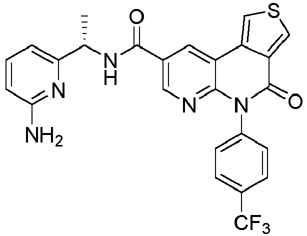 | A | A |
| T-372 | 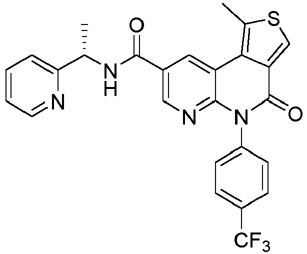 | B | B |
| T-373 | 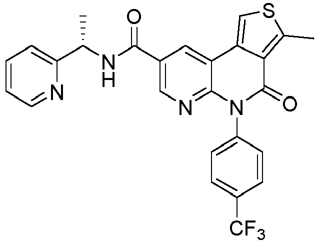 | B | B |
| T-374 | 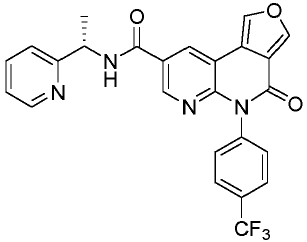 | A | A |
| T-375 | 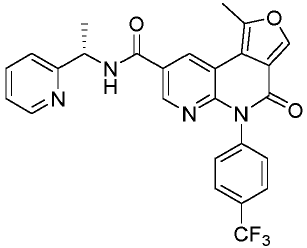 | A | A |
| T-376 | 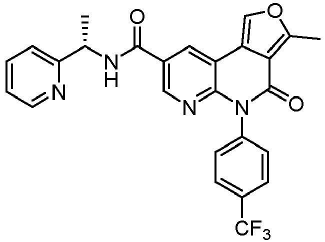 | A | A |
| T-377 | 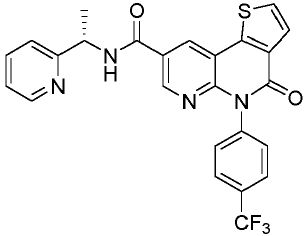 | A | A |
| T-378 | 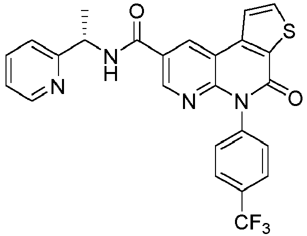 | A | A |
| T-384 | 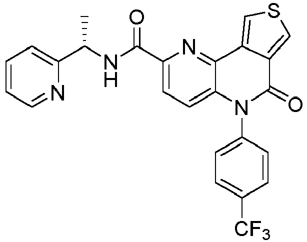 | A | A |
| T-385 | 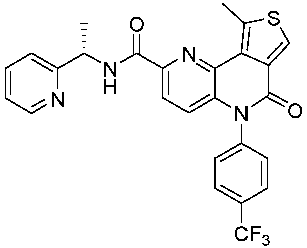 | A | A |
| T-386 | 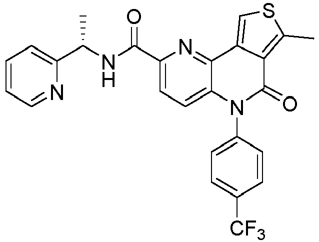 | A | A |
| T-387 | 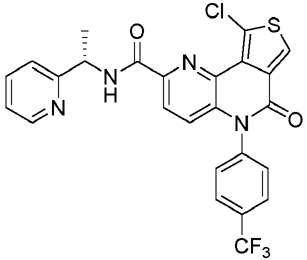 | A | A |
| T-388 | 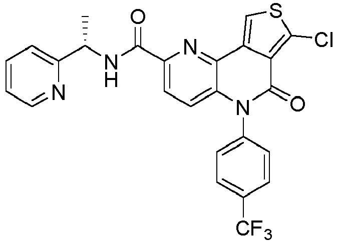 | A | A |
| T-389 | 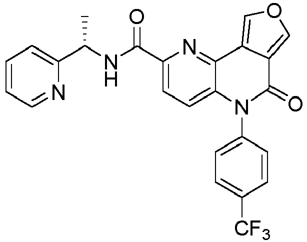 | A | A |
| T-391 | 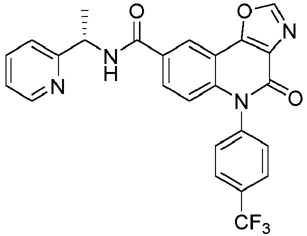 | A | A |
| T-394 | 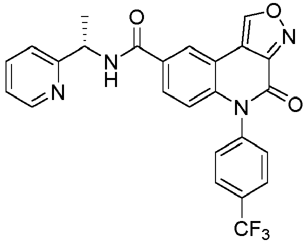 | A | A |
| T-395 | 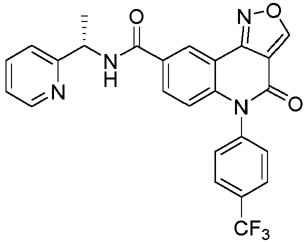 | A | A |
| T-396 | 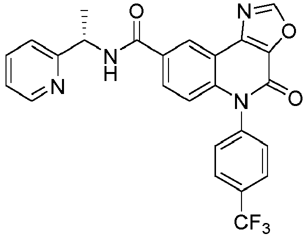 | A | A |
| T-397 | 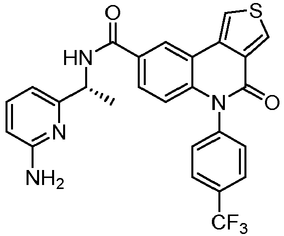 | A | A |
| T-398 | 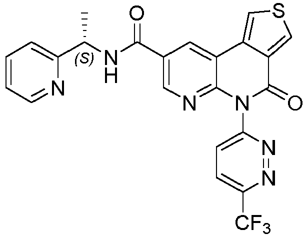 | B | B |
| T-399 | 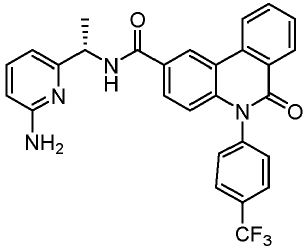 | A | A |
| T-400 | 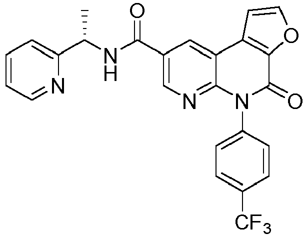 | A | A |
| T-401 | 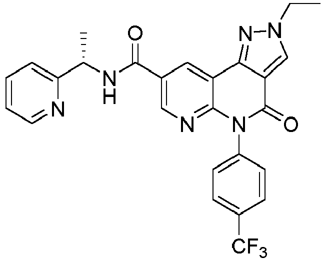 | A | A |
| T-402 | 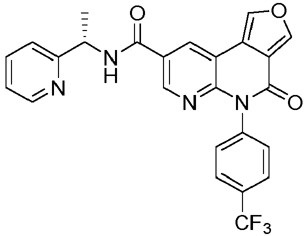 | A | A |
| T-403 | 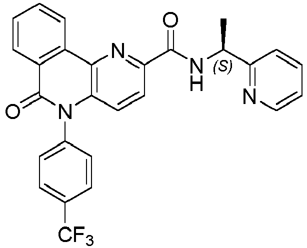 | A | A |
| T-404 | 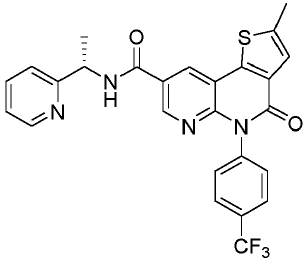 | A | A |
| T-405 | 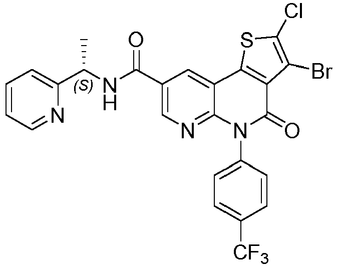 | A | A |
| T-406 | 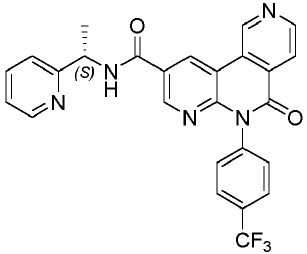 | A | A |
| T-407 | 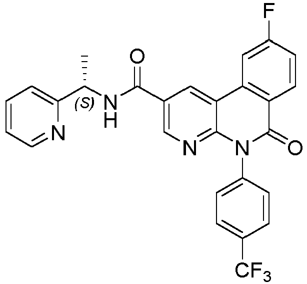 | A | A |
| T-408 | 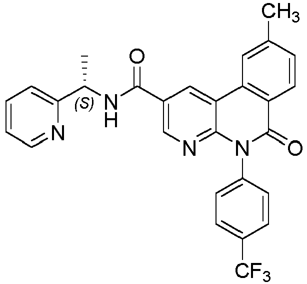 | A | A |
| T-409 | 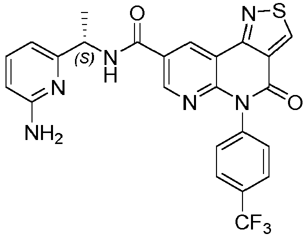 | A | A |
| T-410 | 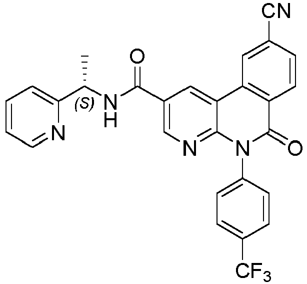 | A | A |
| T-411 | 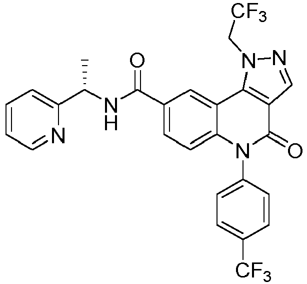 | B | A |
| T-412 | 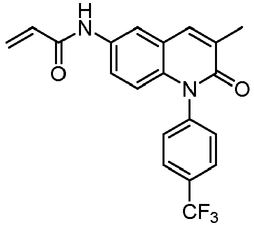 | A | A |
| T-413 | 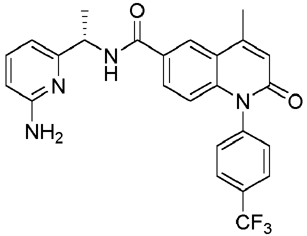 | A | A |
| T-414 | 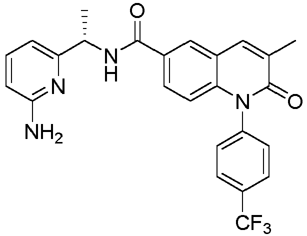 | A | A |
| T-415 | 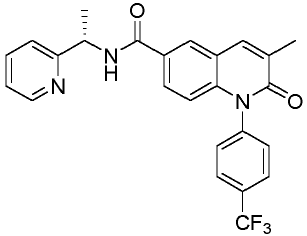 | A | A |
| T-417 | 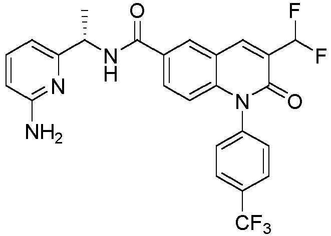 | A | A |
| T-418 | 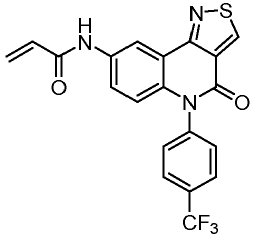 | A | A |
| T-419 | 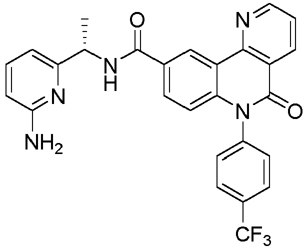 | A | A |
| T-420 | 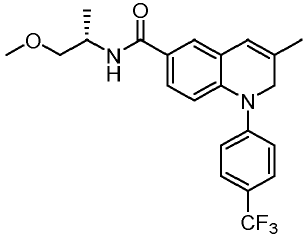 | A | A |
| T-421 | 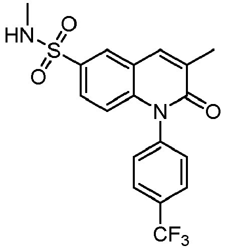 | A | A |
| T-423 | 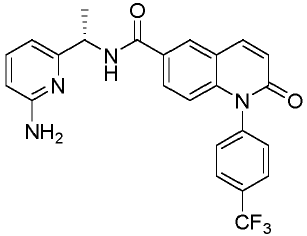 | A | A |
| T-424 | 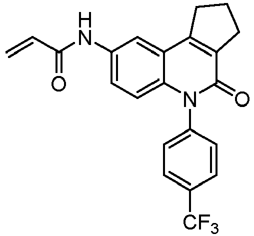 | A | A |
| T-425 | 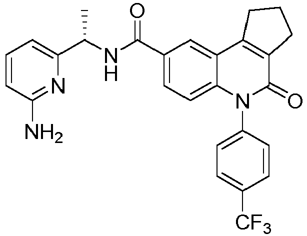 | A | A |
| T-426 | 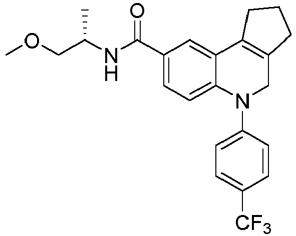 | A | A |
| T-427 | 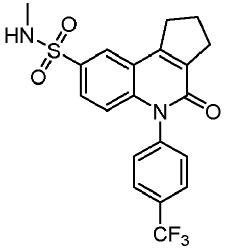 | A | A |
| T-428 | 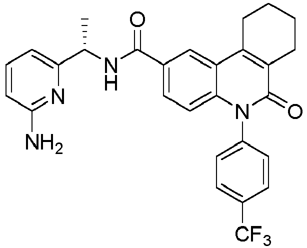 | A | A |
| T-429 | 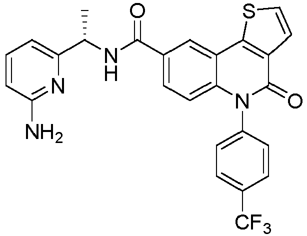 | A | A |
| T-430 | 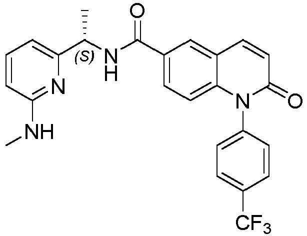 | A | A |
| T-431 | 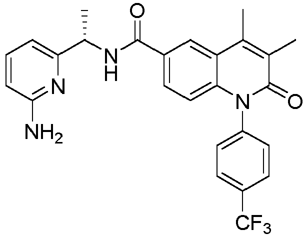 | A | A |
| T-432 | 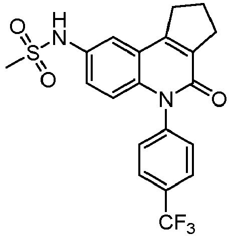 | A | A |
| T-433 | 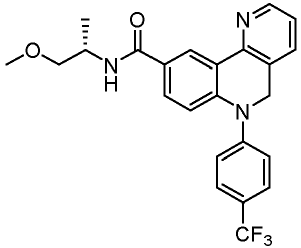 | A | A |
| T-434 | 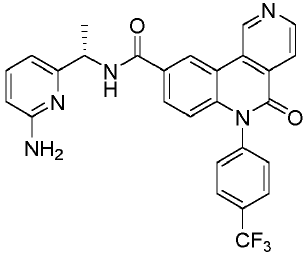 | A | A |
| T-435 | 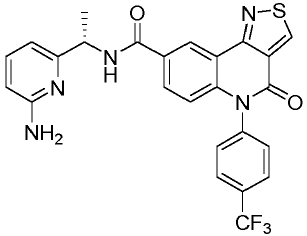 | A | A |
| T-436 | 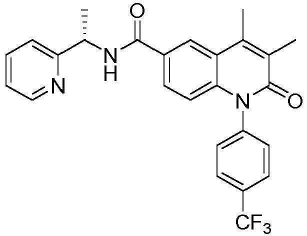 | A | A |
| T-437 | 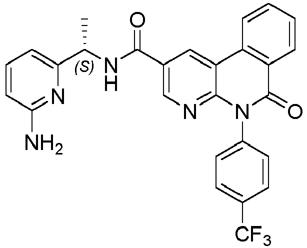 | A | A |
| T-438 | 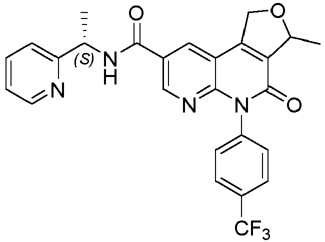 | | B |
| T-439 | 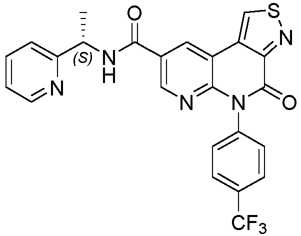 | A | A |
| T-440 | 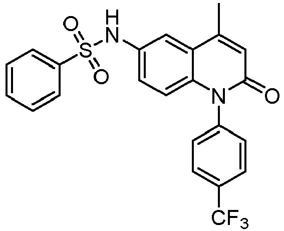 | | |
| T-441 | 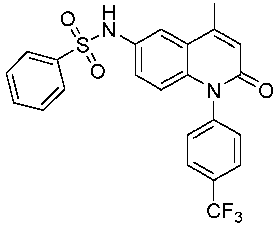 | | |
| T-442 | 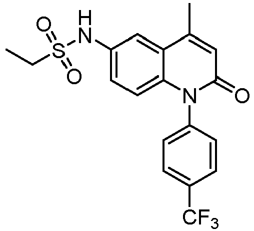 | | A |
| T-443 | 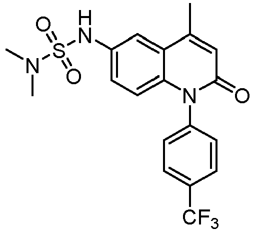 | | |
| T-444 | 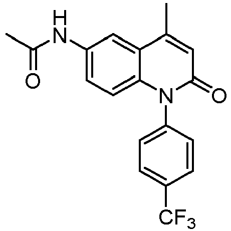 | | B |
| T-445 | 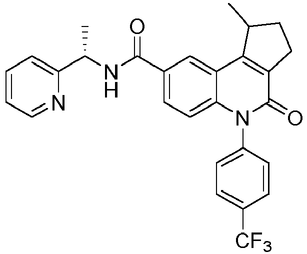 | A | A |
| T-446 | 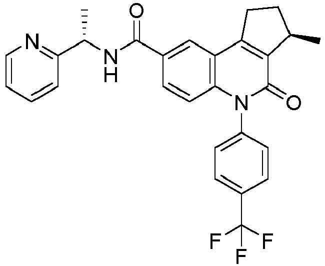 | A | A |
| T-447 | 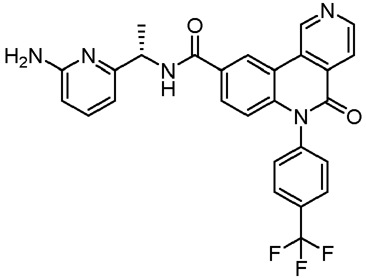 | A | A |
| T-448 | 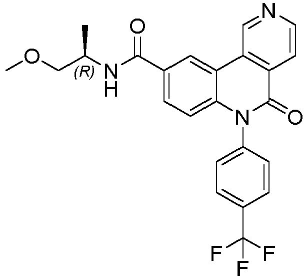 | B | B |
| T-449 | 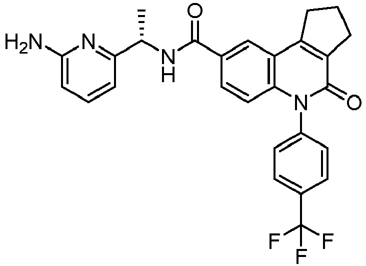 | A | A |
| T-455 | 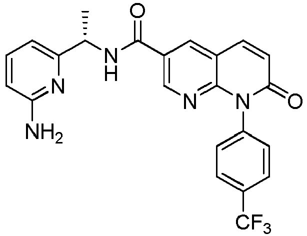 | A | A |
| T-456 | 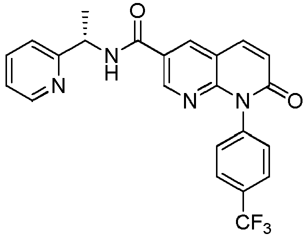 | A | A |
| T-457 | 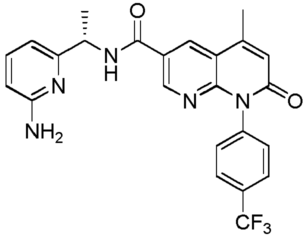 | A | A |
| T-458 | 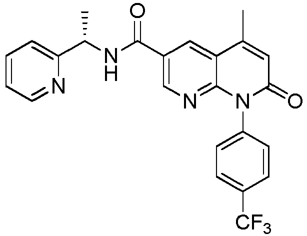 | A | A |
| T-459 | 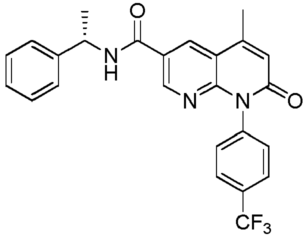 | A | A |
| T-460 | 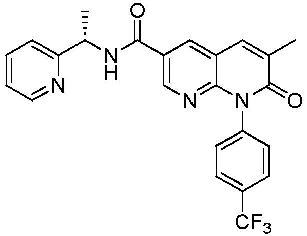 | A | A |
| T-461 | 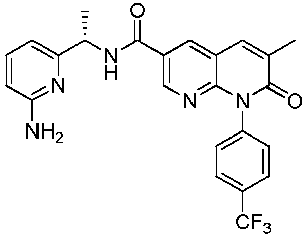 | A | A |
| T-462 | 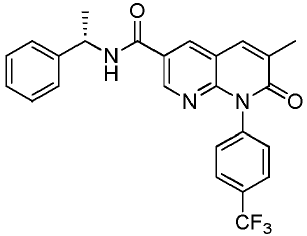 | A | A |

From Table 1, it can be seen that most of the compounds of the present invention have an excellent inhibitory effect on human pleural mesothelioma cells NCI-H226.

### Test Example 3 Pharmacokinetic Experiment of Compounds

Pharmacokinetic test of compounds T-32/T-105/T-253/T-272/T-273/T-277 of the present invention.

### Experimental protocol of rat pharmacokinetics

(1) Required animals: healthy adult SD rats, male, 3 rats, 6-8 weeks old; weighing 200-300g.
(2) Required equipments: analytical balance, animal weight scale, magnetic stirrer, refrigerated centrifuge, single-channel manual pipettor, etc.
(3) Required reagents: EDTA-Na2 anticoagulant, etc. 11.2g of EDTA-Na2 was weighed and placed in a reagent flask, 100 mL of normal saline was added, and the flask was shook to completely dissolve it. After the preparation was completed, it was divided into 1.5 mL centrifuge tubes with 20 uL each for whole blood sample collection.
(3) About 10 mg of the sample to be tested was accurately weighed, 5% DMSO after conversion was added to dissolve, then 30% PEG400 and 65% (10% Hp-β-CD in PBS) were added for sonication, and vortex-mixed evenly to obtain a solution with a concentration of 1 mg/mL which is freshly prepared before use.
(4) 0.1mL of sample was taken into a 1.5 mL centrifuge tube and stored at -80°C for concentration analysis of drug administration solution.
(5) Animals were kept in rat cages, and fasted (not less than 10 h) from the day before the test(but water was allowed). The animals were separately weighed and marked at the tail on the day of the test. Blank blood was collected before administration. The blood was collected from tail vein.
(6) Route of administration: gavage (p.o.); dosage: 10 mg/kg; administration volume: 10mL/kg.
(7) Operation process: Use the left hand with anti-bite gloves to catch the rat, make it stand upright, stretch the 16th gastric lavage needle from the throat of the mouth, insert the needle when feeling out no obvious resistance, and then inject the drug into the stomach.
(8) 0.2 ml of whole blood was collected from the tested animals and added into EDTA-Na2 anticoagulant tubes before administration and 0.5 h, 1 h, 2h, 4 h, 6 h, 8h, 12h and 24 h after administration respectively. The tubes were reversed up and down for 3-4 times and mixed evenly, centrifuged at 4 °C at 10000g for 5 min to separate plasma, and stored at -80°C for testing. The blood was collected from tail vein.
(9) LC-MS/MS method was established to determine the concentration of original drug in plasma, the blood drug concentration-time curve was drawn, and the main pharmacokinetic parameters were calculated by non-compartment model.
(10) Pharmacokinetic parameters of T-32/T-105/T-253/T-272/T-273/T-277 were shown below in Table 2.

**Table 2**

| Parameters of Table 2 | Tₘₐₓ (h) | Cₘₐₓ (ng/mL) | AUCₗₐₛₜ (h*ng/mL) | MRTₗₐₛₜ (h) |
|---|---|---|---|---|
| T-32 | 2.5 | 2220 | 22233.5 | 6.88 |
| T-105 | 4.0 | 3873.02 | 53858.70 | 9.48 |
| T-253 | 4.7 | 2640 | 39243 | 9.78 |
| T-272 | 5.3 | 1407 | 21201 | 9.96 |
| T-273 | 4.0 | 1377 | 11544 | 6.34 |
| T-277 | 4.7 | 2653 | 26919 | 7.28 |
| Control compound 1 | 2.0 | 584 | 2181 | 2.92 |

Wherein control compound 1 is a compound disclosed in patent WO2020097389A1 and has the best properties, and the structure of the compound is shown in the following figure:

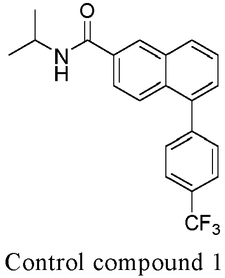

All documents mentioned in the present invention are cited as references in this application, just as each document is individually cited as a reference. In addition, it should be understood that, after reading the above teaching content of the present invention, those skilled in the art can make various changes or modifications to the present invention, and these equivalent forms also fall within the scope defined by the appended claims of the present application.

## Claims

1. A compound of formula I, or a pharmaceutically acceptable salt, a solvate or a prodrug thereof, 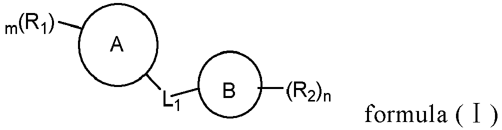 wherein
A is selected from 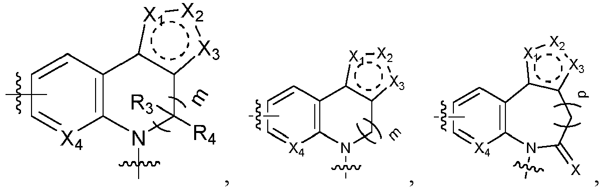 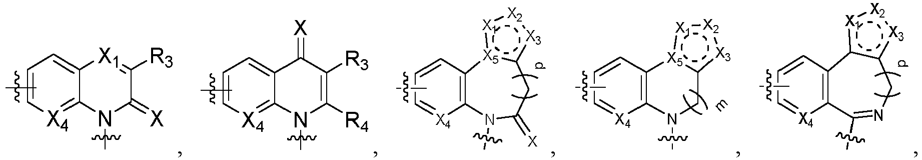 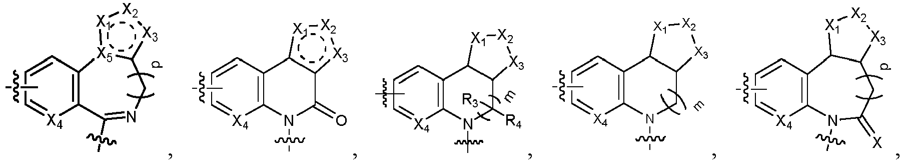 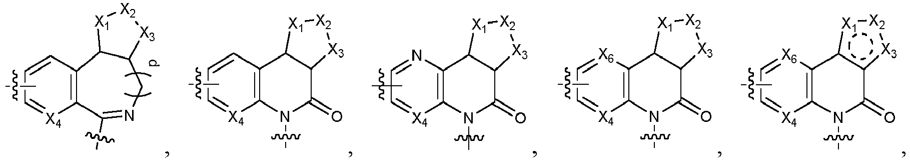 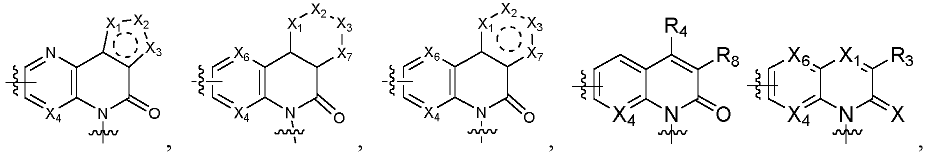 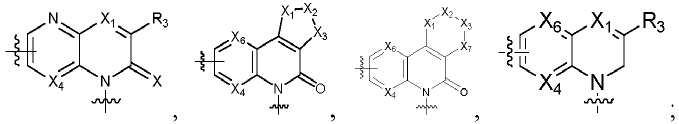
L₁ is selected from absent and CR₃R₄;
B is selected from C6-C10aryl, 5-10 membered heteroaryl containing 1 to 3 heteroatoms selected from N, O and S, C5-C10cycloalkyl;
X is selected from O, NH, CR₃R₄, and S;
X₁, X₂, X₃, X₄, X₅, X₆ and X₇ are each independently selected from CR₃, (CR₃)₂, N, O, S, SR₃, SR₃R₄, NR₄, CR₃R₄, (CR₃R₄)₂;
R₁ is each independently selected from H, D, halogen, CN, NH₂, ureido, carboxyl, carbamate group, C₁₋₆alkyl, C₁₋₆alkoxyl, C₃₋₆cycloalkyl, C₃₋₆cycloalkoxy, C6-C10aryl, 5-10 membered heteroaryl containing 1 to 3 heteroatoms selected from N, O and S, 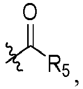 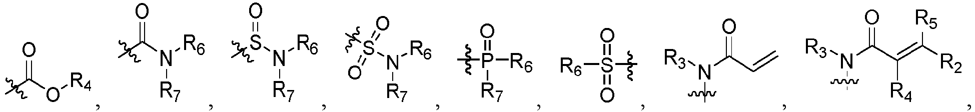 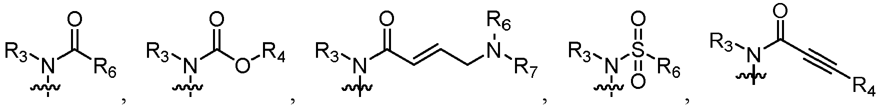 and 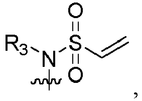 wherein NH₂, ester group, ureido, carbamate group, acylamino, C₁₋₆alkyl, C₁₋₆alkoxyl, C₃₋₆cycloalkyl, C₃₋₆cycloalkoxy, C6-C10aryl, and 5-10 membered heteroaryl containing 1 to 3 heteroatoms selected from N, O and S are optionally substituted with 1, 2 or 3 R;
each of R₂, R₃, R₄ and R₅ are each independently selected from H, D, halogen, CN, NH₂, -CO-(C₁₋₆alkyl), =O, -C(=O)-O-(C1-C6alkyl), -C(=O)-O-OBi, -S(=O)₂-NR₆R₇, 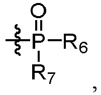 ester group, ureido, carbamate group, acylamino, C₁₋₆alkyl, C₁₋₆haloalkyl, C₁₋₆alkoxyl, C₃₋₆cycloalkyl, C₃₋₆cycloalkoxy, C6-C10aryl, 5-10 membered heteroaryl containing 1 to 3 heteroatoms selected from N, O and S, SF₅, wherein NH₂, ester group, ureido, carbamate group, acylamino, C₁₋₆alkyl, C₁₋₆alkoxyl, C₃₋₆cycloalkyl, C₃₋₆cycloalkoxy, C6-C10aryl, and 5-10 membered heteroaryl containing 1 to 3 heteroatoms selected from N, O and S are optionally substituted with 1, 2 or 3 R; or, X₁ and X₇ are each independently CR₃R₄, and X₁ and X₇ share one R₃, and R₃ is C1-C6alkylene;
R₆ and R₇ are each independently selected from hydrogen, D, C₁₋₆alkyl, C₃₋₆cycloalkyl, C6-C10aryl, NH₂, NH(C₁₋₆alkyl), N(C₁₋₆alkyl)₂, 5-10 membered heteroaryl containing 1 to 3 heteroatoms selected from N, O and S, 5-10 membered heterocyclyl containing 1 to 3 heteroatoms selected from N, O and S, -S(O)₂-(C₁₋₆alkyl), -S(O)₂-(C₂₋₆alkenyl), wherein C₁₋₆alkyl, C₃₋₆cycloalkyl, C6-C10aryl, 5-10 membered heteroaryl containing 1 to 3 heteroatoms selected from N, O and S are optionally substituted with 1, 2 or 3 R, or R₆ and R₇ form a 3-7 membered carbocycle, or R₆ and R₇ form a 3-7 membered heterocycle containing N, O or S;
R₈ is selected from 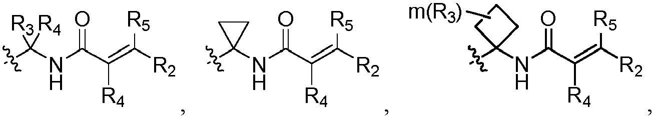 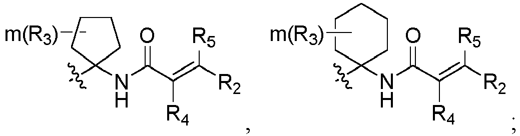
each R is independently selected from halogen, CN, OH, -(C₁₋₆alkylene)-N(C₁₋₆alkyl)₂, NH₂, NH(C₁₋₆alkyl), ester group, ureido, carbamate group, acylamino, C₁₋₆alkyl, C₁₋₆alkoxyl, C₃₋₆cycloalkyl, C₃₋₆cycloalkoxy, C₂₋₆alkenyl, C₂₋₆alkynyl, C6-C10aryl, 5-10 membered heterocyclyl containing 1 to 3 heteroatoms selected from N, O and S, or 5-10 membered heteroaryl containing 1 to 3 heteroatoms selected from N, O and S substituted or unsubstituted with R'; R' is independently selected from the group consisting of: C₁₋₆alkyl, haloC₁₋₆alkyl, C₁₋₆alkoxyl, NH₂, NH(C₁₋₆alkyl), N(C₁₋₆alkyl)₂, CN, halogen, =O;
each m and n are each independently selected from 1, 2, 3 and 4;
p is selected from 0, 1 and 2.

2. The compound of claim 1 or the pharmaceutically acceptable salt, the solvate or the prodrug thereof, wherein:
A is selected from 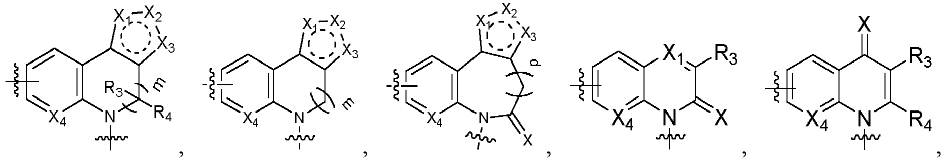 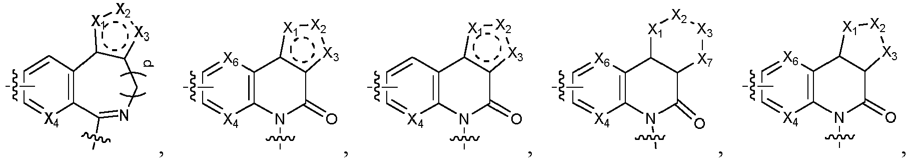 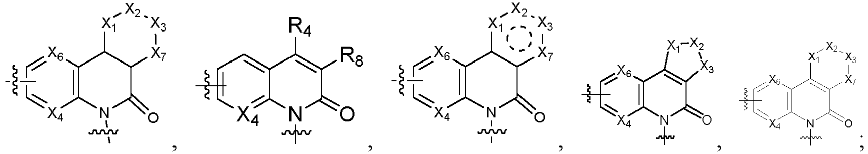
L₁ is selected from absent and CR₃R₄;
B is C6-C10aryl;
X is O;
X₁, X₂, X₃, X₄, X₆ and X₇ are each independently selected from CR₃, N, CR₃R₄, and NR₄;
R₁ is selected from 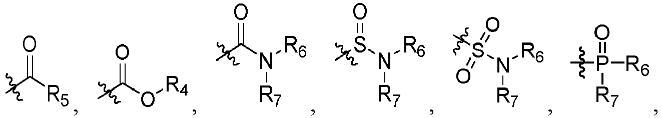 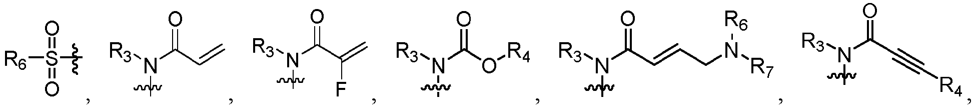 and 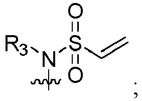
each R₂, R₃, R₄ and R₅ are each independently selected form H, halogen, CN, NH₂, -CO-(C₁₋₆alkyl), C₁₋₆alkyl, C₁₋₆alkoxyl, C₃₋₆cycloalkyl, C₃₋₆cycloalkoxy, C6-C10aryl, 5-10 membered heteroaryl containing 1 to 3 heteroatoms selected from N, O and S, wherein NH₂, C₁₋₆alkyl, C₁₋₆alkoxyl, C₃₋₆cycloalkyl, C₃₋₆cycloalkoxy, C6-C10aryl, 5-10 membered heteroaryl containing 1 to 3 heteroatoms selected from N, O and S are optionally substituted with 1, 2 or 3 R;
R₆ and R₇ are each independently selected from hydrogen, C₁₋₆alkyl, C₃₋₆cycloalkyl, C6-C10aryl, 5-10 membered heteroaryl containing 1 to 3 heteroatoms selected from N, O and S, -S(O)₂-(C₁₋₆alkyl), -S(O)₂-(C₂₋₆alkenyl), wherein C₁₋₆alkyl, C₃₋₆cycloalkyl, C6-C10aryl, 5-10 membered heteroaryl containing 1 to 3 heteroatoms selected from N, O and S are optionally substituted with 1, 2 or 3 R, or R₆ and R₇ form a 3-7 membered carbocycle, or R₆ and R₇ form a 3-7 membered heterocycle containing N, O or S;
R₈ is selected from 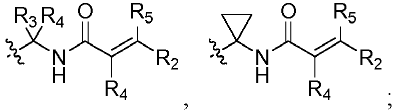
each R is independently selected from halogen, CN, OH, NH₂, C₁₋₆alkyl, C₁₋₆alkoxyl, C₃₋₆cycloalkyl, C₃₋₆cycloalkoxy, C₂₋₆alkenyl, C₂₋₆alkynyl, C6-C10aryl, and 5-10 membered heteroaryl containing 1 to 3 heteroatoms selected from N, O and S;
each m and n are each independently selected from 1, 2, 3 and 4;
p is selected from 0, 1 and 2.

3. The compound of claim 1 or the pharmaceutically acceptable salt, the solvate, or the prodrug thereof, wherein the compound is selected from the group consisting of: 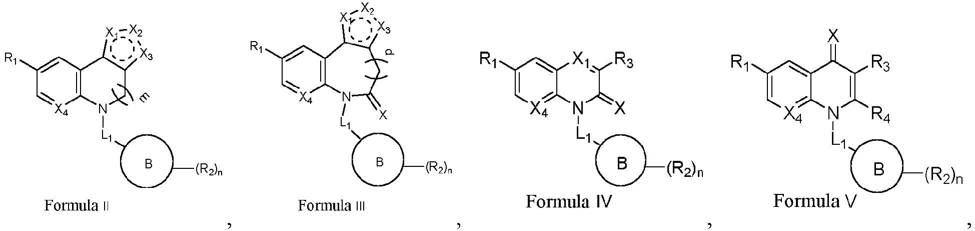 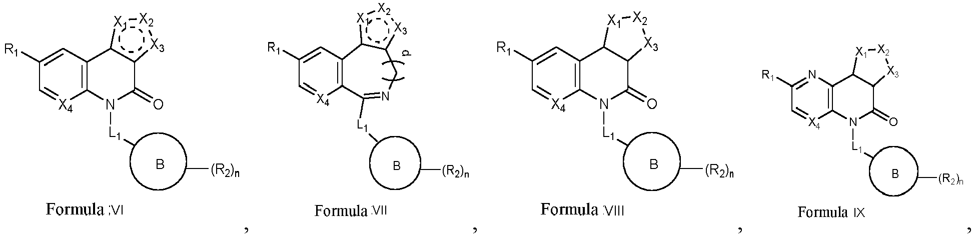 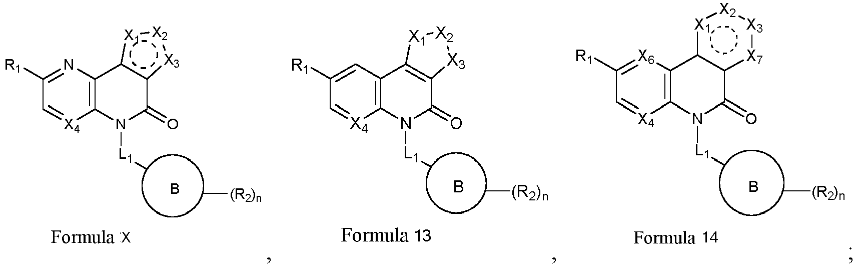 wherein, each group is as defined in claim 1.

4. The compound of claim 1 or the pharmaceutically acceptable salt, the solvate, or the prodrug thereof, wherein the compound is selected from the group consisting of: 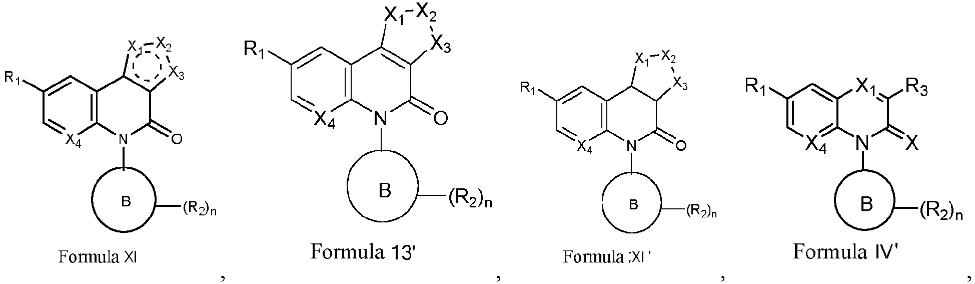 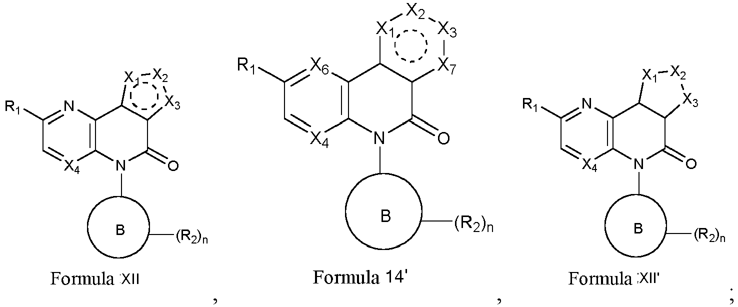
R₁ is selected from CN, ureido, carbamate group, 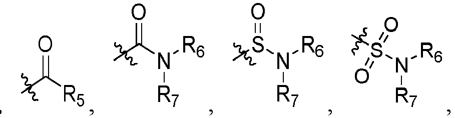 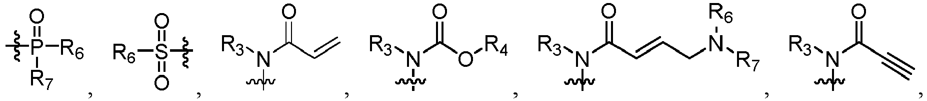 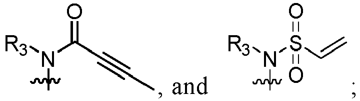
wherein, each group is as defined in claim 1.

5. The compound of claim 1 or the pharmaceutically acceptable salt, the solvate or the prodrug thereof, wherein R₂ is selected from trifluoromethyl, fluorine, chlorine, bromine, iodine, methyl, cyclopentyl, and cyclohexyl and pentafluorothio.

6. The compound of claim 1 or a pharmaceutically acceptable salt, a solvate, or a prodrug thereof, wherein the compound is selected from the group consisting of:

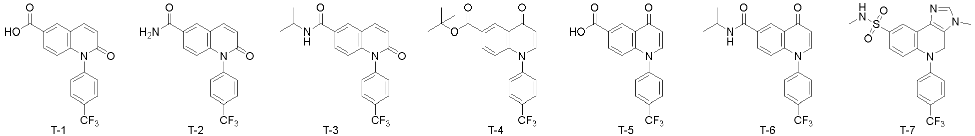

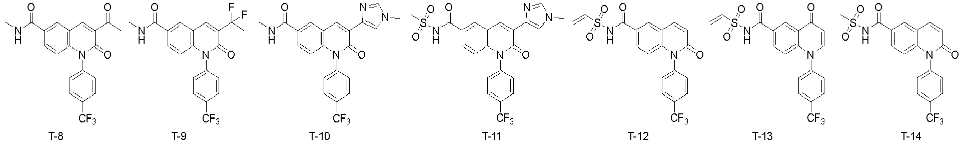

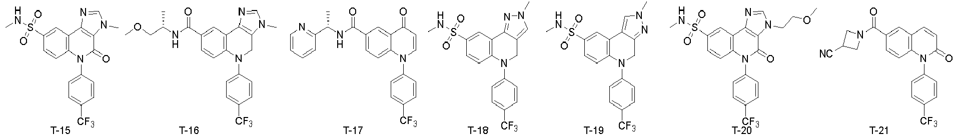

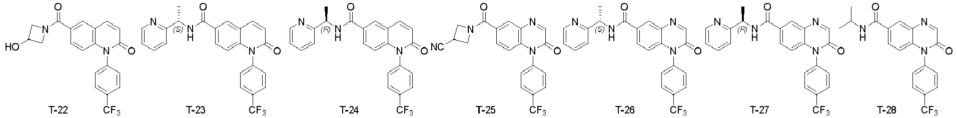

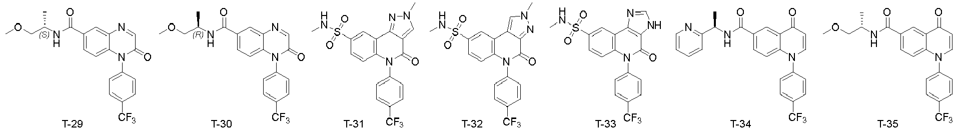

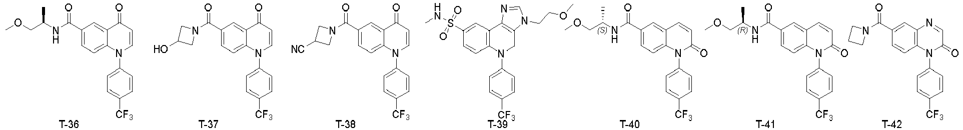

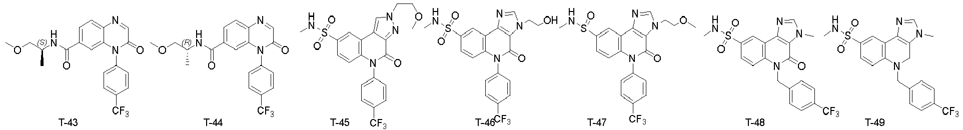

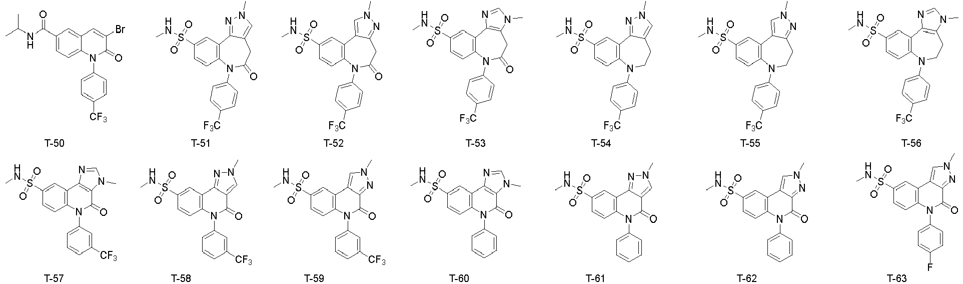

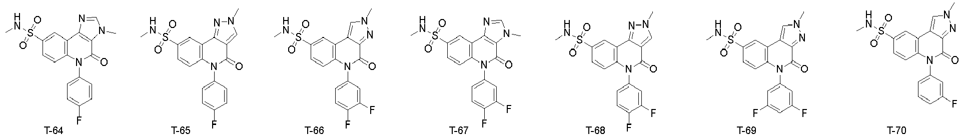

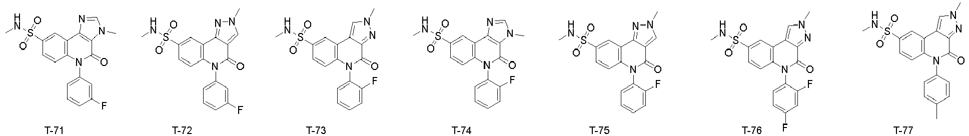

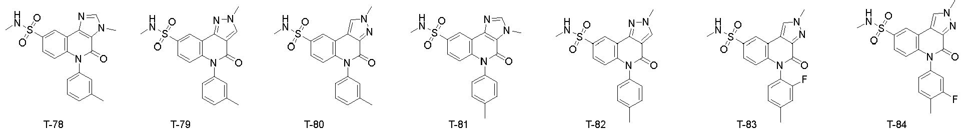

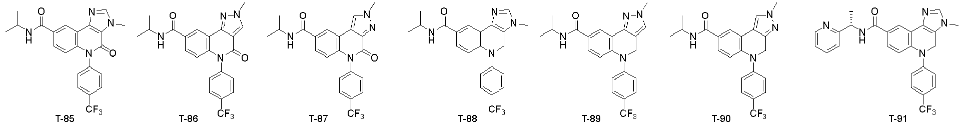

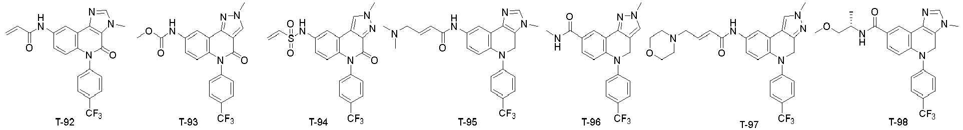

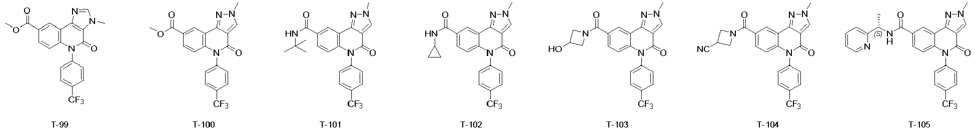

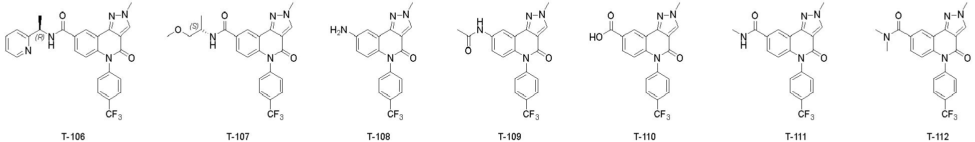

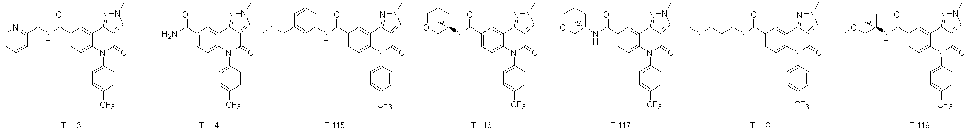

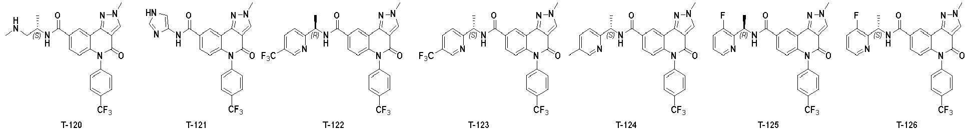

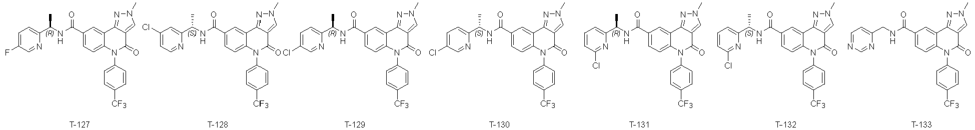

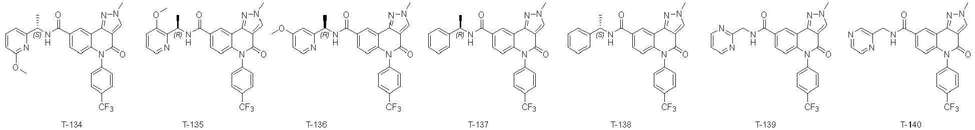

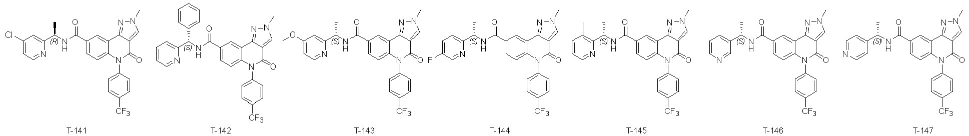

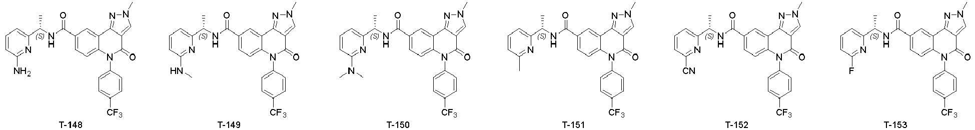

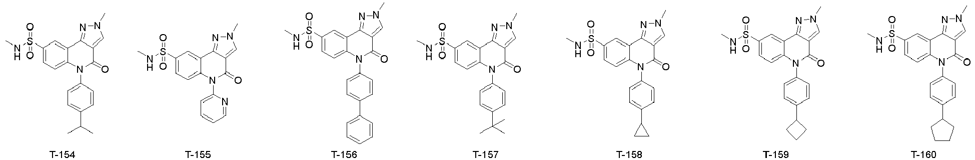

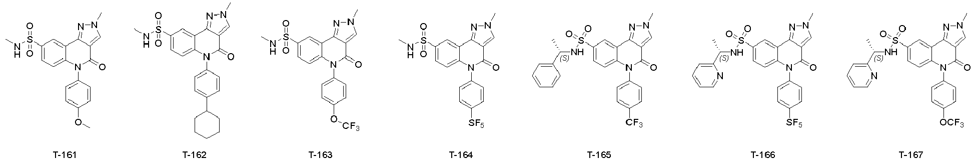

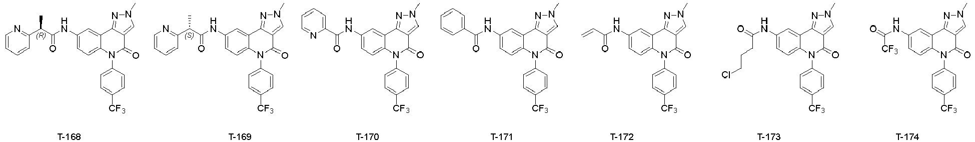

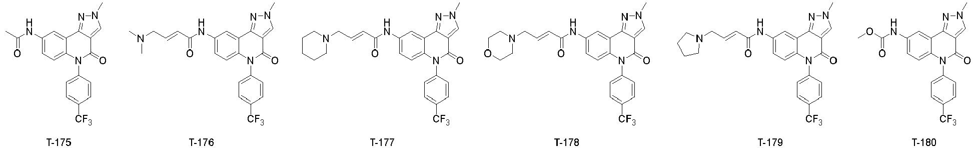

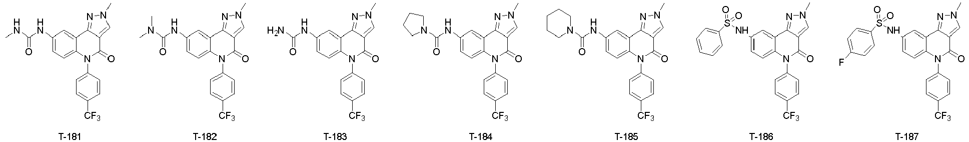

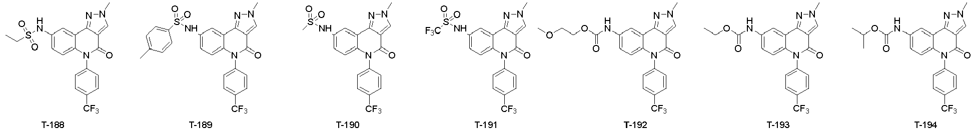

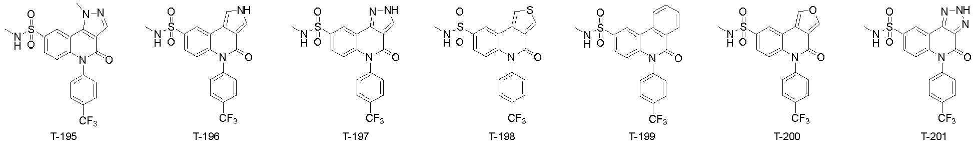

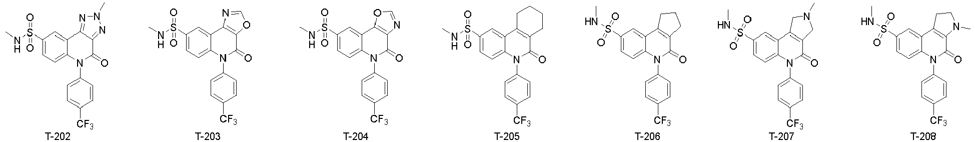

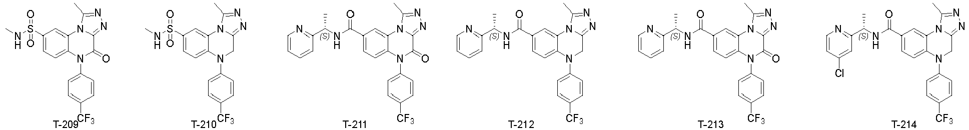

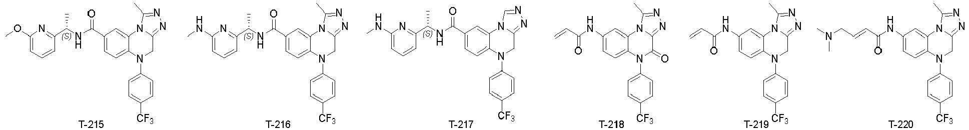

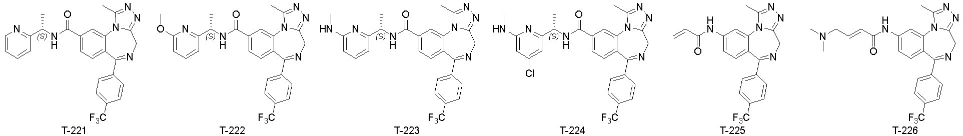

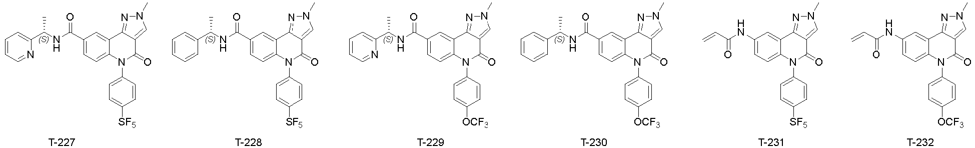

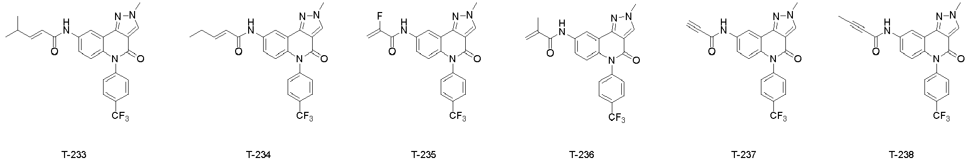

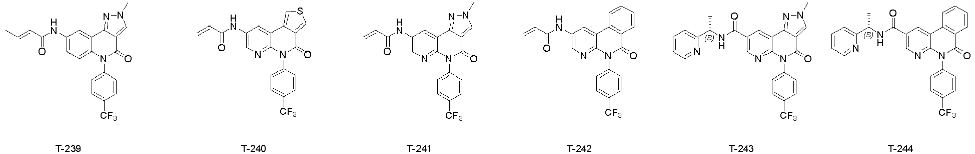

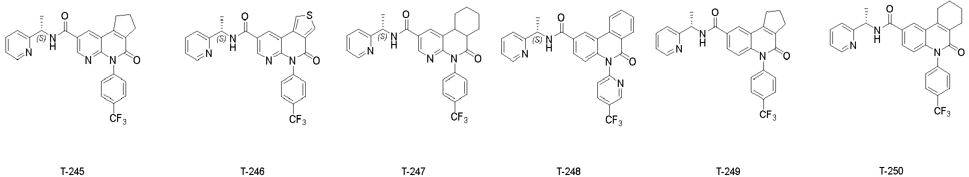

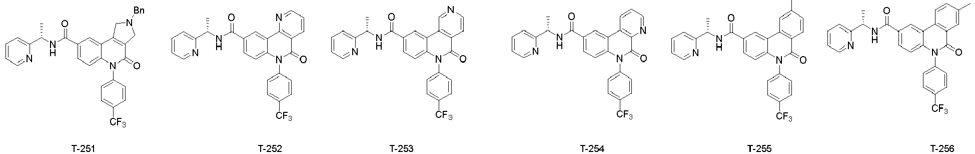

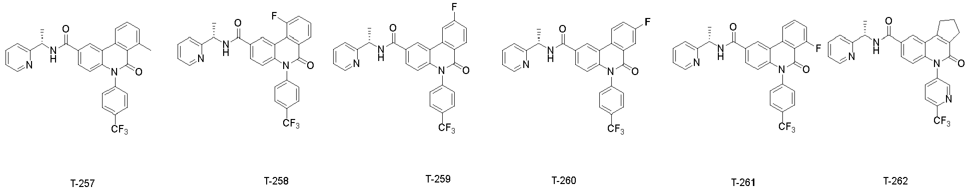

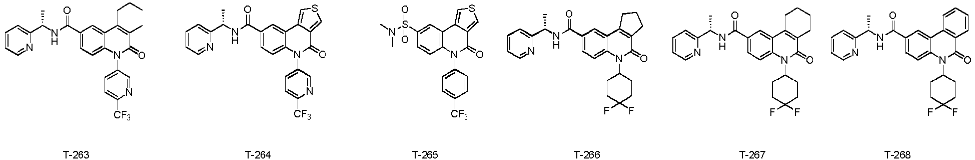

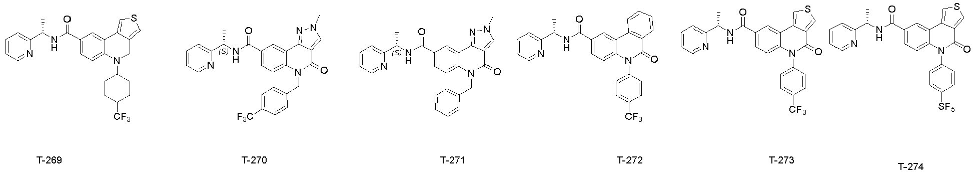

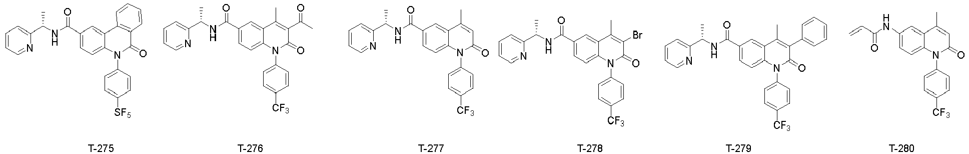

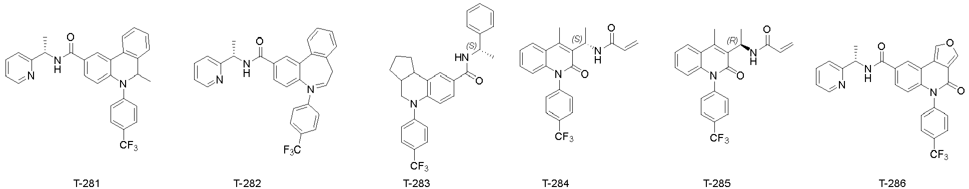

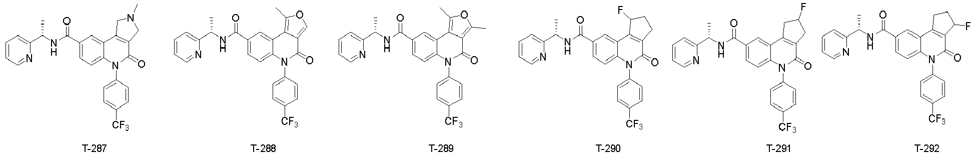

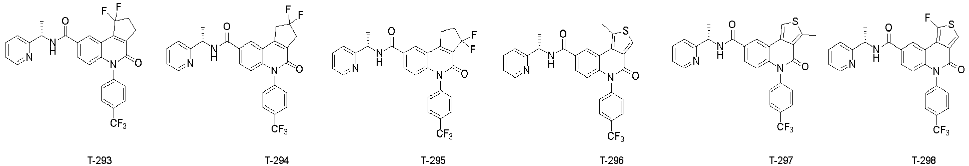

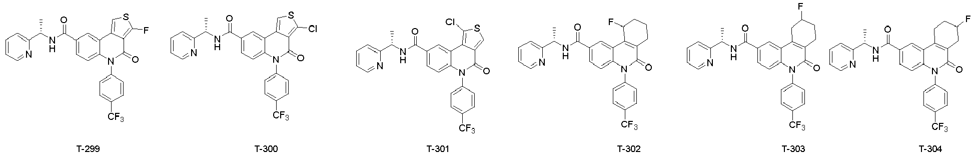

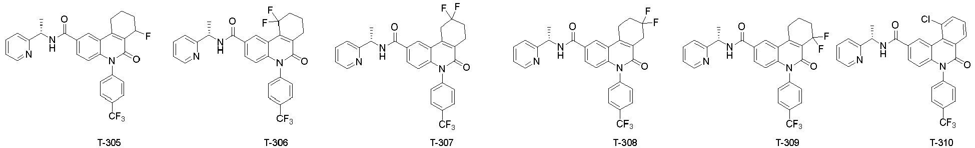

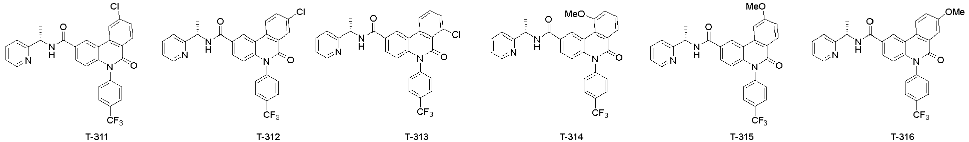

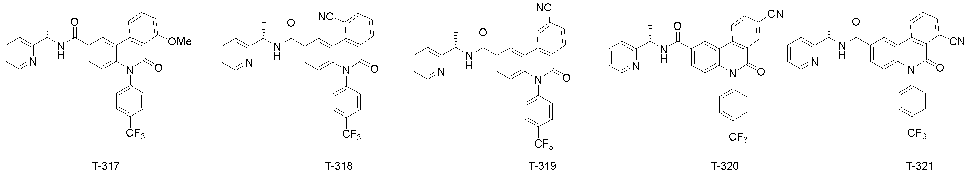

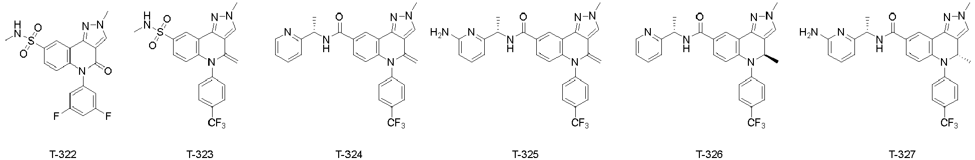

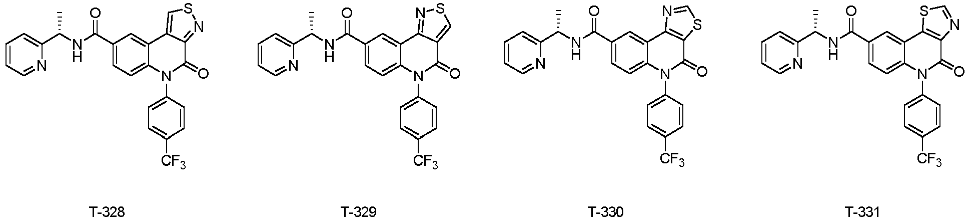

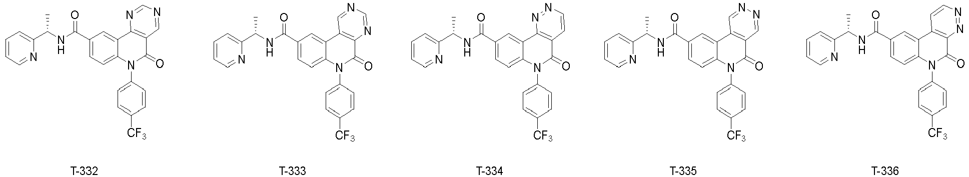

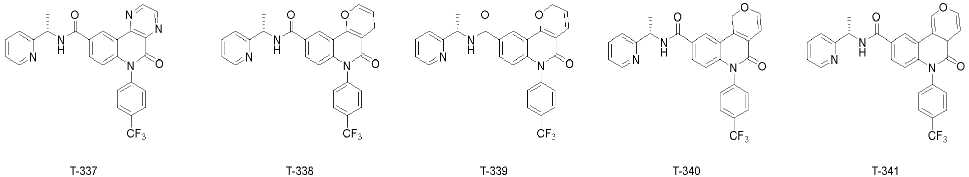

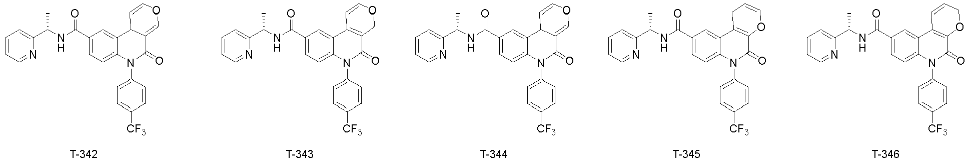

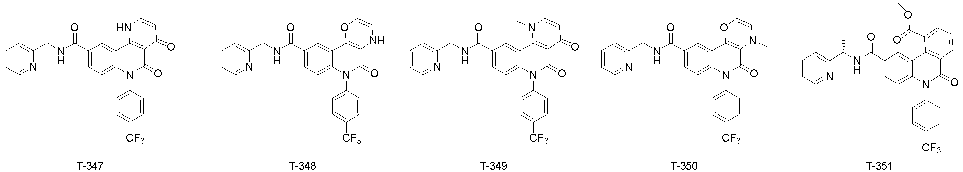

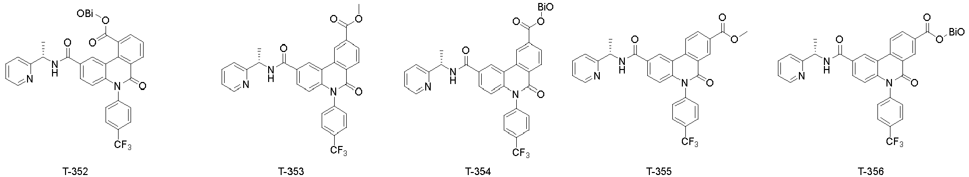

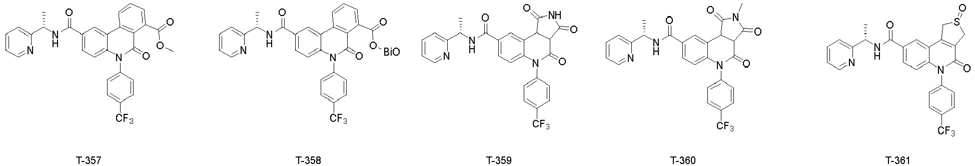

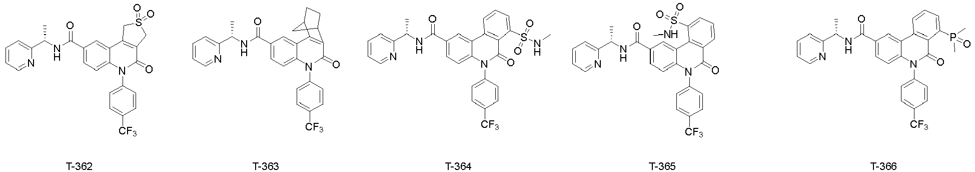

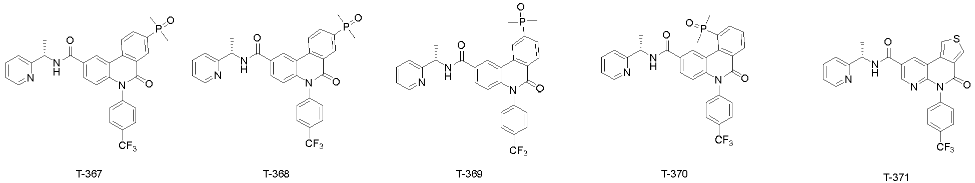

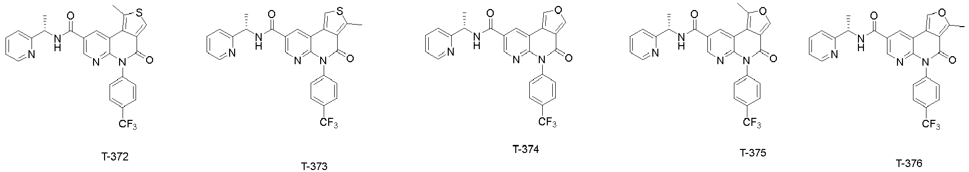

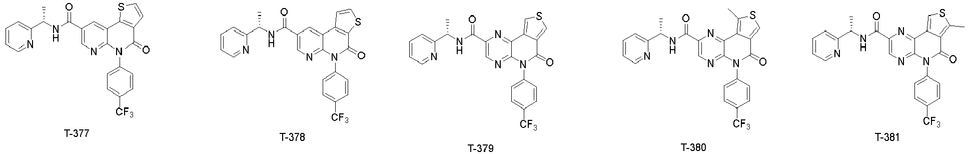

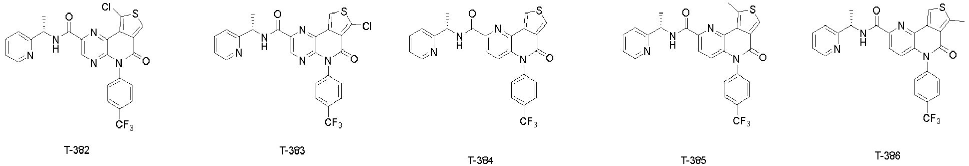

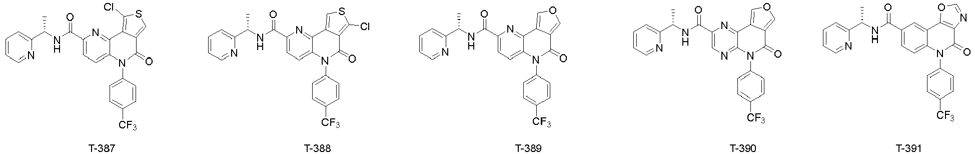

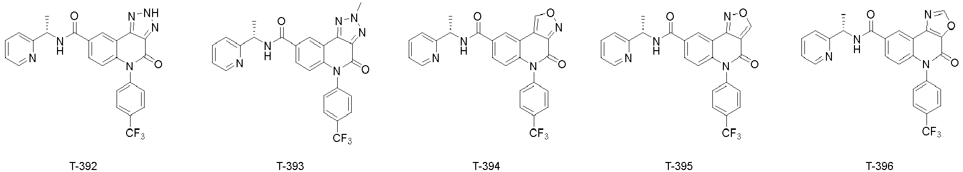

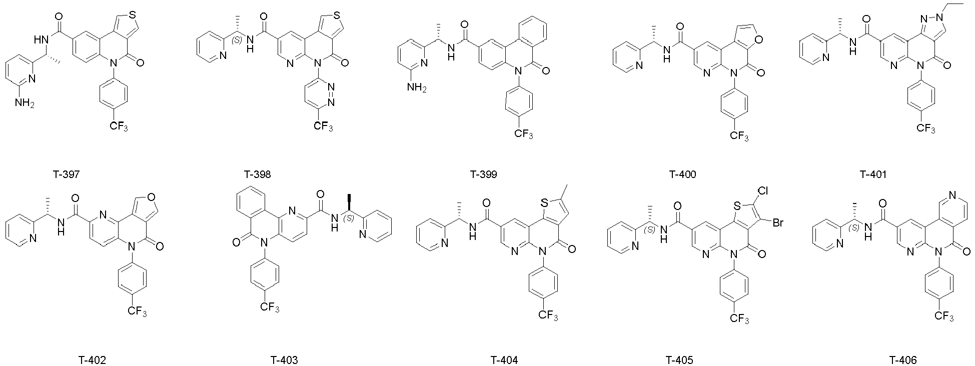

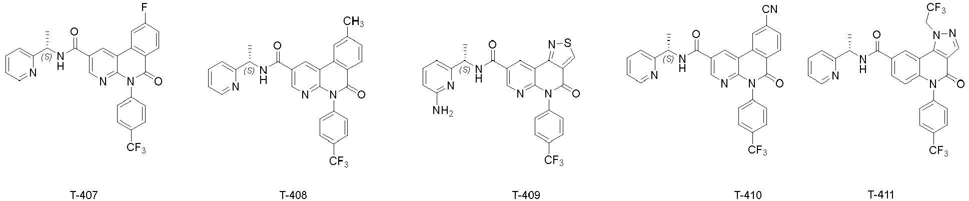

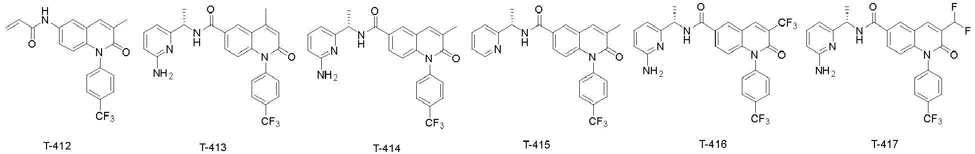

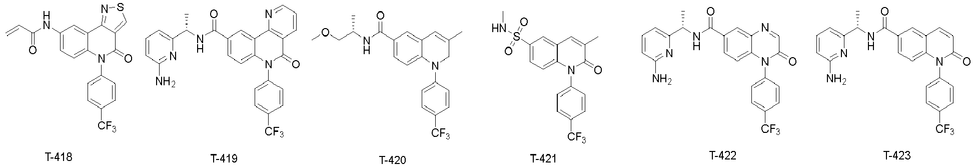

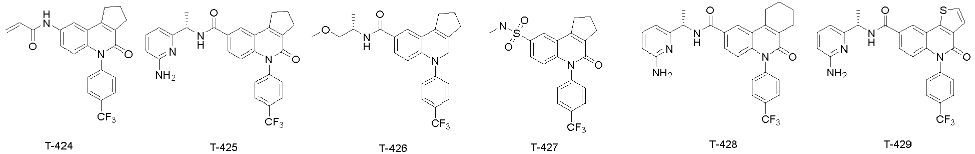

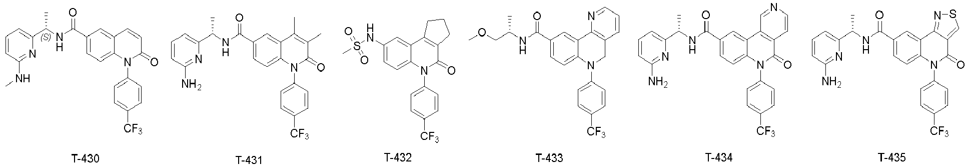

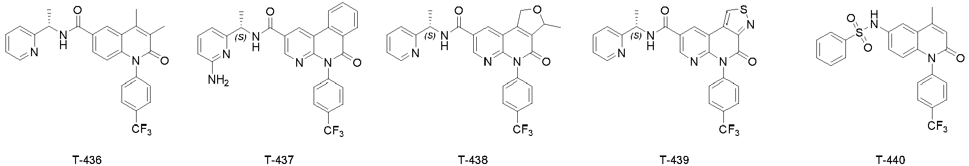

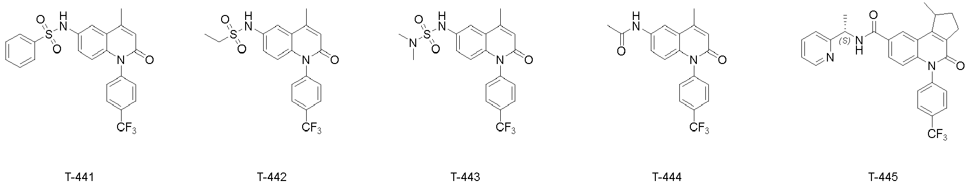

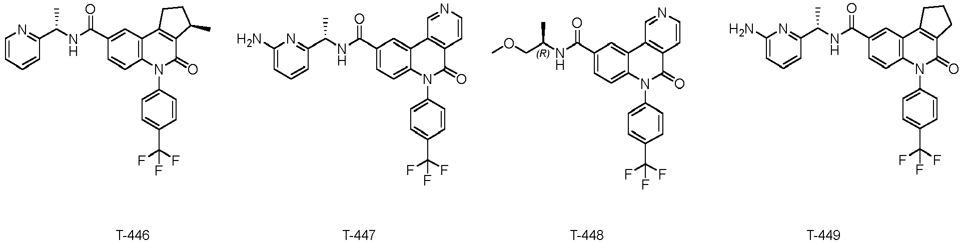

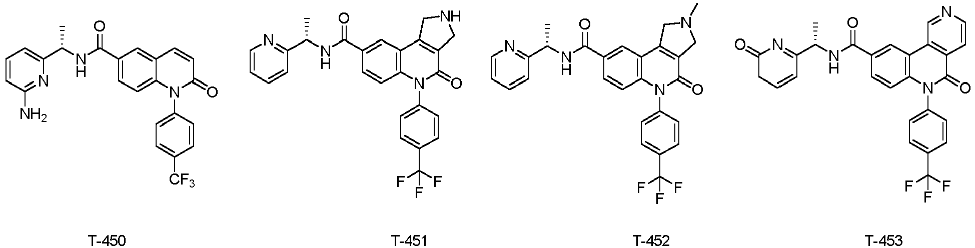

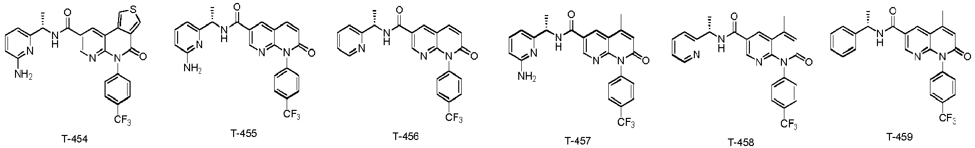

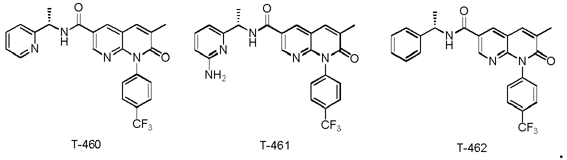

7. A pharmaceutical composition, wherein comprising a pharmaceutically acceptable carrier and one or more of safe and effective amount of the compound of claim 1 or the pharmaceutically acceptable salt, the solvate or the prodrug thereof.

8. A use of the pharmaceutical composition of claim 7 in the preparation of a drug for the prevention and/or treatment of related diseases caused by Hippo pathway dysregulation.

9. A use of the pharmaceutical composition of claim 7 in the preparation of a drug for the prevention and/or treatment of related diseases caused by the dysregulation of YAP or TAZ or YAP/TAZ or YAP/TEAD or YAP/TAZ/TEAD.

10. A combination of the compound of claim 1 with a second drug in the preparation of a drug for the prevention and/or treatment of cancers;
the second drug is selected from the group consisting of: ERK inhibitors, MEK inhibitors, KRAS inhibitors, BRAF inhibitors, EGFR inhibitors, Wnt inhibitors, PD-1 inhibitors, and combinations thereof.
